(19) Europäisches Patentamt
European Patent Office
Office européen des brevets

(11) Veröffentlichungsnummer: **0 058 779**
**B1**

(12) # EUROPÄISCHE PATENTSCHRIFT

(45) Veröffentlichungstag der Patentschrift: **19.09.84**

(21) Anmeldenummer: **81110732.5**

(22) Anmeldetag: **23.12.81**

(51) Int. Cl.[3]: **C 07 C 103/32,** C 07 D 295/12, C 07 D 295/14, C 07 D 227/00, A 61 K 31/195, A 61 K 31/215, A 61 K 31/165, A 61 K 31/395

(54) **Neue Carbonsäureamide, ihre Herstellung und ihre Verwendung als Arzneimittel.**

(30) Priorität: **10.01.81 DE 3100575**

(43) Veröffentlichungstag der Anmeldung: **01.09.82 Patentblatt 82/35**

(45) Bekanntmachung des Hinweises auf die Patenterteilung: **19.09.84 Patentblatt 84/38**

(84) Benannte Vertragsstaaten: **AT BE CH DE FR IT LI LU NL SE**

(56) Entgegenhaltungen:
**BE - A - 764 238**
**FR - A - 2 225 165**
**FR - A - 2 370 723**

(73) Patentinhaber: **Dr. Karl Thomae GmbH, Postfach 1755, D-7950 Biberach (Riss) (DE)**

(72) Erfinder: **Grell, Wolfgang, Dr. Dipl.-Chem., Amriswilstrasse 7, D-7950 Biberach 1 (DE)**
Erfinder: **Griss, Gerhart, Dr. Dipl.-Chem., Schopperweg 1, D-7950 Biberach 1 (DE)**
Erfinder: **Sauter, Robert, Dr. Dipl.-Chem., Albert-Schweitzer-Weg 9, D-7958 Laupheim (DE)**
Erfinder: **Hurnaus, Rudolf, Dr. Dipl.-Chem., Silcherstrasse 19, D-7950 Biberach 1 (DE)**
Erfinder: **Rupprecht, Eckhard, Dr. Dipl.-Bio., Riedbachstrasse 15, D-7960 Aulendorf-Tannhausen (DE)**
Erfinder: **Kaubisch, Nikolaus, Dr. Dipl.-Chem., Moltkestrasse 3, D-6550 Bad Kreuznach (DE)**
Erfinder: **Kähling, Joachim, Dr., Haydnweg 8, D-7950 Biberach (DE)**

**EP 0 058 779 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der vorliegenden Erfindung sind neue Carbonsäureamide der allgemeinen Formel

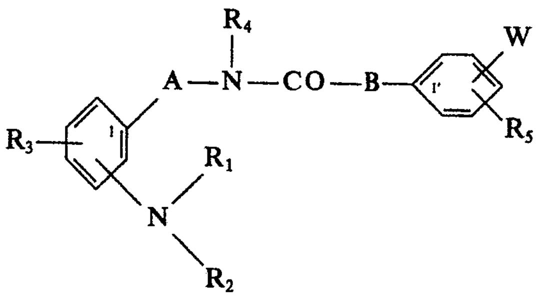

(I)

und deren Salze, insbesondere deren physiologisch verträgliche Salze mit anorganischen oder organischen Säuren und Basen, diese enthaltende Arzneimittel und Verfahren zu ihrer Herstellung.

Die neuen Verbindungen weisen wertvolle pharmakologische Eigenschaften auf, insbesondere eine Wirkung auf den Stoffwechsel, vorzugsweise jedoch eine blutzuckersenkende Wirkung.

In der obigen allgemeinen Formel I bedeuten

$R_1$ und $R_2$, die gleich oder verschieden sein können, Alkylgruppen mit 1 bis 6 Kohlenstoffatomen oder Cycloalkylgruppen mit 5 bis 7 Kohlenstoffatomen oder

$R_1$ und $R_2$ zusammen mit dem dazwischen liegenden Stickstoffatom eine unverzweigte Alkyleniminogruppe mit 3 bis 6 Kohlenstoffatomen, die durch eine oder zwei Alkylgruppen mit jeweils 1 bis 3 Kohlenstoffatomen oder durch eine Hydroxygruppe substituiert sein kann, oder in der eine Methylengruppe durch eine Carbonylgruppe, durch ein Sauerstoff- oder Schwefelatom oder durch eine Iminogruppe, welche durch eine Alkylgruppe mit 1 bis 3 Kohlenstoffatomen, eine Aralkylgruppe mit 7 bis 10 Kohlenstoffatomen, eine Phenyl- oder Halogenphenylgruppe substituiert sein kann, oder in der eine Äthylengruppe durch eine o-Phenylengruppe ersetzt sein kann, eine unverzweigte Alkenyleniminogruppe mit 4 bis 6 Kohlenstoffatomen, eine gesättigte oder teilweise ungesättigte Azabicycloalkylgruppe mit 6 bis 10 Kohlenstoffatomen, eine Aza-1,4-dioxa-spiroalkylgruppe mit 6 bis 8 Kohlenstoffatomen, eine Heptamethylenimino-, Octamethylenimino-, Nonamethylenimino- oder Decamethyleniminogruppe,

$R_3$ ein Wasserstoff- oder Halogenatom, eine Trifluormethyl-, Alkyl-, Hydroxy-, Alkoxy-, Alkanoyloxy-, Mercapto-, Alkylmercapto-, Nitro-, Amino-, Cyano-, Alkanoyl-, Carboxy-, Alkoxycarbonyl-, Aminocarbonyl-, Alkylaminocarbonyl-, Dialkylaminocarbonyl-, Aminosulfonyl-, Alkylamino-, Dialkylamino-, Alkanoylamino-, Alkoxycarbonylamino- oder Alkylsulfonylaminogruppe, wobei jeder Alkylteil in den vorstehend genannten Gruppen jeweils 1 bis 3 Kohlenstoffatome enthalten kann, eine Aralkoxygruppe mit 7 bis 10 Kohlenstoffatomen oder eine Arylcarbonylaminogruppe,

$R_4$ ein Wasserstoffatom oder eine Alkylgruppe mit 1 bis 3 Kohlenstoffatomen,

$R_5$ ein Wasserstoffatom, ein Halogenatom oder eine Alkylgruppe mit 1 bis 3 Kohlenstoffatomen,

A eine Bindung, eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierte Methylen- oder Äthylengruppe, eine durch eine Cycloalkylgruppe mit 3 bis 7 Kohlenstoffatomen, durch eine Hydroxyalkyl-, Alkoxyalkyl-, Cyano-, Carboxyl-, Alkoxycarbonyl-, Aminocarbonyl-, Alkylaminocarbonyl-, Dialkylaminocarbonyl-, Aryl- oder Aralkylgruppe substituierte Methylengruppe, wobei jeder der vorstehend erwähnten Alkylteile 1 bis 3 Kohlenstoffatome enthalten kann, eine Cycloalkylidengruppe mit 3 bis 7 Kohlenstoffatomen oder eine Vinylidengruppe der Formel

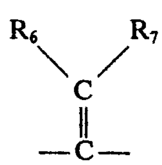

wobei

$R_6$ und $R_7$, die gleich oder verschieden sein können, Wasserstoffatome oder Alkylgruppen mit jeweils 1 bis 3 Kohlenstoffatomen oder auch einer der Reste $R_6$ oder $R_7$ eine Cycloalkylgruppe mit 3 bis 7 Kohlenstoffatomen, eine Aryl- oder Aralkylgruppe oder $R_6$ und $R_7$ zusammen mit dem dazwischen liegenden Kohlenstoffatom einen Cycloalkylidenrest mit 5 bis 7 Kohlenstoffatomen bedeuten,

B eine gegebenenfalls durch eine Alkylgruppe mit 1 bis 3 Kohlenstoffatomen substituierte Methylen- oder Äthylengruppe und

W ein Wasserstoff- oder Halogenatom, eine Nitrogruppe, eine gegebenenfalls durch eine Alkanoylgruppe mit 1 bis 3 Kohlenstoffatomen substituierte Aminogruppe, eine gegebenenfalls durch eine Hydroxy-, Carboxy- oder Alkoxycarbonylgruppe oder zwei Alkoxycarbonylgruppen mit jeweils insgesamt 2 bis 4 Kohlenstoffatomen substituierte Alkylgruppe mit 1 bis 3 Kohlenstoffatomen, eine durch eine Carboxy- oder Alkoxycarbonylgruppe mit insgesamt 2 bis 4 Kohlenstoffatomen substituierte Alkenylgruppe mit 2 bis 5 Kohlenstoffatomen, eine Alkanoylgruppe mit 1 bis 3 Kohlenstoffatomen, eine Dialkoxymethyl- oder Trialkoxymethylgruppe mit 1 bis 3 Kohlenstoffatomen in jedem Alkylteil, eine Alkylendioxymethylgruppe mit 2 oder 3 Kohlenstoffatomen im Alkylenteil, eine 1,3-Oxazolin-2-yl- oder Cyanogruppe, eine gegebenenfalls durch eine oder zwei Alkylgruppen mit jeweils 1 bis 4 Kohlenstoffatomen in jedem Alkylteil substituierte Aminocarbonylgruppe, eine unverzweigte Alkyleniminocarbonylgruppe mit insgesamt 5 bis 8 Kohlenstoffatomen, eine Morpholinocarbonylgruppe, eine (Dialkyl-dioxolanyl)-alkoxycarbonylgruppe mit insgesamt 7 bis 10 Kohlenstoffatomen oder eine Carboxygruppe und deren Ester, wobei Alkylester mit 1 bis 6 Kohlenstoffatomen im Alkylteil mit Ausnahme der $\alpha$-Position jeweils durch eine Hydroxy-, Alkoxy-, Amino-, Alkylamino-, Dialkylamino-, 1,3-Dimethyl-xanthin-7-yl-, Alkanoyloxy-, Aroyloxy-, Aralkanoyloxy- oder Pyridincarbonylgruppe oder durch zwei Hydroxygruppen, wobei eine Methyl- bzw. Methylengruppe jeweils nur durch eine Hydroxygruppe substituiert sein kann, oder durch eine Gruppe der Formel

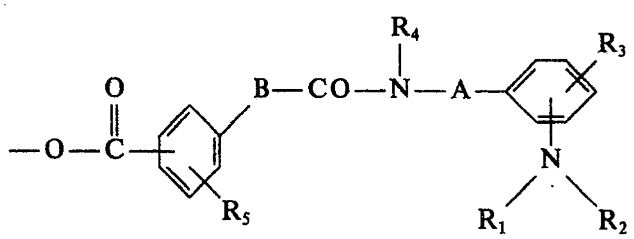

in der
A, B und $R_1$ bis $R_5$ wie eingangs definiert sind, substituiert sein können, wobei jeder Alkylsubstituent in den vorstehend genannten Alkylestern 1 bis 3 Kohlenstoffatome enthalten kann.

Für die bei der Definition der Reste eingangs erwähnten Bedeutungen kommen beispielsweise

für $R_1$ und $R_2$ zusammen mit dem Stickstoffatom die der Dimethylamino-, Diäthylamino-, Dipropylamino-, Dibutylamino-, Diisobutylamino-, Dipentylamino-, Dihexylamino-,N-Methyl-N-äthylamino-, N-Methyl-N-propylamino-, N-Isopropyl-N-propylamino-, N-Isobutyl-N-propylamino-, N-Methyl-N-isopropylamino-, N-Methyl-N-butylamino-, N-Äthyl-N-butylamino-, N-Äthyl-N-isopropylamino-, N-Äthyl-N-pentylamino-, N-Propyl-N-butylamino-, N-Methyl-N-cyclopentylamino-, N-Äthyl-N-cyclopentylamino-, N-Methyl-N-cyclohexylamino-, N-Äthyl-N-cyclohexylamino-, N-Propyl-N-cyclohexylamino-, N-Isobutyl-N-cyclohexylamino-, Pyrrolidino-, Piperidino-, Hexamethylenimino-, Heptamethylenimino-, Octamethylenimino-, Nonamethylenimino-, Decamethylenimino-, Dimethylazetidino-, Methyl-pyrrolidino-, Dimethyl-pyrrolidino-, Äthyl-pyrrolidino-, Methyl-piperidino-, Dimethyl-piperidino-, Äthyl-piperidino-, Diäthyl-piperidino-, Methyl-äthyl-piperidino-, Propyl-piperidino-, Methyl-propyl-piperidino-, Isopropyl-piperidino-, cis-3,5-Dimethyl-piperidino-, trans-3,5-Dimethyl-piperidino-, Morpholino-, Thiomorpholino-, Piperazino-, N-Methyl-piperazino-, N-Äthyl-piperazino-, N-Propyl-piperazino-, N-Isopropyl-piperazino-, N-Benzyl-piperazino-, N-(2-Phenyl-äthyl)-piperazino-, N-(3-Phenylpropyl)-piperazino-, N-Phenylpiperazino-, N-Fluorphenyl-piperazino-, N-Chlorphenyl-piperazino-, N-Bromphenyl-piperazino-, Hydroxy-pyrrolidino-, Hydroxy-piperidino-, Hydroxy-hexamethylentimino-, Pyrrolidon-1-yl-, Piperidon-1-yl-, Hexahydroazepinon-1-yl-, Tetrahydro-isochinolin-2-yl-, Octahydro-isochinolin-2-yl-, Decahydro-isochinolin-2-yl-, Dihydro-isoindol-2-yl-, Hexahydro-isoindol-2-yl-, Octahydro-isoindol-2-yl-, Tetrahydro-3-benzazepin-3-yl-, Decahydro-3-benzazepin-3-yl-, 3-Aza-bicyclo[3.2.0]heptan-3-yl-, 3-Aza-bicyclo[3.2.1]octan-3-yl-, 3-Aza-bicyclo[3.2.2]nonan-3-yl-, 1,4-Dioxa-7-aza-spiro[4,4]nonan-7-yl-, 1,4-Dioxa-7-azaspiro[4,5]decan-7-yl-, 1,4-Dioxa-8-aza-spiro[4,5]decan-8-yl-, 1,4-Dioxa-8-aza-spiro[4,6]undecan-8-yl-, Pyrrolino- oder Tetrahydropyridino-gruppe,

für $R_3$ die des Wasserstoff-, Fluor-, Chlor-, Brom- oder Jodatoms, die der Methyl-, Äthyl-, Propyl-, Isopropyl-, Hydroxy-, Methoxy-, Äthoxy-, Propoxy-, Isopropoxy-, Acetoxy-, Propionyloxy-, Mercapto-, Methylmercapto-, Äthylmercapto-, Propylmercapto-, Isopropylmercapto-, Trifluormethyl-, Nitro-, Cyano-, Formyl-, Acetyl-, Propionyl-, Aminosulfonyl-, Amino-, Methylamino-, Äthylamino-, Propylamino-, Dimethylamino-, Diäthylamino-, Dipropylamino-, Diisopropylamino-, N-Methyl-N-äthylamino-, N-Methyl-N-isopropylamino-, N-Äthyl-N-propylamino-, Formylamino-, Acetylamino-, Propionylamino-, Methylsulfonylamino-, Äthylsulfonylamino-, Propylsulfonylamino-, Isopropylsulfonylamino-, Carboxy-, Methoxycarbonyl-, Äthoxycarbonyl-, Propoxycarbonyl-, Isopropoxycarbonyl-, Methoxycarbonylamino-, Äthoxycarbonylamino-, Propoxycarbonylamino-, Isopropoxycarbonylamino-, Benzoylamino-, Benzyloxy-, 1-Phenyl-äthoxy-, 2-Phenyl-äthoxy-, 3-Phenyl-propoxy-, Aminocarbonyl-, Methylaminocarbonyl-, Äthylaminocarbonyl-, Isopropylaminocarbonyl-, Dimethylaminocarbonyl-, Diäthylaminocarbonyl-, Dipropylaminocarbonyl-, Methyl-äthyl-aminocarbonyl- oder Methyl-propylaminocarbonylgruppe,

für $R_4$ die des Wasserstoffatoms, die der Methyl-, Äthyl-, Propyl- oder Isopropylgruppe,

für $R_5$ die des Wasserstoff-, Fluor-, Chlor-, Brom- oder Jodatoms, die der Methyl-, Äthyl-, Propyl- oder Isopropylgruppe,

für A die der Einfachbindung, die der Methylen-, Äthyliden-, Äthyl-methylen-, Propyl-methylen-, Isopropyl-methylen-, Butyl-methylen-, Pentyl-methylen-, Dimethyl-methylen-, Diäthyl-methylen-, Dipropyl-methylen-, Methyl-äthyl-methylen-, Methyl-propyl-methylen-, Äthyl-propyl-methylen-, Äthyl-isopropyl-methylen-, Äthylen-, Methyl-äthylen-, Äthyl-äthylen-, Propyl-äthylen-, Dimethyl-äthylen-, Cyclopropyl-methylen-, Cyclobutyl-methylen-, Cyclopentyl-methylen-, Cyclohexyl-methylen-, Cycloheptyl-methylen-, Cyclopropyliden-, Cyclobutyliden-, Cyclopentyliden-, Cyclohexyliden-, Cycloheptyliden-, Carboxy-methylen-, Methoxycarbonyl-methylen-, Äthoxycarbonyl-methylen-, Propoxycarbonyl-methylen-, Hydroxymethyl-methylen-, 1-Hydroxyäthyl-methylen-, 2-Hydroxyäthyl-methylen-, 1-Hydroxypropyl-methylen-, 3-Hydroxypropyl-methylen-, Methoxymethyl-methylen-, Äthoxymethyl-methylen-, Propoxymethyl-methylen-, 1-Methoxyäthyl-methylen-, 2-Methoxyäthyl-methylen-, 2-Äthoxyäthyl-methylen-, Cyano-methylen-, Aminocarbonyl-methylen-, Methylaminocarbonyl-methylen-, Dimethylaminocarbonylmethylen-, Äthylaminocarbonyl-methylen-, Diäthylaminocarbonyl-methylen-, Propylaminocarbonyl-methylen-, Phenyl-methylen-, Benzyl-methylen-, 1-Phenyläthyl-methylen-, 2-Phenyläthyl-methylen-, 3-Phenylpropyl-methylen-, 2-Phenylpropyl-methylen-, Vinyliden-, Methylvinyliden-, Dimethyl-vinyliden-, Äthyl-vinyliden-, Diäthyl-vinyliden-, Propyl-vinyliden-, Dipropyl-vinyliden-, Äthyl-methyl-vinyliden-, Äthyl-propyl-vinyliden-, Methyl-propyl-vinyliden-, Cyclopentyl-vinyliden-, Cyclohexyl-vinyliden-, Phenyl-vinyliden-, Benzyl-vinyliden-, 2-Phen-äthyl-vinyliden-, Cyclopropyliden-methylen-, Cyclopentyliden-methylen-, Cyclohexyliden-methylen- oder Cycloheptyliden-methylengruppe,

für B die der Methylen-, Äthylen-, Äthyliden-, Äthyl-methylen-, Propyl-methylen-, oder Isopropyl-methylengruppe und

für W die des Wasserstoff-, Chlor-, Brom- oder Jodatoms, die der Methyl-, Äthyl-, Propyl-, Isopropyl-, Hydroxymethyl-, 1-Hydroxyäthyl-, 2-Hydroxyäthyl-, 1-Hydroxypropyl-, 3-Hydroxypropyl-, Carboxymethyl-, 1-Carboxyäthyl-, 2-Carboxy-äthyl-, 3-Carboxy-propyl-, Methoxycarbonyl-methyl-, Äthoxycarbonyl-methyl-, Propoxycarbonyl-methyl-, 2-Methoxycarbonyl-äthyl-, 2-Äthoxycarbonyl-äthyl-, 3-Äthoxycarbonyl-propyl-, Bis-(methoxycarbonyl)-methyl-, Bis-(äthoxycarbonyl)-methyl-, 2,2-Bis-(äthoxycarbonyl)-äthyl-, Carboxy-vinyl-, Carboxy-propenyl-, Carboxy-pentenyl-, Methoxycarbonyl-vinyl, Äthoxycarbonyl-vinyl-, Propoxycarbonyl-vinyl-, Formyl-, Acetyl-, Propionyl-, Dimethoxy-methyl-, Diäthoxy-methyl-, Dipropoxy-methyl-, Trimethoxy-methyl-, Triäthoxy-methyl-, 1,2-Äthylendioxy-methyl-, 1,3-Propylendioxy-methyl-, Cyano-, Nitro-, Amino-, Formylamino-, Acetamino-, Propionylamino-, 1,3-Oxazolin-2-yl-, Aminocarbonyl-, Methylaminocarbonyl-, Äthylaminocarbonyl-, Propylaminocarbonyl-, Isopropylaminocarbonyl-, Butylaminocarbonyl-, Dimethylaminocarbonyl-, Diäthylaminocarbonyl-, Dipropylaminocarbonyl-, Dibutylaminocarbonyl-, Pyrrolidinocarbonyl-, Piperidinocarbonyl-, Hexamethyleniminocarbonyl-, Heptamethyleniminocarbonyl-, Morpholinocarbonyl-, Carboxy-, Methoxycarbonyl-, Äthoxycarbonyl-, Propoxycarbonyl-, Isopropoxycarbonyl-, Butoxycarbonyl-, tert.-Butoxycarbonyl-, Pentoxycarbonyl-, Hexoxycarbonyl-, Heptoxycarbonyl-, Octoxycarbonyl-, Allyloxycarbonyl-, Butenyloxycarbonyl-,Benzyloxycarbonyl-, 1-Phenyläthoxycarbonyl-, 2-Phenyläthoxycarbonyl-, 3-Phenylpropoxycarbonyl-, 2-Hydroxyäthoxycarbonyl-, 2-Hydroxypropoxycarbonyl-, 3-Hydroxypropoxycarbonyl-, 2-Methoxyäthoxycarbonyl-, 2-Äthoxyäthoxycarbonyl-, (2,2-Dimethyl-dioxolan-4-yl)-methoxycarbonyl-, 2-(2,2-Dimethyl-dioxolan-4-yl)-äthoxycarbonyl-, (2,2-Diäthyl-dioxolan-4-yl)-methoxy-carbonyl-, 2-(2,2-Diäthyl-dioxolan-4-yl)-äthoxycarbonyl-, 3-(2,2-Dimethyl-dioxolan-4-yl)-propoxycarbonyl-, 2-Amino-äthoxycarbonyl-, 2-Dimethylamino-äthoxycarbonyl-, 2-Diäthylamino-äthoxycarbonyl-, 2-(1,3-Dimethyl-xanthin-7-yl)-äthoxycarbonyl-, 2-Acetoxy-äthoxycarbonyl-, 2-Benzoyloxy-äthoxycarbonyl-, 2-Phenylacetoxy-äthoxycarbonyl-, 2-Pyridincarbonyloxy-äthoxycarbonyl-, 2,3-Dihydroxy-propoxycarbonyl-, 3,4-Dihydroxy-butoxycarbonyl-, 2-[4-[(1-(2-Piperidino-phenyl)-äthyl)-aminocarbonylmethyl]-benzoyloxy]-äthoxycarbonyl- oder 3-[4-[(1-(2-Piperidino-phenyl)-äthyl)-aminocarbonylmethyl]-benzoyloxy]-propoxycarbonylgruppe in Betracht.

Bevorzugte Verbindungen der obigen allgemeinen Formel I sind jedoch diejenigen, in denen

$R_1$ und $R_2$ zusammen mit dem dazwischenliegenden Stickstoffatom eine Dialkylamino- oder N-Alkylcyclohexylaminogruppe, in der jeder Alkylteil 1 bis 4 Kohlenstoffatome enthalten kann, eine gegebenenfalls durch eine oder zwei Methylgruppen substituierte unverzweigte Alkyleniminogruppe mit 3 bis 6 Kohlenstoffatomen, eine Hydroxypiperidino-, Piperidon-1-yl-, Tetrahydro-pyridino-, Morpholino-, Thiomorpholino-, N-Methylpiperazino-, N-Benzyl-piperazino-, N-Chlorphenylpiperazino-, Heptamethylenimino- oder Octamethyleniminogruppe, eine gesättigte oder teilweise ungesättigte Azabicycloalkylgruppe mit 7 bis 9 Kohlenstoffatomen, eine unverzweigte Alkyleniminogruppe mit 4 bis 6 Kohlenstoffatomen, in der eine Äthylengruppe durch eine o-Phenylengruppe ersetzt ist, oder eine 1,4-Dioxa-aza-spiro-alkylgruppe mit 7 oder 8 Kohlenstoffatomen,

$R_3$ ein Wasserstoff-, Fluor-, Chlor-, Brom- oder Jodatom, eine Methyl-, Trifluormethyl-, Hydroxy-, Methoxy-, Benzyloxy-, Acetoxy-, Mercapto-, Methylmercapto-, Nitro-, Amino-, Dimethylamino-, Acetylamino-, Methylsulfonylamino-, Benzoylamino-, Äthoxy-carbonylamino-, Cyano-, Carboxy-, Methoxycarbonyl-, Äthoxycarbonyl-, Aminocarbonyl-, Acetyl- oder Aminosulfonylgruppe,

$R_4$ ein Wasserstoffatom oder eine Methylgruppe

$R_5$ ein Wasserstoffatom, ein Chloratom oder eine Methylgruppe,

A eine Bindung, eine gegebenenfalls durch eine Alkylgruppe mit 1 bis 3 Kohlenstoffatomen, eine Phenyl-, Cyclohexyl-, Carboxy-, Methoxycarbonyl- oder Hydroxymethylgruppe substituierte Methylengruppe, eine Dimethyl-methylen-, Cyclopropyliden- oder Äthylengruppe oder eine Vinylidengruppe der Formel

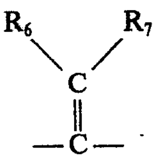

wobei $R_6$ und $R_7$, die gleich oder verschieden sein können Wasserstoffatome oder Methylgruppen oder $R_6$ und $R_7$ zusammen mit den dazwischenliegenden Kohlenstoffatomen einen Cycloalkylidenrest mit 5 oder 6 Kohlenstoffatomen bedeuten,

B eine Methylen-, Äthyliden- oder Äthylengruppe und

W ein Wasserstoffatom, eine Methyl-, Äthyl-, Hydroxymethyl-, Cyano- oder Carboxyvinylengruppe, eine durch eine Carboxygruppe oder durch eine oder zwei Alkoxycarbonylgruppen mit jeweils insgesamt 2 bis 4 Kohlenstoffatomen substituierte Alkylgruppe mit 1 bis 3 Kohlenstoffatomen, eine durch ein Wasserstoffatom, eine Methyl-, Äthyl-, Hydroxy-, Alkoxy-, (2,2-Dimethyl-dioxolan-4-yl)-methoxy-, Benzyloxy-, Pyridylmethoxy-, Amino-, Alkylamino-, Dialkylamino-, Piperidino- oder Morpholinogruppe substituierte Carbonylgruppe, wobei jeder Alkylteil in den vorstehend erwähnten Gruppen 1 bis 3 Kohlenstoffatome enthalten kann, oder eine Gruppe der Formel

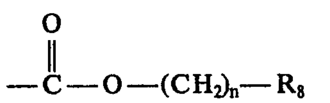

bedeuten, in der n die Zahl 2, 3 oder 4 und

$R_8$ eine Hydroxy-, Methoxy-, Äthoxy-, Acetoxy-, Benzoyloxy-, Pyridincarbonyloxy-, eine Dialkylaminogruppe mit 1 bis 3 Kohlenstoffatomen in jedem Alkylteil, eine 1,3-Dimethylxanthin-7-yl-gruppe oder eine Gruppe der Formel

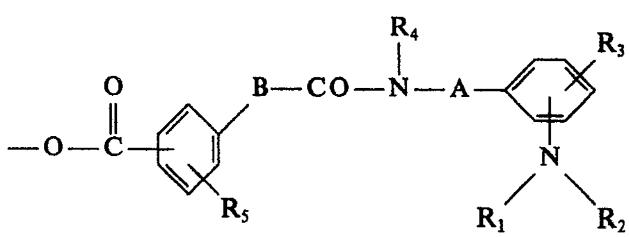

in der
A, B und $R_1$ bis $R_5$ wie vorstehend erwähnt definiert sind, darstellen,
besonders jedoch diejenigen Verbindungen, in denen der Rest

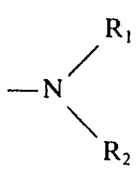

in 2-Position und der Rest W in 4'-Position steht, und, sofern sie ein asymmetrisches Kohlenstoffatom enthalten, deren optisch aktive Antipoden und deren Salze.

Besonders bevorzugte Verbindungen sind jedoch diejenigen der allgemeinen Formel

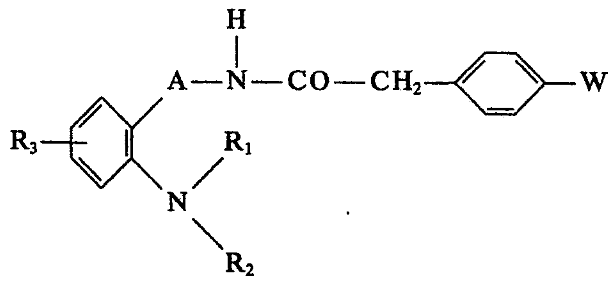

(Ia)

in der

$R_1$ und $R_2$ zusammen mit dem dazwischenliegenden Stickstoffatom eine Dimethylamino-, Pyrrolidino-, Methylpyrrolidino-, Piperidino-, Methylpiperidino-, Dimethylpiperidino-, Tetrahydro-pyridino-, 2-Octahydro-isoindolo- oder Hexamethylen-iminogruppe,

$R_3$ ein Wasserstoff-, Fluor- oder Chloratom oder eine Methylgruppe,

A eine gegebenenfalls durch eine Cyclohexyl-, Phenyl-, Methoxycarbonyl-, Äthoxycarbonyl- oder eine Alkylgruppe mit 1 bis 3 Kohlenstoffatomen substituierte Methylengruppe, eine Dimethyl-methylen- oder eine Vinylidengruppe der Formel

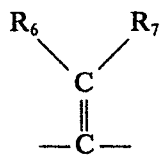

wobei $R_6$ und $R_7$ je ein Wasserstoffatom oder zusammen mit dem dazwischenliegenden Kohlenstoffatom eine Cyclohexylidengruppe darstellen, und

W eine Methyl-, Hydroxymethyl- oder Carboxymethylgruppe, eine durch ein Wasserstoffatom, eine Methyl-, Hydroxy-, Methoxy-, Äthoxy-, Propoxy-, Isopropoxy-, 2-Hydroxyäthoxy-, 2-Methoxyäthoxy-, (2,2-Dimethyl-dioxolan-4-yl)-methoxy- oder 2-Diäthylaminoäthoxy-gruppe substituierte Carbonylgruppe bedeuten, deren optisch aktive Antipoden und deren physiologisch verträgliche Salze mit anorganischen oder organischen Säuren bzw. Basen.

Erfindungsgemäß erhält man die neuen Verbindungen nach folgenden Verfahren:

a) Acylierung eines Amins der allgemeinen Formel

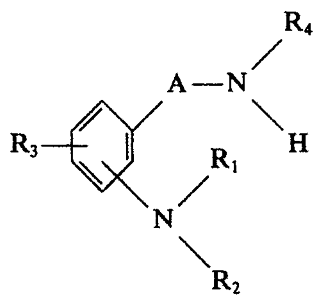

(II)

in der

A und $R_1$ bis $R_4$ wie eingangs definiert sind, bzw., wenn A eine der eingangs erwähnten Vinylidengruppen darstellt, dessen Tautomeren oder dessen Lithium- oder Magnesiumhalogenid-Komplex mit einer Carbonsäure der allgemeinen Formel

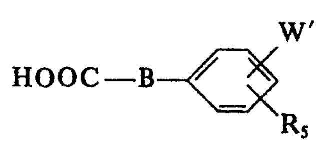

(III)

in der

$R_5$ und B wie eingangs definiert sind und

W' die für W eingangs erwähnten Bedeutungen besitzt oder eine durch einen Schutzrest geschützte Carboxylgruppe darstellt,
oder mit deren gegebenenfalls im Reaktionsgemisch hergestellten reaktionsfähigen Derivaten.
Als reaktionsfähige Derivate einer Verbindung der allgemeinen Formel III kommen beispielsweise deren Ester wie der Methyl-, Äthyl- oder Benzylester, deren Thioester wie der Methylthio- oder Äthylthioester, deren Halogenide wie das Säurechlorid, deren Anhydride oder Imidazolide in Betracht.
Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z. B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z. B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen —25 und 250°C, vorzugsweise jedoch bei Temperaturen zwischen —10°C und der Siedetemperatur des verwendeten Lösungsmittels, durchgeführt. Die Umsetzung kann auch ohne Lösungsmittel durchgeführt werden, des weiteren kann während der Umsetzung entstehendes Wasser durch azeotrope Destillation, z. B. durch Erhitzen mit Toluol am Wasserabscheider, oder durch Zugabe eines Trockenmittels wie Magnesiumsulfat oder Molekularsieb abgetrennt werden.
Erforderlichenfalls wird die anschließende Abspaltung eines Schutzrestes vorzugsweise hydrolytisch durchgeführt, zweckmäßigerweise entweder in Gegenwart einer Säure wie Salzsäure, Schwefelsäure, Phosphorsäure oder Trichloressigsäure oder in Gegenwart einer Base wie Natriumhyxdroxid oder Kaliumhydroxid in einem geeigneten Lösungsmittel wie Wasser, Methanol, Äthanol, Äthanol/Wasser, Wasser/Isopropanol oder Wasser/Dioxan bei Temperaturen zwischen —10 und 120°C, z. B. bei Temperaturen zwischen Raumtemperatur und der Siedetemperatur des Reaktionsgemisches.
Ein als Schutzrest verwendeter tert.Butylrest kann auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure abgespalten werden.
Des weiteren kann ein als Schutzrest verwendeter Benzylrest auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid abgespalten werden.

b) Zur Herstellung einer Verbindung der allgemeinen Formel I, in der W eine Carboxygruppe darstellt:
Spaltung einer Verbindung der allgemeinen Formel

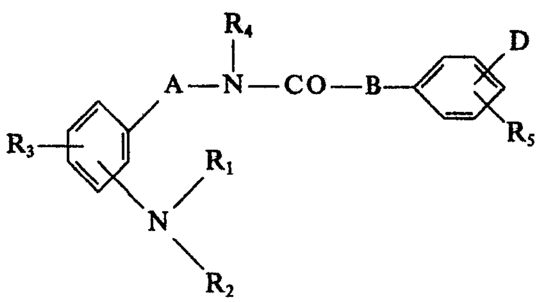

(IV)

in der
$R_1$ bis $R_5$, A und B wie eingangs definiert sind und
D eine durch Hydrolyse, Thermolyse oder Hydrogenolyse in eine Carboxygruppe überführbare Gruppe darstellt.
Als hydrolysierbare Gruppen kommen beispielsweise funktionelle Derivate der Carboxygruppe wie deren unsubstituierte oder substituierte Amide, Ester, Thioester, Orthoester, Iminoäther, Amidine oder Anhydride, die Nitrilgruppe, eine Malonester-(1)-yl-gruppe, die Tetrazolylgruppe, eine gegebenenfalls substituierte 1,3-Oxazol-2-yl- oder 1,3-Oxazolin-2-yl-gruppe und
als thermolytisch abspaltbare Gruppen beispielsweise Ester mit tertiären Alkoholen, z. B. der tert.Butylester, und
als hydrogenolytisch abspaltbare Gruppen beispielsweise Aralkylgruppen, z. B. die Benzylgruppe, in Betracht.
Die Hydrolyse wird zweckmäßigerweise entweder in Gegenwart einer Säure wie Salzsäure, Schwefelsäure, Phosphorsäure oder Trichloressigsäure oder in Gegenwart einer Base wie Nartriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen −10 und 120° C, z. B. bei Temperaturen zwischen Raumtemperatur und der Siedetemperatur des Reaktionsgemisches, durchgeführt.

Bedeutet D in einer Verbindung der allgemeinen Formel IV eine Nitril- oder Aminocarbonylgruppe, so können diese Gruppen auch mit einem Nitrit, z. B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50° C in die Carboxygruppe übergeführt werden.

Bedeutet D in einer Verbindung der allgemeinen Formel IV beispielsweise die tert.Butyloxycarbonylgruppe, so kann die tert.Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z. B. bei Temperaturen zwischen 40 und 100° C, abgespalten werden.

Bedeutet D in einer Verbindung der allgemeinen Formel IV beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50° C, z. B. bei Raumtemperatur, und einem Wasserstoffdruck von 1 bis 5 bar abgespalten werden. Bei der Hydrogenolyse können gleichzeitig andere Gruppen mitreduziert werden, z. B. eine Halogenverbindung enthalogeniert, eine Nitrogruppe in die entsprechende Aminogruppe und eine Vinylidengruppe in die entsprechende Alkylidengruppe übergeführt werden.

c) Umsetzung einer gegebenenfalls im Reaktionsgemisch gebildeten Verbindung der allgemeinen Formel

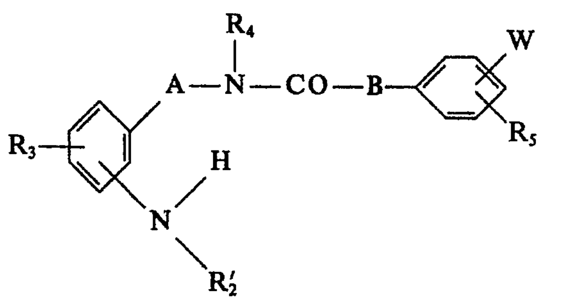

(V)

in der
$R_3$ bis $R_5$, A, B und W wie eingangs definiert sind,
$R_2'$ ein Wasserstoffatom darstellt oder die für $R_2$ vorstehend erwähnten Bedeutungen besitzt, mit einer Verbindung der allgemeinen Formel

$$R_1'-E \qquad (VI)$$

in der
$R_1'$ die für $R_1$ eingangs erwähnten Bedeutungen besitzt oder zusammen mit dem Rest $R_2'$ der Formel V eine gegebenenfalls durch eine oder zwei Alkylgruppen mit jeweils 1 bis 3 Kohlenstoffatomen substituierte geradkettige Alkylengruppe mit 4 bis 6 Kohlenstoffatomen oder eine n-Pentylgruppe, in der die 3. Methylengruppe durch ein Sauerstoff- oder Schwefelatom ersetzt ist, darstellt und
E eine nukleophile Austrittsgruppe wie ein Halogenatom oder eine Sulfonyloxygruppe, z. B. ein Chlor-, Brom- oder Jodatom, eine Methansulfonyloxy- oder p-Toluolsulfonyloxygruppe, oder auch ein Wasserstoffatom, wenn in $R_1'$ eine Methylengruppe durch eine Aldehyd- oder Ketoncarbonylgruppe ersetzt ist, bedeuten, erforderlichenfalls in Gegenwart eines Reduktionsmittels und gegebenenfalls anschließende Hydrolyse.

Als Alkylierungsmittel der Formel VI kommen somit beispielsweise die entsprechenden Halogenide oder Sulfate wie Methyljodid, Äthyljodid, Propylbromid, Dimethylsulfat oder Diäthylsulfat in Betracht.

Die Umsetzung wird zweckmäßigerweise in einem Lösungsmittel wie Aceton, Tetrahydrofuran, Dimethylformamid, Dimethylsulfoxid oder Hexamethylphosphorsäuretriamid, gegebenenfalls in Gegenwart einer anorganischen Base wie Natriumcarbonat, Kaliumkarbonat oder Kalium-tert.butylat oder einer tertiären organischen Base wie Pyridin, bei Temperaturen zwischen 0 und 150° C, vorzugsweise jedoch bei Temperaturen zwischen 20 und 75° C, durchgeführt. Setzt man eine Verbindung der allgemeinen Formel V ein, in der W eine Carboxylgruppe darstellt, so kann diese

Carboxylgruppe je nach den verwendeten Reaktionsbedingungen, z. B. bei Temperaturen oberhalb Raumtemperatur und in Gegenwart einer geeigneten Base, beispielsweise von Natriumcarbonat, gleichzeitig in den entsprechenden Ester übergeführt werden.

Die Methylierung kann auch in der Weise durchgeführt werden, daß eine Verbindung der allgemeinen Formel V mit Formalin in Gegenwart eines Reduktionsmittels, z. B. Ameisensäure oder Wasserstoff in Gegenwart eines Hydrierungskatalysators, z. B. Palladium oder Platin, gegebenenfalls in einem Lösungsmittel wie Ameisensäure oder Eisessig bei Temperaturen bis zur Siedetemperatur des Reaktionsgemisches umgesetzt wird.

Ferner kann die Alkylierung auch mit einer entsprechenden Carbonylverbindung in Gegenwart eines Hydrids wie Natriumcyanoborhydrid in einem geeigneten Lösungsmittel wie Acetonitril/Essigsäure oder Dimethylformamid/Essigsäure vorzugsweise bei pH = 7 und bei Temperaturen zwischen 0 und 50° C durchgeführt werden.

Die gegebenenfalls anschließende Hydrolyse wird vorzugsweise in einem wäßrigen Lösungsmittel wie Wasser/Methanol, Wasser/Äthanol oder Wasser/Dioxan in Gegenwart einer Säure wie Salzsäure oder Schwefelsäure oder in Gegenwart einer Base wie Natrium- oder Kaliumhydroxid bei Temperaturen zwischen 50 und 100° C durchgeführt.

d) Zur Herstellung einer Verbindung der allgemeinen Formel I, in der W eine Carboxygruppe, eine Alkanoylgruppe mit 1 bis 3 Kohlenstoffatomen oder eine Alkylgruppe mit 1 bis 3 Kohlenstoffatomen bedeutet:

Umsetzung einer Verbindung der allgemeinen Formel

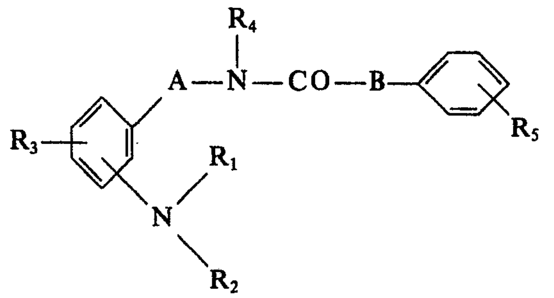

(VII)

in der

$R_1$ bis $R_5$, A und B wie eingangs definiert sind, mit Phosgen, einem Oxalylhalogenid, einem Alkyl- oder Alkanoylhalogenid mit jeweils 1 bis 3 Kohlenstoffatomen im Alkylteil oder mit Cyanwasserstoff/Halogenwasserstoff, vorzugsweise jedoch Chlorwasserstoff, in Gegenwart einer Lewis-Säure.

Hierbei kommen als Halogenide insbesondere die Chloride und Bromide und als Lewis-Säure insbesondere Aluminiumchlorid in Betracht.

Die Umsetzung wird vorzugsweise in einem Lösungsmittel wie Methylenchlorid, Nitrobenzol, Chlorbenzol, Dichlorbenzol, Tetrachloräthan oder Schwefelkohlenstoff oder in Polyphosphorsäure bei Temperaturen zwischen 0 und 120° C, vorzugsweise jedoch bei Temperaturen zwischen 20 und 80° C, durchgeführt. Bedeutet hierbei in einer Verbindung der allgemeinen Formel VII $R_3$ ein Wasserstoffatom, so kann es gleichzeitig durch einen entsprechenden Alkyl- oder Acylrest ersetzt werden.

e) Zur Herstellung einer Verbindung der allgemeinen Formel I, in der W die Carboxygruppe darstellt:

Umsetzung einer Verbindung der allgemeinen Formel

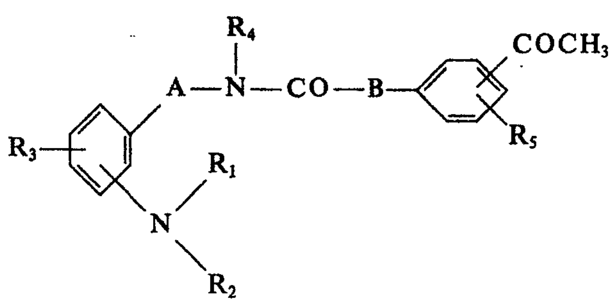

(VIII)

in der

$R_1$ bis $R_5$, A und B wie eingangs definiert sind, mit einem gegebenenfalls im Reaktionsgemisch hergestellten Hypohalogenit.

Die Umsetzung wird zweckmäßigerweise in einem Lösungsmittel wie Wasser/Tetrahydrofuran oder Wasser/Dioxan und in Gegenwart einer Base wie Natriumhydroxid oder Kaliumhydroxid bei Temperaturen zwischen 0 und 80° C, vorzugsweise jedoch bei Temperaturen zwischen 25 und

50°C, durchgeführt.

f) Zur Herstellung einer Verbindung der allgemeinen Formel I, in der W die Carboxygruppe darstellt: Oxidation einer Verbindung der allgemeinen Formel

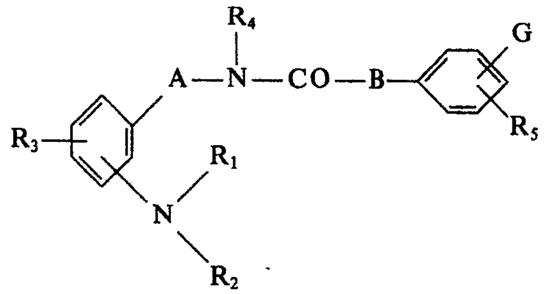

(IX)

in der

$R_1$ bis $R_5$, A und B wie eingangs definiert sind und

G eine durch Oxidation in eine Carboxygruppe überführbare Gruppe darstellt.

Als eine derartige oxidierbare Gruppe kommt beispielsweise die Formylgruppe und deren Acetale, die Hydroxymethylgruppe und deren Äther, eine unsubstituierte oder substituierte Acylgruppe wie die Acetyl-, Chloracetyl-, Propionyl-, die Malonsäure-(1)-yl-gruppe oder eine Malonester-(1)-yl-gruppe in Betracht.

Die Umsetzung wird mit einem Oxidationsmittel in einem geeigneten Lösungsmittel wie Wasser, Eisessig, Pyridin oder Tetrachlorkohlenstoff bei Temperaturen zwischen 0 und 100°C, zweckmäßigerweise jedoch bei Temperaturen zwischen 20 und 50°C, durchgeführt. Die Umsetzung wird jedoch vorzugsweise mit Silberoxid/Natronlauge, Mangandioxid/Aceton oder Methylenchlorid, Wasserstoffperoxid/Natronlauge, Brom oder Chlor/Natron- oder Kalilauge oder Chromtrioxid/Pyridin durchgeführt.

g) Zur Herstellung einer Verbindung der allgemeinen Formel I, in der $R_3$ eine Nitrogruppe bedeutet: Umsetzung einer Verbindung der allgemeinen Formel

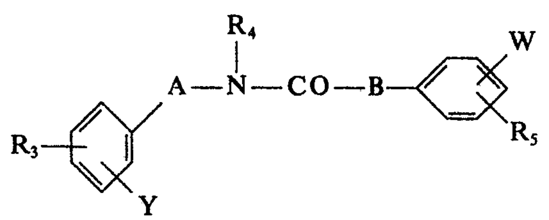

(X)

in der

$R_4$, $R_5$, A, B und W wie eingangs definiert sind,

$R_3$ eine Nitrogruppe und

Y einen nukleophil austauschbaren Rest wie ein Halogenatom darstellen, mit einem Amin der allgemeinen Formel

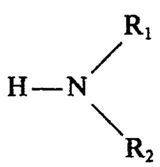

(XI)

in der

$R_1$ und $R_2$ wie eingangs definiert sind, und gegebenenfalls anschließende Hydrolyse.

Unter dem bei der Definition des austauschbaren Restes Y verwendeten Begriff »ein Halogenatom« ist insbesondere ein Fluor-, Chlor- oder Bromatom zu verstehen, vorzugsweise jedoch in o- oder p-Stellung zu der Nitrogruppe.

Die Umsetzung wird zweckmäßigerweise in einem Lösungsmittel wie Wasser, Wasser/Methanol, Wasser/Äthanol, Wasser/Isopropanol, Wasser/Dioxan, Methanol, Äthanol, Dimethylformamid oder in einem Überschuß des eingesetzten Amins der allgemeinen Formel XI und/oder dessen N-Formyl-Derivat gegebenenfalls in Gegenwart einer anorganischen oder tertiären organischen Base, gegebenenfalls in Gegenwart eines Reaktionsbeschleunigers wie Kupfer oder eines Kupfersalzes und gegebenenfalls in einem Druckgefäß bei Temperaturen zwischen 20 und 150°C, vorzugsweise jedoch bei der Siedetemperatur des Reaktionsgemisches, z. B. bei 100°C, durchgeführt. Die Umsetzung kann jedoch auch ohne Lösungsmittel durchgeführt werden.

Die gegebenenfalls anschließende Hydrolyse wird zweckmäßigerweise in einem wäßrigen Lösungsmittel wie Methanol/Wasser, Äthanol/Wasser oder Dioxan/Wasser in Gegenwart einer Säure wie Salzsäure oder Schwefelsäure oder einer Base wie Natrium- oder Kaliumhydroxid bei Temperaturen zwischen 50 und 100°C durchgeführt.

h) Zur Herstellung einer Verbindung der allgemeinen Formel I, in der A eine Gruppe der Formel

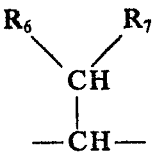

darstellt, wobei $R_6$ und $R_7$ wie eingangs definiert sind:
Reduktion eines Enamides der allgemeinen Formel

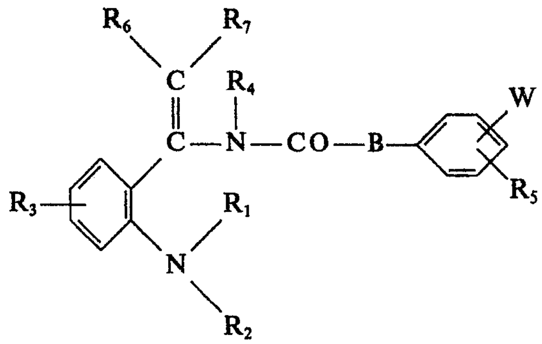

(XII)

in der
$R_1$ bis $R_7$, B und W wie eingangs definiert sind.
Die Reduktion wird vorzugsweise mit Wasserstoff in Gegenwart eines Hydrierungskatalysators wie Palladium/Kohle oder Platin in einem geeigneten Lösungsmittel wie Methanol, Äthanol, Isopropanol, Äthanol/Wasser, Eisessig, Essigsäureäthylester, Dioxan, Tetrahydrofuran, Dimethylformamid, Benzol oder Benzol/Äthanol bei Temperaturen zwischen 0 und 100°C, vorzugsweise jedoch bei Temperaturen zwischen 20 und 50°C, und einem Wasserstoffdruck von 1 bis 5 bar durchgeführt. Bei Verwendung eines geeigneten chiralen Hydrierungskatalysators wie einem Metalligandenkomplex, z. B. einem Komplex aus Rhodiumchlorid und (+)- oder (−) O,O-Isopropyliden-2,3-dihydroxy-1,4-bis-(diphenylphosphino)-butan (=DIOP), erfolgt die Wasserstoffanlagerung enantioselektiv. Des weiteren können bei der katalytischen Hydrierung auch andere reduzierbare Gruppen mitreduziert werden, z. B. eine Nitrogruppe zur Aminogruppe oder ein Chlor- oder Bromatom zum Wasserstoffatom.

i) Zur Herstellung einer Verbindung der allgemeinen Formel I, in der $R_4$ ein Wasserstoffatom und A eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierte Methylen- oder Äthylengruppe, eine durch eine Cycloalkylgruppe mit 3 bis 7 Kohlenstoffatomen, eine Alkoxyalkyl-, Carboxyl-, Alkoxycarbonyl-, Aryl- oder Aralkylgruppe substituierte Methylengruppe, wobei jeder der vorstehend erwähnten Alkylteile 1 bis 3 Kohlenstoffatome enthalten kann, oder eine Cycloalkylidengruppe mit 4 bis 7 Kohlenstoffatomen bedeuten:
Umsetzung einer Verbindung der allgemeinen Formel

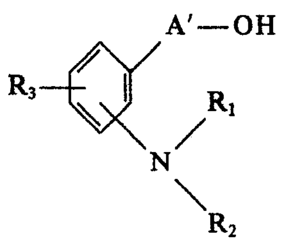

(XIII)

in der
$R_1$ bis $R_3$ wie eingangs definiert sind und
A′ eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierte Methylen- oder Äthylengruppe, eine durch eine Cycloalkylgruppe mit 3 bis 7 Kohlenstoffatomen, eine Alkoxyalkyl-, Carboxyl-, Alkoxycarbonyl-, Aryl- oder Aralkylgruppe substituierte Methylengruppe, wobei jeder der vorstehend erwähnten Alkylteile 1 bis

3 Kohlenstoffatome enthalten kann, oder eine Cycloalkylidengruppe mit 4 bis 7 Kohlenstoffatomen bedeutet, mit einer Verbindung der allgemeinen Formel

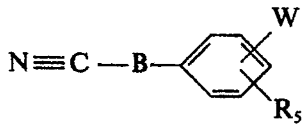

(XIV)

in der

$R_5$, B und W wie eingangs definiert sind.

Die Umsetzung wird in Gegenwart einer starken Säure, welche gleichzeitig als Lösungsmittel dienen kann, vorzugsweise in konzentrierter Schwefelsäure, bei Temperaturen zwischen 20 und 150° C, vorzugsweise bei Temperaturen zwischen 80 und 100° C, durchgeführt.

Erhält man erfindungsgemäß eine Verbindung der allgemeinen Formel I, in der W die Carboxygruppe darstellt, so kann diese gewünschtenfalls mittels Veresterung oder Amidierung in eine entsprechende Verbindung der allgemeinen Formel I übergeführt werden, und/oder

eine Verbindung der allgemeinen Formel I, in der $R_3$ und/oder W eine Nitrogruppe darstellen, so kann diese mittels Reduktion in eine entsprechende Verbindung der allgemeinen Formel I, in der $R_3$ und/oder W eine Aminogruppe darstellen, übergeführt werden, und/oder

eine Verbindung der allgemeinen Formel I, in der $R_3$ und/oder W eine Aminogruppe darstellen, so kann diese über ein entsprechendes Diazoniumsalz in eine entsprechende Verbindung der allgemeinen Formel I, in der $R_3$ ein Wasserstoff- oder Halogenatom, eine Hydroxy-, Alkoxy-, Mercapto-, Alkylmercapto-, Chlorsulfonyl- oder Cyanogruppe und/oder W ein Wasserstoff- oder Halogenatom oder eine Cyanogruppe darstellt, übergeführt werden, wobei eine so gegebenenfalls erhaltene Verbindung der allgemeinen Formel I, in der $R_3$ die Hydroxygruppe darstellt, anschließend mittels Alkylierung in eine entsprechende Verbindung der allgemeinen Formel I übergeführt werden kann, in der $R_3$ eine Alkoxygruppe darstellt, oder eine so gegebenenfalls erhaltene Verbindung der allgemeinen Formel I, in der $R_3$ die Chlorsulfonylgruppe darstellt, anschließend mittels Ammoniak in eine entsprechende Verbindung der allgemeinen Formel I, in der $R_3$ die Aminosulfonylgruppe darstellt, übergeführt werden kann, und/oder

eine Verbindung der allgemeinen Formel I, in der $R_3$ die Aminogruppe darstellt, so kann diese mittels Acylierung in eine entsprechende Verbindung der allgemeinen Formel I, in der $R_3$ eine Alkanoylamino-, Aroylamino-, Alkoxycarbonylamino- oder Alkylsulfonylaminogruppe darstellt, übergeführt werden, und/oder

eine Verbindung der allgemeinen Formel I, in der $R_3$ eine Aminogruppe darstellt, so kann diese mittels Alkylierung in eine entsprechende Verbindung der allgemeinen Formel I, in der $R_3$ eine Alkylamino- oder Dialkylaminogruppe darstellt, übergeführt werden, und/oder

eine Verbindung der allgemeinen Formel I, in der $R_3$ ein Chlor- oder Bromatom darstellt, mittels Enthalogenierung in eine entsprechende Verbindung der allgemeinen Formel I, in der $R_3$ ein Wasserstoffatom darstellt, übergeführt werden, und/oder

eine Verbindung der allgemeinen Formel I, in der $R_3$ die Nitrilgruppe darstellt, so kann diese mittels Hydrolyse bzw. Alkoholyse in eine entsprechende Verbindung der allgemeinen Formel I, in der $R_3$ eine Aminocarbonyl-, Carboxy- bzw. Alkoxycarbonylgruppe darstellt, übergeführt werden, und/oder

eine Verbindung der allgemeinen Formel I, in der $R_3$ eine Carboxy- oder Alkoxycarbonylgruppe und/oder W eine gegebenenfalls veresterte Carboxygruppe darstellen, so kann diese mittels Reduktion in eine entsprechende Verbindung der allgemeinen Formel I, in der $R_3$ und/oder W eine Formyl- oder Hydroxymethylgruppe darstellen, übergeführt werden, und/oder

eine Verbindung der allgemeinen Formel I, in der W eine Gruppe der Formel $-COO-(CH_2)_n-OH$ darstellt, so kann diese mittels Acylierung in eine entsprechende Verbindung der allgemeinen Formel I, in der W eine Gruppe der Formel $-COO-(CH_2)_n-R_8$ darstellt, übergeführt werden, und/oder

eine Verbindung der allgemeinen Formel I, in der W eine Hydroxymethylgruppe darstellt, so kann diese nach Überführung in eine entsprechende Halogenmethylverbindung durch Umsetzung mit einem Malonsäurediester in eine entsprechende Verbindung der allgemeinen Formel I, in der W eine durch zwei Alkoxycarbonylgruppen substituierte Äthylgruppe darstellt, übergeführt werden und/oder

eine Verbindung der allgemeinen Formel I, in der W eine Formylgruppe darstellt, so kann diese mittels Kondensation und gegebenenfalls anschließender Hydrolyse und/oder Decarboxylierung in eine entsprechende Verbindung der allgemeinen Formel I, in der W eine durch eine Hydroxycarbonyl- oder Alkoxycarbonylgruppe substituierte Vinylgruppe darstellt, übergeführt werden und/oder

eine Verbindung der allgemeinen Formel I, in der W eine durch zwei Alkoxycarbonylgruppen substituierte Äthylgruppe darstellt, so kann diese mittels Hydrolyse und Decarboxylierung in eine entsprechende Verbindung der allgemeinen Formel I, in der W eine durch eine Carboxygruppe

substituierte Äthylgruppe darstellt, übergeführt werden und/oder

eine Verbindung der allgemeinen Formel I, in der W die Carboxylgruppe darstellt, so kann diese mittels Überführung in eine Sulfonsäurehydrazid und anschließende Disproportionierung in eine entsprechende Verbindung der allgemeinen Formel I, in der W die Formylgruppe darstellt, übergeführt werden, und/oder

eine Verbindung der allgemeinen Formel I, in der $R_1$ und $R_2$ zusammen mit dem dazwischenliegenden Stickstoffatom eine Aza-1,4-dioxa-spiro-alkylgruppe mit 6 bis 8 Kohlenstoffatomen darstellen, so kann diese mittels Hydrolyse in Gegenwart einer Säure in eine entsprechende Verbindung der allgemeinen Formel I, in der $R_1$ und $R_2$ zusammen mit dem dazwischenliegenden Stickstoffatom eine unverzweigte Alkyleniminogruppe mit 4 bis 6 Kohlenstoffatomen darstellen, in der eine Methylengruppe durch eine Carbonylgruppe ersetzt ist, übergeführt werden, und/oder

eine Verbindung der allgemeinen Fornmel I, in der $R_1$ und $R_2$ zusammen mit dem dazwischenliegenden Stickstoffatom eine unverzweigte Alkyleniminogruppe mit 4 bis 6 Kohlenstoffatomen darstellen, in der eine Methylengruppe durch eine Carbonylgruppe ersetzt ist, so kann diese mittels Reduktion in eine entsprechende Hydroxy-alkyleniminoverbindung der allgemeinen Formel I übergeführt werden, und/oder

eine Verbindung der allgemeinen Formel I, in der W eine Aminocarbonylgruppe darstellt, so kann diese mittels Dehydratisierung in eine entsprechende Verbindung der allgemeinen Formel I, in der W eine Cyangruppe darstellt, übergeführt werden.

Die nachträgliche Dehydratisierung wird mit einem wasserentziehenden Mittel wie Phosphorpentoxid, Schwefelsäure oder p-Toluolsulfonsäurechlorid gegebenenfalls in einem Lösungsmittel wie Methylenchlorid oder Pyridin bei Temperaturen zwischen 0 und 100°C, vorzugsweise bei Temperaturen zwischen 20 und 80°C, durchgeführt.

Die nachträgliche Veresterung wird zweckmäßigerweise in einem geeigneten Lösungsmittel, z. B. in einem entsprechenden Alkohol, Pyridin, Toluol, Methylenchlorid, Tetrahydrofuran oder Dioxan, in Gegenwart eines säureaktivierenden und/oder wasserentziehenden Mittels wie Thionylchlorid, Chlorameisensäureäthylester, Carbonyldiimidazol oder N,N'-Dicyclohexylcarbodiimid oder dessen Isoharnstoffäthern, gegebenenfalls in Gegenwart eines Reaktionsbeschleunigers wie Kupfer-chlorid, oder durch Umesterung, z. B. mit einem entsprechenden Kohlensäurediester, bei Temperaturen zwischen 0 und 100°C, vorzugsweise jedoch bei Temperaturen zwischen 20°C und der Siedetemperatur des betreffenden Lösungsmittels, durchgeführt.

Die nachträgliche Amidierung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z. B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol, N,N'-Thionyldiimidazol oder Triphenylphosphin/Tetrachlorkohlenstoff, oder eines die Aminogruppe aktivierenden Mittels, z. B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen −25 und 250°C, vorzugsweise jedoch bei Temperaturen zwischen −10°C und der Siedetemperatur des verwendeten Lösungsmittels, durchgeführt. Die Umsetzung kann auch ohne Lösungsmittel durchgeführt werden, des weiteren kann während der Umsetzung entstehendes Wasser durch azeotrope Destillation, z. B. durch Erhitzen mit Toluol am Wasserabscheider, oder durch Zugabe eines Trockenmittels wie Magnesiumsulfat oder Molekularsieb abgetrennt werden.

Die nachträgliche Reduktion der Nitroverbindung wird vorzugsweise in einem Lösungsmittel wie Wasser, Wasser/Äthanol, Methanol, Eisessig, Essigsäureäthylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)-chlorid oder Natriumdithionit, oder mit Hydrazin in Gegenwart von Raney-Nikkel bei Temperaturen zwischen 0 und 50°C, vorzugsweise jedoch bei Raumtemperatur, durchgeführt.

Die nachträgliche Umsetzung eines Diazoniumsalzes, z. B. des Fluoroborats, des Hydrosulfates in Schwefelsäure, des Hydrochlorids oder des Hydrojodids, erforderlichenfalls in Gegenwart von Kupfer oder eines entsprechenden Kupfer-(I)-Salzes wie Kupfer-(I)-chlorid/Salzsäure, Kupfer-(I)-bromid/Bromwasserstoffsäure, Trinatrium-kupfer-(I)tetracyanid bei pH 7 oder eines Alkalixanthogenats, oder von Kupfer-(II)-chlorid/Schwefeldioxid in Eisessig gegebenenfalls unter Zusatz von Magnesiumchlorid, wird bei leicht erhöhten Temperaturen, z. B. bei Temperaturen zwischen 15 und 100°C, durchgeführt; die nachträgliche Umsetzung mit unterphosphoriger Säure wird vorzugsweise bei −5 bis 0°C durchgeführt. Das hierzu erforderliche Diazoniumsalz wird zweckmäßigerweise in einem geeigneten Lösungsmittel, z. B. in Wasser/Salzsäure, Methanol/Salzsäure, Äthanol/Salzsäure oder Dioxan/Salzsäure, durch Diazotierung einer entsprechenden Aminoverbindung mit einem Nitrit, z. B. Natriumnitrit oder einem Ester der salpetrigen Säure, bei niederen Temperaturen, z. B. bei Temperaturen zwischen −10 und 5°C, hergestellt.

Die nachträgliche Acylierung wird zweckmäßigerweise in einem Lösungsmittel wie Methylenchlorid, Äther, Tetrahydrofuran oder in einem Überschuß des verwendeten Acylierungsmittels, z. B. Ameisensäure, Essigsäure oder Propionsäure bzw. deren Anhydriden, Säurechloriden oder Estern, gegebenenfalls in Gegenwart einer anorganischen oder einer tertiären organischen Base, welche gleichzeitig auch als Lösungsmittel dienen kann, und gegebenenfalls in Gegenwart eines die Säure aktivierenden oder wasserentziehenden Mittels bei Temperaturen zwischen —25 und 150°C, vorzugsweise jedoch bei Temperaturen zwischen —10°C und der Siedetemperatur des Reaktionsgemisches, durchgeführt.

Die nachträgliche N-Alkylierung wird zweckmäßigerweise mit einem entsprechenden Halogenid oder Sulfonsäureester, z. B. mit Methyljodid, Dimethylsulfat, Äthylbromid oder p-Toluolsulfonsäureäthylester, gegebenenfalls in Gegenwart einer Base wie Natriumhydrid, Kaliumhydroxid oder Kalium-tert.butylat und vorzugsweise in einem Lösungsmittel wie Diäthyläther, Tetrahydrofuran, Dioxan, Äthanol, Pyridin oder Dimethylformamid bei Temperaturen zwischen 0 und 75°C, vorzugsweise bei Raumtemperatur, durchgeführt. Die Methylierung kann ferner mit Formaldehyd/Ameisensäure zweckmäßigerweise bei der Siedetemperatur des Reaktionsgemisches und die Alkylierung mit einer entsprechenden Carbonylverbindung in Gegenwart eines Hydrids wie Natriumcyanoborhydrid in einem Lösungsmittel wie Acetonitril/Essigsäure oder Dimethylformamid/Essigsäure vorzugsweise bei pH = 7 und bei Temperaturen zwischen 0 und 50°C durchgeführt werden.

Die nachträgliche Enthalogenierung wird zweckmäßigerweise in einem Lösungsmittel wie Methanol, Äthanol, Essigester, Eisessig oder Dimethylformamid mittels katalytisch angeregtem Wasserstoff, z. B. mit Wasserstoff in Gegenwart von Platin oder Palladium/Kohle, bei Temperaturen zwischen 0 und 75°C, vorzugsweise jedoch bei Raumtemperatur, und bei einem Wasserstoffdruck von 1—5 bar durchgeführt.

Die nachträgliche Hydrolyse wird zweckmäßigerweise entweder in Gegenwart einer Säure wie Salzsäure, Schwefelsäure, Phosphorsäure, Polyphosphorsäure oder Trifluoressigsäure oder in Gegenwart einer Base wie Natriumhydroxid oder Kaliumhydroxid in einem geeigneten Lösungsmittel wie Wasser, Äthanol, Wasser/Äthanol, Wasser/Isopropanol oder Wasser/Dioxan bei erhöhten Temperaturen, z. B. bei der Siedetemperatur des Reaktionsgemisches, durchgeführt. Diese kann jedoch auch mit einem Nitrit, z. B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C durchgeführt werden. Die nachträgliche Alkoholyse wird zweckmäßigerweise in Gegenwart von Halogenwasserstoff, vorzugsweise Chlorwasserstoff, bei Temperaturen zwischen 20°C und der Siedetemperatur des verwendeten Alkohols durchgeführt.

Die nachträgliche Reduktion wird vorzugsweise mit einem Metallhydrid, z. B. mit einem komplexen Metallhydrid wie Lithiumaluminiumhydrid, in einem geeigneten Lösungsmittel wie Diäthyläther, Tetrahydrofuran oder Dioxan bei Temperaturen zwischen 0 und 100°C, vorzugsweise jedoch bei Temperaturen zwischen 20 und 60°C, durchgeführt.

Die nachträgliche O-Alkylierung wird zweckmäßigerweise mit einem entsprechenden Halogenid, Sulfonsäureester oder Diazoalkan, z. B. mit Methyljodid, Dimethylsulfat, Äthylbromid, p-Toluolsulfonsäure-äthylester, Methansulfonsäure-isopropylester oder Diazomethan, gegebenenfalls in Gegenwart einer Base wie Natriumhydrid, Kaliumhydroxid oder Kalium-tert.butylat und vorzugsweise in einem Lösungsmittel wie Diäthyläther, Tetrahydrofuran, Dioxan, Methanol, Äthanol, Pyridin oder Dimethylformamid bei Temperaturen zwischen 0 und 75°C, vorzugsweise jedoch bei Raumtemperatur, durchgeführt.

Die nachträgliche Überführung einer Hydroxymethylgruppe in eine Halogenmethylgruppe wird mit einem Halogenierungsmittel wie Thionylchlorid, Phosphortrichlorid, Phosphortribromid oder Phosphorpentachlorid in einem Lösungsmittel wie Methylenchlorid, Tetrachlorkohlenstoff, Benzol oder Nitrobenzol und deren anschließende Umsetzung mit einem Malonsäureester, z. B. mit einem Alkalisalz des Malonsäurediäthylester, bei Temperaturen zwischen 0 und 100°C, vorzugsweise jedoch bei Temperaturen zwischen 20 und 50°C, durchgeführt.

Die nachträgliche Kondensation einer Formyl-Verbindung wird zweckmäßigerweise in einem Lösungsmittel wie Pyridin oder Tetrahydrofuran mit Malonsäure, mit einem Malonsäureester, mit einem Dialkylphosphono-essigsäureester oder einem Alkoxycarbonylmethylen-triphenyl-phosphon, gegebenenfalls in Gegenwart einer Base als Kondensationsmittel, z. B. in Gegenwart von Piperidin, Kalium-tert.butylat oder Natriumhydrid, bei Temperaturen zwischen 0 und 100°C durchgeführt; durch anschließendes Ansäuern, z. B. mit Salzsäure oder Schwefelsäure bzw. durch anschließende alkalische Hydrolyse erhält man die gewünschte Säure.

Die nachträgliche Hydrolyse und Decarboxylierung wird zweckmäßigerweise in Gegenwart einer Säure wie Salzsäure, Schwefelsäure, Phosphorsäure, Polyphosphorsäure oder Trifluoressigsäure in einem geeigneten Lösungsmittel wie Wasser, Äthanol, Wasser/Äthanol, Wasser/Isopropanol oder Wasser/Dioxan bei erhöhten Temperaturen, z. B. bei der Siedetemperatur des Reaktionsgemisches durchgeführt.

Die nachträgliche Disproportionierung eines Sulfonsäurehydrazides, welches man durch Umsetzung eines entsprechenden Hydrazins mit einem entsprechenden reaktionsfähigen Carbonsäurederivat erhält, wird in Gegenwart einer Base wie Natriumkarbonat in einem Lösungsmittel wie Äthylenglykol bei Temperaturen zwischen 100 und 200°C, vorzugsweise jedoch bei 160—170°C, durchgeführt.

Die erhaltenen Verbindungen der allgemeinen Formel I lassen sich ferner in ihre Additionssalze,

insbesondere in ihre physiologisch verträglichen Salze mit anorganischen oder organischen Säuren oder auch Basen überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure und als Basen Natriumhydroxid, Kaliumhydroxid oder Cyclohexylamin in Betracht.

Die als Ausgangsstoffe verwendeten Verbindungen der allgemeinen Formeln II bis XIV sind teilweise literaturbekannt bzw. erhält man nach an sich bekannten Verfahren.

So erhält man beispielsweise eine Verbindung der allgemeinen Formel II, in der A eine Bindung darstellt, durch Reduktion einer entsprechenden Nitroverbindung, beispielsweise mittels katalytisch angeregtem oder nascierendem Wasserstoff oder mittels Natriumdithionit oder durch Hofmann-, Curtius-, Lossen- oder Schmidt-Abbau einer entsprechenden Verbindung.

Man erhält beispielsweise eine Verbindung der allgemeinen Formel II, in der A eine Vinylidengruppe bedeutet bzw. deren tautomeres Ketimin durch Umsetzung eines entsprechenden Nitrils mit einer entsprechenden Grignard- oder Lithium-Verbindung und anschließende Hydrolyse oder durch Umsetzung eines entsprechenden Ketons mit einem entsprechenden Amin in Gegenwart von Titantetrachlorid. Zur weiteren Umsetzung mit einer Verbindung der allgemeinen Formel III bzw. deren reaktionsfähigen Derivaten, insbesondere deren Säurechloride, kann auch der metallorganische Ketimin-Komplex verwendet werden.

Man erhält beispielsweise eine Verbindung der allgemeinen Formel II, in der A keine Bindung und keine Vinylidengruppe darstellt, durch Reduktion eines entsprechenden Nitrils mit Lithiumaluminiumhydrid, durch Umsetzung eines entsprechenden Nitrils mit einer entsprechenden Grignard- oder Lithium-Verbindung und gegebenenfalls anschließender Lithiumaluminiumhydrid-Reduktion oder anschließender Hydrolyse zum Ketimin, welches anschließend mit katalytisch angeregtem Wasserstoff, mit einem komplexen Metallhydrid oder mit nascierendem Wasserstoff reduziert wird, durch Hydrolyse bzw. durch Hydrazinolyse einer entsprechenden Phthalimidoverbindung, durch Umsetzung eines entsprechenden Ketons mit Ammoniumformiat und anschließender Hydrolyse bzw. mit einem Ammoniumsalz in Gegenwart von Natriumcyanoborhydrid, durch Reduktion eines entsprechenden Oxims mit Lithiumaluminiumhydrid, mit katalytisch angeregtem oder nascierendem Wasserstoff, durch Reduktion einer entsprechenden N-Benzyl- oder N-1-Phenyläthyl-Schiffschen Base, z. B. mit einem komplexen Metallhydrid in Äther oder Tetrahydrofuran bei Temperaturen zwischen —78° und der Siedetemperatur des verwendeten Lösungsmittels und anschließender Abspaltung der Benzyl- oder 1-Phenyläthylgruppe mittels katalytischer Hydrierung, durch Ritter-Reaktion eines entsprechenden Alkohols mit Kaliumcyanid in Schwefelsäure, oder durch Hofmann-, Curtius-, Lossen- oder Schmidt-Abbau einer entsprechenden Verbindung. Ein so erhaltenes Amin der allgemeinen Formel II mit einem chiralen Zentrum kann durch Racematspaltung, z. B. mittels fraktionierter Kristallisation der diastereomeren Salze mit optisch aktiven Säuren und anschließender Zerlegung der Salze oder durch Bildung von diastereomeren Verbindungen, deren Trennung und anschließende Spaltung in dessen Enantiomeren aufgetrennt werden. Ferner kann ein optisch aktives Amin der allgemeinen Formel II auch durch enantio-selektive Reduktion eines entsprechenden Ketimins mittels komplexer Bor- oder Aluminiumhydride, in denen ein Teil der Hydridwasserstoffatome durch optisch aktive Alkoholreste ersetzt ist, oder mittels Wasserstoff in Gegenwart eines geeigneten chiralen Hydrierungskatalysators hergestellt werden bzw. analog ausgehend von einer entsprechenden N-Benzyl- oder gegebenenfalls optisch aktiven N-1-Phenäthyl-Schiffschen Base und gegebenenfalls anschließender Abspaltung des Benzyl- oder 1-Phenäthylrestes.

Eine Verbindung der allgemeinen Formel II, in der $R_4$ einen niederen Alkylrest darstellt, erhält man durch Reduktion einer entsprechenden N-Acyl-Verbindung, z. B. mittels Lithiumaluniniumhydrid.

Die als Ausgangsstoffe verwendeten Verbindungen der allgemeinen Formeln IV, V und VII bis X erhält man durch Umsetzung eines entsprechenden Amins mit einer entsprechenden Carbonsäure bzw. deren aktiven Derivaten und gegebenenfalls anschließende Hydrolyse. Eine Verbindung der allgemeinen Formel VIII erhält man auch durch Friedel-Crafts-Acetylierung einer entsprechenden Verbindung.

Eine als Ausgangsstoff verwendete Verbindung der allgemeinen Formel XII erhält man vorzugsweise durch Acylierung eines entsprechenden Ketimins mit einer entsprechenden Carbonsäure bzw. deren reaktiven Derivaten unter Tautomerisierung.

Eine als Ausgangsstoff verwendete Verbindung der allgemeinen Formel XIII erhält man durch Reduktion einer entsprechenden Carbonylverbindung oder durch Umsetzung einer entsprechenden Carbonylverbindung mit einem entsprechenden Grignard- oder Lithium-Reagens.

Wie bereits eingangs erwähnt, weisen die neuen Verbindungen der allgemeinen Formel I wertvolle pharmakologische Eigenschaften auf, nämlich eine Wirkung auf den Intermediärstoffwechsel, insbesondere jedoch eine blutzuckersenkende Wirkung.

Beispielsweise wurden die Verbindungen

A = 4-[(2-Pyrrolidino-benzyl)-aminocarbonylmethyl]-benzoesäure,
B = 4-[(1-(2-Pyrrolidino-phenyl)-äthyl)-aminocarbonylmethyl]-benzoesäure,
C = 4-[(1-(5-Chlor-2-pyrrolidino-phenyl)-äthyl)-aminocarbonylmethyl]-benzoesäure,

D = 4-[(2-Piperidino-benzyl)-aminocarbonylmethyl]-benzoesäure,
E = 4-[(1-(2-Piperidino-phenyl)-äthenyl)-aminocarbonylmethyl]-benzoesäure,
F = 4-[(1-(6-Chlor-2-piperidino-phenyl)-äthenyl)-aminocarbonylmethyl]-benzoesäure,
G = 4-[(1-(6-Methyl-2-piperidino-phenyl)-äthenyl)-aminocarbonylmethyl]-benzoesäure,
H = 4-[(1-(2-Piperidino-phenyl)-äthyl)-aminocarbonylmethyl]-benzoesäure,
I = 4-[(1-(2-Piperidino-phenyl)-äthyl)-aminocarbonylmethyl]-benzoesäure-äthylester,
K = (+) 4-[(1-(2-Piperidino-phenyl)-äthyl)-aminocarbonylmethyl]-benzoesäure-äthylester,
L = 4-[(1-(2-Piperidino-phenyl)-äthyl)-aminocarbonylmethyl]-benzoesäure-(2,2-dimethyl-dioxolan-4-yl)-methylester,
M = 4-[(1-(2-Piperidino-phenyl)-äthyl)-aminocarbonylmethyl]-toluol,
N = 4-[(1-(2-Piperidino-phenyl)-äthyl)-aminocarbonylmethyl]-benzylalkohol,
O = 4-[(1-(2-Piperidino-phenyl)-äthyl)-aminocarbonylmethyl]-benzaldehyd,
P = 4-[(1-(2-Piperidino-phenyl)-äthyl)-aminocarbonylmethyl]-phenylessigsäure,
Q = 4-[(1-(4-Chlor-2-piperidino-phenyl)-äthyl)-aminocarbonylmethyl]-benzoesäure,
R = 4-[(1-(5-Chlor-2-piperidino-phenyl)-äthyl)-aminocarbonylmethyl]-benzoesäure,
S = 4-[(1-(6-Chlor-2-piperidino-phenyl)-äthyl)-aminocarbonylmethyl]-benzoesäure-äthylester,
T = 4-[(1-(5-Fluor-2-piperidino-phenyl)-äthyl)-aminocarbonylmethyl]-benzoesäure,
U = 4-[(1-(4-Methyl-2-piperidino-phenyl)-äthyl)-aminocarbonylmethyl]-benzoesäure,
V = 4-[(1-(5-Methyl-2-piperidino-phenyl)-äthyl)-aminocarbonylmethyl]-benzoesäure,
W = 4-[(2-(2-Piperidino-phenyl)-2-propyl)-aminocarbonylmethyl]-benzoesäure,
X = 4-[(1-(2-Piperidino-phenyl)-2-methyl-propyl)-aminocarbonylmethyl]-benzoesäure,
Y = 4-[(2-Piperidino-benzhydryl)-aminocarbonylmethyl]-benzoesäure,
Z = 4-[(1-(2-(1,2,3,6-Tetrahydro-pyridino)-phenyl)-äthyl)-aminocarbonylmethyl]-benzoesäure,
AA = 4-[(1-(2-(3-Methyl-piperidino)-phenyl)-äthyl)-aminocarbonylmethyl]-benzoesäure,
AB = 4-[(1-(2-Hexahydroazepino-phenyl)-äthyl)-aminocarbonylmethyl]-benzoesäure,
AC = 4-[(1-(2-Octahydroisoindolo-phenyl)-äthyl)-aminocarbonylmethyl]-benzoesäure,
AD = 4-(α-Methoxycarbonyl-2-piperidino-benzyl)-aminocarbonylmethyl]-benzoesäure-äthylester und
AE = (+)-4-[(1-(2-Piperidino-phenyl)-äthyl)-aminocarbonylmethyl]-benzoesäure

auf ihre blutzuckersenkenden Eigenschaften wie folgt untersucht:

### 1. Blutzuckersenkende Wirkung

Die blutzuckersenkende Wirkung der zu untersuchenden Substanzen wurde an weiblichen Ratten eigener Zucht mit dem Gewicht von 180—220 g geprüft, welche 24 Stunden vor Versuchsbeginn nüchtern gesetzt wurden. Die zu untersuchenden Substanzen wqurden unmittelbar vor Versuchsbeginn in 1,5%iger Methylcellulose suspendiert und per Schlundsonde appliziert.

Die Blutentnahme erfolgte unmittelbar vor Substanzapplikation, sowie 1, 2, 3 und 4 Stunden danach jeweils aus dem retroorbitalen Venenplexus. Hiervon wurden jeweils 50 µl mit 0,5 ml 0,33 N Perchlorsäure enteiweißt und zentrifugiert. Im Überstand wurde Glukose nach der Hexokinase-Methode mit Hilfe eines Analysenphotometers bestimmt. Die statistische Auswertung erfolgte nach dem t-Test nach Student mit p = 0,05 als Signifikanzgrenze.

Die nachfolgende Tabelle enthält die gefundenen Werte in Prozent gegenüber Kontrolle:

Tabelle 1

| Substanz | 25 mg/kg 1 | 2 | 3 | 4 | 10 mg/kg 1 | 2 | 3 | 4 | 5 mg/kg 1 | 2 | 3 | 4 |
|---|---|---|---|---|---|---|---|---|---|---|---|---|
| | Stunden | | | | Stunden | | | | Stunden | | | |
| A | | | | | –36 | –23 | –14– | n.s. | –22 | n.s. | –10 | n.s. |
| B | | | | | –42 | –35 | –31 | –13 | –38 | –18 | n.s. | n.s. |
| C | –40 | –30 | –26 | –22 | –26 | –17 | n.s. | n.s. | | | | |
| D | | | | | –38 | –36 | –25 | –14 | –27 | –16 | –11 | –13 |
| E | | | | | –42 | –39 | –34 | –32 | –45 | –41 | –36 | –21 |
| F | –45 | –42 | –38 | –32 | –44 | –39 | –32 | –24 | –47 | –33 | –26 | n.s. |
| G | | | | | | | | | –31 | –15 | n.s. | n.s. |
| H | –40 | –43 | –45 | –38 | –45 | –38 | –35 | –30 | –45 | –45 | –36 | –32 |
| I | | | | | –24 | –27 | – 17 | –13 | –22 | –22 | n.s. | n.s. |
| K | | | | | | | | | –47 | –42 | –31 | –22 |
| L | | | | | –39 | –37 | – 32 | –24 | –43 | –34 | –29 | –19 |
| M | –45 | –44 | –38 | –32 | | | | | | | | |
| N | | | | | –40 | –40 | –30 | –31 | –35 | –29 | n.s. | n.s. |
| O | –46 | –47 | –37 | –36 | –46 | –41 | –39 | –35 | –43 | –35 | –26 | –23 |
| P | | | | | –41 | –26 | –19 | n.s. | –27 | –18 | n.s. | n.s. |
| Q | | | | | –35 | –39 | –33 | –30 | | | | |
| R | –36 | –36 | –34 | –28 | –36 | –34 | –26 | –20 | –17 | –18 | –11 | n.s. |
| S | –44 | –46 | –39 | –37 | | | | | | | | |
| T | | | | | –49 | –47 | –46 | –46 | –43 | –36 | –29 | –29 |
| U | | | | | –37 | –18 | n.s. | n.s. | –42 | –15 | n.s. | n.s. |
| V | –28 | –23 | –25 | –20 | | | | | | | | |
| W | | | | | –32 | –34 | –27 | –20 | –19 | –24 | –16 | n.s. |
| X | –46 | – 45 | –43 | –36 | –43 | –41 | –36 | –28 | –36 | –40 | –32 | –32 |
| Y | –44*) | –44*) | –41*) | –42*) | | | | | –44 | –38 | –41 | –37 |
| Z | | | | | | | | | –45 | –39 | –35 | –31 |
| AA | –46 | –38 | –44 | –46 | –42 | –32 | –26 | –35 | –48 | –36 | –33 | –20 |
| AB | –45 | –46 | –39 | –34 | –41 | –35 | –24 | –17 | –29 | –18 | n.s. | n.s. |
| AC | –41 | –44 | –32 | –26 | | | | | | | | |
| AD | | | | | –40 | –32 | –31 | –17 | | | | |
| AE**) | | | | | | | | | –41 | –34 | –20 | n.s. |

*) Dosis: 20 mg/kg.
**) Dosis: 1 mg/kg.
n.s. ≙ statistisch nicht signifikant.

2. Akute Toxizität

Bei weiblichen und männlichen Mäusen eigener Zucht mit dem Gewicht von 20—26 g wurde die toxische Wirkung der Substanzen H, R und Y nach oraler Gabe (Suspension in 1%iger Methylcellulose) einer einmaligen Dosis bei einer Nachbeobachtungszeit von 14 Tagen geprüft. Die nachfolgende Tabelle enthält die gefundenen Werte:

| Substanz | Orientierende Toxizität |
|---|---|
| H | >2000 mg/kg p.o. (1 von 10 Tieren gestorben) |
| R | >2000 mg/kg p.o. (0 von 10 Tieren gestorben) |
| Y | >2000 mg/kg p.o. (0 von 6 Tieren gestorben) |

Aufgrund ihrer pharmakologischen Eigenschaften eignen sich die erfindungsgemäß hergestellten Verbindungen der allgemeinen Formel I und deren physiologisch verträgliche Salze zur Behandlung des Diabetes mellitus. Hierzu lassen sie sich, gegebenenfalls in Kombination mit anderen Wirksubstanzen, in die üblichen galenischen Zubereitungsformen wie Tabletten, Dragées, Kapseln, Pulver oder Suspensionen einarbeiten. Die Einzeldosis am Erwachsenen beträgt hierbei 1—50 mg, vorzugsweise jedoch 2,5—20 mg, 1- oder 2mal täglich.

Die nachfolgenden Beispiele sollen die Erfindung näher erläutern:

## Beispiel 1

4-[(1-(5-Chlor-2-dimethylamino-phenyl)-äthyl)-aminocarbonylmethyl]-benzoesäure-methylester

Zu einer Lösung von 2,00 g (0,0103 Mol) 4-Methoxycarbonyl-phenyl-essigsäure in 13,5 ml absolutem Tetrahydrofuran gibt man bei 20°C unter Rühren 1,67 g (0,0103 Mol) Carbonyldiimidazol und erhitzt anschließend unter Feuchtigkeitsausschluß 45 Minuten auf Rückfluß. Nach Abkühlen auf Raumtemperatur versetzt man mit 2,05 g (0,0103 Mol) 1-(5-Chlor-2-dimethylamino-phenyl)-äthylamin in 7 ml absolutem Tetrahydrofuran und rührt über Nacht bei 20°C. Man dampft im Vakuum ein und reinigt den Eindampfrückstand durch Säulenchromatographie an Kieselgel (Toluol/Aceton = 10/1).

Ausbeute: 2,6 g (66,7% der Theorie),
Schmelzpunkt: 153—155°C (aus Äther).

| | | | | |
|---|---|---|---|---|
| Ber.: | C 64,08 | H 6,18 | Cl 9,46 | N 7,47 |
| Gef.: | C 64,30 | H 6,04 | Cl 9,70 | N 7,39 |

Analog Beispiel 1 wurden folgende Verbindungen hergestellt:

4-[(1-(5-Chlor-2-dipropylamino-phenyl)-äthyl)-aminocarbonylmethyl]-benzoesäure-methylester

Ausbeute: 42% der Theorie,
Schmelzpunkt: 135—137°C (aus Äther/Petroläther).

| | | | | |
|---|---|---|---|---|
| Ber.: | C 66,83 | H 7,25 | Cl 8,23 | N 6,50 |
| Gef.: | C 66,95 | H 7,35 | Cl 8,35 | N 6,05 |

4-[(1-(5-Chlor-2-dibutylamino-phenyl)-äthyl)-aminocarbonylmethyl]-benzoesäure-methylester

Ausbeute: 64,8% der Theorie,
Schmelzpunkt: 110—112° C.

| | | | | |
|---|---|---|---|---|
| Ber.: | C 68,03 | H 7,69 | Cl 7,72 | N 6,10 |
| Gef.: | C 67,86 | H 7,61 | Cl 7,73 | N 6,17 |

4-[(1-(5-Chlor-2-cyclohexyl-N-methylamino-phenyl)-äthyl)-aminocarbonylmethyl]-benzoesäure-methylester

Ausbeute: 63,9% der Theorie,
Schmelzpunkt: 152—153° C (Äther).

| | | | | |
|---|---|---|---|---|
| Ber.: | C 67,78 | H 7,05 | Cl 8,00 | N 6,32 |
| Gef.: | C 67,70 | H 6,92 | Cl 8,24 | N 6,46 |

4-[(5-Chlor-2-pyrrolidino-benzyl)-aminocarbonylmethyl]-benzoesäure-methylester

Ausbeute: 68,1% der Theorie,
Schmelzpunkt: 139—141° C (Methanol).

| | | | | |
|---|---|---|---|---|
| Ber.: | C 65,19 | H 5,99 | Cl 9,17 | N 7,24 |
| Gef.: | C 65,46 | H 5,91 | Cl 9,26 | N 7,41 |

4-[(1-(5-Chlor-2-pyrrolidino-phenyl)-äthyl)-aminocarbonylmethyl]-benzoesäure-methylester

Ausbeute: 58,3% der Theorie,
Schmelzpunkt: 133—135° C (Methanol).

| | | | | |
|---|---|---|---|---|
| Ber.: | C 65,91 | H 6,29 | Cl 8,84 | N 6,99 |
| Gef.: | C 66,24 | H 6,19 | Cl 8,75 | N 7,13 |

4-[(5-Chlor-2-piperidino-benzyl)-aminocarbonylmethyl]-benzoesäure-methylester

Ausbeute: 75,1% der Theorie,
Schmelzpunkt: 123—125° C (Äther).

| | | | | |
|---|---|---|---|---|
| Ber.: | C 65,91 | H 6,29 | Cl 8,84 | N 6,99 |
| Gef.: | C 66,05 | H 6,13 | Cl 8,86 | N 7,21 |

4-[(1-(5-Chlor-2-piperidino-benzyl)-aminocarbonyl)-äthyl]-benzoesäure-methylester

Ausbeute: 70,4% der Theorie,
Schmelzpunkt: 142—144° C (Äther).

| | | | | |
|---|---|---|---|---|
| Ber.: | C 66,57 | H 6,56 | Cl 8,55 | N 6,75 |
| Gef.: | C 66,50 | H 6,49 | Cl 8,44 | N 6,86 |

4-[(1-(5-Chlor-2-piperidino-phenyl)-äthyl)-aminocarbonylmethyl]-benzoesäure-methylester

Ausbeute: 69,5% der Theorie,
Schmelzpunkt: 147—149° C (Äther).

| | | | | |
|---|---|---|---|---|
| Ber.: | C 66,57 | H 6,56 | Cl 8,55 | N 6,75 |
| Gef.: | C 66,33 | H 6,54 | Cl 8,67 | N 6,85 |

4-[(1-(5-Chlor-2-(3-methyl-piperidino)-phenyl)-äthyl)-aminocarbonylmethyl]-benzoesäure-methylester

Ausbeute: 54,3% der Theorie,
Schmelzpunkt: 160—162° C (Methanol).

| | | | | |
|---|---|---|---|---|
| Ber.: | C 67,20 | N 6,81 | Cl 8,27 | N 6,53 |
| Gef.: | C 67,27 | N 6,81 | Cl 8,13 | N 6,45 |

4-[(1-(5-Chlor-2-(3,5-cis-dimethyl-piperidino)-phenyl)-äthyl)-aminocarbonylmethyl]-benzoesäure-methylester

Ausbeute: 44% der Theorie,
Schmelzpunkt: 190—193° C (Methanol).

| | | | | |
|---|---|---|---|---|
| Ber.: | C 67,78 | H 7,05 | Cl 8,00 | N 6,32 |
| Gef.: | C 67,50 | H 7,05 | Cl 8,25 | N 6,48 |

4-[(1-(5-Chlor-2-piperidino-phenyl)-propyl)-aminocarbonylmethyl]-benzoesäure-methylester

Ausbeute: 65,9% der Theorie,
Schmelzpunkt: 142—144° C (Äther).

| | | | | |
|---|---|---|---|---|
| Ber.: | C 67,20 | H 6,81 | Cl 8,26 | N 6,53 |
| Gef.: | C 67,45 | H 6,63 | Cl 8,38 | N 6,63 |

4-[(1-(5-Chlor-2-piperidino-phenyl)-2-methyl-propyl)-aminocarbonylmethyl]-benzoesäure-methylester

Ausbeute: 61,4% der Theorie,
Schmelzpunkt: 156—158° C (Äther).

| | | | | |
|---|---|---|---|---|
| Ber.: | C 67,78 | H 7,05 | Cl 8,00 | N 6,32 |
| Gef.: | C 67,80 | H 7,17 | Cl 7,89 | N 6,28 |

4-[(1-(5-Chlor-2-morpholino-phenyl)-äthyl)-aminocarbonylmethyl]-benzoesäure-methylester

Ausbeute: 69,8% der Theorie,
Schmelzpunkt: 156—158° C (Äther).

| | | | | |
|---|---|---|---|---|
| Ber.: | C 63,38 | H 6,04 | Cl 8,50 | N 6,72 |
| Gef.: | C 63,24 | H 6,12 | Cl 8,70 | N 6,85 |

4-[(1-(5-Chlor-2-thiomorpholino-phenyl)-äthyl)-aminocarbonylmethyl]-benzoesäure-methylester

Ausbeute: 68,2% der Theorie,
Schmelzpunkt: 167—169° C (Äther).

| | | | | | |
|---|---|---|---|---|---|
| Ber.: | C 61,03 | H 5,82 | Cl 8,19 | N 6,47 | S 7,41 |
| Gef.: | C 60,83 | H 5,77 | Cl 8,33 | N 6,49 | S 7,39 |

4-[(1-(5-Chlor-2-(hexahydro-1H-azepino)-phenyl)-äthyl)-aminocarbonylmethyl]-benzoesäure-methylester

Ausbeute: 41,7% der Theorie,
Schmelzpunkt: 146—147° C (Methylenchlorid/Petroläther).

| | | | | |
|---|---|---|---|---|
| Ber.: | C 67,19 | H 6,81 | Cl 8,27 | N 6,53 |
| Gef.: | C 66,90 | H 6,66 | Cl 8,30 | N 6,39 |

4-[(1-(5-Chlor-2-octahydroazocino-phenyl)-äthyl)-aminocarbonylmethyl]-benzoesäure-methylester

Ausbeute: 30% der Theorie,
Schmelzpunkt: 154—156° C.

| | | |
|---|---|---|
| Ber.: | Molpeak | m/e = 442/444 (1 Chlor) |
| Gef.: | | m/e = 442/444 (1 Chlor) |

4-[(1-(5-Chlor-2-(octahydro-1H-azonino)-phenyl)-äthyl)-aminocarbonylmethyl]-benzoesäure-methylester

Ausbeute: 38% der Theorie,
Schmelzpunkt: 184—185° C (Chloroform/Toluol).

| | | | |
|---|---|---|---|
| Ber.: | C 68,32 | H 7,28 | N 6,13 |
| Gef.: | C 68,10 | H 7,30 | N 6,28 |

4-[(2-(5-Chlor-2-piperidino-phenyl)-2-propyl)-aminocarbonylmethyl]-benzoesäure-methylester

Ausbeute: 84,4% der Theorie,
Schmelzpunkt: 162—164° C.

| | | |
|---|---|---|
| Ber.: | Molpeak | m/e = 428/430 (1 Chlor) |
| Gef.: | | m/e = 428/430 (1 Chlor) |

4-[(1-(5-Nitro-2-piperidino-phenyl)-äthyl)-aminocarbonylmethyl]-benzoesäure-methylester

Ausbeute: 68,3% der Theorie,
Schmelzpunkt: 178—180° C (Toluol).

| | | | |
|---|---|---|---|
| Ber.: | C 64,93 | H 6,40 | N 9,88 |
| Gef.: | C 65,05 | H 6,43 | N 9,87 |

4-[(1-(2-Piperidino-phenyl)-äthyl)-aminocarbonylmethyl]-benzoesäure-methylester

Ausbeute: 59,1% der Theorie,
Schmelzpunkt: 145—147° C.

| | | | |
|---|---|---|---|
| Ber.: | C 72,61 | H 7,42 | N 7,36 |
| Gef.: | C 72,35 | H 7,39 | N 7,40 |

4-[(5-Methyl-2-piperidino-benzyl)-aminocarbonylmethyl]-benzoesäure-methylester

Ausbeute: 32,9% der Theorie,
Schmelzpunkt: 124—126° C (Petroläther/Aceton.

| | | |
|---|---|---|
| Ber.: | Molpeak | m/e = 380 |
| Gef.: | | m/e = 380 |

N(4-Nitro-phenacetyl)-N-[1-(2-piperidino-phenyl)-äthyl]-amin

Ausbeute: 62,4% der Theorie,
Schmelzpunkt: 165—167° C (Äther).

| | | | |
|---|---|---|---|
| Ber.: | C 68,64 | H 6,86 | N 11,44 |
| Gef.: | C 68,73 | H 6,88 | N 11,63 |

N-(4-Acetyl-phenacetyl)-N-[1-(2-piperidino-phenyl)-äthyl]-amin

Ausbeute: 32,4% der Theorie,
Schmelzpunkt: 162—164° C (Äther).

| | | | |
|---|---|---|---|
| Ber.: | C 75,79 | H 7,74 | N 7,69 |
| Gef.: | C 75,51 | H 7,86 | N 7,38 |

N-(4-Acetyl-phenacetyl)-N-[1-(5-chlor-2-piperidino-phenyl)-äthyl]-amin

Ausbeute: 50,3% der Theorie,
Schmelzpunkt: 162—163° C (Äther).

| | | | |
|---|---|---|---|
| Ber.: | C 69,24 | H 6,82 | N 7,02 |
| Gef.: | C 68,88 | H 6,63 | N 6,70 |

2-[(1-(2-Piperidino-phenyl)-äthyl)-aminocarbonylmethyl]-benzoesäure-methylester

Ausbeute: 82% der Theorie,
Schmelzpunkt: 107—108° C.

| | | | |
|---|---|---|---|
| Ber.: | C 72,60 | H 7,42 | N 7,36 |
| Gef.: | C 72,79 | H 7,38 | N 7,53 |

3-[(1-(2-Piperidino-phenyl)-äthyl)-aminocarbonylmethyl]-benzoesäure-methylester

Ausbeute: 47% der Theorie,
Schmelzpunkt: 155° C.

| | | | |
|---|---|---|---|
| Ber.: | C 73,07 | H 7,67 | N 7,10 |
| Gef.: | C 73,30 | H 7,58 | N 7,17 |

3-Chlor-4-[(1-(2-piperidino-phenyl)-äthyl)-aminocarbonylmethyl]-benzoesäure-methylester

Ausbeute: 63% der Theorie,
Schmelzpunkt: 123—124° C.

| | | | | |
|---|---|---|---|---|
| Ber.: | C 67,20 | H 6,81 | Cl 8,27 | N 6,53 |
| Gef.: | C 67,28 | H 6,84 | Cl 8,36 | N 6,50 |

4-[(1-(2-(1,2,3,4-Tetrahydro-isochinolin-2-yl)-phenyl)-äthyl)-aminocarbonylmethyl]-benzoesäure-methylester

Ausbeute: 43% der Theorie,
Schmelzpunkt: 142—144° C.

| | | | |
|---|---|---|---|
| Ber.: | C 75,99 | H 6,83 | N 6,33 |
| Gef.: | C 75,64 | H 6,75 | N 6,35 |

4-[(1-(2-Piperidino-phenyl)-äthyl)-aminocarbonylmethyl]-toluol

Ausbeute: 59% der Theorie,
Schmelzpunkt: 136—138° C.

| | | | |
|---|---|---|---|
| Ber.: | C 78,53 | H 8,39 | N 8,33 |
| Gef.: | C 78,58 | H 8,16 | N 8,26 |

4-[(5-Chlor-2-piperidino-anilino)-carbonylmethyl]-benzoesäuremethylester

Ausbeute: 40,3% der Theorie,
Schmelzpunkt: 156—158° C (Methanol/Toluol).

| | | | |
|---|---|---|---|
| Ber.: | C 65,19 | H 5,99 | Cl 9,16 |
| Gef.: | C 65,20 | H 6,15 | Cl 9,40 |

4-[2-(2-Piperidino-anilino-carbonyl)-äthyl]-benzoesäure-methylester

Ausbeute: 26,9% der Theorie,
Schmelzpunkt: 71—73° C (Petroläther).

| | | | |
|---|---|---|---|
| Ber.: | C 72,10 | H 7,15 | N 7,65 |
| Gef.: | C 72,00 | H 7,09 | N 7,94 |

4-[(1-(2-(1,2,3,6-Tetrahydro-pyridino)-phenyl)-äthyl)-aminocarbonylmethyl]-benzoesäure-äthylester

Ausbeute: 63,4% der Theorie,
Schmelzpunkt: 125—127° C (Äther).

| | | | |
|---|---|---|---|
| Ber.: | C 73,44 | H 7,19 | N 7,14 |
| Gef.: | C 73,38 | H 7,13 | N 7,13 |

4-[(2-(5-Chlor-2-piperidino-phenyl)-äthyl)-aminocarbonylmethyl]-benzoesäure-äthylester

Ausbeute: 68% der Theorie,
Schmelzpunkt: 95—97° C (Äthanol).

| | | | | |
|---|---|---|---|---|
| Ber.: | C 67,20 | H 6,81 | Cl 8,27 | N 6,53 |
| Gef.: | C 67,75 | H 6,76 | Cl 8,22 | N 6,24 |

4-[(1-(5-Fluor-2-piperidino-phenyl)-äthyl)-aminocarbonylmethyl]-benzoesäure-äthylester

Ausbeute: 47,3% der Theorie,
Schmelzpunkt: 138—140° C (Äther).

| | | | |
|---|---|---|---|
| Ber.: | C 69,88 | H 7,09 | N 6,79 |
| Gef.: | C 70,10 | H 7,10 | N 6,87 |

4-[(1-(5-Nitro-2-piperidino-phenyl)-äthyl)-aminocarbonylmethyl]-benzoesäure-äthylester

Ausbeute: 56,5% der Theorie,
Schmelzpunkt: 144—147° C (Äthanol).

| | | | |
|---|---|---|---|
| Ber.: | C 65,59 | H 6,65 | N 9,56 |
| Gef.: | C 65,78 | H 6,56 | N 9,73 |

4-[(2-(1-(2-Piperidino-phenyl)-äthyl)-aminocarbony)-äthyl]-benzoesäure-methylester

Ausbeute: 90% der Theorie,
Schmelzpunkt: 129—131° C.

| | | | |
|---|---|---|---|
| Ber.: | C 73,06 | H 7,67 | N 7,10 |
| Gef.: | C 72,61 | H 7,77 | N 7,52 |

4-[(2-Hydroxy-1-(2-piperidino-phenyl)-äthyl)-aminocarbonylmethyl]-benzoesäure-äthylester

Ausbeute: 44,4% der Theorie,
Schmelzpunkt: 132—135° C (Petroläther/Aceton).

| | | | | |
|---|---|---|---|---|
| Ber.: | C 70,22 | H 7,37 | N 6,82 | m/e = 410 |
| Gef.: | C 70,02 | H 7,25 | N 6,77 | m/e = 410 |

4-[(1-(5-Hydroxy-2-piperidino-phenyl)-äthyl)-aminocarbonylmethyl]-benzoesäure-äthylester

Ausbeute: 64,2% der Theorie,
Schmelzpunkt: 150—151° C (Äther).

| | | | | |
|---|---|---|---|---|
| Ber.: | C 70,22 | H 7,37 | N 6,82 | m/e = 410 |
| Gef.: | C 70,37 | H 7,17 | N 6,81 | m/e = 410 |

4-[(α-Methoxycarbonyl-2-piperidino-benzyl)-aminocarbonylmethyl]-benzoesäure-äthylester

Ausbeute: 59% der Theorie,
Schmelzpunkt: 110—112° C (Petroläther/Aceton).

| | | | | |
|---|---|---|---|---|
| Ber.: | C 68,47 | H 6,90 | N 6,39 | m/e = 438 |
| Gef.: | C 68,57 | H 6,64 | N 6,46 | m/e = 438 |

4-[(1-(5-Chlor-2-(2-methyl-piperidino)-phenyl)-äthyl)-aminocarbonylmethyl]-benzoesäure-äthylester

Ausbeute: 71,3% der Theorie,
Schmelzpunkt: <20° C.

Ber.: m/e = 442/444 (1 Chlor)
Gef.: m/e = 442/444 (1 Chlor)

4-[(1-(2-Hexahydroazepino-phenyl)-äthyl)-aminocarbonylmethyl]-benzoesäure-äthylester

Ausbeute: 68% der Theorie,
Schmelzpunkt: 145—148° C (Toluol).

| | | | |
|---|---|---|---|
| Ber.: | C 73,50 | H 7,90 | N 6,86 |
| Gef.: | C 73,35 | H 8,04 | N 6,89 |

4-[(1-(2-[1,4-Dioxa-8-azaspiro[4,5]decyl-(8)]-phenyl)-äthyl)-aminocarbonylmethyl]-benzoesäure-äthylester

Ausbeute: 64,3% der Theorie,
Schmelzpunkt: 143—145° C (Petroläther/Aceton).

| | | | |
|---|---|---|---|
| Ber.: | C 69,01 | H 7,13 | N 6,19 |
| Gef.: | C 69,30 | H 7,38 | N 6,21 |

4-[(1-(2-(2-Methyl-pyrrolidino)-phenyl)-äthyl)-aminocarbonylmethyl]-benzoesäure-äthylester

Ausbeute: 72,0% der Theorie,
Schmelzpunkt: 94—97° C.

| | | | |
|---|---|---|---|
| Ber.: | C 73,07 | H 7,66 | N 7,10 |
| Gef.: | C 73,25 | H 7,67 | N 7,11 |

4-[(1-(3-Methyl-2-piperidino-phenyl)-äthyl)-aminocarbonylmethyl]-benzoesäure-äthylester

Ausbeute: 39,5% der Theorie,
Schmelzpunkt: 178—179° C.

Ber.: m/e = 408
Gef.: m/e = 408

4-[(1-(3-Chlor-2-piperidino-phenyl)-äthyl)-aminocarbonylmethyl]-benzoesäure-äthylester

Ausbeute: 52,6% der Theorie.

Ber.: m/e = 428/430 (1 Chlor)
Gef.: m/e = 428/430 (1 Chlor)

Beispiel 2

(+) 4-[(1-(2-Piperidino-phenyl)-äthyl)-aminocarbonylmethyl]-benzoesäure-äthylester

Zu einer Lösung von 290,9 mg (1,40 mMol) 4-Äthoxycarbonylphenylessigsäure in 6 ml Tetrahydrofuran gibt man 231,4 mg (1,43 mMol) Carbonyldiimidazol und erhitzt anschließend unter Feuchtigkeitsausschluß 1,5 Stunden auf Rückfluß. Nach Abkühlen auf Raumtemperatur setzt man 0,385 ml (≙2,78 mMol) Triäthylamin (über Kaliumhydroxid getrocknet) und 360 mg (1,30 mMol) (+)-1-(2-Piperidino-phenyl)-äthylamin-dihydrochlorid [Schmelzpunkt 242°C (Zers.); $[\alpha]_D^{20}$ = +14,8°C (c=1; Methanol)] zusammen mit 2 ml Tetrahydrofuran zu. Man rührt in einem 50°C warmen Ölbad 4 Stunden lang. Man dampft im Vakuum ein und verteilt den Eindampfrückstand zwischen Chloroform und Wasser. Den Chloroformextrakt trocknet man über Natriumsulfat, filtriert ihn durch eine G3-Glasfritte und dampft ihn im Vakuum zur Trockne ein. Den erhaltenen Rückstand reinigt man durch Säulenchromatographie an Kieselgel (Chloroform/Methanol = 6 : 1).
Ausbeute: 229 mg (44,7% der Theorie),
Schmelzpunkt: 89—90°C (Äther).
$[\alpha]_D^{20}$= +8,2° (c=1; Methanol).

| | | | | |
|---|---|---|---|---|
| Ber.: | C 73,07 | H 7,66 | N 7,10 | m/e = 394 |
| Gef.: | C 73,20 | H 7,68 | N 7,14 | m/e = 394 |

Analog Beispiel 2 wurde hergestellt:

(—)-4-[(1-(2-Piperidino-phenyl)-äthyl)-aminocarbonylmethyl]-
benzoesäure-äthylester

aus (—)-1-(2-Piperidino-phenyl)-äthylamin-dihydrochlorid. [Schmelzpunkt: 239—242° (Zers.); $[\alpha]_D^{20}$: —19,6° (c=1; Methanol)].
Ausbeute: 41,1% der Theorie,
Schmelzpunkt: 77—79°C (Äther/Cyclohexan).
$[\alpha]_D^{20}$= —6,2° (c=1; Methanol).

| | | | | |
|---|---|---|---|---|
| Ber.: | C 73,07 | H 7,66 | N 7,10 | m/e = 394 |
| Gef.: | C 72,67 | H 7,75 | N 6,82 | m/e = 394 |

Beispiel 3

4-[(1-(4-Chlor-2-piperidino-phenyl)-äthyl)-aminocarbonylmethyl]-
benzoesäure-äthylester

Zu einer Lösung von 5,5 g (0,023 Mol) 1-(4-Chlor-2-piperidino-phenyl)-äthylamin, 4,8 g (0,023 Mol) 4-Äthoxycarbonyl-phenylessigsäure, 7,3 g (0,028 Mol) Triphenylphosphin und 3,2 ml (0,023 Mol) Triäthylamin in 50 ml Acetonitril gibt man 2,3 ml (0,023 Mol) Tetrachlorkohlenstoff und rührt 24 Stunden bei Raumtemperatur. Anschließend dampft man im Vakuum ein und verteilt zwischen 100 ml Wasser und Essigsäureäthylester. Die vereinigten, über Natriumsulfat getrockneten organischen Extrakte filtriert man, dampft sie im Vakuum ein und reinigt den Eindampfrückstand durch Säulenchromatographie an Kieselgel (Toluol/Essigsäureäthylester = 4/1).

Ausbeute: 6,1 g (62% der Theorie),
Schmelzpunkt: 126—128°C.

| | | | | |
|---|---|---|---|---|
| Ber.: | C 67,20 | H 6,81 | Cl 8,27 | N 6,53 |
| Gef.: | C 67,43 | H 6,97 | Cl 8,16 | N 6,40 |

Analog Beispiel 3 wurden folgende Verbindungen erhalten:

4-[(1-(4-Methyl-2-piperidino-phenyl)-äthyl)-aminocarbonylmethyl]-
benzoesäure-äthylester

Ausbeute: 48,2% der Theorie,
Schmelzpunkt: 120—122°C.

| | | | |
|---|---|---|---|
| Ber.: | C 73,50 | H 7,89 | N 6,86 |
| Gef.: | C 73,61 | H 7,95 | N 6,73 |

4-[(1-(2-(4-Methyl-piperidino)-phenyl)-äthyl)-aminocarbonylmethyl]-benzoesäure-äthylester

Ausbeute: 55,8% der Theorie,
Schmelzpunkt: 125—128° C (Äther).

| | | | |
|---|---|---|---|
| Ber.: | C 73,50 | H 7,90 | N 6,86 |
| Gef.: | C 73,30 | H 7,99 | N 7,20 |

4-[(1-(2-Piperidino-phenyl)-äthyl)-aminocarbonylmethyl]-benzoesäure-äthylester

Ausbeute: 71% der Theorie,
Schmelzpunkt: 147—148° C.

| | | | |
|---|---|---|---|
| Ber.: | C 73,06 | H 7,67 | N 7,10 |
| Gef.: | C 73,54 | H 8,04 | N 6,95 |

4-[(1-(2-Piperidino-phenyl)-äthyl)-aminocarbonylmethyl]-phenyl-essigsäure

Hergestellt aus 1-(2-Piperidino-phenyl)-äthylamin und p-Phenylen-diessigsäure.

Ausbeute: 27% der Theorie,
Schmelzpunkt: 186—189° C.

| | | | |
|---|---|---|---|
| Ber.: | C 72,60 | H 7,42 | N 7,36 |
| Gef.: | C 72,75 | H 7,65 | N 7,11 |

4-[(2-Piperidino-benzhydryl)-aminocarbonylmethyl]-benzoesäure-äthylester

Ausbeute: 87,4% der Theorie,
Schmelzpunkt: 160—162° C.

| | | | |
|---|---|---|---|
| Ber.: | C 76,29 | H 7,06 | N 6,14 |
| Gef.: | C 76,44 | H 7,08 | N 6,17 |

4-[(5-Chlor-2-piperidino-benzhydryl)-aminocarbonylmethyl]-benzoesäure-äthylester

Ausbeute: 78% der Theorie,
Schmelzpunkt: 202—204° C

| | | | | |
|---|---|---|---|---|
| Ber.: | C 70,93 | H 6,36 | Cl 7,22 | N 5,71 |
| Gef.: | C 70,85 | H 6,40 | Cl 7,11 | N 5,45 |

4-[(1-(4-Piperidino-phenyl)-äthyl)-aminocarbonylmethyl]-benzoesäure-äthylester

Ausbeute: 39% der Theorie,
Schmelzpunkt: 118—120° C.

| | | | |
|---|---|---|---|
| Ber.: | C 73,07 | H 7,67 | N 7,10 |
| Gef.: | C 73,20 | H 7,78 | N 7,11 |

4-[(1-(2-(4-Methyl-piperazino)-phenyl)-äthyl)-aminocarbonylmethyl]-benzoesäure-äthylester

Ausbeute: 53% der Theorie,
Schmelzpunkt: 130—132° C.

| | | | |
|---|---|---|---|
| Ber.: | C 70,38 | H 7,63 | N 10,26 |
| Gef.: | C 70,41 | H 7,53 | N 10,13 |

4-[(1-(2-4-Benzyl-piperazino)-phenyl)-äthyl)-aminocarbonylmethyl]-benzoesäure-äthylester

Ausbeute: 75% der Theorie,
Schmelzpunkt: 135—136° C.

| | | | |
|---|---|---|---|
| Ber.: | C 74,20 | H 7,26 | N 8,66 |
| Gef.: | C 74,45 | H 7,34 | N 8,54 |

4-[(1-(2-(4-p-Chlorphenyl-piperazino)-phenyl)-äthyl)-aminocarbonylmethyl]-benzoesäure-äthylester

Ausbeute: 48,5% der Theorie,
Schmelzpunkt: 178—180° C.

| | | | | |
|---|---|---|---|---|
| Ber.: | C 68,83 | H 6,37 | Cl 7,01 | N 8,30 |
| Gef.: | C 68,71 | H 6,22 | Cl 6,82 | N 8,41 |

4-[(α-Cyclohexyl-2-piperidino-benzyl)-aminocarbonylmethyl]-benzoesäure-äthylester

Ausbeute: 75% der Theorie,
Schmelzpunkt: 135° C.

| | | | |
|---|---|---|---|
| Ber.: | C 75,29 | H 8,28 | N 6,06 |
| Gef.: | C 75,11 | H 8,13 | N 5,99 |

N-(4-Chlor-phenacetyl)-N-[1-(2-piperidino-phenyl)-äthyl)-amin

Ausbeute: 79% der Theorie,
Schmelzpunkt: 150—152° C.

| | | | | |
|---|---|---|---|---|
| Ber.: | C 70,67 | H 7,06 | Cl 9,93 | N 7,85 |
| Gef.: | C 70,94 | H 7,84 | Cl 10,09 | N 7,90 |

4-[(2-Pyrrolidino-benzhydryl)-aminocarbonylmethyl]-benzoesäure-äthylester

Ausbeute: 57% der Theorie,
Schmelzpunkt: 163—165° C.

| | | | |
|---|---|---|---|
| Ber.: | C 75,99 | H 6,83 | N 6,33 |
| Gef.: | C 75,45 | H 6,52 | N 6,10 |

4-[(2-Hexamethylenimino-benzhydryl)-aminocarbonylmethyl]-
benzoesäure-äthylester

Ausbeute: 68% der Theorie,
Schmelzpunkt: 151—154° C.

| | | | |
|---|---|---|---|
| Ber.: | C 76,56 | H 7,28 | N 5,95 |
| Gef.: | C 76,43 | H 7,19 | N 6,01 |

Beispiel 4

4-[(1-(2-Piperidino-phenyl)-äthenyl)-aminocarbonylmethyl]-
benzoesäure-äthylester

Zu einer Lösung von 10,9 g (0,0539 Mol) frisch hergestelltem (2-Piperidinophenyl)-methyl-ketimin in 110 ml Acetonitril gibt man unter Rühren nacheinander 11,2 g (0,0539 Mol) 4-Äthoxycarbonyl-phenylessigsäure, 17 g (0,0647 Mol) Triphenylphosphin, 22,6 ml (0,162 Mol) Triäthylamin und 5,2 ml (0,0539 Mol) Tetrachlorkohlenstoff. Die nach kurzer Zeit klare Lösung rührt man 20 Stunden bei 20° C. Man filtriert vom ausgefallenen Niederschlag (Triäthylaminhydrochlorid) ab und dampft das Filtrat im Vakuum ein. Den Eindampfrückstand reinigt man durch Säulenchromatographie an Kieselgel (Toluol/Aceton = 10/1).

Ausbeute: 15 g (70,1% der Theorie),
Schmelzpunkt: 112—115° C (Äther).

| | | | |
|---|---|---|---|
| Ber.: | C 73,44 | H 7,19 | N 7,14 |
| Gef.: | C 73,28 | H 7,32 | N 6,96 |

Analog Beispiel 4 wurden folgende Verbindungen erhalten:

4-[(α-Cyclohexyliden-2-piperidino-benzyl)-aminocarbonylmethyl]-
benzoesäure-äthylester

Ausbeute: 24% der Theorie,
Schmelzpunkt: 131—133° C.

| | | | |
|---|---|---|---|
| Ber.: | C 75,62 | H 7,88 | N 6,08 |
| Gef.: | C 75,59 | H 7,47 | N 6,01 |

4-[(1-(2-Piperidino-phenyl)-propenyl)-aminocarbonylmethyl]-
benzoesäure-äthylester

Ausbeute: 65,0% der Theorie (E- und Z-Isomeren-Gemisch).
Schmelzpunkt des polaren Isomeren: 82—84° C.

| | | | |
|---|---|---|---|
| Ber.: | C 73,86 | H 7,44 | N 6,89 |
| Gef.: | C 73,73 | H 7,57 | N 7,01 |

Beispiel 5

4-[(1-(2-Piperidino-phenyl)-äthyl)-aminocarbonylmethyl]-
benzoesäure-äthylester

Zu einer gerührten Lösung von 49,6 g (0,243 Mol) 1-(2-Piperidino-phenyl)-äthylamin [$Kp_{0,6}$: 100—107° C; Schmelzpunkt des Dihydrochlorids: 234—237° C (Zers.)] und 37,3 ml (0,267 Mol) Triäthylamin in 245 ml Methylenchlorid tropft man unter leichter Eiskühlung bei einer Innentemperatur von 20—30° C die Lösung von 60,6 g (0,267 Mol) 4-Äthoxycarbonyl-phenacetylchlorid in 120 ml Methylenchlorid. Anschließend rührt man noch 2 Stunden bei Raumtemperatur. Den ausgefallenen Niederschlag filtriert man ab, wäscht ihn einmal mit Methylenchlorid und schüttelt die vereinigten Methylenchlorid-Phasen nacheinander zweimal mit Wasser, einmal mit 10%igem wässrigem Ammoniak, zweimal mit Wasser, einmal mit 100 ml 3%iger Salzsäure und zweimal mit Wasser. Man trocknet die Methylenchlorid-Phase über Natriumsulfat, filtriert sie und dampft sie im Vakuum ein. Den Eindampfrückstand kristallisiert man aus Äther.

Ausbeute: 88,8 g (92,7% der Theorie),
Schmelzpunkt: 148—150° C.

Analog Beispiel 5 wurden folgende Verbindungen erhalten:

4-[(5-Methyl-2-piperidino-benzyl)-aminocarbonylmethyl]-benzoesäure-äthylester

Ausbeute: 22,5% der Theorie,
Schmelzpunkt: 116,5—117° C (Äthanol/Petroläther).

| | | | |
|---|---|---|---|
| Ber.: | C 73,07 | H 7,66 | N 7,10 |
| Gef.: | C 73,48 | H 7,62 | N 7,15 |

4-[(1-(5-Methyl-2-piperidino-phenyl)-äthyl)-aminocarbonylmethyl]-benzoesäure-äthylester

Ausbeute: 20,2% der Theorie,
Schmelzpunkt: 132—132,5° C (Äthanol).

| | | | |
|---|---|---|---|
| Ber.: | C 73,50 | H 7,90 | N 6,86 |
| Gef.: | C 73,49 | H 7,74 | N 6,94 |

4-[(1-(5-Methoxy-2-piperidino-phenyl)-äthyl)-aminocarbonylmethyl]-benzoesäure-äthylester

Ausbeute: 35,8% der Theorie,
Schmelzpunkt: 131—132° C (Äthanol).

| | | | |
|---|---|---|---|
| Ber.: | C 70,73 | H 7,60 | N 6,60 |
| Gef.: | C 70,98 | H 7,59 | N 6,38 |

4-[(1-(2-Piperidino-phenyl)-äthyl)-N-methylamino-carbonylmethyl]-benzoesäure-äthylester

Ausbeute: 65,2% der Theorie,
Schmelzpunkt: <20° C.

| | | | |
|---|---|---|---|
| Ber.: | C 73,50 | H 7,90 | N 6,86 |
| Gef.: | C 72,99 | H 7,60 | N 6,87 |

4-[(1-(2-(Decahydro-isochinolin-2-yl)-phenyl)-äthyl)-aminocarbonylmethyl]-benzoesäure-äthylester

Ausbeute: 44% der Theorie,
Schmelzpunkt: 159° C.

| | | | |
|---|---|---|---|
| Ber.: | C 74,96 | H 8,08 | N 6,24 |
| Gef.: | C 75,09 | H 8,01 | N 6,01 |

4-[(1-(2-(1,2,3,4,5,6,7,8-Octahydro-isochinolin-2-yl)-phenyl)-äthyl)-aminocarbonylmethyl]-benzoesäure-äthylester

Ausbeute: 35% der Theorie,
Schmelzpunkt: 115—117° C.

| | | | |
|---|---|---|---|
| Ber.: | C 75,30 | H 7,67 | N 6,27 |
| Gef.: | C 75,18 | H 7,37 | N 5,89 |

4-[(1-(2-(Octahydro-isoindol-2-yl)-phenyl)-äthyl)-aminocarbonylmethyl]-benzoesäure-äthylester

Ausbeute: 36% der Theorie,
Schmelzpunkt: 141° C.

| | | | |
|---|---|---|---|
| Ber.: | C 74,62 | H 7,88 | N 6,44 |
| Gef.: | C 74,70 | H 7,97 | N 6,42 |

4-[(1-(3-Piperidino-phenyl)-äthyl-aminocarbonylmethyl]-benzoesäure-äthylester

Ausbeute: 24% der Theorie,
Schmelzpunkt: 164° C.

| | | | |
|---|---|---|---|
| Ber.: | C 73,07 | H 7,66 | N 7,10 |
| Gef.: | C 72,80 | H 7,48 | N 7,13 |

4-[(1-(6-Chlor-2-piperidino-phenyl)-äthyl)-aminocarbonylmethyl]-benzoesäure-äthylester

Ausbeute: 17% der Theorie,
Schmelzpunkt: <20° C.

| | | | | | |
|---|---|---|---|---|---|
| Ber.: | C 67,20 | H 6,81 | Cl 8,26 | N 6,53 | m/e=428/30 |
| Gef.: | C 67,96 | H 6,56 | Cl 8,80 | N 6,67 | m/e=428/30 |

4-[(1-(6-Methyl-2-piperidino-phenyl)-äthyl)-aminocarbonylmethyl]-benzoesäure-äthylester

Ausbeute: 3,5% der Theorie,
Schmelzpunkt: <20° C.

| | | | | |
|---|---|---|---|---|
| Ber.: | C 73,49 | H 7,89 | N 6,85 | m/e=408 |
| Gef.: | C 73,80 | H 7,61 | N 7,01 | m/e=408 |

4-[(1-(2-(3-Aza-bicyclo[3,2,2]nonan-3-yl)-phenyl)-äthyl)-aminocarbonylmethyl]-benzoesäure-äthylester

Ausbeute: 0,5% der Theorie,
Schmelzpunkt: <20° C.

| | |
|---|---|
| Ber.: | m/e=434 |
| Gef.: | m/e=434 |

N-[1-(5-Chlor-2-piperidino-phenyl)-äthyl]-N-phenacetyl-amin

Ausbeute: 53,5% der Theorie,
Schmelzpunkt: 134—136° C (Äthanol).

| | | | | |
|---|---|---|---|---|
| Ber.: | C 70,67 | H 7,06 | Cl 9,94 | N 7,85 |
| Gef.: | C 70,40 | H 7,32 | Cl 9,77 | N 7,68 |

Beispiel 6

4-[(1-(2-Piperidino-phenyl)-äthenyl)-aminocarbonylmethyl]-benzoesäure-äthylester

Zu einer gerührten Lösung von 2,02 g (0,010 Mol) frisch hergestelltem Methyl-(2-piperidino-phenyl)-ketimin und 1,53 ml (0,011 Mol) Triäthylamin in 10 ml Methylenchlorid tropft man unter Eiskühlung bei einer Innentemperatur von 1 bis 6° C binnen 15 Minuten die Lösung von 2,49 g (0,011 Mol) 4-Äthoxycarbonylphenacetylchlorid in 10 ml Methylenchlorid. Man rührt noch 20 Stunden bei 20° C und gießt das Reaktionsgemisch in kalte Natriumhydrogencarbonat-Lösung ein. Man extrahiert mehrfach mit Methylenchlorid, wäscht den organischen Extrakt einmal mit Wasser, trocknet über Natriumsulfat, filtriert und dampft im Vakuum ein. Den Eindampfrückstand reinigt man durch Säulenchromatographie an Kieselgel (Toluol/Aceton = 50/1).

Ausbeute: 1,86 g (47,7% der Theorie),
Schmelzpunkt: 113—116° C (Äthanol).

| | | | | |
|---|---|---|---|---|
| Ber.: | C 73,44 | H 7,19 | N 7,14 | m/e = 392 |
| Gef.: | C 72,95 | H 6,98 | N 7,22 | m/e = 392 |

Analog Beispiel 6 wurden folgende Verbindungen hergestellt:

4-[(1-(6-Chlor-2-piperidino-phenyl)-äthenyl)-aminocarbonylmethyl]-benzoesäure-äthylester

Ausbeute: 37% der Theorie,
Schmelzpunkt: 102—105° C.

| | | | | | |
|---|---|---|---|---|---|
| Ber.: | C 67,51 | H 6,37 | Cl 8,30 | N 6,56 | m/e = 426/28 |
| Gef.: | C 67,86 | H 6,36 | Cl 8,58 | N 6,23 | m/e = 426/28 |

4-[(1-(6-Methyl-2-piperidino-phenyl)-äthenyl)-aminocarbonylmethyl]-benzoesäure-äthylester

Ausbeute: 41% der Theorie,
Schmelzpunkt: 116—118° C.

| | | | |
|---|---|---|---|
| Ber.: | C 73,86 | H 7,43 | N 6,89 |
| Gef.: | C 73,75 | H 7,43 | N 6,77 |

Beispiel 7

4-[(1-(2-Piperidino-phenyl)-äthenyl)-aminocarbonylmethyl]-benzoesäure-äthylester

Zu einer Suspension von 2,20 g (6,24 mMol) Jodmagnesium-[methyl-(2-piperidino-phenyl)-ketimino]-Komplex in 15 ml Methylenchlorid tropft man unter Rühren die Lösung von 1,55 g (6,86 mMol) 4-Äthoxycarbonyl-phenacetylchclorid in 5 ml Methylenchlorid. Dabei steigt die Innentemperatur von 20° C auf 30° C an. Man rührt noch 2 Stunden bei Raumtemperatur, versetzt unter Rühren mit Wasser und extrahiert mehrfach mit Methylenchlorid. Die Methylenchlorid-Lösung wäscht man dreimal mit

Wasser, trocknet sie über Natriumsulfat, filtriert sie und dampft sie im Vakuum ein. Den Eindampfrückstand reinigt man durch Säulenchromatographie an Kieselgel (Toluol/Aceton = 50/2).

Ausbeute: 1,1 g (45,8% der Theorie),
Schmelzpunkt: 115—118° C (Äthanol).

| | | | |
|---|---|---|---|
| Ber.: | C 73,44 | H 7,19 | N 7,14 |
| Gef.: | C 73,30 | H 7,06 | N 7,16 |

Analog Beispiel 7 wurde folgende Verbindung erhalten:

4-[(1-(5-Chlor-2-piperidino-phenyl)-äthenyl)-aminocarbonylmethyl]-benzoesäure-äthylester

Ausbeute: 39,5% der Theorie,
Schmelzpunkt: 142—145° C (Äthanol).

| | | | | |
|---|---|---|---|---|
| Ber.: | C 67,51 | H 6,37 | Cl 8,30 | N 6,56 |
| Gef.: | C 67,51 | H 6,37 | Cl 8,36 | N 6,49 |

## Beispiel 8

4-[(1-(5-Chlor-2-dimethylamino-phenyl)-äthyl)-aminocarbonylmethyl]-benzoesäure

Eine Lösung von 2,0 g (0,00534 Mol) 4-[(1-(5-Chlor-2-dimethyl-amino-phenyl)-äthyl)-aminocarbonylmethyl]-benzoesäure-methylester und 0,32 g (0,00801 Mol) Natriumhydroxid in 23 ml Äthanol und 7 ml Wasser rührt man 2 Stunden bei 50° C. Man dampft im Vakuum ein, nimmt in Wasser auf, stellt mit 2 N-Salzsäure auf pH 6 ein und extrahiert mit Äthylacetat. Die organische Phase schüttelt man mit Wasser, trocknet über Natriumsulfat, filtriert sie und dampft sie im Vakuum ein. Den Eindampfrückstand kristallisiert man aus Äther um.

Ausbeute: 1,7 g (88% der Theorie),
Schmelzpunkt: 190—192° C.

| | | | | |
|---|---|---|---|---|
| Ber.: | C 63,24 | H 5,87 | Cl 9,83 | N 7,76 |
| Gef.: | C 62,90 | H 5,81 | Cl 10,02 | N 7,90 |

Analog Beispiel 8 wurden folgende Verbindungen hergestellt:

4-[(1-(5-Chlor-2-dipropylamino-phenyl)-äthyl)-aminocarbonylmethyl]-benzoesäure

Ausbeute: 87,6% der Theorie,
Schmelzpunkt: 203—205° C.

| | | | | |
|---|---|---|---|---|
| Ber.: | C 66,25 | H 7,01 | Cl 8,50 | N 6,72 |
| Gef.: | C 65,97 | H 6,96 | Cl 8,52 | N 6,55 |

4-[(1-(5-Chlor-2-dibutylamino-phenyl)-äthyl)-aminocarbonylmethyl]-benzoesäure

Ausbeute: 77,3% der Theorie,
Schmelzpunkt: 200—202° C.

| | | | | |
|---|---|---|---|---|
| Ber.: | C 67,47 | H 7,48 | Cl 7,97 | N 6,30 |
| Gef.: | C 67,45 | H 7,60 | Cl 8,28 | N 6,44 |

4-[(1-(5-Chlor-2-N-cyclohexyl-N-methylamino-phenyl)-äthyl)-aminocarbonylmethyl]-benzoesäure

Ausbeute: 88,2% der Theorie,
Schmelzpunkt: 198—200° C (Äther).

| | | | | |
|---|---|---|---|---|
| Ber.: | C 67,20 | H 6,81 | Cl 8,27 | N 6,53 |
| Gef.: | C 67,10 | H 6,73 | Cl 8,16 | N 6,47 |

4-[(5-Chlor-2-pyrrolidino-benzyl)-aminocarbonylmethyl]-benzoesäure

Ausbeute: 84,2% der Theorie,
Schmelzpunkt: 208—210° C (Äthylacetat).

| | | | | |
|---|---|---|---|---|
| Ber.: | C 64,42 | H 5,68 | Cl 9,51 | N 7,51 |
| Gef.: | C 64,70 | H 5,68 | Cl 9,58 | N 7,60 |

4-[(1-(5-Chlor-2-pyrrolidino-phenyl)-äthyl)-aminocarbonylmethyl]-benzoesäure

Ausbeute: 81,1% der Theorie,
Schmelzpunkt: 202—204° C (Äthylacetat).

| | | | | |
|---|---|---|---|---|
| Ber.: | C 65,20 | H 5,99 | Cl 9,17 | N 7,24 |
| Gef.: | C 65,02 | H 6,12 | Cl 9,32 | N 7,10 |

4-[(5-Chlor-2-piperidino-benzyl)-aminocarbonylmethyl]-benzoesäure

Ausbeute: 78% der Theorie,
Schmelzpunkt: 164—166° C.

| | | | | |
|---|---|---|---|---|
| Ber.: | C 65,19 | H 5,99 | Cl 9,17 | N 7,24 |
| Gef.: | C 65,50 | H 5,76 | Cl 9,24 | N 7,36 |

4-[(1-(5-Chlor-2-piperidino-benzyl)-aminocarbonyl)-äthyl]-benzoesäure

Ausbeute: 81,1% der Theorie,
Schmelzpunkt: 213—216° C (Aceton/Äther).

| | | | | |
|---|---|---|---|---|
| Ber.: | C 65,90 | H 6,29 | Cl 8,84 | N 6,99 |
| Gef.: | C 66,30 | H 6,40 | Cl 9,00 | N 7,04 |

4-[(1-(5-Chlor-2-piperidino-phenyl)-äthyl)-aminocarbonylmethyl]-benzoesäure

Ausbeute: 84,9% der Theorie,
Schmelzpunkt: 213—215° C (Äther).

| | | | | |
|---|---|---|---|---|
| Ber.: | C 65,91 | H 6,29 | Cl 8,85 | N 6,99 |
| Gef.: | C 66,18 | H 6,19 | Cl 8,88 | N 7,12 |

4-[(1-(5-Chlor-2-(3-methyl-piperidino)-phenyl)-äthyl)-aminocarbonylmethyl]-benzoesäure

Ausbeute: 69,2% der Theorie,
Schmelzpunkt: 208—210° C (Äthylacetat).

| | | | | |
|---|---|---|---|---|
| Ber.: | C 66,57 | H 6,56 | Cl 8,55 | N 6,75 |
| Gef.: | C 66,36 | H 6,77 | Cl 8,58 | N 6,80 |

4-[(1-(5-Chlor-2-(3,5-cis-dimethyl-piperidino)-phenyl)-äthyl)-aminocarbonylmethyl]-benzoesäure

Ausbeute: 82,2% der Theorie,
Schmelzpunkt: 212—214° C (Äther).

| | | | | |
|---|---|---|---|---|
| Ber.: | C 67,20 | H 6,81 | Cl 8,26 | N 6,53 |
| Gef.: | C 66,95 | H 6,69 | Cl 8,43 | N 6,68 |

4-[(1-(5-Chlor-2-piperidino-phenyl)-propyl)-aminocarbonylmethyl]-benzoesäure

Ausbeute: 81,5% der Theorie,
Schmelzpunkt: 200—203° C (Äther).

| | | | | |
|---|---|---|---|---|
| Ber.: | C 66,57 | H 6,56 | Cl 8,55 | N 6,75 |
| Gef.: | C 66,74 | H 6,35 | Cl 8,59 | N 6,45 |

4-[(1-(5-Chlor-2-piperidino-phenyl)-2-metyhl-propyl)-aminocarbonylmethyl]-benzoesäure

Ausbeute: 82,7% der Theorie,
Schmelzpunkt: 236—240° C (Äthylacetat).

| | | | | |
|---|---|---|---|---|
| Ber.: | C 67,20 | H 6,81 | Cl 8,27 | N 6,53 |
| Gef.: | C 67,19 | H 6,56 | Cl 8,14 | N 6,39 |

4-[(1-(5-Chlor-2-morpholino-phenyl)-äthyl)-aminocarbonylmethyl]-benzoesäure

Ausbeute: 85,6% der Theorie,
Schmelzpunkt: 201—203° C (Äther).

| | | | | |
|---|---|---|---|---|
| Ber.: | C 62,60 | H 5,75 | Cl 8,80 | N 6,95 |
| Gef.: | C 62,30 | H 5,82 | Cl 8,83 | N 6,85 |

4-[(1-(5-Chlor-2-thiomorpholino-phenyl)-äthyl)-aminocarbonylmethyl]-benzoesäure

Ausbeute: 87,6% der Theorie,
Schmelzpunkt: 216—217° C (Äther).

| | | | | |
|---|---|---|---|---|
| Ber.: | C 60,20 | H 5,53 | Cl 8,46 | N 6,69 |
| Gef.: | C 59,90 | H 5,51 | Cl 8,61 | N 6,53 |

4-[(1-(5-Chlor-2-(hexahydro-1H-azepino)-phenyl)-äthyl)-aminocarbonylmethyl]-benzoesäure

Ausbeute: 81,2% der Theorie,
Schmelzpunkt: 202—204° C (Chloroform/Toluol).

| | | | | |
|---|---|---|---|---|
| Ber.: | C 66,58 | H 6,56 | Cl 8,55 | N 6,75 |
| Gef.: | C 66,60 | H 6,37 | Cl 8,50 | N 6,59 |

4-[(1-(5-Chlor-2-octahydroazocino-phenyl)-äthyl)-aminocarbonylmethyl]-benzoesäure

Ausbeute: 44,4% der Theorie,
Schmelzpunkt: 196–197° C (Chloroform/Petroläther).

| | | | |
|---|---|---|---|
| Ber.: | C 67,19 | H 6,81 | N 6,53 |
| Gef.: | C 67,10 | H 6,97 | N 6,37 |

4-[(1-(5-Chlor-2-(octahydro-1H-azonino)-phenyl)-äthyl)-aminocarbonylmethyl]-benzoesäure

Ausbeute: 74,7% der Theorie,
Schmelzpunkt: 204–206° C (Äthylacetat/Petroläther).

| | | | |
|---|---|---|---|
| Ber.: | C 67,78 | H 7,05 | N 6,32 |
| Gef.: | C 67,50 | H 7,03 | N 6,04 |

4-[(2-(5-Chlor-2-piperidino-phenyl)-2-propyl)-aminocarbonylmethyl]-benzoesäure

Ausbeute: 82,9% der Theorie,
Schmelzpunkt: 227–229° C (Aceton).

| | | | | |
|---|---|---|---|---|
| Ber.: | C 66,57 | H 6,56 | Cl 8,55 | N 6,75 |
| Gef.: | C 66,03 | H 6,66 | Cl 8,67 | N 6,59 |

4-[(1-(5-Nitro-2-piperidino-phenyl)-äthyl)-aminocarbonylmethyl]-benzoesäure

Ausbeute: 95,6% der Theorie,
Schmelzpunkt: 252–254° C (Äther).

| | | | |
|---|---|---|---|
| Ber.: | C 64,22 | H 6,12 | N 10,21 |
| Gef.: | C 64,20 | H 6,17 | N 10,12 |

4-[(1-(2-Piperidino-phenyl)-äthyl)-aminocarbonylmethyl]-benzoesäure

Ausbeute: 85% der Theorie,
Schmelzpunkt: 170–172° C.

| | | | |
|---|---|---|---|
| Ber.: | C 72,11 | H 7,15 | N 7,64 |
| Gef.: | C 71,94 | H 7,03 | N 7,72 |

4-[(2-(2-Piperidino-phenyl)-2-propyl)-aminocarbonylmethyl]-benzoesäure

Ausbeute: 72,7% der Theorie,
Schmelzpunkt: 213–215° C.

| | | | |
|---|---|---|---|
| Ber.: | C 72,61 | H 7,42 | N 7,36 |
| Gef.: | C 72,52 | H 7,31 | N 7,45 |

4-[(5-Methyl-2-piperidino-benzyl)-aminocarbonylmethyl]-benzoesäure

Ausbeute: 64,6% der Theorie,
Schmelzpunkt: 120–122° C.

| | | | | |
|---|---|---|---|---|
| Ber.: | C 72,11 | H 7,15 | N 7,64 | m/e = 366 |
| Gef.: | C 72,42 | H 7,38 | N 7,45 | m/e = 366 |

Schmelzpunkt des Hydrochlorids: 266° C (Zers.)

| | | | | |
|---|---|---|---|---|
| Ber.: | C 65,58 | H 6,76 | Cl 8,80 | N 6,95 |
| Gef.: | C 65,00 | H 6,62 | Cl 9,40 | N 7,00 |

4-[(2-Piperidino-anilino)-carbonylmethyl]-benzoesäure x 0,25 HCl

Ausbeute: 72,5% der Theorie,
Schmelzpunkt: 216—217° C.

| | | | | | |
|---|---|---|---|---|---|
| Ber.: | (x 0,25 HCl) | C 69,11 | H 6,45 | Cl 2,55 | N 8,06 |
| Gef.: | | C 69,40 | H 6,32 | Cl 3,08 | N 8,37 |

4-[(5-Chlor-2-piperidino-anilino)-carbonylmethyl]-benzoesäure-hydrochlorid

Ausbeute: 51,3% der Theorie,
Schmelzpunkt: 232° C (Zers.).

| | | | | |
|---|---|---|---|---|
| Ber.: | C 58,68 | H 5,42 | Cl 17,32 | N 6,84 |
| Gef.: | C 58,26 | H 5,44 | Cl 17,97 | N 6,74 |

4-[(2-(2-Piperidino-anilino-carbonyl)-äthyl]-benzoesäure-Semihydrat

Ausbeute: 69,9% der Theorie,
Schmelzpunkt: 151—153° C (Petroläther/Aceton).

| | | | | |
|---|---|---|---|---|
| Ber.: | (x 0,5 $H_2O$) | C 69,78 | H 6,97 | N 7,75 |
| Gef.: | | C 69,30 | H 6,82 | N 7,46 |

4-[(2-(1-(2-Piperidino-phenyl)-äthyl)-aminocarbonyl)-äthyl]-benzoesäure x 0,2 $H_2O$

Ausbeute: 71,4% der Theorie,
Schmelzpunkt: 171—172° C (Aceton/Petroläther).

| | | | | |
|---|---|---|---|---|
| Ber.: | (x 0,2 $H_2O$) | C 71,91 | H 7,45 | N 7,29 |
| Gef.: | | C 71,90 | H 7,30 | N 7,03 |

## Beispiel 9

4-[(1-(5-Benzyloxy-2-piperidino-phenyl)-äthyl)-aminocarbonylmethyl]-benzoesäure

244 mg (0,487 mMol) 4-[(1-(5-Benzyloxy-2-piperidino-phenyl)-äthyl)-aminocarbonylmethyl]-benzoesäure-äthylester in 2,5 ml Äthanol erhitzt man unter Rühren mit 0,73 ml 1 N-Natronlauge in einem Bad von 50° C, bis (nach 3 Stunden) dünnschichtchromatographisch kein Ester mehr nachzuweisen ist. Man gibt 0,73 ml 1N-Salzsäure zu, dampft im Vakuum ein und verteilt zwischen Äthylacetat und Wasser. Den organischen Extrakt trocknet man über Natriumsulfat, filtriert ihn und dampft ihn im Vakuum ein. Den Eindampfrückstand kristallisiert man aus Methanol um.

Ausbeute: 191 mg (83% der Theorie),
Schmelzpunkt: 220—222° C.

| | | | |
|---|---|---|---|
| Ber.: | C 73,71 | H 6,83 | N 5,93 |
| Gef.: | C 73,21 | H 6,67 | N 5,80 |

Analog Beispiel 9 wurden folgende Verbindungen erhalten:

4-[(1-(2-Hexahydroazepino-phenyl)-äthyl)-aminocarbonylmethyl]-benzoesäure

Ausbeute: 68,5% der Theorie,
Schmelzpunkt: 174—176° C (Äthylacetat).

| | | | |
|---|---|---|---|
| Ber.: | C 72,61 | H 7,42 | N 7,36 |
| Gef.: | C 72,36 | H 7,34 | N 7,38 |

4-[(1-(2-(1,2,3,6-Tetrahydro-pyridino)-phenyl)-äthyl)-aminocarbonylmethyl]-benzoesäure

Ausbeute: 68,2% der Theorie,
Schmelzpunkt: 158—160° C (Äthylacetat).

| | | | |
|---|---|---|---|
| Ber.: | C 72,51 | H 6,64 | N 7,69 |
| Gef.: | C 72,20 | H 6,66 | N 7,74 |

4-[(2-(5-Chlor-2-piperidino-phenyl)-äthyl)-aminocarbonylmethyl]-benzoesäure

Ausbeute: 75% der Theorie,
Schmelzpunkt: 192—195° C (Äthylacetat).

| | | | | |
|---|---|---|---|---|
| Ber.: | C 65,91 | H 6,29 | Cl 8,84 | N 6,99 |
| Gef.: | C 66,39 | H 6,17 | Cl 8,45 | N 6,78 |

4-[(1-(5-Fluor-2-piperidino-phenyl)-äthyl)-aminocarbonylmethyl]-benzoesäure

Ausbeute: 52,9% der Theorie,
Schmelzpunkt: 174—176° C (Äthylacetat).

| | | | |
|---|---|---|---|
| Ber.: | C 68,73 | H 6,55 | N 7,29 |
| Gef.: | C 68,30 | H 6,48 | N 7,45 |

4-[(5-Methyl-2-piperidino-benzyl)-aminocarbonylmethyl]-benzoesäure

Ausbeute: 53,9% der Theorie,
Schmelzpunkt: 120—122° C (Äthanol).

| | | | | |
|---|---|---|---|---|
| Ber.: | C 72,11 | H 7,15 | N 7,64 | m/e = 366 |
| Gef.: | C 72,45 | H 7,04 | N 7,64 | m/e = 366 |

4-[(1-(5-Cyano-2-piperidino)-phenyl)-äthyl)-aminocarbonylmethyl]-benzoesäure

Ausbeute: 71,6% der Theorie,
Schmelzpunkt: 198—200° C (Äther).

| | | | |
|---|---|---|---|
| Ber.: | C 70,57 | H 6,44 | N 10,73 |
| Gef.: | C 70,17 | H 6,38 | N 11,00 |

4-[(1-(5-Carboxy-2-piperidino)-phenyl-äthyl)-aminocarbonyl-methyl]-benzoesäure

Hergestellt aus dem entsprechenden Diäthylester durch Verseifung mit 2,5 Äquivalenten Natriumhydroxid.

Ausbeute: 73,5% der Theorie,
Schmelzpunkt: 260° C (Zers.).

| | | | |
|---|---|---|---|
| Ber.: | C 67,30 | H 6,38 | N 6,82 |
| Gef.: | C 67,76 | H 6,62 | N 6,85 |

4-[(1-(2-[1,4-Dioxa-8-azaspiro-[4.5]decan-8-yl]-phenyl)-äthyl)-aminocarbonylmethyl]-benzoesäure-Semihydrat

Ausbeute: 85,7% der Theorie,
Schmelzpunkt: 130—135° C (Petroläther/Aceton).

| | | | | |
|---|---|---|---|---|
| Ber.: | (x 0,5 $H_2O$) | C 66,49 | H 6,74 | N 6,46 |
| Gef.: | | C 66,56 | H 6,65 | N 6,46 |

4-[2-Hydroxy-1-(2-piperidino-phenyl)-äthyl)-aminocarbonyl-methyl]-benzoesäure

Ausbeute: 65% der Theorie,
Schmelzpunkt: 155—157° C (Zers.) (Petroläther/ + Aceton).

| | |
|---|---|
| Ber.: | m/e = 382 |
| Gef.: | m/e = 382 |

4-[(1-(5-Chlor-2-(2-methyl-piperidino)-phenyl)-äthyl)-amino-carbonylmethyl]-benzoesäure

Ausbeute: 64,1% der Theorie,
Schmelzpunkt: 195—198° C (Äthylacetat).

| | | | | |
|---|---|---|---|---|
| Ber.: | C 66,57 | H 6,56 | Cl 8,54 | N 6,75 |
| Gef.: | C 66,01 | H 6,25 | Cl 8,32 | N 6,90 |

4-[(1-(5-Aminocarbonyl-2-piperidino-phenyl)-äthyl)-aminocarbonylmethyl]-benzoesäure

Ausbeute: 86% der Theorie,
Schmelzpunkt: 231—235° C (Äthylacetat).

| | | | |
|---|---|---|---|
| Ber.: | C 67,46 | H 6,65 | N 10,26 |
| Gef.: | C 67,96 | H 6,68 | N 10,11 |

4-[(1-(2-(4-Methyl-piperidino)-phenyl)-äthyl)-aminocarbonyl-methyl]-benzoesäure

Ausbeute: 67,7% der Theorie,
Schmelzpunkt: 173—175° C (Chloroform).

| | | | |
|---|---|---|---|
| Ber.: | C 72,61 | H 7,42 | N 7,36 |
| Gef.: | C 72,20 | H 7,36 | N 7,45 |

4-[(1-(2-Piperidino-phenyl)-äthyl)-N-methylamino-carbonylmethyl]-benzoesäure-Hydrochlorid

Überführung des viskosen Betains (72% roh) ins Hydrochlorid mittels Chlorwasserstoff in isopropanolischer Lösung.

Ausbeute: 32% der Theorie,
Schmelzpunkt: 222—230° C (Zers.) (Äthanol).

| | | | | |
|---|---|---|---|---|
| Ber.: | C 66,25 | H 7,01 | Cl 8,50 | N 6,71 |
| Gef.: | C 66,07 | H 6,96 | Cl 8,37 | N 6,58 |

2-[(1-(2-Piperidino-phenyl)-äthyl)-aminocarbonylmethyl]-benzoesäure

Ausbeute: 7% der Theorie,
Schmelzpunkt: 135° C (Zers.).

| | | | |
|---|---|---|---|
| Ber.: | C 72,10 | H 7,15 | N 7,64 |
| Gef.: | C 72,29 | H 7,03 | N 7,37 |

3-[(1-(2-Piperidino-phenyl)-äthyl)-aminocarbonylmethyl]-benzoesäure

Ausbeute: 86% der Theorie,
Schmelzpunkt: 205—207° C.

| | | | |
|---|---|---|---|
| Ber.: | C 72,11 | H 7,15 | N 7,64 |
| Gef.: | C 72,30 | H 7,29 | N 7,71 |

3-Chlor-4-[(1-(2-piperidino-phenyl)-äthyl)-aminocarbonyl-methyl]-benzoesäure

Ausbeute: 38% der Theorie,
Schmelzpunkt: ab 175° C Sintern, ab 190° C klare Schmelze.

| | | | | |
|---|---|---|---|---|
| Ber.: | C 65,91 | H 6,29 | Cl 8,84 | N 6,99 |
| Gef.: | C 65,42 | H 6,32 | Cl 9,05 | N 6,77 |

4-[(1-(2-(1,2,3,4-Tetrahydro-isochinolin-2-yl)-phenyl)-äthyl)-aminocarbonylmethyl]-benzoesäure

Ausbeute: 59% der Theorie,
Schmelzpunkt: 207—209° C.

| | | | |
|---|---|---|---|
| Ber.: | C 75,34 | H 6,32 | N 6,76 |
| Gef.: | C 75,30 | H 6,29 | N 6,67 |

4-[(1-(3-Piperidino-phenyl)-äthyl)-aminocarbonylmethyl]-benzoesäure

Ausbeute: 33% der Theorie,
Schmelzpunkt: 206—208° C.

| | | | |
|---|---|---|---|
| Ber.: | C 72,09 | H 7,15 | N 7,64 |
| Gef.: | C 72,04 | H 7,14 | N 7,57 |

4-[(1-(6-Chlor-2-piperidino-phenyl)-äthyl)-aminocarbonylmethyl]-benzoesäure

Ausbeute: 35% der Theorie,
Schmelzpunkt: 148—150° C.

| | | | | |
|---|---|---|---|---|
| Ber.: | C 65,91 | H 6,28 | Cl 8,84 | N 6,98 |
| Gef.: | C 65,45 | H 6,36 | Cl 9,63 | N 6,84 |

4-[(1-(6-Methyl-2-piperidino-phenyl)-äthyl)-aminocarbonylmethyl]-benzoesäure

Ausbeute: 33% der Theorie,
Schmelzpunkt: 170° C.

| | | | |
|---|---|---|---|
| Ber.: | C 72,60 | H 7,41 | N 7,36 |
| Gef.: | C 72,45 | H 7,34 | N 7,32 |

4-[(1-(2-(Octahydro-isoindol-2-yl)-phenyl)-äthyl)-aminocarbonylmethyl]-benzoesäure

Ausbeute: 64% der Theorie,
Schmelzpunkt: 130° C.

| | | | |
|---|---|---|---|
| Ber.: | C 73,86 | H 7,43 | N 6,89 |
| Gef.: | C 73,60 | H 7,47 | N 6,72 |

4-[(1-(2-(Decahydro-isochinolin-2-yl)-phenyl)-äthyl)-aminocarbonylmethyl]-benzoesäure

Ausbeute: 71% der Theorie,
Schmelzpunkt: 220—221° C.

| | | | | |
|---|---|---|---|---|
| Ber.: | C 74,25 | H 7,66 | N 6,66 | m/e = 420 |
| Gef.: | C 74,45 | H 7,50 | N 6,58 | m/e = 420 |

4-[(1-(2-(1,2,3,4,5,6,7,8-Octahydro-isochinolin-2-yl)-phenyl-äthyl)-aminocarbonylmethyl]-benzoesäure-Semihydrat

Ausbeute: 99% der Theorie,
Schmelzpunkt: 70° C (Zers.).

| | | | | |
|---|---|---|---|---|
| Ber.: (x 0,5 $H_2O$): | C 73,05 | H 7,30 | N 6,54 | m/e = 418 |
| Gef.: | C 73,00 | H 7,16 | N 5,98 | m/e = 418 |

4-[(1-(4-Chlor-2-piperidino-phenyl)-äthyl)-aminocarbonylmethyl]-benzoesäure

Ausbeute: 82,1% der Theorie,
Schmelzpunkt: 200—202° C.

| | | | | |
|---|---|---|---|---|
| Ber.: | C 65,91 | H 6,29 | Cl 8,84 | N 6,99 |
| Gef.: | C 66,06 | H 6,40 | Cl 9,01 | N 6,93 |

4-[(1-(4-Methyl-2-piperidino-phenyl)-äthyl)-aminocarbonylmethyl]-benzoesäure

Ausbeute: 66,5% der Theorie,
Schmelzpunkt: 110—115° C.

| | | | |
|---|---|---|---|
| Ber.: | C 72,60 | H 7,42 | N 7,36 |
| Gef.: | C 72,50 | H 7,52 | N 7,46 |

4-[(2-Piperidino-benzhydryl)-aminocarbonylmethyl]-benzoesäure

Ausbeute: 88% der Theorie,
Schmelzpunkt: 232—234° C.

| | | | |
|---|---|---|---|
| Ber.: | C 75,68 | H 6,59 | N 6,54 |
| Gef.: | C 75,16 | H 6,52 | N 6,74 |

4-[(5-Chlor-2-piperidino-benzhydryl)-aminocarbonylmethyl]-benzoesäure

Ausbeute: 78,5% der Theorie,
Schmelzpunkt: 255—260° C.

| | | | | |
|---|---|---|---|---|
| Ber.: | C 70,05 | H 5,88 | Cl 7,66 | N 6,05 |
| Gef.: | C 70,50 | H 5,76 | Cl 7,36 | N 6,06 |

4-[(1-(4-Piperidino-phenyl)-äthyl)-aminocarbonylmethyl]-benzoesäure

Ausbeute: 81% der Theorie,
Schmelzpunkt: 208—210° C.

| | | | |
|---|---|---|---|
| Ber.: | C 72,11 | H 7,15 | N 7,64 |
| Gef.: | C 72,24 | H 7,26 | N 7,54 |

4-[(1-(2-(4-Methyl-piperazino)-phenyl)-äthyl)-aminocarbonylmethyl]-benzoesäure

Ausbeute: 65% der Theorie,
Schmelzpunkt: 150—153° C.

| | | | |
|---|---|---|---|
| Ber.: | C 69,27 | H 7,13 | N 11,02 |
| Gef.: | C 69,62 | H 7,65 | N 10,64 |

4-[(1-(2-(4-Benzyl-piperazino)-phenyl)-äthyl)-aminocarbonylmethyl]-benzoesäure-Hydrochlorid

Ausbeute: 32% der Theorie,
Schmelzpunkt: 180° C.

| | | | | |
|---|---|---|---|---|
| Ber.: | C 68,07 | H 6,53 | Cl 7,18 | N 8,51 |
| Gef.: | C 67,85 | H 6,56 | Cl 7,18 | N 8,51 |

4-[(1-(2-(4-p-Chlorphenyl-piperazino)-phenyl)-äthyl)-aminocarbonylmethyl]-benzoesäure

Ausbeute: 75% der Theorie,
Schmelzpunkt: 212° C (Zers.).

| | | | | |
|---|---|---|---|---|
| Ber.: | C 67,84 | H 5,90 | Cl 7,42 | N 8,79 |
| Gef.: | C 67,74 | H 6,22 | Cl 7,59 | N 8,82 |

4-[(α-Cyclohexyl-2-piperidino-benzyl)-aminocarbonylmethyl]-benzoesäure

Ausbeute: 33% der Theorie,
Schmelzpunkt: 199—202° C.

| | | | |
|---|---|---|---|
| Ber.: | C 74,62 | H 7,89 | N 6,45 |
| Gef.: | C 74,60 | H 7,54 | N 6,66 |

(+)-4-[(1-(2-Piperidino-phenyl)-äthyl)-aminocarbonylmethyl]-benzoesäure x 0,3 $H_2O$

Ausbeute: 40% der Theorie,
Schmelzpunkt: 107° C (Zers.) (Isopropanol/Äther)
$[\alpha]_D^{20}$ = +7,3° (c=1; Methanol).

| | | | | | |
|---|---|---|---|---|---|
| Ber.: | (x 0,3 $H_2O$) | C 71,02 | H 7,25 | N 7,52 | m/e = 366 |
| Gef.: | | C 70,90 | H 7,22 | N 7,42 | m/e = 366 |

(—)-4-[(1-(2-Piperidino-phenyl)-äthyl)-aminocarbonylmethyl]-benzoesäure-Natriumsalz

Rohausbeute an Betain: 77% der Theorie.

| | |
|---|---|
| Ber.: | m/e = 366 |
| Gef.: | m/e = 366 |

Überführung ins Natriumsalz mittels 1 Äquivalent Natronlauge in Äthanol.
Schmelzpunkt des Natriumsalzes: 190° (Zers.).

4-[(1-(2-Piperidino-phenyl)-äthenyl)-aminocarbonylmethyl]-benzoesäure

Ausbeute: 53,6% der Theorie,
Schmelzpunkt: 158—160° C (Äthanol).

| | | | |
|---|---|---|---|
| Ber.: | C 72,51 | H 6,64 | N 7,69 |
| Gef.: | C 72,40 | H 6,34 | N 7,51 |

4-[(1-(5-Chlor-2-piperidino-phenyl)-äthenyl)-aminocarbonylmethyl]-benzoesäure

Ausbeute: 78,7% der Theorie,
Schmelzpunkt: 198—200° C (Aceton).

| | | | | |
|---|---|---|---|---|
| Ber.: | C 66,24 | H 5,81 | Cl 8,88 | N 7,02 |
| Gef.: | C 65,74 | H 5,72 | Cl 9,37 | N 7,10 |

4-[($\alpha$-Cyclohexyliden-2-piperidino-benzyl)-aminocarbonylmethyl]-benzoesäure

Ausbeute: 21% der Theorie,
Schmelzpunkt: 213—216° C.

| | | | |
|---|---|---|---|
| Ber.: | C 74,97 | H 7,46 | N 6,48 |
| Gef.: | C 74,73 | H 7,52 | N 6,48 |

4-[(1-(6-Chlor-2-piperidino-phenyl)-äthenyl)-aminocarbonylmethyl]-benzoesäure

Ausbeute: 39% der Theorie,
Schmelzpunkt: 162° C.

| | | | | | |
|---|---|---|---|---|---|
| Ber.: | C 66,24 | H 5,81 | Cl 8,88 | N 7,02 | m/e = 398/400 |
| Gef.: | C 66,48 | H 5,84 | Cl 8,88 | N 6,85 | m/e = 398/400 |

4-[(1-(6-Methyl-2-piperidino-phenyl)-äthenyl)-aminocarbonylmethyl]-benzoesäure

Ausbeute: 49% der Theorie,
Schmelzpunkt: 128—130° C.

| | |
|---|---|
| Ber.: | m/e = 378 |
| Gef.: | m/e = 378 |

4-[(1-(2-Piperidino-phenyl)-propenyl)-aminocarbonylmethyl]-benzoesäure

Ausbeute: 65% der Theorie,
Schmelzpunkt (Z-Form): 185—187° C (Äthylacetat).
Schmelzpunkt (E-Form): 108—110° C

| | | | | |
|---|---|---|---|---|
| Ber.: | | C 72,99 | H 6,92 | N 7,40 |
| Gef.: | (Z-Form): | C 73,10 | H 6,99 | N 7,56 |

4-[(1-(5-Hydroxy-2-piperidino-phenyl)-äthyl)-aminocarbonylmethyl]-benzoesäure-Semihydrat

Verseifung mit 2,5 Äquivalenten Natriumhydroxid.
Ausbeute: 55,9% der Theorie,
Schaum (aus Äther).

| | | | | |
|---|---|---|---|---|
| Ber.: | (x 0,5 $H_2O$) | C 67,50 | H 6,95 | N 7,16 |
| Gef.: | | C 67,11 | H 7,15 | N 6,87 |

4-[(1-(2-(2-Methyl-pyrrolidino)-phenyl)-äthyl)-aminocarbonylmethyl]-benzoesäure

Ausbeute: 62% der Theorie,
Schmelzpunkt: 169—172° C.

| | | | |
|---|---|---|---|
| Ber.: | C 72,11 | H 7,15 | N 7,64 |
| Gef.: | C 71,96 | H 6,82 | N 7,51 |

4-[(1-(5-Aminosulfonyl-2-piperidino-phenyl)-äthyl)-aminocarbonylmethyl]-benzoesäure

Ausbeute: 19,2% der Theorie,
Schmelzpunkt: 210° C (Zers.).

| | | | | |
|---|---|---|---|---|
| Ber.: | C 59,30 | H 6,11 | N 9,43 | m/e = 445 |
| Gef.: | C 58,80 | H 5,87 | N 9,06 | m/e = 445 |

4-[(1-(2-Piperidino-phenyl)-propyl)-aminocarbonylmethyl]-benzoesäure

Ausbeute: 71,4% der Theorie,
Schmelzpunkt: 208—210° C (Äthanol).

| | | | |
|---|---|---|---|
| Ber.: | C 72,61 | H 7,42 | N 7,36 |
| Gef.: | C 72,30 | H 7,44 | N 7,45 |

## Beispiel 10

4-[(1-(2-Piperidino-phenyl)-äthyl)-aminocarbonylmethyl]-benzoesäure

Zu 88,8 g (0,225 Mol) 4-[(1-(2-Piperidino-phenyl)-äthyl)-aminocarbonylmethyl]-benzoesäure-äthylester in 890 ml Äthanol gibt man eine Lösung von 13,5 g (0,338 Mol) Natriumhydroxid in 50 ml Wasser

und rührt bei einer Innentemperatur von 60° C, bis dünnschichtchromatographisch kein Ausgangsprodukt mehr nachweisbar ist (ca. 45 Minuten). Dann setzt man 400 ml Wasser zu und stellt bei ~25° C mit halbkonzentrierter Salzsäure unter pH-metrischer Kontrolle auf pH = 5,8 ein. Nach kurzer Zeit setzt Kristallisation ein. Nach Stehen über Nacht bei 20° C filtriert man ab, wäscht das Kristallisat mehrmals mit Wasser, löst das Kristallisat in Methylenchlorid und trennt von der wäßrigen Phase. Die Methylenchlorid-Lösung trocknet man über Natriumsulfat, filtriert sie, dampft sie im Vakuum ein und erhält so 57,5 g festen Eindampfrückstand.

Das äthanolisch-salzsaure Filtrat (pH = 5,8) stellt man mit halbkonzentrierter Salzsäure auf pH = 5,0 ein, destilliert dann das Äthanol im Vakuum weitestgehend ab und kühlt die eingeengte Lösung in Eis ab. Den ausgefallenen Niederschlag filtriert man ab, löst ihn in Methylenchlorid, trennt von der wäßrigen Phase ab, trocknet die Methylenchlorid-Lösung, filtriert sie und dampft sie im Vakuum ein. Der feste Eindampfrückstand beträgt 13,0 g. Die beiden Eindampfrückstände (zusammen 70,5 g) kristallisiert man aus der 5- bis 6fachen Menge Äthanol/Wasser (80/20) unter Aktivkohle-Zusatz um.

Ausbeute: 62% der Theorie,
Schmelzpunkt: 163—164° C.

| | | | |
|---|---|---|---|
| Ber.: | C 72,11 | H 7,15 | N 7,64 |
| Gef.: | C 72,13 | H 7,25 | N 7,75 |

Stellt man nach beendeter Verseifung, nach Zugabe von Wasser und Abkühlen auf 25° C gleich auf pH = 5,0 ein, so erhält man — wie vorstehend beschrieben, aber ohne Aufarbeitung des äthanolischsalzsauren Filtrats — 75,9% getrockneten Eindampfrückstand, der schon vor der abschließenden Umkristallisation eine richtige Elementaranalyse liefert.

Schmelzpunkt: 172—176° C.

| | | | |
|---|---|---|---|
| Ber.: | C 72,11 | H 7,15 | N 7,64 |
| Gef.: | C 71,90 | H 7,08 | N 7,52 |

Analog Beispiel 10 wurden folgende Verbindungen erhalten:

4-[(1-(5-Methyl-2-piperidino-phenyl)-äthyl)-aminocarbonylmethyl]-benzoesäure

Ausbeute: 56,6% der Theorie,
Schmelzpunkt: 215—217° C (Äthanol).

| | | | |
|---|---|---|---|
| Ber.: | C 72,61 | H 7,42 | N 7,36 |
| Gef.: | C 72,71 | H 7,49 | N 7,25 |

4-[(α-Carboxy-2-piperidino-benzyl)-aminocarbonylmethyl]-
benzoesäure x 0,66 $H_2O$

Hergestellt durch Verseifung des 4-[(α-Methoxycarbonyl-2-piperidino)-benzyl)-aminocarbonylmethyl]-benzoesäure-äthylesters mit 2,5 Äquivalenten Natriumhydroxid.

Ausbeute: 72,2% der Theorie,
Schmelzpunkt: 235—240° C (Zers.) (Methanol/Chloroform).

| | | | | |
|---|---|---|---|---|
| Ber.: | (x 0,66 $H_2O$) | C 64,69 | H 6,33 | N 6,85 |
| Gef.: | | C 64,64 | H 6,23 | N 6,61 |

## Beispiel 11

4-[(1-(2-Piperidino-phenyl)-äthyl)-aminocarbonylmethyl]-
benzoesäure-Natriumsalz-Monohydrat

Man rührt 500 mg (1,26 mMol) 4-[(2-Piperidino-phenyl)-äthyl)-aminocarbonylmethyl]-benzoesäure-äthylester in 5 ml Äthanol zusammen mit 1,26 ml 1 N-Natronlauge 1 Stunde bei 50° C. Dann kühlt man auf 0° C auf, filtriert vom ausgefallenen Kristallisat ab und wäscht es mit kaltem Äthanol und mit Äther.

Ausbeute: 238 mg (48,6% der Theorie),
Schmelzpunkt: 245—250° C.

| | | | | |
|---|---|---|---|---|
| Ber.: | (x 1 $H_2O$): | C 65,01 | H 6,69 | N 6,89 |
| Gef.: | | C 65,40 | H 6,83 | N 6,72 |

Analog Beispiel 11 wurde folgende Verbindung erhalten:

4-[(1-(5-Methoxy-2-piperidino-phenyl)-äthyl)-aminocarbonylmethyl]-benzoesäure-Natriumsalz-Monohydrat

Ausbeute: 17,5% der Theorie,
Schmelzpunkt: 212—215° C.

| | | | | |
|---|---|---|---|---|
| Ber.: | (x 1 $H_2O$): | C 63,28 | H 6,70 | N 6,42 |
| Gef.: | | C 63,20 | H 6,82 | N 6,51 |

Aus diesem Natriumsalz erhält man analog Beispiel 9 die entsprechende Säure als Monohydrat:

Schmelzpunkt: 187—189° C (Äthanol/Wasser).

| | | | | |
|---|---|---|---|---|
| Ber.: | (x 1 $H_2O$): | C 66,40 | H 7,29 | N 6,76 |
| Gef.: | | C 66,87 | H 6,97 | N 6,80 |

## Beispiel 12

4-[(1-(2-Piperidino-phenyl)-äthyl)-aminocarbonylmethyl]-benzoesäure-Natriumsalz x 0,6 $H_2O$

Man löst 8,4 g (0,0229 Mol) 4-[(1-(2-Piperidino-phenyl)-äthyl)-aminocarbonylmethyl]-benzoesäure-bei 60 bis 65°C in 80 ml Äthanol, fügt unter Rühren 22,9 ml 1N-Natronlauge zu und rührt noch 30 Minuten. Dann läßt man auf 20° C abkühlen, wobei ein Niederschlag ausfällt. Man kühlt auf 0° C ab, filtriert und wäscht den Niederschlag mit kaltem Äthanol und Äther. Den so erhaltenen Niederschlag vom Schmelzpunkt 250—251° C kristallisiert man aus Äthanol/Wasser (7/3) um.

Ausbeute: 7,2 g (78,6% der Theorie),
Schmelzpunkt: 253—255° C.

| | | | | |
|---|---|---|---|---|
| Ber.: | (x 0,6 $H_2O$): | C 66,18 | H 6,61 | N 7,02 |
| Gef.: | | C 66,10 | H 6,64 | N 7,13 |

## Beispiel 13

4-[(1-(2-Piperidino-phenyl)-äthyl)-aminocarbonylmethyl]-benzoesäure

Man erhitzt 100 mg (0,237 mMol) 4-[(1-(2-Piperidino)-phenyl)-äthyl)-aminocarbonylmethyl]-benzoesäure-tert.butylester in 5 ml Benzol zusammen mit einigen Kristallen p-Toluolsulfonsäure-hydraht einen halben Tag auf Rückfluß. Dann ist laut Dünnschichtchromatogramm kein Ausgangsprodukt mehr vorhanden, dafür ist nach $R_f$-Wert und Massenspektrum das gewünschte Produkt entstanden.

Schmelzpunkt: 163—165° C.

| | |
|---|---|
| Ber.: | m/e = 366 |
| Gef.: | m/e = 366 |

## Beispiel 14

4-[(1-(2-Piperidino-phenyl)-äthyl)-aminocarbonylmethyl]-benzoesäure

Man hydriert 0,46 g (1 mMol) 4-[(1-(2-Piperidino-phenyl)-äthyl)-aminocarbonylmethyl]-benzoesäure-benzylester in 20 ml Äthanol an 0,25 g Pallaidum/Kohle (10%ig) bei 50° C und 5 bar Wasserstoffdruck. Nach 5 Stunden filtriert man vom Katalysator über Celite ab und dampft im Vakuum ein. Den Eindampfrückstand kristallisiert man aus Äthanol/Wasser (8/2) um.

Ausbeute: 0,26 g (71% der Theorie),
Schmelzpunkt: 163—165° C.

| | | | |
|---|---|---|---|
| Ber.: | C 72,11 | H 7,15 | N 7,64 |
| Gef.: | C 72,30 | H 7,25 | N 7,81 |

## Beispiel 15

### 4-[(1-(5-Chlor-2-piperidino-phenyl)-äthyl)-aminocarbonylmethyl]-benzoesäure

In eine gerührte Lösung von 3,57 g (0,01 Mol) N-[1-(5-Chlor-2-piperidino-phenyl)-äthyl]-N-[phenacetyl]-amin in 16 ml Schwefelkohlenstoff werden bei 0 bis 5° C 2,54 g (0,02 Mol) Oxalylchlorid getropft und anschließend 2,67 g (0,02 Mol) Aluminiumchlorid eingetragen. Nach einer Stunde gibt man nochmals die gleichen Mengen Oxalylchlorid und Aluminiumchlorid zu und erhitzt anschließend 3 Stunden auf 50° C. Man kühlt ab, versetzt mit Eiswasser und Salzsäure und extrahiert mit Chloroform. Den organischen Extrakt trocknet und filtriert man und dampft ihn im Vakuum ein. Den Eindampfrückstand reinigt man durch Säulenchromatographie an Kieselgel (Chloroform/Methanol = 10/1).

Ausbeute: 0,60 g (15% der Theorie),
Schmelzpunkt: 213—214° C (Äther).

| | | | | |
|---|---|---|---|---|
| Ber.: | C 65,91 | H 6,29 | Cl 8,85 | N 6,99 |
| Gef.: | C 66,13 | H 6,05 | Cl 8,97 | N 7,25 |

## Beispiel 16

### N-[4-Acetyl-phenacetyl]-N-[1-(5-chlor-2-piperidino-phenyl)-äthyl]-amin

Zu 1,12 g (8,43 mMol) Aluminiumchlorid in 10 ml Methylenchlorid gibt man bei einer Innentemperatur von 0 bis 5° C die Lösung von 0,6 ml (8,43 mMol) Acetylchlorid in 5 ml Methylenchlorid. Anschließend tropft man unter Rühren bei 0 bis 5° C die Lösung von 1 g (2,81 mMol) N-[1-(5-Chlor-2-piperidino-phenyl)-äthyl]-N-[phenacetyl]-amin in 5 ml Methylenchlorid zu. Man rührt eine Stunde bei 3° C und 2 Tage bei 20° C. Man zersetzt unter Kühlen mit Eiswasser und Salzsäure, trennt die Methylenchloridphase ab und extrahiert mit Chloroform. Die vereinigten organischen Phasen trocknet man über Natriumsulfat, filtriert und dampft sie im Vakuum ein. Den Eindampfrückstand reinigt man durch Säulenchromatographie an Kieselgel (Toluol/Aceton = 4/1).

Ausbeute: 0,28 g (25% der Theorie),
Schmelzpunkt: 160—161° C.

| | | | | | |
|---|---|---|---|---|---|
| Ber.: | C 69,24 | H 6,82 | Cl 8,89 | N 7,02 | m/e = 398/400 |
| Gef.: | C 69,55 | H 6,99 | Cl 9,45 | N 6,85 | m/e = 398/400 |

## Beispiel 17

### 4-[(1-(5-Chlor-2-piperidino-phenyl)-äthyl)-aminocarbonylmethyl]-benzoesäure

Zu einer gerührten Natriumhypobromit-Lösung [hergestellt aus 1,84 g (0,046 Mol) Natriumhydroxid, gelöst in 9 ml Wasser, und 0,72 ml (0,014 Mol) Brom unter Eiskühlung] tropft man innerhalb 15 Minuten bei 35—40° C die Lösung von 1,23 g (0,0031 Mol) N-[4-Acetyl-phenacetyl]-N-[1-(5-chlor-2-piperidino-phenyl)-äthyl]-amin in 12 ml Dioxan. Nach 40 Minuten bei 35—40° C gibt man wäßrige Natriumhydrogensulfit-Lösung und Wasser zu und dampft das Gemisch im Vakuum ein. Den Rückstand löst man in Wasser, säuert unter Kühlung mit 2N-Salzsäure an und extrahiert mit Äther/Äthylacetat. Die organische Phase trocknet und filtriert man und dampft sie im Vakuum ein. Den Eindampfrückstand kristallisiert man aus Äther.

Ausbeute: 0,14 g (11% der Theorie),
Schmelzpunkt: 213—215° C.

| | | | | |
|---|---|---|---|---|
| Ber.: | C 65,91 | H 6,29 | Cl 8,85 | N 6,99 |
| Gef.: | C 65,78 | H 5,98 | Cl 8,95 | N 7,17 |

Analog Beispiel 17 wurde folgende Verbindung erhalten:

4-[(1-(2-Piperidino-phenyl)-äthyl)-aminocarbonylmethyl]-benzoesäure

Ausbeute: 15% der Theorie,
Schmelzpunkt: 170—171° C.

| | | | |
|---|---|---|---|
| Ber.: | C 72,11 | H 7,15 | N 7,64 |
| Gef.: | C 72,45 | H 7,01 | N 7,48 |

## Beispiel 18

### 4-[(1-(2-Piperidino-phenyl)-äthyl)-aminocarbonylmethyl]-benzaldehyd

Hergestellt aus 4-[(1-(2-Piperidino-phenyl)-äthyl)-aminocarbonylmethyl]-benzylalkohol durch Oxidation mit aktivem Mangandioxid in absolutem Aceton und anschließende Reinigung durch Säulenchromatographie an Kieselgel (Chloroform/Aceton = 20/1).

Ausbeute: 4% der Theorie,
Schmelzpunkt: 159° C.

| | | | |
|---|---|---|---|
| Ber.: | C 75,40 | H 7,48 | N 7,99 |
| Gef.: | C 75,05 | H 7,18 | N 7,67 |

## Beispiel 19

### 4-[(1-(2-Piperidino-phenyl)-äthyl)-aminocarbonylmethyl]-benzoesäure

Hergestellt aus 4-[(1-(2-Piperidino-phenyl)-äthyl)-aminocarbonylmethyl]-benzaldehyd durch Erwärmen mit Silberoxid in Gegenwart von 1 N-Natronlauge für 20 Minuten auf dem Dampfbad, anschließendes Ansäuern mit 2 N-Schwefelsäure bei pH = 5, Extraktion mit Äthylacetat und Reinigung durch Säulenchromatographie an Kieselgel (Toluol/Aceton = 1/1).

Ausbeute: 3% der Theorie,
Schmelzpunkt: 168—170° C.

| | |
|---|---|
| Ber.: | m/e = 366 |
| Gef.: | m/e = 366 |

## Beispiel 20

### 4-[(1-(2-Piperidino-phenyl)-äthyl)-aminocarbonylmethyl]-benzoesäure-äthylester

Man hydriert 5,5 g (0,014 Mol) 4-[(1-(2-Piperidino-phenyl)-äthenyl)-aminocarbonylmethyl]-benzoesäure-äthylester in 110 ml Äthanol an 1,5 g Palladium/Kohle (10%ig) bei 20° C und 5 bar Wasserstoff. Nach 30 Minuten filtriert man vom Katalysator über Celite ab und dampft das Filtrat im Vakuum ein.

Ausbeute: 4,7 g (85,5% der Theorie,
Schmelzpunkt: 152—154° C (Äthanol/Petroläther).

| | | | |
|---|---|---|---|
| Ber.: | C 73,07 | H 7,66 | N 7,10 |
| Gef.: | C 72,80 | H 7,63 | N 7,08 |

Analog Beispiel 20 wurde folgende Verbindung erhalten:

4-[(1-(2-Piperidino-phenyl)-propyl)-aminocarbonylmethyl]-
benzoesäure-äthylester

Ausbeute: 70,8% der Theorie,
Schmelzpunkt: 132—134° C (Äther).

| | | | |
|---|---|---|---|
| Ber.: | C 73,50 | H 7,90 | N 6,86 |
| Gef.: | C 73,71 | H 7,88 | N 6,77 |

Beispiel 21

4-[(1-(2-Piperidino-phenyl)-äthyl)-aminocarbonylmethyl]-benzoesäure

Man hydriert 100 mg (0,2744 mMol) 4-[(1-(2-Piperidino-phenyl)-äthenyl)-aminocarbonylmethyl]-benzoesäure in 5 ml absolutem Äthanol an 50 mg Palladium/Kohle (10%ig) bei 20° C und 1 bar Wasserstoff unter Schütteln. Nach 1,5 Stunden filtriert man und dampft im Vakuum ein.

Ausbeute: 91% der Theorie,
Schmelzpunkt: 170—171° C.

| | |
|---|---|
| Ber.: | m/e = 366 |
| Gef.: | m/e = 366 |

Beispiel 22

4-[(1-(2-Piperidino-phenyl)-äthyl)-aminocarbonylmethyl]-
benzoesäure-Semihydrat

Man hydriert 200 mg (0,5014 mMol) 4-[(1-(5-Chlor-2-piperidino-phenyl)-äthenyl)-aminocarbonylmethyl]-benzoesäure in 10 ml absolutem Äthanol an 100 mg Palladium/Kohle (10%ig) bei 50° C und 1 bar Wasserstoff unter Schütteln. Nach 1,5 Stunden filtriert man, gibt 5 ml Wasser zu und stellt mit 1N-Natronlauge auf pH=6 ein und dampft im Vakuum vom Äthanol ab. Es fällt ein farbloser Niederschlag aus, den man nach Abkühlen abfiltriert.

Ausbeute: 100 mg (53,1% der Theorie),
Schmelzpunkt: 135° C.

| | | | | |
|---|---|---|---|---|
| Ber. (x 0,5 $H_2O$): | C 70,36 | H 7,24 | N 7,46 | m/e = 366 |
| Gef.: | C 70,31 | H 7,44 | N 7,78 | m/e = 366 |

Beispiel 23

4-[(1-(2-Piperidino-phenyl)-äthyl)-aminocarbonylmethyl]-
benzoesäure-äthylester

Zu einem Gemisch von 2 g (9,74 mMol) 1-(2-Piperidino-phenyl)-äthanol und 4 g (21,1 mMol) 4-Cyanomethyl-benzoesäure-äthylester gibt man unter Rühren und Kühlen mit Eis 1,6 ml konz. Schwefelsäure in kleinen Tropfen so zu, daß die Innentemperatur 35 bis 40° C nicht übersteigt. Anschließend erhitzt man 2,5 Stunden im Bad von 80° C, setzt dann weitere 2 g (10,5 mMol) 4-Cyanomethyl-benzoesäure-äthylester und 0,8 ml konz. Schwefelsäure zu und erhitzt noch eine Stunde bei 80° C und 3 Stunden bei 100° C. Dann ist dünnschichtchromatographisch kein Ausgangs-Alkohol mehr zu erkennen.

Nach Abkühlen auf 20° C überschichtet man mit Äthylacetat und gibt unter Rühren und Kühlen Eiswasser zu. Man extrahiert mehrfach mit Äthylacetat, trocknet den organischen Extrakt über Natriumsulfat, filtriert und dampft im Vakuum ein. Den Eindampfrückstand reinigt man durch Säulenchromatographie an Kieselgel (Toluol/Aceton=10/1). Aus den Vorfraktionen isoliert man 0,5 g 2-Piperidino-styrol.

Ausbeute: 0,66 g (17% der Theorie),
Schmelzpunkt: 147—150° C (Äthanol).

| | | | |
|---|---|---|---|
| Ber.: | C 73,07 | H 7,66 | N 7,10 |
| Gef.: | C 73,26 | H 7,55 | N 6,90 |

Beispiel 24

4-[(1-(5-Chlor-2-piperidino-phenyl)-äthyl)-aminocarbonylmethyl]-benzoesäure

Zu einer gerührten Lösung von 1 g (5,55 mMol) 4-Carboxy-phenyl-essigsäure und von 1,32 g (5,55 mMol) 1-(5-Chlor-2-piperidino-phenyl)-äthylamin in 10 ml absolutem Pyridin tropft man 0,4 ml (5,55 mMol) Thionylchlorid, wobei die Innentemperatur von 20° C auf 35° C ansteigt. Das tiefbraune Reaktionsgemisch rührt man noch 3 Stunden bei 20° C und dampft es dann im Vakuum ein. Den Eindampfrückstand verteilt man zwischen Wasser (bei pH=3 nach Zusatz von 2N-Salzsäure) und Chloroform. Den organischen Extrakt trocknet und filtriert man und dampft ihn im Vakuum ein. Den Eindampfrückstand reinigt man durch Säulenchromatographie an Kieselgel (Chloroform/Methanol=10/1).

Ausbeute: 1,06 g (48% der Theorie),
Schmelzpunkt: 212—214° C (Äther).

| | | | | |
|---|---|---|---|---|
| Ber.: | C 65,91 | H 6,29 | Cl 8,85 | N 6,99 |
| Gef.: | C 65,79 | H 6,01 | Cl 8,69 | N 6,87 |

Analog Beispiel 24 wurden folgende Verbindungen erhalten:

4-[(1-(2-Piperidino-phenyl)-äthyl)-aminocarbonylmethyl]-benzoesäure

Ausbeute: 52% der Theorie,
Schmelzpunkt: 169—171° C.

| | | | |
|---|---|---|---|
| Ber.: | C 72,11 | H 7,15 | N 7,64 |
| Gef.: | C 71,84 | H 6,87 | N 7,72 |

4-[(1-(2-(4-Oxo-piperidino)-phenyl)-äthyl)-aminocarbonylmethyl]-benzoesäure

Ausbeute: 32% der Theorie,
Schmelzpunkt: 177—180° C (Zers.) (Aceton/Petroläther).

| | | | |
|---|---|---|---|
| Ber.: | C 69,46 | H 6,36 | N 7,36 |
| Gef.: | C 69,62 | H 6,41 | N 7,50 |

4-[(1-(2-(4-Hydroxy-piperidino)-phenyl)-äthyl)-aminocarbonylmethyl]-benzoesäure x 0,66 $H_2O$

Ausbeute: 23,5% der Theorie,
Schmelzpunkt: 176—179° C (Zers.) (Aceton/Petroläther).

| | | | |
|---|---|---|---|
| Ber. (x 0,66 $H_2O$): | C 66,97 | H 6,81 | N 7,10 |
| Gef.: | C 67,12 | H 6,78 | N 7,26 |

4-[(1-(2-Piperidino-phenyl)-äthyl)-aminocarbonylmethyl]-benzonitril

Hergestellt mit 4-Cyano-phenylessigsäure.

Ausbeute: 51% der Theorie,
Schmelzpunkt: 155—157° C (Äthylacetat).

| | | | |
|---|---|---|---|
| Ber.: | C 76,05 | H 7,25 | N 12,09 |
| Gef.: | C 76,41 | H 7,10 | N 12,20 |

Beispiel 25

4-[(1-(2-Piperidino-phenyl)-äthyl)-aminocarbonylmethyl]-benzylalkohol

Hergestellt aus 4-[(1-(2-Piperidino-phenyl)-äthyl)-aminocarbonylmethyl]-benzoesäure-äthylester durch Lithiumaluminiumhydrid-Reduktion in Tetrahydrofuran.

Ausbeute: 39% der Theorie,
Schmelzpunkt: 104—106° C.

| | | | |
|---|---|---|---|
| Ber.: | C 74,96 | H 8,00 | N 7,94 |
| Gef.: | C 74,80 | H 7,80 | N 7,80 |

Beispiel 26

4-[(1-(2-Piperidino-phenyl)-äthyl)-aminocarbonylmethyl]-benzylmalonsäure-diäthylester

Zu einer Lösung von Natrium-malonsäure-diäthylester [bereitet aus 0,7 g (30 mMol) Natrium in 25 ml absolutem Äthanol und 4,8 g (30 mMol) Malonsäure-diäthylester] tropft man eine Lösung von 3,7 g (10 mMol) 4-[(1-(2-Piperidino-phenyl)-äthyl)-aminocarbonylmethyl]-benzoylchlorid [Schmelzpunkt: 123—125° C; hergestellt aus dem in Beispiel 25 beschriebenen Alkohol mittels Thionylchlorid in Chloroform] in 25 ml absolutem Äthanol. Man setzt eine katalytische Menge Kaliumjodid zu und erhitzt 16 Stunden auf Rückfluß. Dann engt man im Vakuum ein, stellt mit Salzsäure neutral und extrahiert mit Methylenchlorid. Den organischen Extrakt trocknet man über Natriumsulfat, filtriert und dampft ihn im Vakuum ein. Den Eindampfrückstand reinigt man durch Säulenchromatographie an Kieselgel (Toluol/Aceton = 6/1).

Ausbeute: 3,0 g (60% der Theorie),
Schmelzpunkt: < 20° C.

| | |
|---|---|
| Ber.: | m/e = 494 |
| Gef.: | m/e = 494 |

Beispiel 27

3-/4-/(1-(2-Piperidino-phenyl)-äthyl)-aminocarbonylmethyl/-phenyl/-propionsäure

Zu einer Lösung von 0,85 g (1,7 mMol) 4-/(1-(2-Piperidino-phenyl)-äthyl)-aminocarbonylmethyl/-benzylmalonsäure-diäthylester in 18 ml Äthanol gibt man 5 ml 1N-Natronlauge und rührt 2 Stunden bei 50° C. Dann engt man im Vakuum ein, setzt Wasser und 5 ml 1N-Salzsäure zu. Den gebildeten Niederschlag filtriert man ab, trocknet ihn im Vakuum und erhitzt ihn dann 30 Minuten auf 120° C, wobei sich Kohlendioxid abspaltet. Anschließend reinigt man durch Säulenchromatographie an Kieselgel (Chloroform/Methanol = 20/1).

Ausbeute: 0,15 g (22,4% der Theorie),
Schmelzpunkt: 68—70° C.

| | | | | |
|---|---|---|---|---|
| Ber.: | C 73,06 | H 7,67 | N 7,10 | m/e = 394 |
| Gef.: | C 72,64 | H 7,42 | N 6,81 | m/e = 394 |

Beispiel 28

4-[(1-(2-Piperidino-phenyl)-äthyl)-aminocarbonylmethyl]-benzaldehyd

Hergestellt durch Erhitzen von rohem $N^1$-[4-[(1-(2-Piperidino-phenyl)-äthyl)-aminocarbonylmethyl]-benzoyl]-$N^2$-tosyl-hydrazin [hergestellt aus 4-[(1-(2-Piperidino-phenyl)-äthyl)-aminocarbonylmethyl]-benzoesäure und Tosyl-hydrazin mit Carbonyldiimidazol in Tetrahydrofuran] mit wasserfreiem Natriumkarbonat bei 160—170° C in Äthylenglykol.

Ausbeute: 10% der Theorie,
Schmelzpunkt: 159° C.

| | | | |
|---|---|---|---|
| Ber.: | C 75,40 | H 7,48 | N 7,99 |
| Gef.: | C 74,99 | H 7,24 | N 7,60 |

Beispiel 29

4-[(1-(2-Piperidino-phenyl)-äthyl)-aminocarbonylmethyl]-benzoesäure

Man hydriert 0,50 g (1,247 mMol) 4-[(1-(5-Chlor-2-piperidino-phenyl)-äthyl)-aminocarbonylmethyl]-benzoesäure in 20 ml absolutem Äthanol an 0,25 g Palladium/Kohle (10%ig) bei 50° C und 5 bar Wasserstoffdruck. Nach 2 Stunden filtriert man vom Katalysator über Celite ab und dampft im Vakuum ein. Den Eindampfrückstand verteilt man bei pH = 6 zwischen Wasser und Äthylacetat. Den organischen Extrakt wäscht man mit Wasser, trocknet und filtriert ihn und dampft im Vakuum ein.

Ausbeute: 0,31 g (67% der Theorie),
Schmelzpunkt: 170—172° C (Äther).

| | | | |
|---|---|---|---|
| Ber.: | C 72,11 | H 7,15 | N 7,64 |
| Gef.: | C 71,76 | H 6,98 | N 7,51 |

Analog Beispiel 29 wurden folgende Verbindungen hergestellt:

4-[(2-(2-Piperidino-phenyl)-2-propyl)-aminocarbonylmethyl]-benzoesäure

Ausbeute: 68,5% der Theorie,
Schmelzpunkt: 213—215° C.

| | | | |
|---|---|---|---|
| Ber.: | C 72,61 | H 7,42 | N 7,36 |
| Gef.: | C 72,43 | H 7,25 | N 7,40 |

4-[(1-(2-Dimethylamino-phenyl)-äthyl)-aminocarbonylmethyl]-benzoesäure

Ausbeute: 53,3% der Theorie,
Schmelzpunkt: 165—168° C (Aceton/Petroläther).

| | | | |
|---|---|---|---|
| Ber.: | C 69,92 | H 6,79 | N 8,59 |
| Gef.: | C 69,88 | H 6,83 | N 8,49 |

4-[(2-Pyrrolidino-benzyl)-aminocarbonylmethyl]-benzoesäure

Ausbeute: 55% der Theorie,
Schmelzpunkt: 212—215° C (Methanol).

| | | | |
|---|---|---|---|
| Ber.: | C 70,99 | H 6,55 | N 8,28 |
| Gef.: | C 70,97 | H 6,91 | N 8,15 |

4-[(1-(2-Pyrrolidino-phenyl)-äthyl)-aminocarbonylmethyl]-benzoesäure

Ausbeute: 25% der Theorie,
Schmelzpunkt: 155—157° C (Aceton/Äther).

| | | | |
|---|---|---|---|
| Ber.: | C 71,57 | H 6,86 | N 7,95 |
| Gef.: | C 71,22 | H 6,75 | N 8,42 |

4-[(2-Piperidino-benzyl)-aminocarbonylmethyl]-benzoesäure

Ausbeute: 60,4% der Theorie,
Schmelzpunkt: 175—177° C (Aceton).

| | | | |
|---|---|---|---|
| Ber.: | C 71,57 | H 6,86 | N 7,95 |
| Gef.: | C 71,48 | H 7,00 | N 8,09 |

4-[(2-(2-Piperidino-phenyl)-äthyl)-aminocarbonylmethyl]-benzoesäure

Ausbeute: 60,4% der Theorie,
Schmelzpunkt: 164—166° C (Äthylacetat).

| | | | |
|---|---|---|---|
| Ber.: | C 72,11 | H 7,15 | N 7,64 |
| Gef.: | C 72,35 | H 7,18 | N 7,76 |

4-[(1-(2-(2-Methyl-piperidino)-phenyl)-äthyl)-aminocarbonylmethyl]-benzoesäure

Ausbeute: 90,9% der Theorie,
Schmelzpunkt: 171—173° C (Petroläther/Aceton).

| | | | |
|---|---|---|---|
| Ber.: | C 72,61 | H 7,42 | N 7,36 |
| Gef.: | C 72,30 | H 7,39 | N 7,43 |

4-[(1-(2-(3-Methyl-piperidino)-phenyl)-äthyl)-aminocarbonylmethyl]-benzoesäure

Ausbeute: 86,3% der Theorie,
Schmelzpunkt: 170—173° C (Petroläther/Aceton).

| | | | |
|---|---|---|---|
| Ber.: | C 72,61 | H 7,42 | N 7,36 |
| Gef.: | C 72,20 | H 7,28 | N 7,12 |

4-[(1-(2-Dipropylamino-phenyl)-äthyl)-aminocarbonylmethyl]-benzoesäure

Ausbeute: 51,1% der Theorie,
Schmelzpunkt: 175—178° C (Äthylacetat).

| | | | |
|---|---|---|---|
| Ber.: | C 72,22 | H 7,91 | N 7,32 |
| Gef.: | C 72,10 | H 8,05 | N 7,69 |

4-[(1-(2-Piperidino-phenyl)-2-methyl-propyl)-aminocarbonylmethyl]-benzoesäure

Ausbeute: 68% der Theorie,
Schmelzpunkt: 215—217° C (Aceton).

| | | | |
|---|---|---|---|
| Ber.: | C 73,06 | H 7,67 | N 7,10 |
| Gef.: | C 73,10 | H 7,55 | N 6,99 |

4-[(1-(2-Piperidino-phenyl)-äthyl)-aminocarbonylmethyl]-benzoesäure-methylester

Hergestellt aus 4-[(1-(5-Chlor-2-piperidino-phenyl)-äthyl)-aminocarbonylmethyl]-benzoesäure-methylester

Ausbeute: 37,2% der Theorie,
Schmelzpunkt: 145—147° C.

| | | | |
|---|---|---|---|
| Ber.: | C 72,61 | H 7,42 | N 7,36 |
| Gef.: | C 72,47 | H 7,30 | N 7,56 |

4-[(2-Piperidino-anilino)-carbonylmethyl]-benzoesäure-methylester

Hergestellt aus 4-[(5-Chlor-2-piperidino-anilino)-carbonyl-methyl]-benzoesäure-methylester.

Ausbeute: 60% der Theorie,
Schmelzpunkt: 85—86° C (Toluol/Petroläther).

| | | | |
|---|---|---|---|
| Ber.: | C 71,57 | H 6,86 | N 7,96 |
| Gef.: | C 71,48 | H 6,92 | N 8,39 |

N-Phenacetyl-N-[1-(2-piperidino-phenyl)-äthyl]-amin

Hergestellt aus N-[1-(5-Chlor-2-piperidino-phenyl)-äthyl]-N-phenacetyl-amin.

Ausbeute: 54,6% der Theorie,
Schmelzpunkt: 120—121° C (Petroläther/Aceton).

| | | | |
|---|---|---|---|
| Ber.: | C 78,22 | H 8,13 | N 8,69 |
| Gef.: | C 77,90 | H 8,24 | N 8,75 |

## Beispiel 30

4-[(1-(5-Amino-2-piperidino-phenyl)-äthyl)-aminocarbonylmethyl]-benzoesäure-methylester

Man hydriert 2,0 g (0,0047 Mol) 4-[(1-(5-Nitro-2-piperidino-phenyl)-äthyl)-aminocarbonylmethyl]-benzoesäure-methylester in 20 ml Dimethylformamid an 0,2 g Palladium/Kohle (10%ig) in einer Parr-Apparatur bei 20° C und 1 bar Wasserstoffdruck. Nach Ende der Wasserstoffaufnahme (2 Stunden) filtriert man vom Katalysator über Celite ab und dampft im Vakuum zur Trockne ein.

Ausbeute: 1,8 g (95% der Theorie),
Schmelzpunkt: 140—142° C (Toluol).

Analog Beispiel 30 wurden folgende Verbindungen hergestellt:

4-[(1-(5-Amino-2-piperidino-phenyl)-äthyl)-aminocarbonylmethyl]-benzoesäure-äthylester

Ausbeute: 97,8% der Theorie,
Schmelzpunkt: 148—149,5° C (Cyclohexan).

| | | | |
|---|---|---|---|
| Ber.: | C 70,39 | H 7,63 | N 10,26 |
| Gef.: | C 70,20 | H 7,67 | N 9,60 |

4-[(1-(5-Amino-2-piperidino-phenyl)-äthyl)-aminocarbonylmethyl]-benzoesäure

Hergestellt aus 4-[(1-(5-Nitro-2-piperidino-phenyl)-äthyl)-aminocarbonylmethyl]-benzoesäure.

Ausbeute: 85,7% der Theorie,
Schmelzpunkt: 223—225° C (Äther).

| | | | |
|---|---|---|---|
| Ber.: | C 69,27 | H 7,13 | N 11,02 |
| Gef.: | C 69,18 | H 7,04 | N 11,35 |

N-[4-Amino-phenacetyl]-N-[1-(2-piperidino-phenyl)-äthyl]-amindihydrochlorid-Semihydrat

Hergestellt aus N-[4-Nitro-phenacetyl]-N-[1-(2-piperidino-phenyl)-äthyl]-amin. Überführung der rohen Amino-Verbindung ins Dihydrochlorid in Äthanol mittels ätherischer Salzsäure.

Ausbeute: 17,5% der Theorie,
Schmelzpunkt: 238° C (Zers.).

| | | | | |
|---|---|---|---|---|
| Ber.: | (× 2 HCl × 0,5 $H_2O$): | C 60,12 | H 7,21 | Cl 16,91 |
| Gef.: | | C 60,52 | H 7,52 | Cl 17,05 |

## Beispiel 31

### 4-[(1-(5-Brom-2-piperidino-phenyl)-äthyl)-aminocarbonylmethyl]-benzoesäure

Zu 0,40 g (1,05 mMol) 4-[(1-(5-Amino-2-piperidino-phenyl)-äthyl)-aminocarbonylmethyl]-benzoesäure in 2 ml halbkonzentrierter wäßriger Bromwasserstoffsäure tropft man bei einer Innentemperatur von 0 bis 5°C die Lösung von 0,072 g (1,05 mMol) Natriumnitrit in 0,5 ml Wasser. Die so erhaltene Diazoniumsalz-Lösung tropft man dann zu 0,196 g Kupfer(I)bromid in 2 ml 48%iger Bromwasserstoffsäure, wobei heftige Gasentwicklung erfolgt. Man rührt noch 1,5 Stunden bei einer Innentemperatur von 45—50°C nach, kühlt ab und stellt mit 4N-Natronlauge auf pH 4 ein. Man extrahiert mit warmem Äthylacetat, wäscht den Extrakt mit Wasser, trocknet und filtriert ihn. Nach Eindampfen im Vakuum reinigt man den erhaltenen Rückstand durch Säulenchromatographie an Kieselgel (Chloroform/Methanol = 7/1).

Ausbeute: 0,08 g (17% der Theorie),
Schmelzpunkt: 212—213°C (Äthylacetat/Petroläther.

| | | | | |
|---|---|---|---|---|
| Ber.: | C 59,32 | H 5,66 | Br 17,94 | N 6,29 |
| Gef.: | C 59,30 | H 5,71 | Br 17,85 | N 6,48 |

Analog Beispiel 31 wurde folgende Verbindung hergestellt:

### 4-[(1-(5-Chlor-2-piperidino-phenyl)-äthyl)-aminocarbonylmethyl]-benzoesäure

Hergestellt durch Diazotierung von 4-[(1-(5-Amino-2-piperidino-phenyl)-äthyl)-aminocarbonylmethyl]-benzoesäure in konz. HCl und Sandmeyer-Reaktion mit Kupfer(I)chlorid.

Ausbeute: 25,2% der Theorie,
Schmelzpunkt: 213—215°C.

| | | | | |
|---|---|---|---|---|
| Ber.: | C 65,91 | H 6,29 | Cl 8,85 | N 6,99 |
| Gef.: | C 66,20 | H 6,31 | Cl 8,87 | N 6,82 |

Falls man die Reaktion in Salzsäure ohne Kupfer(I)chlorid ausführt, beträgt die Ausbeute 19% der Theorie. Daneben entstehen noch 9% der entsprechenden 5-Hydroxy-Verbindung.

## Beispiel 32

### 4-[(1-(5-Jod-2-piperidino-phenyl)-äthyl)-aminocarbonylmethyl]-benzoesäure-äthylester

Zu 1,0 g (2,44 mMol) 4-[(1-(5-Amino-2-piperidino-phenyl)-äthyl)-aminocarbonylmethyl]-benzoesäure-äthylester in 1,9 ml halbkonzentrierter Jodwasserstoffsäure tropft man unter Rühren langsam bei 0 bis 5°C die Lösung von 0,17 g (2,44 mMol) Natriumnitrit in 0,52 ml Wasser und läßt dann innerhalb einer Stunde auf 20°C erwärmen. Anschließend erhitzt man 2 Stunden bei 100°C. Nach Abkühlen extrahiert man mit Äthylacetat, wäscht die organische Phase mit verdünnter Natriumbikarbonat-Lösung und dann mit Wasser, trocknet über Natriumsulfat, filtriert und dampft im Vakuum ein. Den Eindampfrückstand reinigt man durch Säulenchromatographie an Kieselgel (Toluol/Aceton = 5/1).

Ausbeute: 0,011 g (0,93% der Theorie),
Schmelzpunkt: 145—147°C (Äther).

| | | | | |
|---|---|---|---|---|
| Ber.: | C 55,39 | H 5,62 | N 5,38 | m/e = 520 |
| Gef.: | C 55,95 | H 5,53 | N 5,05 | m/e = 520 |

Beispiel 33

4-[(1-(5-Cyano-2-piperidino-phenyl)-äthyl)-aminocarbonylmethyl]-benzoesäure-äthylester

Zu 2,0 g (4,88 mMol) 4-[(1-(5-Amino-2-piperidino-phenyl)-äthyl)-aminocarbonylmethyl]-benzoesäure-äthylester in 4,0 ml Wasser +3,5 ml konzentrierter Salzsäure tropft man unter Rühren bei —5 bis 0°C die Lösung von 0,34 g (4,88 mMol) Natriumnitrit in 2,3 ml Wasser. Man rührt 15 Minuten nach und neutralisiert dann mit 1,1 g Calciumkarbonat. Die so erhaltene Suspension spült man mit Hilfe von 2 × 15 ml Wasser in eine 0°C kalte Lösung, die man aus 0,568 g (6,34 mMol) Kupfer(I)cyanid, 1,24 g (19 mMol) Kaliumcyanid und 5,8 ml Wasser bereitet hat. Dabei fällt sofort ein rotgefärbter Niederschlag aus. Unter Rühren erwärmt man das Reaktionsgemisch 30 Minuten bei einer Innentemperatur von 45°C, dann 30 Minuten bei 70°C und 60 Minuten bei 95°C. Im Dünnschichtchromatogramm fehlt jetzt der rotgefärbte Fleck, der vorher vorhanden war. Man kühlt auf 20°C ab und extrahiert mit Äthylacetat. Den organischen Extrakt trocknet man über Natriumsulfat, filtriert und dampft ihn im Vakuum ein. Den Eindampfrückstand reinigt man durch zwei Säulenchromatographien an Kieselgel ((a) Toluol/Aceton = 10/1, (b) Methylenchlorid/Acetonitril/Eisessig = 10/1/0,05). Neben den entsprechenden 5-Cl- und 5-H-Verbindungen erhält man die gewünschte 5-Cyano-Verbindung.

Ausbeute: 0,186 g (9% der Theorie),
Schmelzpunkt: 165—167°C (Äther).

| | | | | |
|---|---|---|---|---|
| Ber.: | C 71,58 | H 6,97 | N 10,02 | m/e = 419 |
| Gef.: | C 71,64 | H 6,94 | N 9,72 | m/e = 419 |

Beispiel 34

4-[(1-(5-Aminosulfonyl-2-piperidino-phenyl)-äthyl)-aminocarbonylmethyl]-benzoesäure-äthylester

a) Zu einer Suspension von 2,0 g (4,88 mMol) 4-[(1-(5-Amino-2-piperidino-phenyl)-aminocarbonylmethyl]-benzoesäure-äthylester in 2,02 ml halbkonzentrierter Salzsäure tropft man unter Rühren bei 4 bis 6°C die Lösung von 0,37 g (5,36 mMol) Natriumnitrit in 0,7 ml Wasser. Anschließend setzt man 0,37 g (3,89 mMol) Magnesiumchlorid zu. Die so erhaltene Mischung tropft man anschließend bei 30°C zu einer Lösung, die man aus 4,9 ml Eisessig (gesättigt mit Schwefeldioxid) und 0,27 g Kupfer(II)chlorid-dihydrat bereitet hat. Dabei steigt die Innentemperatur auf 40°C, und es entwickelt sich lebhaft Stickstoff. Man rührt noch 15 Minuten im Bad von 50°C, setzt dann 7,5 ml Wasser zu und extrahiert mit Chloroform. Den organischen Extrakt trocknet man über Natriumsulfat, filtriert und dampft im Vakuum ein. Der viskose, rotbraune Eindampfrückstand (2,7 g; noch Chloroform-haltig) enthält neben der entsprechenden 5-Chlor-Verbindung den gewünschten 4-[(1-(5-Chlorsulfonyl-2-piperidino-phenyl)-äthyl)-aminocarbonylmethyl]-benzoesäure-äthylester.

b) Die Lösung des unter a) erhaltenen Eindampfrückstandes in 10 ml Chloroform tropft man bei 2°C unter Rühren zu 50 ml konz. Ammoniak. 30 Minuten später setzt man gesättigte Kochsalz-Lösung zu, um Phasentrennung zu erreichen. Man extrahiert nochmals mit Chloroform, trocknet und filtriert den organischen Extrakt und dampft ihn im Vakuum ein. Den Eindampfrückstand reinigt man durch Säulenchromatographie an Kieselgel (Chloroform/Methanol = 10/1). Neben 55% der entsprechenden 5-Chlor-Verbindung erhält man die gewünschte 5-Aminosulfonyl-Verbindung als Schaum.

Ausbeute: 33% der Theorie.

| | |
|---|---|
| Ber.: | m/e = 473 |
| Gef.: | m/e = 473 |

Beispiel 35

4-[(1-(5-Dimethylamino-2-piperidino-phenyl)-äthyl)-aminocarbonylmethyl]-benzoesäure

Zu einer gerührten Lösung von 0,20 g (0,5242 mMol) 4-[(1-(5-Amino-2-piperidino-phenyl)-äthyl)-aminocarbonylmethyl]-benzoesäure und 0,45 ml 40%igem Formalin in 2 ml Acetonitril +1 ml absolutem Dimethylformamid gibt man bei 20°C 0,10 g (1,589 mMol) Natrium-cyanborhydrid und nach 2 Minuten 0,056 ml Eisessig. Nach 1,5 Stunden dampfte man im Vakuum ein. Den Eindampfrückstand löst man in Wasser unter Zusatz von Salzsäure bei pH 2—3. Nach mehrfacher Extraktion mit Chloroform stellt man die wäßrige Phase mit gesättigter Natriumhydrogencarbonat-Lösung auf pH 6 bis 7 ein und extrahiert

mehrfach mit Chloroform. Diesen organischen Extrakt trocknet und filtriert man. Nach Eindampfen im Vakuum kristallisiert man den Eindampfrückstand aus Isopropanol um. Die farblosen Kristalle wäscht man mit absolutem Äther.

Ausbeute: 0,09 g (42,8% der Theorie),
Schmelzpunkt: 185° C (Zers. ab 175° C).

| | | | |
|---|---|---|---|
| Ber.: | C 70,39 | H 7,63 | N 10,26 |
| Gef.: | C 70,10 | H 7,63 | N 10,47 |

## Beispiel 36

4-[(1-(5-Acetylamino-2-piperidino-phenyl)-äthyl)-aminocarbonylmethyl]-benzoesäure

Man rührt 0,10 g (0,262 mMol) 4-[(1-(5-Amino-2-piperidino-phenyl)-äthyl)-aminocarbonylmethyl]-benzoesäure in 1 ml Acetanhydrid 6 Stunden bei 20° C, dampfte dann im Vakuum ein, destilliert mehrfach mit Toluol ab und kristallisiert den Eindampfrückstand aus Äther.

Ausbeute: 0,08 g (72,7% der Theorie),
Schmelzpunkt: 241—243° C.

| | | | |
|---|---|---|---|
| Ber.: | C 68,07 | H 6,90 | N 9,92 |
| Gef.: | C 67,53 | H 6,83 | N 9,72 |

## Beispiel 37

4-[(1-(5-Benzoylamino-2-piperidino-phenyl)-äthyl)-aminocarbonylmethyl]-benzoesäure

Zur Lösung von 1 g (2,62 mMol) 4-[(1-(5-Amino-2-piperidino-phenyl)-äthyl)-aminocarbonylmethyl]-benzoesäure und von 0,37 ml (2,62 mMol) Triäthylamin in 10 ml wasserfreiem Dimethylformamid tropft man 0,30 ml (2,62 mMol) Benzoylchlorid. Man rührt 2 Stunden bei 20—30° C, dampft im Vakuum ein und verteilt zwischen Wasser und Äthylacetat. Die organische Phase trocknet und filtriert man und dampft sie im Vakuum ein. Den Eindampfrückstand (1,12 g) kristallisiert man aus Äthanol unter Aktivkohle-Zusatz um.

Ausbeute: 0,5 g (39,4% der Theorie),
Schmelzpunkt: 225—227° C.

| | | | |
|---|---|---|---|
| Ber.: | C 71,73 | H 6,43 | N 8,65 |
| Gef.: | C 71,70 | H 6,50 | N 8,66 |

Analog Beispiel 37 wurde folgende Verbindung hergestellt:

4-[(1-(5-Äthoxycarbonylamino-2-piperidino-phenyl)-äthyl)-aminocarbonylmethyl]-benzoesäure

Ausbeute: 34,2% der Theorie,
Schmelzpunkt: 220° C (Zers.).

| | | | |
|---|---|---|---|
| Ber.: | C 66,21 | H 6,89 | N 9,26 |
| Gef.: | C 65,97 | H 6,83 | N 9,57 |

## Beispiel 38

4-[(1-(5-Methylsulfonylamino-2-piperidino-phenyl)-äthyl)-aminocarbonylmethyl]-benzoesäure

Zur Lösung von 0,10 g (0,262 mMol) 4-[(1-(5-Amino-2-piperidino-phenyl)-äthyl)-aminocarbonylmethyl]-benzoesäure in 1 ml wasserfreiem Pyridin gibt man 0,20 ml (0,262 mMol) Mesylchlorid. Nach Abklingen der exothermen Reaktion läßt man noch 4 Stunden bei 20° C stehen. Dann dampft man im Vakuum ein. Den Eindampfrückstand verteilt man bei pH 2—3 zwischen Wasser und Chloroform. Die

saure wäßrige Phase stellt man mit Natriumhydrogencarbonat-Lösung auf pH 6 bis 7 ein und extrahiert mit Chloroform. Diesen Chloroform-Extrakt trocknet und filtriert man. Den durch Eindampfen im Vakuum erhaltenen Rückstand reinigt man durch Säulenchromatographie an Kieselgel (Chloroform/Methanol = 4/1).

Ausbeute: 0,03 g (25% der Theorie),
Schmelzpunkt: 210—220° C (Zers) (Äther).

| | | |
|---|---|---|
| Ber.: | Molpeak | m/e = 459 |
| Gef.: | | m/e = 459 |

Beispiel 39

4-[(1-(5-Acetoxy-2-piperidino-phenyl)-äthyl)-aminocarbonylmethyl]-benzoesäure

Man erhitzt 0,35 g (0,915 mMol) 4-[(1-(5-Hydroxy-2-piperidino-phenyl)-äthyl)-aminocarbonylmethyl]-benzoesäure zusammen mit 0,103 ml (1,098 mMol) Acetanhydrid auf dem Dampfbad und läßt noch 4 Tage bei 20° C stehen. Man kristallisiert aus Methanol um.

Ausbeute: 0,16 g (41,2% der Theorie),
Schmelzpunkt: 218—221° C.

| | | | |
|---|---|---|---|
| Ber.: | C 67,91 | H 6,65 | N 6,60 |
| Gef.: | C 67,70 | H 6,95 | N 6,55 |

Beispiel 40

4-[(1-(5-Methoxy-2-piperidino-phenyl)-äthyl)-aminocarbonylmethyl]-benzoesäure-methylester

Eine Lösung von 60 mg (0,157 mMol) 4-[(1-(5-Hydroxy-2-piperidino-phenyl)-äthyl)-aminocarbonylmethyl]-benzoesäure in 1 ml Methanol (+1 Tropfen Wasser) wird tropfenweise mit einer ätherischen Diazomethan-Lösung versetzt, bis keine Gasentwicklung mehr erfolgt. Zur Zerstörung überschüssigen Diazomethans fügt man 2N-Essigsäure zu. Dann dampft man im Vakuum ein. Den Eindampfrückstand verteilt man zwischen Toluol/Äther und verdünnter Natronlauge. Nach Trocknen, Filtrieren und Eindampfen der organischen Phase im Vakuum reinigt man den Eindampfrückstand durch Säulenchromatographie an Kieselgel (Chloroform/Methanol = 5/1).

Ausbeute: 27% der Theorie,
Schmelzpunkt: Schaum.

| | | |
|---|---|---|
| Ber.: | Molpeak | m/e = 410 |
| Gef.: | | m/e = 410 |

Beispiel 41

4-[(1-(5-Benzyloxy-2-piperidino-phenyl)-äthyl)-aminocarbonylmethyl]-benzoesäure-äthylester

Zu einer Suspension von 1,353 mMol Natriumhydrid (32,5 mg einer 50%igen Suspension in Öl) in 2 ml wasserfreiem Dimethylformamid tropft man zügig die Lösung von 0,50 g (1,218 mMol) 4-[(1-(5-Hydroxy-2-piperidino-phenyl)-äthyl)-aminocarbonylmethyl]-benzoesäure-äthylester in 10 ml wasserfreiem Dimethylformamid. Nach 1,5 Stunden Rühren bei 20° C tropft man 0,16 ml (1,353 mMol) Benzylbromid, gelöst in 2,3 ml wasserfreiem Dimethylformamid zu und rührt 16 Stunden bei 20° C. Nach Eindampfen im Vakuum verteilt man zwischen Wasser und Äther. Den organischen Extrakt trocknet und filtriert man und dampft ihn im Vakuum ein. Den Eindampfrückstand reinigt man durch Säulenchromatographie an Kieselgel (Toluol/Aceton = 10/1).

Ausbeute: 0,34 g (55,7% der Theorie),
Schmelzpunkt: 155—157° C (Äther).

| | | | |
|---|---|---|---|
| Ber.: | C 74,37 | H 7,25 | N 5,60 |
| Gef.: | C 74,11 | H 7,41 | N 5,39 |

Beispiel 42

4-[(1-(5-Aminocarbonyl-2-piperidino-phenyl)-äthyl)-aminocarbonylmethyl]-benzoesäure-äthylester

Man rührt 3,8 g (9,06 mMol) 4-[(1-(5-Cyano-2-piperidino-phenyl)-äthyl)-aminocarbonylmethyl]-benzoesäure-äthylester und 38 g Polyphosphorsäure 2,5 Stunden bei 80—90° C. Unter Kühlung mit Eis gibt man vorsichtig Wasser zu. Man überschichtet mit Äthylacetat und stellt mit konz. Ammoniak alkalisch. Die organische Phase wäscht man mit Wasser, trocknet sie und dampft sie im Vakuum ein. Den Eindampfrückstand reinigt man durch Säulenchromatographie an Kieselgel (Chloroform/Methanol = 20/1).

Ausbeute: 1 g (25,2% der Theorie),
Schmelzpunkt: 188—189° C (Äthanol).

| | | | |
|---|---|---|---|
| Ber.: | C 68,63 | H 7,14 | N 9,60 |
| Gef.: | C 68,42 | H 6,95 | N 9,46 |

Beispiel 43

4-[(1-(5-Äthoxycarbonyl-2-piperidino-phenyl)-äthyl)-aminocarbonylmethyl]-benzoesäure-äthylester

In eine auf Rückfluß erhitzte Lösung von 1,1 g (2,62 mMol) 4-[(1-(5-Cyano-2-piperidino-phenyl)-äthyl)-aminocarbonylmethyl]-benzoesäure-äthylester in 22 ml absolutem Äthanol leitet man trokkenen Chlorwasserstoff ein, bis nach 4 Stunden kein Nitril mehr nachweisbar ist. Man dampft im Vakuum ein, versetzt mit Wasser und Äther, und stellt mit Natriumhydrogencarbonat-Lösung alkalisch. Die abgetrennte Ätherphase schüttelt man einmal mit Wasser, trocknet und filtriert sie und dampft im Vakuum ein. Den Eindampfrückstand reinigt man durch Säulenchromatographie an Kieselgel (Methylenchlorid/Acetonitril/Eisessig = 10/1/0,05).

Ausbeute: 0,6 g (49,2% der Theorie),
Schmelzpunkt: 136—138° C (Äther).

| | | | |
|---|---|---|---|
| Ber.: | C 69,51 | H 7,35 | N 6,00 |
| Gef.: | C 69,28 | H 7,34 | N 5,83 |

Beispiel 44

4-[(1-(2-(4-Oxo-piperidino-phenyl)-äthyl)-aminocarbonylmethyl]-benzoesäure

Eine Lösung von 2,9 g (6,86 mMol) 4-[(1-(2-[1,4-Dioxa-8-aza-spiro[4.5]decan-8-yl]-phenyl)-äthyl)-aminocarbonylmethyl]-benzoesäure-semihydrat in 40 ml Aceton stellt man durch Zugabe von 2N-Salzsäure auf pH = 2 ein. Nach 6 Stunden Rühren bei 50° C gibt man 5 Tropfen konz. Salzsäure zu und läßt 16 Stunden bei 20° C stehen.Man dampft im Vakuum ein, setzt Wasser und Äthylacetat zu und stellt durch Zugabe von 2N-Ammoniak auf pH = 6 ein.Man schüttelt mehrmals mit Äthylacetat aus, wäscht die vereinigten organischen Extrakte mit Wasser, trocknet und filtriert sie und dampft im Vakuum ein. Den Eindampfrückstand kristallisiert man aus Aceton/Petroläther um.

Ausbeute: 1,9 g (73,1% der Theorie),
Schmelzpunkt: 177—180° C (Zers.).

| | | | |
|---|---|---|---|
| Ber.: | C 69,46 | H 6,36 | N 7,36 |
| Gef.: | C 69,75 | H 6,33 | N 7,29 |

Beispiel 45

4-[(1-(2-(4-Hydroxy-piperidino-phenyl)-äthyl)-aminocarbonylmethyl]-benzoesäure × 0,66 $H_2O$

Zu einer Lösung von 1 g (2,63 mMol) 4-[(1-(2-(4-Oxo-piperidino)-phenyl)-äthyl)-aminocarbonylmethyl]-benzoesäure in 20 ml absolutem Äthanol gibt man unter Rühren portionsweise 0,224 g

(5,92 mMol) Natriumborhydrid. Nach 1,5 Stunden Rühren bei Raumtemperatur stellt man zunächst mit 2N-Salzsäure sauer, dampft im Vakuum ein, gibt Wasser und Äthylacetat zu und stellt mit 2N-Natronlauge auf pH = 6 ein. Man extrahiert mehrfach mit Äthylacetat, trocknet und filtriert den Extrakt und dampft ihn im Vakuum ein. Den Eindampfrückstand kristallisiert man aus Petroläther.

Ausbeute: 0,78 g (75% der Theorie),
Schmelzpunkt: 175—180° C (Zers.).

| | | | | |
|---|---|---|---|---|
| Ber.: | (× 0,66 $H_2O$): | C 66,97 | H 6,81 | N 7,10 |
| Gef.: | | C 66,72 | H 6,62 | N 6,98 |

Beispiel 46

4-[(1-(2-Piperidino-phenyl)-äthyl)-aminocarbonylmethyl]-benzoesäure-propylester

Zu einer Lösung von 2 g (5,46 mMol) 4-[(1-(2-Piperidino-phenyl)-äthyl)-aminocarbonylmethyl]-benzoesäure in 20 ml absolutem Tetrahydrofuran gibt man 0,94 g (5,80 mMol) Carbonyl-diimidazol und erhitzt 30 Minuten unter Feuchtigkeitsausschluß auf Rückfluß. Anschließend setzt man 1,64 ml (22 mMol) 1-Propanol zu, rührt 18 Stunden bei 20° C und erhitzt noch 8 Stunden auf Rückfluß. Anschließend dampft man im Vakuum ein und reinigt den Eindampfrückstand durch Säulenchromatographie an Kieselgel (Toluol/Aceton = 10/1).

Ausbeute: 1,3 g (58,3% der Theorie),
Schmelzpunkt: 150—151° C (Äthylacetat).

| | | | |
|---|---|---|---|
| Ber.: | C 73,51 | H 7,90 | N 6,86 |
| Gef.: | C 73,70 | H 7,78 | N 6,92 |

Analog Beispiel 46 wurden folgende Verbindungen erhalten:

4-[(1-(2-Piperidino-phenyl)-äthyl)-aminocarbonylmethyl]-benzoesäure-isopropylester

Ausbeute: 45% der Theorie,
Schmelzpunkt: 141—143° C (Äther).

| | | | |
|---|---|---|---|
| Ber.: | C 73,51 | H 7,90 | N 6,86 |
| Gef.: | C 73,20 | H 7,79 | N 6,70 |

4-[(1-(2-Piperidino-phenyl)-äthyl)-aminocarbonylmethyl]-benzoesäure-butylester

Ausbeute: 49% der Theorie,
Schmelzpunkt: 148° C (Äther/Toluol).

| | | | |
|---|---|---|---|
| Ber.: | C 73,90 | H 8,11 | N 6,63 |
| Gef.: | C 74,10 | H 7,99 | N 6,70 |

4-[(1-(5-Chlor-2-piperidino-phenyl)-äthyl)-aminocarbonylmethyl]-benzoesäure-äthylester

Ausbeute: 41% der Theorie,
Schmelzpunkt: 130—133° C (Äther).

| | | | | |
|---|---|---|---|---|
| Ber.: | C 67,21 | H 6,81 | Cl 8,26 | N 6,53 |
| Gef.: | C 66,90 | H 6,65 | Cl 8,32 | N 6,67 |

4-[(1-(5-Chlor-2-piperidino-phenyl)-äthyl)-aminocarbonylmethyl]-
benzoesäure-butylester

Ausbeute: 30,7% der Theorie,
Schmelzpunkt: 115—118° C.

| | | | | |
|---|---|---|---|---|
| Ber.: | C 68,33 | H 7,27 | Cl 7,75 | N 6,12 |
| Gef.: | C 68,20 | H 7,23 | Cl 7,68 | N 5,95 |

4-[(1-(5-Chlor-2-piperidino-phenyl)-äthyl)-aminocarbonylmethyl]-
benzoesäure-tert.butylester

Ausbeute: 1% der Theorie.

| | | |
|---|---|---|
| Ber.: | Molpeak | m/e = 456/8 |
| Gef.: | | m/e = 456/8 |

4-[(1-(2-Piperidino-phenyl)-äthyl)-aminocarbonylmethyl]-
benzoesäure-(2-methoxy-äthylester)

Ausbeute: 56% der Theorie,
Schmelzpunkt: 155—157° C (Äthylacetat).

| | | | |
|---|---|---|---|
| Ber.: | C 70,74 | H 7,60 | N 6,60 |
| Gef.: | C 70,55 | H 7,38 | N 6,47 |

4-[(1-(2-Piperidino-phenyl)-äthyl)-aminocarbonylmethyl]-
benzoesäure-[(2,2-dimethyl-dioxolan-4-yl)-methyl]-ester

Ausbeute: 30,5% der Theorie,
Schmelzpunkt: 110—112° C (Äther).

| | | | | |
|---|---|---|---|---|
| Ber.: | C 69,98 | H 7,55 | N 5,83 | m/e = 480 |
| Gef.: | C 69,80 | H 7,50 | N 5,76 | m/e = 480 |

4-[(1-(2-Piperidino-phenyl)-äthyl)-aminocarbonylmethyl]-
benzoesäure-benzylester

Ausbeute: 73,7% der Theorie,
Schmelzpunkt: 126—128° C (Äthylacetat).

| | | | |
|---|---|---|---|
| Ber.: | C 76,28 | H 7,06 | N 6,14 |
| Gef.: | C 76,33 | H 7,20 | N 6,03 |

4-[(1-(2-Piperidino-phenyl)-äthyl)-aminocarbonylmethyl]-
benzoesäure-(2-hydroxy-äthyl)-ester

Es wurde nach Zusatz von 10 Äquivalenten Äthylenglykol 17 Stunden auf Rückfluß erhitzt.

Ausbeute: 71,4% der Theorie,
Schmelzpunkt: 128—129° C (Äthylacetat/Äther).

| | | | | |
|---|---|---|---|---|
| Ber.: | C 70,21 | H 7,36 | N 6,82 | m/e = 410 |
| Gef.: | C 70,14 | H 7,42 | N 6,70 | m/e = 410 |

1,2-Bis[4-[(1-(2-Piperidino-phenyl)-äthyl)-aminocarbonylmethyl]-benzoyloxy]-äthan

Es wurde nach Zusatz von 0,5 Äquivalenten Äthylenglykol 17 Stunden auf Rückfluß erhitzt.

Ausbeute: 43,5% der Theorie,
Schmelzpunkt: 188—191° C (Toluol).

| | | | | |
|---|---|---|---|---|
| Ber.: | C 72,80 | H 7,17 | N 7,38 | m/e = 758 |
| Gef.: | C 72,85 | H 7,07 | N 7,37 | m/e = 758 |

4-[(1-(2-Piperidino-phenyl)-äthyl)-aminocarbonylmethyl]-benzoesäure-(2-diäthylamino-äthyl)-ester

Ausbeute: 56,7% der Theorie,
Schmelzpunkt: 99—101° C (Petroläther.

| | | | |
|---|---|---|---|
| Ber.: | C 72,23 | H 8,44 | N 9,03 |
| Gef.: | C 72,40 | H 8,37 | N 8,95 |

4-[(1-(2-Piperidino-phenyl)-äthyl)-aminocarbonylmethyl]-benzoesäure-[2-(1,3-dimethyl-xanthin-7-yl)-äthyl]-ester

Als Lösungsmittel wurde absolutes Pyridin verwendet. Nach Zusatz von 1 Äquivalent 7-(2-Hydroxyäthyl)-theophyllin und nach Zusatz eines winzigen Stückchens metallischen Natriums wurde 4 Stunden im Bad von130° C gerührt.

Ausbeute: 40,9% der Theorie,
Schmelzpunkt: 121—123° C (Äther).

| | | | | |
|---|---|---|---|---|
| Ber.: | C 65,01 | H 6,34 | N 14,68 | m/e = 572 |
| Gef.: | C 64,78 | H 6,38 | N 14,90 | m/e = 572 |

Beispiel 47

4-[(1-(2-Piperidino-phenyl)-äthyl)-aminocarbonylmethyl]-benzoesäure-methylester

Man erhitzt das Gemisch von 2 g (5,46 mMol) 4-[(1-(2-Piperidino-phenyl)-äthyl)-aminocarbonylmethyl]-benzoesäure, 0,53 g Methanol, 0,38 ml konz. Schwefelsäure und 1,65 ml 1,2-Dichlor-äthan 24 Stunden auf Rückfluß, dampft im Vakuum ein, löst in Chloroform und schüttelt mit verdünnter Natriumhydrogencarbonat-Lösung aus. Die organische Phase wäscht man mit Wasser, trocknet und filtriert sie und dampft im Vakuum ein. Den Eindampfrückstand reinigt man durch Säulenchromatographie an Kieselgel (Toluol/Aceton = 5/1.

Ausbeute: 0,93 g (44% der Theorie),
Schmelzpunkt: 146—147° C.

| | | | |
|---|---|---|---|
| Ber.: | C 72,60 | H 7,42 | N 7,36 |
| Gef.: | C 72,19 | H 7,33 | N 7,01 |

Beispiel 48

4-[(1-(2-Piperidino-phenyl)-2-propyl)-aminocarbonylmethyl]-benzoesäure-äthylester

Man rührt 0,20 g (0,526 mMol) 4-[(2-(2-Piperidino-phenyl)-2-propyl)-aminocarbonylmethyl]-benzoesäure und 2 ml 4N-äthanolische Salzsäure bei 20° C. Nach 36 Stunden dampft man im Vakuum ein und verteilt den Eindampfrückstand zwischen Wasser (bei pH = 8 durch Zugabe von 10%igem Ammoniak) und Äthylacetat. Man wäscht die organische Phase mit Wasser, trocknet und filtriert sie und dampft

sie im Vakuum ein. Den Eindampfrückstand reinigt man durch Säulenchromatographie an Kieselgel (Toluol/Aceton = 10/1).

Ausbeute: 0,079 g (36,7% der Theorie),
Schmelzpunkt: 151—153°C (Äther).

| | | | |
|---|---|---|---|
| Ber.: | C 73,50 | H 7,90 | N 6,86 |
| Gef.: | C 73,40 | H 7,95 | N 6,96 |

Beispiel 49

4-[(1-(2-Piperidino-phenyl)-äthyl)-aminocarbonylmethyl]-benzoesäure-tert.butylester

Man rührt das Gemisch aus 3,60 g (17,4 mMol) N,N'-Dicyclohexylcarbodiimid, 1,9 ml (20,4 mMol) tert.Butanol und 0,036 g (0,36 mMol) Kupfer(I)chlorid 3 Tage bei Raumtemperatur, gibt dann 12 ml Methylenchlorid zu und tropft die so erhaltene Lösung zur Lösung von 2 g (5,46 mMol) 4-[(1-(2-Piperidino-phenyl)-äthyl)-aminocarbonylmethyl]-benzoesäure in 80 ml Methylenchlorid. Nach 16 Stunden Rühren bei 20°C filtriert man vom ausgefallenen Niederschlag ab, wäscht ihn mit Methylenchlorid und dampft die Methylenchlorid-Lösung im Vakuum ein. Den Eindampfrückstand reinigt man durch Säulenchromatographie an Kieselgel (Toluol/Aceton = 15/1).

Ausbeute: 0,45 g (19,7% der Theorie),
Schmelzpunkt: 125—127°C (Äther).

| | | | |
|---|---|---|---|
| Ber.: | C 73,90 | H 8,11 | N 6,63 |
| Gef.: | C 74,20 | H 8,09 | N 6,77 |

Beispiel 50

4-[(1-(2-Piperidino-phenyl)-äthyl)-aminocarbonylmethyl]-benzoesäure-(2-nicotinoyloxy-äthyl)-ester

Zur Lösung von 0,45 g (1,10 mMol) 4-[(1-(2-Piperidino-phenyl)-äthyl)-aminocarbonylmethyl]-benzoesäure-(2-hydroxy-äthyl)-ester und 0,16 ml (1,16 mMol) Triäthylamin in 10 ml Methylenchlorid tropft man zügig die Lösung von 0,16 g (1,13 mMol) Nikotinsäurechlorid in 5 ml Methylenchlorid. Nach 4 Stunden Rühren bei 20°C schüttelt man mit Wasser aus, trocknet und filtriert die Methylenchlorid-Lösung und dampft sie im Vakuum ein. Den Eindampfrückstand reinigt man durch Säulenchromatographie an Kieselgel (Chloroform/Aceton = 3/1).

Ausbeute: 0,34 g (60% der Theorie),
Schmelzpunkt: 103—105°C (Äther).

| | | | |
|---|---|---|---|
| Ber.: | C 69,88 | H 6,45 | N 8,15 |
| Gef.: | C 70,13 | H 6,55 | N 8,13 |

Beispiel 51

4-[(1-(2-Piperidino-phenyl)-äthyl)-aminocarbonylmethyl]-benzamid

Zu 4,76 g (0,013 Mol) 4-[(1-(2-Piperidino-phenyl)-äthyl)-aminocarbonylmethyl]-benzoesäure in 60 ml absolutem Pyridin gibt man 2,3 g (0,0142 Mol) Carbonyldiimidazol und erhitzt anschließend 45 Minuten auf 50°C. Nach Abkühlen in einem Kohlensäure-Methanol-Bad gibt man 7 ml flüssigem Ammoniak dazu und erhitzt im Autoklaven 20 Stunden auf 80°C. Danach kühlt man das Reaktionsgemisch ab und dampft im Vakuum ein. Den Rückstand löst man in 50 ml heißem Methanol, gibt 200 ml Wasser dazu und läßt über Nacht stehen. Der kristalline Niederschlag wird abgesaugt und aus Methanol unter Zusatz von Aktivkohle umkristallisiert.

Ausbeute: 3,5 g (73,6% der Theorie),
Schmelzpunkt: 197—199°C.

| | | | |
|---|---|---|---|
| Ber.: | C 72,30 | H 7,45 | N 11,50 |
| Gef.: | C 72,30 | H 7,45 | N 11,32 |

Beispiel 52

4-[(1-(2-Piperidino-phenyl)-äthyl)-aminocarbonylmethyl]-
N-methyl-benzamid

Man erhitzt 2 g (5,46 mMol) 4-[(1-(2-Piperidino-phenyl)-äthyl)-aminocarbonylmethyl]-benzoesäure und 0,94 g (5,80 mMol) Carbonyldiimidazol in 20 ml absolutem Pyridin eine Stunde auf Rückfluß. Anschließend gibt man 0,41 g (6,07 mMol) Methylaminhydrochlorid zu und rührt eine Stunde bei 20°C und 2 Stunden unter Rückfluß. Man dampft im Vakuum ein, verteilt zwischen Wasser und Methylenchlorid, trocknet und filtriert den organischen Extrakt und dampft ihn im Vakuum ein. Den Eindampfrückstand reinigt man durch Säulenchromatographie an Kieselgel (Chloroform/Methanol/konzentriertem Ammoniak = 10/1/0,05).

Ausbeute: 1,7 g (82% der Theorie),
Schmelzpunkt: 218—220°C (Isopropanol).

| | | | |
|---|---|---|---|
| Ber.: | C 72,77 | H 7,70 | N 11,07 |
| Gef.: | C 72,88 | H 7,67 | N 10,91 |

Analog Beispiel 52 wurde folgende Verbindung hergestellt:

4-[(1-(2-Piperidino-phenyl)-äthyl)-aminocarbonylmethyl]-
N,N-dimethyl-benzamid

Ausbeute: 52,5% der Theorie,
Schmelzpunkt: 148—150°C (Äthylacetat).

| | | | |
|---|---|---|---|
| Ber.: | C 73,26 | H 7,94 | N 10,68 |
| Gef.: | C 73,60 | H 7,85 | N 10,73 |

Beispiel 53

4-[(1-(2-Piperidino-phenyl)-äthyl)-aminocarbonylmethyl]-
N-butyl-benzamid

Zur Lösung von 2 g (5,46 mMol) 4-[(1-(2-Piperidino-phenyl)-äthyl)-aminocarbonylmethyl]-benzoesäure in 20 ml absolutem Tetrahydrofuran gibt man 0,94 g (5,80 mMol) Carbonyldiimidazol. Man erhitzt 30 Minuten auf Rückfluß, setzt 0,44 g (6,1 mMol) 1-Butylamin zu und erhitzt 2 Stunden auf Rückfluß. Man dampft im Vakuum ein und reinigt den Eindampfrückstand durch Säulenchromatographie an Kieselgel (Chloroform/Aceton = 6/1).

Ausbeute: 1,65 g (71,7% der Theorie),
Schmelzpunkt: 178—181°C (Äthylacetat).

| | | | |
|---|---|---|---|
| Ber.: | C 74,09 | H 8,37 | N 9,97 |
| Gef.: | C 74,34 | H 8,26 | N 9,95 |

Analog Beispiel 53 wurden folgende Verbindungen erhalten:

4-[(1-(2-Piperidino-phenyl)-äthyl)-aminocarbonylmethyl]-
benzoesäure-piperidid

Ausbeute: 73,8% der Theorie,
Schmelzpunkt: 131—133°C (Toluol).

| | | | | |
|---|---|---|---|---|
| Ber.: | C 74,79 | H 8,14 | N 9,69 | m/e = 433 |
| Gef.: | C 75,13 | H 7,99 | N 9,48 | m/e = 433 |

4-[(1-(2-Piperidino-phenyl)-äthyl)-aminocarbonylmethyl]-benzoesäure-morpholid

Ausbeute: 60,5% der Theorie,
Schmelzpunkt: 148—150° C (Äthylacetat/Äther).

| | | | |
|---|---|---|---|
| Ber.: | C 71,69 | H 7,64 | N 9,65 |
| Gef.: | C 71,60 | H 7,80 | N 9,57 |

Beispiel 54

4-[(1-(2-Piperidino-phenyl)-äthyl)-aminocarbonylmethyl]-benzonitril

Zu einem Gemisch von 2,19 g (6 mMol) 4-[(1-(2-Piperidino-phenyl)-äthyl)-aminocarbonylmethyl]-benzamid und 1,07 g (13,5 mMol) absolutem Pyridin gibt man bei Raumtemperatur unter Rühren 1,14 g (6 mMol) p-Toluolsulfonsäurechlorid in 2 Portionen. Man rührt 15 Minuten bei 20° C und dann 2 Stunden bei 50° C. Nach Abkühlen versetzt man mit Wasser, stellt mit konzentriertem Ammoniak alkalisch und extrahiert 3mal mit Chloroform. Die vereinigten Chloroformextrakte werden mit Wasser gewaschen, über Natriumsulfat getrocknet, filtriert und im Vakuum eingedampft. Den Eindampfrückstand reinigt man durch Säulenchromatographie an Kieselgel (Chloroform/Äthylacetat = 4/1).

Ausbeute: 1,15 g (55,3% der Theorie),
Schmelzpunkt: 155—157° C (Äthylacetat).

| | | | |
|---|---|---|---|
| Ber.: | C 76,05 | H 7,25 | N 12,09 |
| Gef.: | C 76,30 | H 7,07 | N 11,90 |

Beispiel A

Tabletten mit 5 mg 4-[(1-(2-Piperidino-phenyl)-äthyl)-aminocarbonylmethyl]-benzoesäure

Zusammensetzung:
1 Tablette enthält:

| | |
|---|---|
| Wirksubstanz (1) | 5,0 mg |
| Maisstärke (2) | 62,0 mg |
| Milchzucker (3) | 48,0 mg |
| Polyvinylpyrrolidon (4) | 4,0 mg |
| Magnesiumstearat (5) | 1,0 mg |
| | 120,0 mg |

Herstellungsverfahren

1, 2, 3 und 4 werden gemischt und mit Wasser befeuchtet. Die feuchte Mischung wird durch ein Sieb mit 1,5-mm-Maschenweite gedrückt und bei ca. 45° C getrocknet. Das Granulat wird durch ein Sieb mit 1,0-mm-Maschenweite geschlagen und mit 5 vermischt. Die fertige Mischung preßt man auf einer Tablettenpresse mit Stempeln von 7 mm Durchmesser, die mit einer Teilkerbe versehen sind, zu Tabletten.

Tablettengewicht: 120 mg.

Beispiel B

Dragées mit 2,5 mg 4-[(1-(2-Piperidino-phenyl)-äthyl)-aminocarbonylmethyl]-benzoesäure

1 Dragéekern enthält:

| | |
|---|---|
| Wirksubstanz (1) | 2,5 mg |
| Kartoffelstärke (2) | 44,0 mg |
| Milchzucker (3) | 30,0 mg |
| Polyvinylpyrrolidon (4) | 3,0 mg |
| Magnesiumstearat (5) | 0,5 mg |
| | 80,0 mg |

Herstellungsverfahren

1, 2, 3 und 4 werden gut gemischt und mit Wasser befeuchtet. Die feuchte Masse drückt man durch ein Sieb mit 1 mm Maschenweite, trocknet bei ca. 45° C und schlägt das Granulat anschließend durch dasselbe Sieb. Nach dem Zumischen von 5 werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 120 mg.

Beispiel C

Tabletten mit 10 mg 4-[(1-(2-Piperidino-phenyl)-äthyl)-aminocarbonylmethyl]-benzoesäure

Zusammensetzung:
1 Tablette enthält:

| | |
|---|---|
| Wirksubstanz | 10,0 mg |
| Milchzucker pulv. | 70,0 mg |
| Maisstärke | 31,0 mg |
| Polyvinylpyrrolidon | 8,0 mg |
| Magnesiumstearat | 1,0 mg |
| | 120,0 mg |

Herstellungsverfahren

Die Mischung aus der Wirksubstanz, Milchzucker und Maisstärke wird mit einer 20%igen Lösung von Polyvinylpyrrolidon in Wasser befeuchtet. Die feuchte Masse wird durch ein Sieb mit 1,5-mm-Maschenweite granuliert und bei 45° C getrocknet. Das getrocknete Granulat wird durch ein Sieb mit 1-mm-Maschenweite gerieben und mit Magnesiumstearat homogen vermischt.

| | |
|---|---|
| Tablettengewicht: | 120 mg |
| Stempel: | 7 mm ∅ mit Teilkerbe |

Beispiel D

Dragées mit 5 mg 4-[(1-(2-Piperidino-phenyl)-äthyl)-aminocarbonylmethyl]-benzoesäure

1 Dragéekern enthält:

| | |
|---|---|
| Wirksubstanz | 5,0 mg |
| Calciumphosphat sekundär | 70,0 mg |
| Maisstärke | 50,0 mg |
| Polyvinylpyrrolidon | 4,0 mg |
| Magnesiumstearat | 1,0 mg |
| | 130,0 mg |

Herstellungsverfahren

Die Mischung aus der Wirksubstanz, Calciumphosphat und Maisstärke wird mit einer 15%igen Lösung von Polyvinylpyrrolidon in Wasser befeuchtet. Die feuchte Masse wird durch ein Sieb mit 1-mm-Maschenweite geschlagen,bei 45°C getrocknet und anschließend durch dasselbe Sieb gerieben. Nach dem Vermischen mit der angegebenen Menge Magnesiumstearat werden daraus Dragéekerne gepreßt.

| | |
|---|---|
| Kerngewicht | 130 mg |
| Stempel | 7 mm Ø |

Auf die so hergestellten Dragéekerne wird auf bekannte Art eine Schicht aus Zucker und Talkum aufgetragen. Die fertigen Dragées werden mit Wachs poliert.

Dragéegewicht: 180 mg

**Patentansprüche für die Vertragsstaaten: BE, CH, DE, FR, LT, LI, LU, NL, SE**

1. Neue Carbonsäureamide der allgemeinen Formel

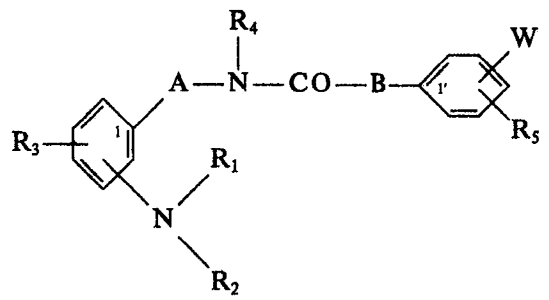

(I)

in der

$R_1$ und $R_2$, die gleich oder verschieden sein können, Alkylgruppen mit 1 bis 6 Kohlenstoffatomen oder Cycloalkylgruppen mit 5 bis 7 Kohlenstoffatomen oder

$R_1$ und $R_2$ zusammen mit dem dazwischenliegenden Stickstoffatom eine unverzweigte Alkyleniminogruppe mit 3 bis 6 Kohlenstoffatomen, die durch eine oder zwei Alkylgruppen mit jeweils 1 bis 3 Kohlenstoffatomen oder durch eine Hydroxygruppe substituiert sein kann, oder in der eine Methylengruppe durch eine Carbonylgruppe, durch ein Sauerstoff- oder Schwefelatom oder durch eine Iminogruppe, welche durch eine Alkylgruppe mit 1 bis 3 Kohlenstoffatomen, eine Aralkylgruppe mit 7 bis 10 Kohlenstoffatomen, eine Phenyl- oder Halogenphenylgruppe substituiert sein kann, oder in der eine Äthylengruppe durch eine o-Phenylengruppe ersetzt sein kann, eine unverzweigte Alkenyleniminogruppe mit 4 bis 6 Kohlenstoffatomen, eine gesättigte oder teilweise ungesättigte Azabicycloalkylgruppe mit 6 bis 10 Kohlenstoffatomen, eine Aza-1,4-dioxa-spiro-alkylgruppe mit 6 bis 8 Kohlenstoffatomen, eine Heptamethylenimino-, Octamethylenimino-, Nonamethylenimino- oder Decamethyleniminogruppe,

$R_3$ ein Wasserstoff- oder Halogenatom, eine Trifluormethyl-, Alkyl-, Hydroxy-, Alkoxy-, Alkanoyloxy-, Mercapto-, Alkylmercapto-, Nitro-, Amino-, Cyano-, Alkanoyl-, Carboxy-, Alkoxycarbonyl-, Aminocarbonyl-, Alkylaminocarbonyl-, Dialkylaminocarbonyl-, Aminosulfonyl-, Alkylamino-, Dialkylamino-, Alkanoylamino-, Alkoxycarbonylamino- oder Alkylsulfonylaminogruppe, wobei jeder Alkylteil in den vorstehend genannten Gruppen jeweils 1 bis 3 Kohlenstoffatome enthalten kann, eine Araloxygruppe mit 7 bis 10 Kohlenstoffatomen oder eine Arylcarbonylaminogruppe,

$R_4$ ein Wasserstoffatom oder eine Alkylgruppe mit 1 bis 3 Kohlenstoffatomen,

$R_5$ ein Wasserstoffatom, ein Halogenatom oder eine Alkylgruppe mit 1 bis 3 Kohlenstoffatomen,

A eine Bindung, eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierte Methylen- oder Äthylengruppe, eine durch eine Cycloalkylgruppe mit 3 bis 7 Kohlenstoffatomen, durch eine Hydroxyalkyl-, Alkoxyalkyl-, Cyano-, Carboxyl-, Alkoxycarbonyl-, Aminocarbonyl-, Alkylaminocarbonyl-, Dialkylaminocarbonyl-, Aryl- oder Aralkylgruppe substituierte Methylengruppe, wobei jeder der vorstehend erwähnten Alkylteile 1 bis 3 Kohlenstoffatomen enthalten kann, eine Cycloalkylidengruppe mit 3 bis 7 Kohlenstoffatomen oder eine Vinylidengruppe der Formel

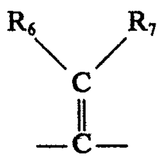

wobei
$R_6$ und $R_7$, die gleich oder verschieden sein können, Wasserstoffatome oder Alkylgruppen mit jeweils 1 bis 3 Kohlenstoffatomen oder auch einer der Reste $R_6$ oder $R_7$ eine Cycloalkylgruppe mit 3 bis 7 Kohlenstoffatomen, eine Aryl- oder Aralkylgruppe oder $R_6$ und $R_7$ zusammen mit dem dazwischenliegenden Kohlenstoffatom einen Cycloalkylidenrest mit 5 bis 7 Kohlenstoffatomen bedeuten,

B eine gegebenenfalls durch eine Alkylgruppe mit 1 bis 3 Kohlenstoffatomen substituierte Methylen- oder Äthylengruppe und

W ein Wasserstoff- oder Halogenatom, eine Nitrogruppe, eine gegebenenfalls durch eine Alkanoylgruppe mit 1 bis 3 Kohlenstoffatomen substituierte Aminogruppe, eine gegebenenfalls durch eine Hydroxy-, Carboxy- oder Alkoxycarbonylgruppe oder zwei Alkoxycarbonylgruppen mit jeweils insgesamt 2 bis 4 Kohlenstoffatomen substituierte Alkylgruppe mit 1 bis 3 Kohlenstoffatomen, eine durch eine Carboxy- oder Alkoxycarbonylgruppe mit insgesamt 2 bis 4 Kohlenstoffatomen substituierte Alkenylgruppe mit 2 bis 5 Kohlenstoffatomen, eine Alkanoylgruppe mit 1 bis 3 Kohlenstoffatomen, eine Dialkoxymethyl- oder Trialkoxymethylgruppe mit 1 bis 3 Kohlenstoffatomen in jedem Alkylteil, eine Alkylendioxymetylgruppe mit 2 oder 3 Kohlenstoffatomen im Alkylenteil, eine 1,3-Oxazolin-2-yl- oder Cyanogruppe, eine gegebenenfalls durch eine oder zwei Alkylgruppen mit jeweils 1 bis 4 Kohlenstoffatomen in jedem Alkylteil substituierte Aminocarbonylgruppe, eine unverzweigte Alkyleniminocarbonylgruppe mit insgesamt 5 bis 8 Kohlenstoffatomen, eine Morpholinocarbonylgruppe, eine (Dialkyldioxolan-yl)-alkoxycarbonylgruppe mit insgesamt 7 bis 10 Kohlenstoffatomen oder eine Carboxygruppe und deren Ester, wobei Alkylester mit 1 bis 6 Kohlenstoffatomen im Alkylteil mit Ausnahme der $\alpha$-Position jeweils durch eine Hydroxy-, Alkoxy-, Amino-, Alkylamino-, Dialkylamino-, 1,3,-Dimethyl-xanthin-7-yl-, Alkanoyloxy-, Aroyloxy-, Aralkanoyloxy- oder Pyridincarbonyloxygruppe oder durch zwei Hydroxygruppen, wobei eine Methyl- bzw. Methylengruppe jeweils nur durch eine Hydroxygruppe substituiert sein kann, oder duch eine Gruppe der Formel

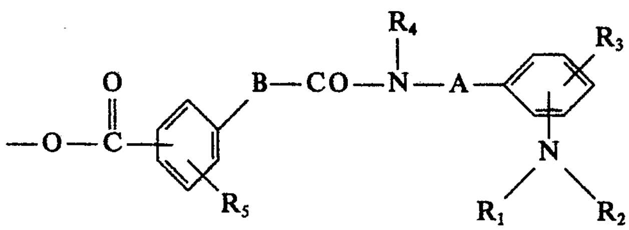

in der
A, B und $R_1$ bis $R_5$ wie eingangs definiert sind, substituiert sein können, wobei jeder Alkylsubstituent in den vorstehend genannten Alkylestern 1 bis 3 Kohlenstoffatome enthalten kann, bedeuten, und, sofern diese ein asymmetrisches Kohlenstoffatomen enthalten, deren optisch aktive Antipoden und deren Salze, insbesondere deren physiologisch verträgliche Salze mit anorganischen oder organischen Säuren oder Basen.

2. Neue Carbonsäureamide der allgemeinen Formel I gemäß Anspruch 1, in der

$R_1$ und $R_2$ zusammen mit dem dazwischenliegenden Stickstoffatom eine Dialkylamino- oder N-Alkylcyclohexylaminogruppe, in der jeder Alkylteil 1 bis 4 Kohlenstoffatome enthalten kann, eine gegebenenfalls durch eine oder zwei Methylgruppen substituierte unverzweigte Alkyleniminogruppe mit 3 bis 6 Kohlenstoffatomen, eine Hydroxypiperidino-, Piperidon-1-yl-, Tetrahydro-pyridino-, Morpholino-, Thiomorpholino-, N-Methyl-piperazino-, N-Benzyl-piperazino-, N-Chlorphenyl-piperazino-, Heptamethylenimino- oder Octamethyleniminogruppe, eine gesättigte oder teilweise ungesättigte Azabicycloalkylgruppe mit 7 bis 9 Kohlenstoffatomen, eine unverzweigte Alkyleniminogruppe mit 4 bis 6 Kohlenstoffatomen, in der eine Äthylengruppe durch eine o-Phenylengruppe ersetzt ist, oder eine 1,4-Dioxa-aza-spiro-alkylgruppe mit 7 oder 8 Kohlenstoffatomen,

$R_3$ ein Wasserstoff-, Fluor-, Chlor-, Brom- oder Jodatom, eine Methyl-, Trifluormethyl-, Hydroxy-, Methoxy-, Benzyloxy-, Acetoxy-, Mercapto-, Methylmercapto-, Nitro-, Amino-, Dimethylamino-, Acetylamino-, Methylsulfonylamino-, Benzoylamino-, Äthoxy-carbonylamino-, Cyano-, Carboxy-, Methoxycarbonyl-, Äthoxycarbonyl-, Aminocarbonyl-, Acetyl- oder Aminosulfonylgruppe,

$R_4$ ein Wasserstoffatom oder eine Methylgruppe,
$R_5$ ein Wasserstoffatom, ein Chloratom oder eine Methylgruppe,
A eine Bindung, eine gegebenenfalls durch eine Alkylgruppe mit 1 bis 3 Kohlenstoffatomen, eine Phenyl-, Cyclohexyl-, Carboxy-, Methoxycarbonyl- oder Hydroxymethylgruppe substituierte Methylengruppe, eine Dimethyl-methylen-, Cyclopropyliden- oder Äthylengruppe oder eine Vinylidengruppe der Formel

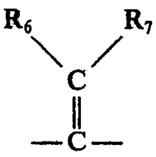

wobei $R_6$ und $R_7$, die gleich oder verschieden sein können, Wasserstoffatome oder Methylgruppen oder $R_6$ und $R_7$ zusammen mit dem dazwischenliegenden Kohlenstoffatomen einen Cycloalkylidenrest mit 5 oder 6 Kohlenstoffatomen bedeuten,
B eine Methylen-, Äthyliden- oder Äthylengruppe und
W ein Wasserstoffatom, eine Methyl-, Äthyl-, Hydroxymethyl-, Cyano- oder Carboxyvinylengruppe, eine durch eine Carboxygruppe oder durch eine oder zwei Alkoxycarbonylgruppen mit jeweils insgesamt 2 bis 4 Kohlenstoffatomen substituierte Alkylgruppe mit 1 bis 3 Kohlenstoffatomen, eine durch ein Wasserstoff, eine Methyl-, Äthyl-, Hydroxy-, Alkoxy-, (2,2-Dimethyl-dioxolan-4-yl)-methoxy-, Benzyloxy-, Pyridylmethoxy-, Amino-, Alkylamino-, Dialkylamino-, Piperidino- oder Morpholinogruppe substituierte Carbonylgruppe, wobei jeder Alkylteil in den vorstehend erwähnten Gruppen 1 bis 3 Kohlenstoffatomen enthalten kann, oder eine Gruppe der Formel

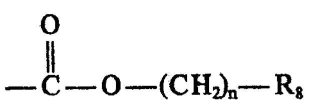

bedeuten, in der n die Zahl 2, 3 oder 4 und
$R_8$ eine Hydroxy-, Methoxy-, Äthoxy-, Acetoxy-, Benzoyloxy-, Pyridincarbonyloxy- oder eine Dialkylaminogruppe mit 1 bis 3 Kohlenstoffatomen in jedem Alkylteil, eine 1,3-Di-methyl-xanthin-7-yl-gruppe oder eine Gruppe der Formel

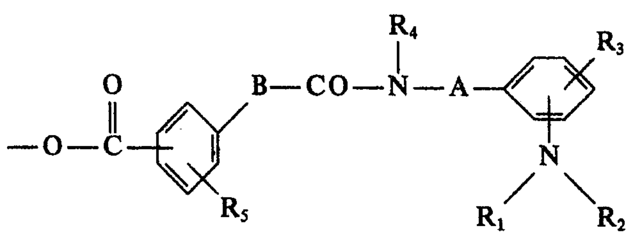

in der
A, B und $R_1$ bis $R_5$ wie im Anspruch 2 vorstehend erwähnt definiert sind, bedeuten, und, sofern diese ein asymmetrisches Kohlenstoffatom enthalten, deren optisch aktive Antipoden und deren Salze, insbesondere deren physiologisch verträgliche Salze mit anorganischen oder organischen Säuren oder Basen.

3. Neue Carbonsäureamide der allgemeinen Formel I gemäß Anspruch 1, in der
$R_1$ bis $R_5$, A, B und W wie im Anspruch 2 definiert sind und der Rest

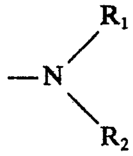

in 2-Position und der Rest W in 4'-Position steht, und, sofern diese ein asymmetrisches Kohlenstoffatom enthalten, deren optisch aktive Antipoden und deren physiologisch verträgliche Additionssalze mit anorganischen oder organischen Säuren oder Basen.

4. Neue Carbonsäureamide der allgemeinen Formel

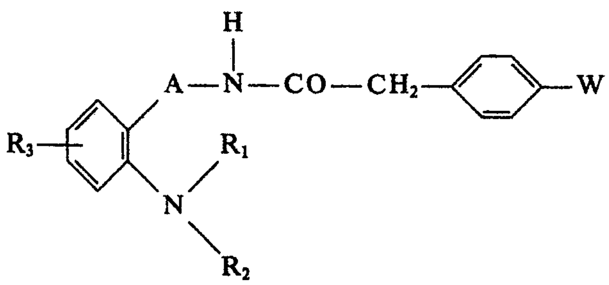

(Ia)

in der

$R_1$ und $R_2$ zusammen mit dem dazwischenliegenden Stickstoffatom eine Dimethylamino-, Pyrrolidino-, Methylpyrrolidino-, Piperidino-, Methylpiperidino-, Dimethylpiperidino-, Tetrahydro-pyridino-, 2-Octahydro-isoindolo- oder Hexamethyleniminogruppe,
$R_3$ ein Wasserstoff-, Fluor- oder Chloratom oder eine Methylgruppe,
A eine gegebenenfalls durch eine Cyclohexyl-, Phenyl-, Methoxycarbonyl-, Äthoxycarbonyl- oder eine Alkylgruppe mit 1 bis 3 Kohlenstoffatomen substituierte Methylengruppe, eine Dimethyl-methylen- oder eine Vinylidengruppe der Formel

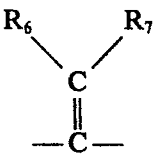

wobei $R_6$ und $R_7$ je ein Wasserstoffatom oder zusammen mit dem dazwischenliegenden Kohlenstoffatom eine Cyclohexylidengruppe darstellen, und
W eine Methyl-, Hydroxymethyl- oder Carboxymethylgruppe, eine durch ein Wasserstoffatom, eine Methyl-, Hydroxy-, Methoxy-, Äthoxy-, Propoxy-, Isopropoxy-, 2-Hydroxyäthoxy-, 2-Methoxyäthoxy- oder (2,2-Dimethyl-dioxolan-4-yl)-methoxy- oder 2-Diäthylaminoäthoxy-gruppe substituierte Carbonylgruppe bedeuten, und, sofern diese ein asymmetrisches Kohlenstoffatom enthalten, deren optisch aktive Antipoden und deren physiologisch verträgliche Additionssalze mit anorganischen oder organischen Säuren oder Basen.

5. Neue Carbonsäureamide der allgemeinen Formel Ia gemäß Anspruch 4, in der

$R_1$ und $R_2$ zusammen mit dem dazwischenliegenden Stickstoffatom eine Pyrrolidino-, Piperidino-, Methyl-piperidino-, Hexamethylenimino-, Tetrahydro-pyridino- oder 2-Octahydroisoindologruppe,
$R_3$ ein Wasserstoff-, Fluor- oder Chloratom oder eine Methylgruppe,
A eine gegebenenfalls durch eine Methyl-, Isopropyl-, Phenyl- oder Methoxycarbonylgruppe substituierte Methylengruppe, eine Dimethyl-methylen- oder Vinylidengruppe und
W eine Methyl-, Hydroxymethyl-, Carboxymethyl-, Formyl- oder Carboxylgruppe oder eine gegebenenfalls durch eine (2,2-Dimethyl-dioxolan-4-yl)-gruppe substituierte Alkoxycarbonylgruppe, in der die Alkoxygruppe 1 bis 3 Kohlenstoffatome enthalten kann, bedeuten, und, sofern diese ein asymmetrisches Kohlenstoffatomen enthalten, deren optisch aktive Antipoden und deren physiologisch verträgliche Additionssalze mit anorganischen oder organischen Säuren oder Basen.

6. 4-[(1-(2-Piperidino-phenyl)-äthyl)-aminocarbonylmethyl]-benzoesäure, deren Alkylester mit 1 bis 3 Kohlenstoffatomen, deren optisch aktive Antipoden und deren Salze.

7. 4-[(2-Piperidino-benzhydril)-aminocarbonylmethyl]-benzoesäure, deren Alkylester mit 1 bis 3 Kohlenstoffatomen, deren optisch aktive Antipoden und deren Salze.

8. Arzneimittel, enthaltend eine Verbindung gemäß den Ansprüchen 1 bis 7 neben einem oder mehreren inerten Trägerstoffen und/oder Verdünnungsmitteln.

9. Arzneimittel gemäß Anspruch 8 mit einer blutzuckersenkenden Wirkung.

10. Verfahren zur Herstellung von neuen Carbonsäureamiden der allgemeinen Formel

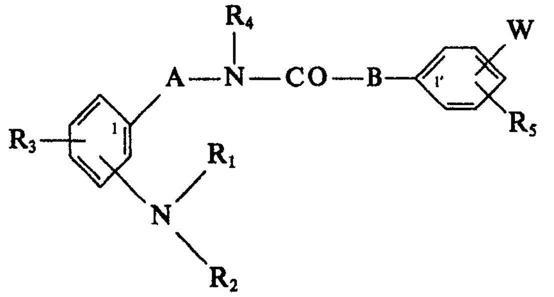

(I)

in der

$R_1$ und $R_2$, die gleich oder verschieden sein können, Alkylgruppen mit 1 bis 6 Kohlenstoffatomen oder Cycloalkylgruppen mit 5 bis 7 Kohlenstoffatomen oder

$R_1$ und $R_2$ zusammen mit dem dazwischenliegenden Stickstoffatom eine unverzweigte Alkyleniminogruppe mit 3 bis 6 Kohlenstoffatomen, die durch eine oder zwei Alkylgruppen mit jeweils 1 bis 3 Kohlenstoffatomen oder durch eine Hydroxygruppe substituiert sein kann, oder in der eine Methylengruppe durch eine Carbonylgruppe, durch ein Sauerstoff- oder Schwefelatom oder durch eine Iminogruppe, welche durch eine Alkylgruppe mit 1 bis 3 Kohlenstoffatomen, eine Aralkylgruppe mit 7 bis 10 Kohlenstoffatomen, eine Phenyl- oder Halogenphenylgruppe substituiert sein kann, oder in der eine Äthylengruppe durch eine o-Phenylengruppe ersetzt sein kann, eine unverzweigte Alkenyleniminogruppe mit 4 bis 6 Kohlenstoffatomen, eine gesättigte oder teilweise ungesättigte Azabicycloalkylgruppe mit 6 bis 10 Kohlenstoffatomen, eine Aza-1,4-dioxa-spiroalkylgruppe mit 6 bis 8 Kohlenstoffatomen, eine Heptamethylenimino-, Octamethylenimino-, Nonamethylenimino- oder Decamethyleniminogruppe,

$R_3$ ein Wasserstoff- oder Halogenatom, eine Trifluormethyl-, Alkyl-, Hydroxy-, Alkoxy-, Alkanoyloxy-, Mercapto-, Alkylmercapto-, Nitro-, Amino-, Cyano-, Alkanoyl-, Carboxy-, Alkoxycarbonyl-, Aminocarbonyl-, Alkylaminocarbonyl-, Dialkylaminocarbonyl-, Aminosulfonyl-, Alkylamino-, Dialkylamino-, Alkanoylamino-, Alkoxycarbonylamino- oder Alkylsulfonylaminogruppe, wobei jeder Alkylteil in den vorstehend genannten Gruppen jeweils 1 bis 3 Kohlenstoffatome enthalten kann, eine Aralkyloxygruppe mit 7 bis 10 Kohlenstoffatomen oder eine Arylcarbonylaminogruppe,

$R_4$ ein Wasserstoffatom oder eine Alkylgruppe mit 1 bis 3 Kohlenstoffatomen,

$R_5$ ein Wasserstoffatom, ein Halogenatom oder eine Alkylgruppe mit 1 bis 3 Kohlenstoffatomen,

A eine Bindung, eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierten Methylen- oder Äthylengruppe, eine durch eine Cycloalkylgruppe mit 3 bis 7 Kohlenstoffatomen, durch eine Hydroxyalkyl-, Alkoxyalkyl-, Cyano-, Carboxyl-, Alkoxycarbonyl-, Aminocarbonyl-, Alkylaminocarbonyl-, Dialkylaminocarbonyl-, Aryl- oder Aralkylgruppe substituierte Methylengruppe, wobei jeder der vorstehend erwähnten Alkylteile 1 bis 3 Kohlenstoffatomen enthalten kann, eine Cycloalkylidengruppe mit 3 bis 7 Kohlenstoffatomen oder eine Vinylidengruppe der Formel

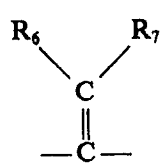

wobei

$R_6$ und $R_7$, die gleich oder verschieden sein können, Wasserstoffatome oder Alkylgruppen mit jeweils 1 bis 3 Kohlenstoffatomen oder auch einer der Reste $R_6$ oder $R_7$ eine Cycloalkylgruppe mit 3 bis 7 Kohlenstoffatomen, eine Aryl- oder Aralkylgruppe oder $R_6$ und $R_7$ zusammen mit dem dazwischenliegenden Kohlenstoffatomen einen Cycloalkylidenrest mit 5 bis 7 Kohlenstoffatomen bedeuten,

B eine gegebenenfalls durch eine Alkylgruppe mit 1 bis 3 Kohlenstoffatomen substituierte Methylen- oder Äthylengruppe und

W ein Wasserstoff- oder Halogenatom, eine Nitrogruppe, eine gegebenenfalls durch eine Alkanoylgruppe mit 1 bis 3 Kohlenstoffatomen substituierte Aminogruppe, eine gegebenenfalls durch eine Hydroxy-, Carboxy- oder Alkoxycarbonylgruppe oder zwei Alkoxycarbonylgruppen mit jeweils insgesamt 2 bis 4 Kohlenstoffatomen substituierte Alkylgruppe mit 1 bis 3 Kohlenstoffatomen, eine durch eine Carboxy- oder Alkoxycarbonylgruppe mit insgesamt 2 bis 4 Kohlenstoffatomen substituierte Alkenylgruppe mit 2 bis 5 Kohlenstoffatomen, eine Alkanoylgruppe mit 1 bis 3 Kohlenstoffatomen, eine Dialkoxymethyl- oder Trialkoxymethylgruppe mit 1 bis 3 Kohlenstoffatomen in jedem Alkylteil, eine Alkylendioxymethylgruppe mit 2 oder 3 Kohlenstoffatomen im Alkylenteil, eine 1,3-Oxazolin-2-yl- oder Cyanogruppe, eine gegebenenfalls durch eine oder zwei Alkylgruppen mit jeweils 1 bis 4 Kohlenstoffatomen in jedem Alkylteil substituierte Aminocarbonylgruppe, eine unverzweigte Alkyleniminocarbonylgruppe mit insgesamt 5 bis 8 Kohlenstoffatomen, eine Morpholinocarbonylgruppe, eine (Dialkyl-dioxolan-yl)-alkoxycarbonylgruppe mit insgesamt 7 bis 10 Kohlenstoffatomen oder eine Carboxygruppe und deren Ester, wobei Alkylester mit 1 bis 6 Kohlenstoffatomen im Alkylteil mit Ausnahme der $\alpha$-Position jeweils durch eine Hydroxy-, Alkoxy-, Amino-, Alkylamino-, Dialkylamino-, 1,3-Dimethyl-xanthin-7-yl-, Alkanoyloxy-, Arylcarbonyloxy-, Aralkanoyloxy- oder Pyridincarbonyloxygruppe oder durch zwei Hydroxygruppen, wobei eine Methyl- bzw. Methylengruppe jeweils nur durch eine Hydroxygruppe substituiert sein kann, oder durch eine Gruppe der Formel

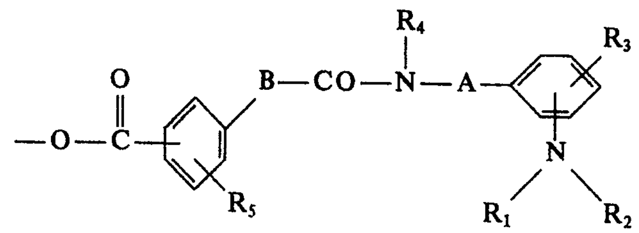

in der
A, B und $R_1$ bis $R_5$ wie eingangs definiert sind, substituiert sein können, wobei jeder Alkylsubstituent in den vorstehend genannten Alkylestern 1 bis 3 Kohlenstoffatome enthalten kann, bedeuten, sowie von deren optisch aktiven Antipoden und von deren Salzen, insbesondere von deren physiologisch verträglichen Salzen mit anorganischen oder organischen Säuren oder Basen, dadurch gekennzeichnet, daß

a) ein Amin der allgemeinen Formel

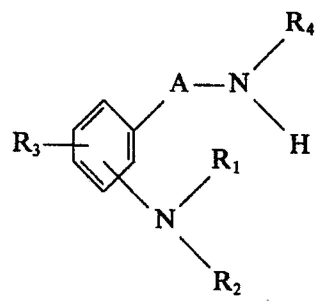

(II)

in der
A und $R_1$ bis $R_4$ wie eingangs definiert sind, bzw. wenn A eine der eingangs erwähnten Vinylidengruppen darstellt, dessen Tautomeres, dessen Lithium- oder Magnesiumhalogenid-Komplex mit einer Carbonsäure der allgemeinen Formel

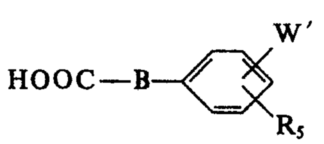

(III)

in der
$R_5$ und B wie eingangs definiert sind und
W' die für W eingangs erwähnten Bedeutungen besitzt oder eine durch einen Schutzrest geschützte Carboxylgruppe darstellt,
oder mit deren gegebenenfalls im Reaktionsgemisch hergestellten reaktionsfähigen Derivaten umgesetzt und erforderlichenfalls ein verwendeter Schutzrest abgespalten wird, oder

b) zur Herstellung einer Verbindung der allgemeinen Formel I, in der W eine Carboxygruppe dargestellt, eine Verbindung der allgemeinen Formel

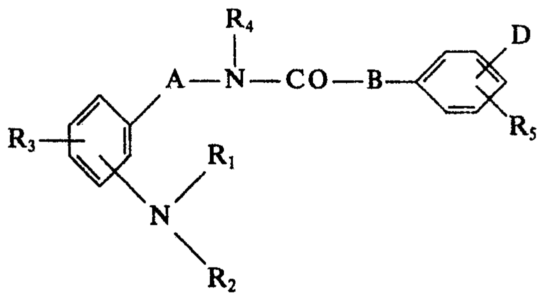

(IV)

in der
$R_1$ bis $R_5$, A und B wie eingangs definiert sind und D eine durch Hydrolyse, Thermolyse oder Hydrogenolyse in eine Carboxygruppe überführbare Gruppe darstellt, hydrolytisch, thermolytisch oder hydrogenolytisch gespalten wird, oder

c) eine gegebenenfalls im Reaktionsgemisch gebildete Verbindung der allgemeinen Formel

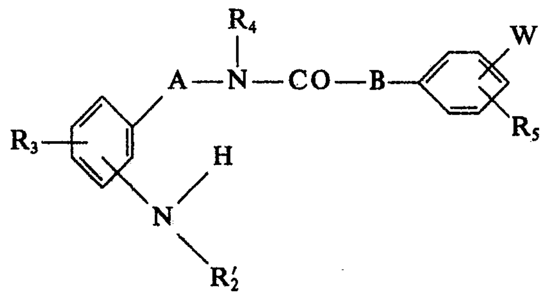

(V)

in der
$R_3$ bis $R_5$, A B und W wie eingangs definiert sind und $R_2$ ein Wasserstoffatom darstellt oder die für $R_2$ eingangs erwähnten Bedeutungen besitzt, mit einer Verbindung der allgemeinen Formel

$R_1' - E$ (VI)

in der
$R_1'$ die für $R_1$ eingangs erwähnten Bedeutungen besitzt oder zusammen mit dem Rest $R_2'$ der Formel V eine gegebenenfalls durch eine oder zwei Alkylgruppen mit jeweils 1 bis 3 Kohlenstoffatomen substituierte geradkettige Alkylengruppe mit 4 bis 6 Kohlenstoffatomen oder eine n-Pentylengruppe, in der die 3. Methylengruppe durch ein Sauerstoff- oder Schwefelatom ersetzt ist, und
E eine nukleophile Austrittsgruppe oder auch ein Wasserstoffatom, wenn in $R_1'$ eine Methylengruppe durch eine Aldehyd- oder Ketoncarbonylgruppe ersetzt ist, bedeuten, erforderlichenfalls in Gegenwart eines Reduktionsmittels alkyliert und gegebenenfalls anschließend hydrolysiert wird, oder

d) zur Herstellung einer Verbindung der allgemeinen Formel I, in der W eine Carboxylgruppe, eine Alkanoylgruppe mit 1 bis 3 Kohlenstoffatomen oder eine Alkylengruppe mit 1 bis 3 Kohlenstoffatomen bedeutet, eine Verbindung der allgemeinen Formel

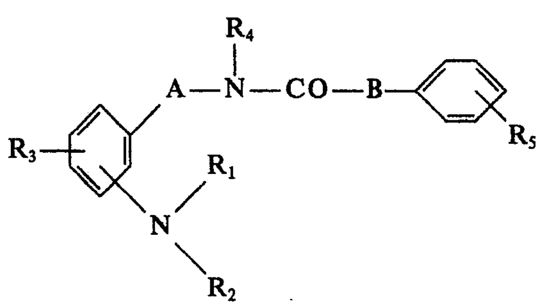

(VII)

in der
$R_1$ bis $R_5$, A und B wie eingangs definiert sind, mit Phosgen, einem Oxalhylhalogenid, einem Alkyl- oder Alkanoylhalogenid mit jeweils 1 bis 3 Kohlenstoffatomen im Alkylteil oder mit Cyanwasserstoff/Halogenwasserstoff in Gegenwart einer Lewis-Säure umgesetzt wird, oder

e) zur Herstellung einer Verbindung der allgemeinen Formel I, in der W die Carboxygruppe darstellt, eine Verbindung der allgemeinen Formel

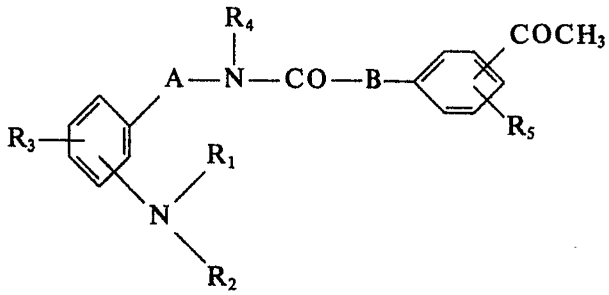

(VIII)

in der
$R_1$ bis $R_5$, A und B wie eingangs definiert sind, mit einem gegebenenfalls im Reaktionsgemisch hergestellten Hypohalogenit in Gegenwart einer Alkalibase umgesetzt wird, oder

f) zur Herstellung einer Verbindung der allgemeinen Formel I, in der W die Carboxygruppe darstellt, eine Verbindung der allgemeinen Formel

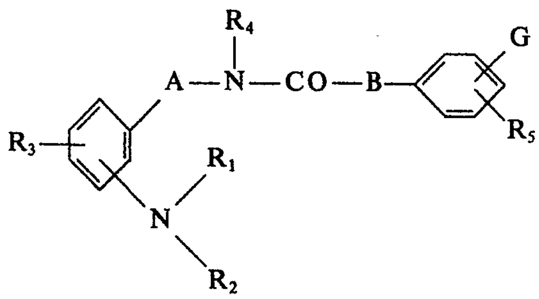

(IX)

in der
$R_1$ bis $R_5$, A und B wie eingangs definiert sind und G eine durch Oxidation in eine Carboxygruppe überführbare Gruppe darstellt,
oxidiert wird, oder

g) zur Herstellung einer Verbindung der allgemeinen Formel I, in der $R_3$ die Nitrogruppe darstellt, eine Verbindung der allgemeinen Formel

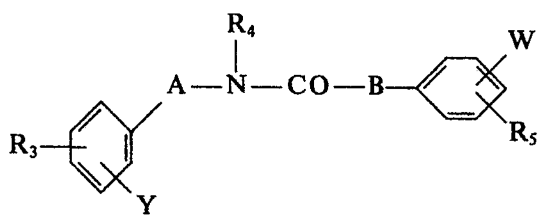

(X)

in der
$R_4$, $R_5$, A, B und W wie eingangs definiert sind,
$R_3$ eine Nitrogruppe und
Y einen nukleophil austauschbaren Rest bedeuten, mit einem Amin der allgemeinen Formel

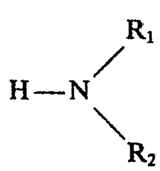

(XI)

in der
$R_1$ und $R_2$ wie eingangs definiert sind,
umgesetzt und gegebenenfalls anschließend hydrolysiert wird, oder

h) zur Herstellung einer Verbindung der allgemeinen Formel I, in der A eine Gruppe der Formel

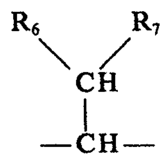

darstellt, wobei $R_6$ und $R_7$ wie eingangs definiert sind, eine Verbindung der allgemeinen Formel

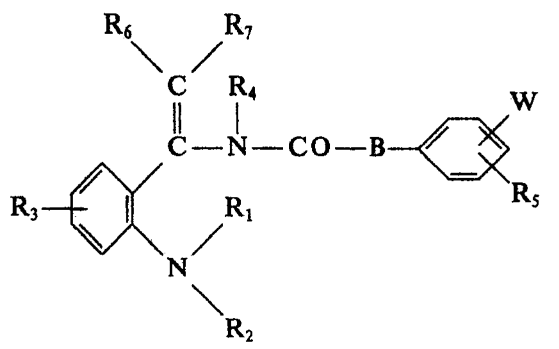

(XII)

in der
$R_1$ bis $R_7$, B und W wie eingangs definiert sind, mit Wasserstoff in Gegenwart eines Hydrierungskatalysators reduziert wird, oder

i) zur Herstellung einer Verbindung der allgemeinen Formel I, in der $R_4$ ein Wasserstoffatom und A eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierte Methylen- oder Äthylengruppe, eine durch eine Cycloalkylgruppe mit 3 bis 7 Kohlenstoffatomen, eine Alkoxyalkyl-, Carboxyl-, Alkoxycarbonyl-, Aryl- oder Aralkylgruppe substituierte Methylengruppe, wobei jeder der vorstehend erwähnten Alkylteile 1 bis 3 Kohlenstoffatom enthalten kann, oder eine Cycloalkylidengruppe mit 4 bis 7 Kohlenstoffatomen bedeuten, eine Verbindung der allgemeinen Formel

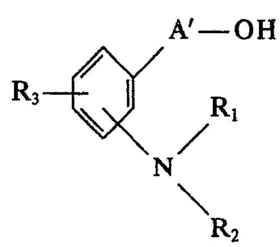

(XIII)

in der
$R_1$ bis $R_3$ wie eingangs definiert sind und
A'eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierte Methylen- oder Äthylengruppe, eine durch eine Cycloalkylgruppe mit 3 bis 7 Kohlenstoffatomen, eine Alkoxyalkyl-, Carboxyl-, Alkoxycarbonyl-, Aryl- oder Aralkylgruppe substituierte Methylengruppe, wobei jeder der vorstehend erwähnten Alkylteile 1 bis 3 Kohlenstoffatome enthalten kann, oder eine Cycloalkylidengruppe mit 4 bis 7 Kohlenstoffatomen bedeutet,
mit einer Verbindung der allgemeinen Formel

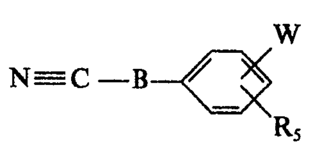

(XIV)

in der
$R_5$, B und W wie eingangs definiert sind, in Gegenwart einer starken Säure umgesetzt wird und gewünschtenfalls anschlienßend eine so erhaltene Verbindung der allgemeinen Formel I, in der W die Carboxylgruppe darstellt, mittels Veresterung oder Amidierung in eine entsprechende Verbindung der allgemeinen Formel I übergeführt wird und/oder
eine erhaltene Verbindung der allgemeinen Formel I, in der $R_3$ und/oder W eine Nitrogruppe

darstellen, mittels Reduktion in eine entsprechende Verbindung der allgemeinen Formel I, in der $R_3$ und/oder W eine Aminogruppe darstellen, übergeführt wird, und/oder
eine erhaltene Verbindung der allgemeinen Formel I, in der $R_3$ und/oder W eine Aminogruppe darstellen, über ein entsprechendes Diazoniumsalz in eine entsprechende Verbindung der allgemeinen Formel I, in der $R_3$ ein Wasserstoff- oder Halogenatom, eine Hydroxy-, Alkoxy-, Mercapto-, Alkylmercapto-, Chlorsulfonyl- oder Cyangruppe und/oder W ein Wasserstoff- oder Halogenatom oder eine Cyangruppe darstellen, übergeführt wird, wobei eine so gegebenenfalls erhaltene Verbindung der allgemeinen Formel I, in der $R_3$ die Hydroxygruppe darstellt, mittels Alkylierung in eine entsprechende Verbindung der allgemeinen Formel I, in der $R_3$ eine Alkoxygruppe darstellt oder eine so gegebenenfalls erhaltene Verbindung der allgemeinen Formel I, in der $R_3$ die Chlorsulfonylgruppe darstellt, mittels Ammoniak in eine entsprechende Verbindung der allgemeinen Formel I, in der $R_3$ die Aminosulfonylgruppe darstellt, übergeführt werden kann, und/oder
eine erhaltene Verbindung der allgemeinen Formel I, in der $R_3$ eine Aminogruppe darstellt, mittels Acylierung in eine entsprechende Verbindung der allgemeinen Formel I, in der $R_3$ eine Alkanoylamino-, Aroylamino-, Alkoxycarbonylamino- oder Alkylsulfonylaminogruppe darstellt, übergeführt wird, und/oder
eine erhaltene Verbindung der allgemeinen Formel I, in der $R_3$ eine Aminogruppe darstellt, mittels Alkylierung in eine entsprechende Verbindung der allgemeinen Formel I, in der $R_3$ eine Alkylamino- oder Dialkylaminogruppe darstellt, übergeführt wird und/oder
eine erhaltene Verbindung der allgemeinen Formel I, in der $R_3$ ein Chlor- oder Bromatom darstellt, mittels Enthalogenierung in eine entsprechende Verbindung der allgemeinen Formel I, in der $R_3$ ein Wasserstoffatom darstellt, übergeführt wird, und/oder
eine erhaltene Verbindung der allgemeinen Formel I, in der $R_3$ eine Nitrilgruppe darstellt, mittels Hydrolyse bzw. Alkoholyse in eine entsprechende Verbindung der allgemeinen Formel I, in der $R_3$ eine Aminocarbonyl-, Carboxy- bzw. Alkoxycarbonylgruppe darstellt, übergeführt wird, und/oder
eine erhaltene Verbindung der allgemeinen Formel I, in der $R_3$ eine Carboxy- oder Alkoxycarbonylgruppe und/oder W eine gegebenenfalls veresterte Carboxygruppe darstellen, mittels Reduktion in eine entsprechende Verbindung der allgemeinen Formel I, in der $R_3$ und/oder W eine Formyl- oder Hydroxymethylgruppe darstellen, übergeführt wird und/oder
eine erhaltene Verbindung der allgemeinen Formel I, in der W eine Alkoxycarbonylgruppe darstellt, in der die Alkoxygruppe 2 bis 6 Kohlenstoffatome enthalten kann und mit Ausnahme der $\alpha$-Position durch eine Hydroxygruppe substituiert ist, mittels Acylierung in eine entsprechende Verbindung der allgemeinen Formel I übergeführt wird und/oder
eine erhaltene Verbindung der allgemeinen Formel I, in der W eine Hydroxymethylgruppe darstellt, mittels Halogenierung und anschließender Umsetzung mit einem Malonsäurediester in eine entsprechende Verbindung der allgemeinen Formel I, in der W eine durch zwei Alkoxycarbonylgruppen substituierte Äthylgruppe darstellt, übergeführt wird und/oder
eine erhaltene Verbindung der allgemeinen Formel I, in der W eine Formylgruppe darstellt, mittels Kondensation und gegebenenfalls anschließender Hydrolyse und/oder Decarboxylierung in eine entsprechende Verbindung der allgemeinen Formel I, in der W eine durch eine Hydroxycarbonyl- oder Alkoxycarbonylgruppe substituierte Vinylgruppe darstellt, übergeführt wird und/oder
eine erhaltene Verbindung der allgemeinen Formel I, in der W eine durch zwei Alkoxycarbonylgruppen substituierte Äthylgruppe darstellt, mittels Hydrolyse und Decarboxylierung in eine entsprechende Verbindung der allgemeinen Formel I, in der W eine durch eine Carboxygruppe substituierte Äthylgruppe darstellt, übergeführt wird, und/oder
eine erhaltene Verbindung der allgemeinen Formel I, in der W eine Carboxygruppe darstellt, mittels Überführung in ein Sulfonsäurehydrazid und anschließend Disproportionierung in eine entsprechende Verbindung der allgemeinen Formel I, in der W eine Formylgruppe darstellt, übergeführt wird, und/oder
eine erhaltene Verbindung der allgemeinen Formel I, in der $R_1$ und $R_2$ zusammen mit dem dazwischenliegenden Stickstoffatom eine Aza-1,4-dioxa-spiro-alkylgruppe mit 6 bis 8 Kohlenstoffatomen darstellen, mittels Hydrolyse in eine entsprechende Verbindung der allgemeinen Formel I, in der $R_1$ und $R_2$ zusammen mit dem dazwischenliegenden Stickstoffatom eine unverzweigte Alkyleniminogruppe mit 4 bis 6 Kohlenstoffatomen darstellen, in der eine Methylengruppe durch eine Carbonylgruppe ersetzt ist, übergeführt wird, und/oder
eine erhaltene Verbindung der allgemeinen Formel I, in der $R_1$ und $R_2$ zusammen mit dem dazwischenliegenden Stickstoffatom eine unverzweigte Alkyleniminogruppe mit 4 bis 6 Kohlenstoffatomen darstellen, in der eine Methylengruppe durch eine Carbonylgruppe ersetzt ist, mittels Reduktion in eine entsprechende Hydroxy-alkylen-iminoverbindung der allgemeinen Formel I übergeführt wird, und/oder
eine erhaltene Verbindung der allgemeinen Formel I, in der W eine Aminocarbonylgruppe darstellt, mittels Dehydratisierung in eine entsprechende Verbindung der allgemeinen Formel

I, in der W eine Cyangruppe darstellt, übergeführt wird, und/oder
eine erhaltene Verbindung der allgemeinen Formel I in ihre Salze, insbesondere in ihre physiologisch verträglichen Salze mit anorganischen oder organischen Säuren und Basen übergeführt wird.

**Patentansprüche für den Vertragsstaat: AT**

1. Verfahren zur Herstellung von neuen Carbonsäureamiden der allgemeinen Formel

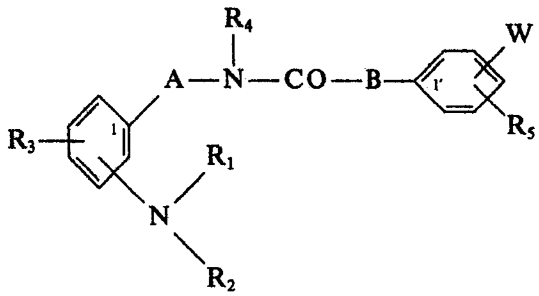

(I)

in der

$R_1$ und $R_2$, die gleich oder verschieden sein können, Alkylgruppen mit 1 bis 6 Kohlenstoffatomen oder Cycloalkylgruppen mit 5 bis 7 Kohlenstoffatomen oder

$R_1$ und $R_2$ zusammen mit dem dazwischenliegenden Stickstoffatom eine unverzweigte Alkyleniminogruppe mit 3 bis 6 Kohlenstoffatomen, die durch eine oder zwei Alkylgruppen mit jeweils 1 bis 3 Kohlenstoffatomen oder durch eine Hydroxygruppe substituiert sein kann, oder in der eine Methylengruppe durch eine Carbonylgruppe, durch ein Sauerstoff- oder Schwefelatom oder durch eine Iminogruppe, welche durch eine Alkylgruppe mit 1 bis 3 Kohlenstoffatomen, eine Aralkylgruppe mit 7 bis 10 Kohlenstoffatomen, eine Phenyl- oder Halogenphenylgruppe substituiert sein kann, oder in der eine Äthylengruppe durch eine o-Phenylengruppe ersetzt sein kann, eine unverzweigte Alkenyleniminogruppe mit 4 bis 6 Kohlenstoffatomen, eine gesättigte oder teilweise ungesättigte Azabicycloalkylgruppe mit 6 bis 10 Kohlenstoffatomen, eine Aza-1,4-dioxa-spiro-alkylgruppe mit 6 bis 8 Kohlenstoffatomen, eine Heptamethylenimino-, Octamethylenimino-, Nonamethylenimino- oder Decamethyleniminogruppe,

$R_3$ ein Wasserstoff- oder Halogenatom, eine Trifluormethyl-, Alkyl-, Hydroxy-, Alkoxy-, Alkanoyloxy-, Mercapto-, Alkylmercapto-, Nitro-, Amino-, Cyano-, Alkanoyl-, Carboxy-, Alkoxycarbonyl-, Aminocarbonyl-, Alkylaminocarbonyl-, Dialkylaminocarbonyl-, Aminosulfonyl-, Alkylamino-, Dialkylamino-, Alkanoylamino-, Alkoxycarbonylamino- oder Alkylsulfonylaminogruppe, wobei jeder Alkylteil in den vorstehend genannten Gruppen jeweils 1 bis 3 Kohlenstoffatome enthalten kann, eine Araloxygruppe mit 7 bis 10 Kohlenstoffatomen oder eine Arylcarbonylaminogruppe,

$R_4$ ein Wasserstoffatom oder eine Alkylgruppe mit 1 bis 3 Kohlenstoffatomen,

$R_5$ ein Wasserstoffatom, ein Halogenatom oder eine Alkylgruppe mit 1 bis 3 Kohlenstoffatomen,

A eine Bindung, eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierte Methylen- oder Äthylengruppe, eine durch eine Cycloalkylgruppe mit 3 bis 7 Kohlenstoffatomen, durch eine Hydroxyalkyl-, Alkoxyalkyl-, Cyano-, Carboxyl-, Alkoxycarbonyl-, Aminocarbonyl-, Alkylaminocarbonyl-, Dialkylaminocarbonyl-, Aryl- oder Aralkylgruppe substituierte Methylengruppe, wobei jeder der vorstehend erwähnten Alkylteile 1 bis 3 Kohlenstoffatomen enthalten kann, eine Cycloalkylidengruppe mit 3 bis 7 Kohlenstoffatomen oder eine Vinylidengruppe der Formel

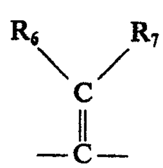

wobei
$R_6$ und $R_7$, die gleich oder verschieden sein können, Wasserstoffatome oder Alkylgruppen mit jeweils 1 bis 3 Kohlenstoffatomen oder auch einer der Reste $R_6$ oder $R_7$ eine Cycloalkylgruppe mit 3 bis 7 Kohlenstoffatomen, eine Aryl- oder Aralkylgruppe oder $R_6$ und $R_7$ zusammen mit dem dazwischenliegenden Kohlenstoffatom einen Cycloalkylidenrest mit 5 bis 7 Kohlenstoffatomen bedeuten,

B eine gegebenenfalls durch eine Alkylgruppe mit 1 bis 3 Kohlenstoffatomen substituierte Methylen- oder Äthylengruppe und

W ein Wasserstoff- oder Halogenatom, eine Nitrogruppe, eine gegebenenfalls durch eine Alkanoylgruppe mit 1 bis 3 Kohlenstoffatomen substituierte Aminogruppe, eine gegebenenfalls durch eine Hydroxy-, Carboxy- oder Alkoxycarbonylgruppe oder zwei Alkoxycarbonylgruppen mit jeweils insgesamt 2 bis 4 Kohlenstoffatomen substituierte Alkylgruppe mit 1 bis 3 Kohlenstoffatomen, eine durch eine Carboxy- oder Alkoxycarbonylgruppe mit insgesamt 2 bis 4 Kohlenstoffatomen substituierte Alkenylgruppe mit 2 bis 5 Kohlenstoffatomen, eine Alkanoylgruppe mit 1 bis 3 Kohlenstoffatomen, eine Dialkoxymethyl- oder Trialkoxymethylgruppe mit 1 bis 3 Kohlenstoffatomen in jedem Alkylteil, eine Alkylendioxymetylgruppe mit 2 oder 3 Kohlenstoffatomen im Alkylenteil, eine 1,3-Oxazolin-2-yl- oder Cyanogruppe, eine gegebenenfalls durch eine oder zwei Alkylgruppen mit jeweils 1 bis 4 Kohlenstoffatomen in jedem Alkylteil substituierte Aminocarbonylgruppe, eine unverzweigte Alkyleniminocarbonylgruppe mit insgesamt 5 bis 8 Kohlenstoffatomen, eine Morpholinocarbonylgruppe, eine (Dialkyl-dioxolan-yl)-alkoxycarbonylgruppe mit insgesamt 7 bis 10 Kohlenstoffatomen oder eine Carboxygruppe und deren Ester, wobei Alkylester mit 1 bis 6 Kohlenstoffatomen im Alkylteil mit Ausnahme der $\alpha$-Position jeweils durch eine Hydroxy-, Alkoxy-, Amino-, Alkylamino-, Dialkylamino-, 1,3,-Dimethyl-xanthin-7-yl-, Alkanoyloxy-, Arylcarbonyloxy-, Aralkanoyloxy- oder Pyridincarbonyloxygruppe oder durch zwei Hydroxygruppen, wobei eine Methyl- bzw. Methylengruppe jeweils nur durch eine Hydroxygruppe substituiert sein kann, oder durch eine Gruppe der Formel

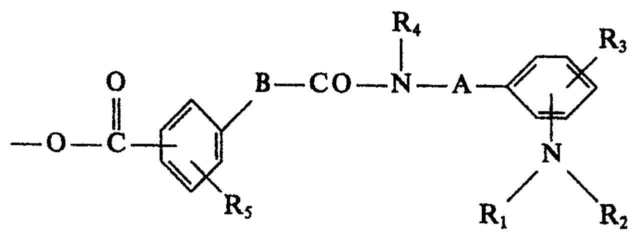

in der

A, B und $R_1$ bis $R_5$ wie eingangs definiert sind, substituiert sein können, wobei jeder Alkylsubstituent in den vorstehend genannten Alkylestern 1 bis 3 Kohlenstoffatome enthalten kann, bedeuten, sowie von deren optisch aktiven Antipoden und von deren Salzen, insbesondere von deren physiologisch verträglichen Salzen mit anorganischen oder organischen Säuren oder Basen, dadurch gekennzeichnet, daß

a) ein Amin der allgemeinen Formel

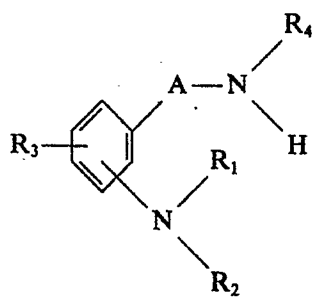

(II)

in der

A und $R_1$ bis $R_4$ wie eingangs definiert sind bzw. wenn A eine der eingangs erwähnten Vinylidengruppen darstellt, dessen Tautomeres, dessen Lithium- oder Magnesiumhalogenid-Komplex mit einer Carbonsäure der allgemeinen Formel

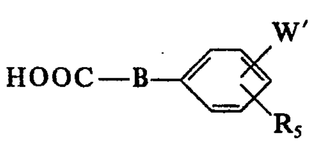

(III)

in der

$R_5$ und B wie eingangs definiert sind und

W' die für W eingangs erwähnten Bedeutungen besitzt oder eine durch einen Schutzrest geschützte Carboxylgruppe darstellt,

oder mit deren gegebenenfalls im Reaktionsgemisch hergestellten reaktionsfähigen Derivaten umgesetzt und erforderlichenfalls ein verwendeter Schutzrest abgespalten wird, oder

b) zur Herstellung einer Verbindung der allgemeinen Formel I, in der W eine Carboxygruppe darstellt, eine Verbindung der allgemeinen Formel

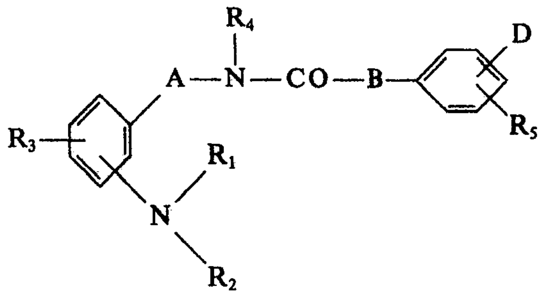

(IV)

in der
$R_1$ bis $R_5$, A und B wie eingangs definiert sind und D eine durch Hydrolyse, Thermolyse oder Hydrogenolyse in eine Carboxygruppe überführbare Gruppe darstellt, hydrolytisch, thermolytisch oder hydrogenolytisch gespalten wird, oder

c) eine gegebenenfalls im Reaktionsgemisch gebildete Verbindung der allgemeinen Formel

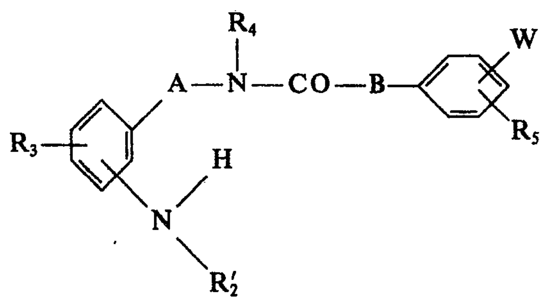

(V)

in der
$R_3$ bis $R_5$, A, B und W wie eingangs definiert sind und $R_2'$ ein Wasserstoffatom darstellt oder die für $R_2$ eingangs erwähnten Bedeutungen besitzt, mit einer Verbindung der allgemeinen Formel

$R_1'-E$ (VI)

in der
$R_1'$ die für $R_1$ eingangs erwähnten Bedeutungen besitzt oder zusammen mit dem Rest $R_2'$ der Formel V eine gegebenenfalls durch eine oder zwei Alkylgruppen mit jeweils 1 bis 3 Kohlenstoffatomen substituierte geradkettige Alkylengruppe mit 4 bis 6 Kohlenstoffatomen oder eine n-Pentylengruppe, in der die 3. Methylengruppe durch ein Sauerstoff- oder Schwefelatom ersetzt ist, und
E eine nukleophile Austrittsgruppe oder auch, ein Wasserstoffatom, wenn in $R_1'$ eine Methylengruppe durch eine Aldehyd- oder Ketoncarbonylgruppe ersetzt ist, bedeuten, erforderlichenfalls in Gegenwart eines Reduktionsmittels alkyliert und gegebenenfalls anschließend hydrolysiert wird, oder

d) zur Herstellung einer Verbindung der allgemeinen Formel I, in der W eine Carboxygruppe, eine Alkanoylgruppe mit 1 bis 3 Kohlenstoffatomen oder eine Alkylgruppe mit 1 bis 3 Kohlenstoffatomen bedeutet, eine Verbindung der allgemeinen Formel

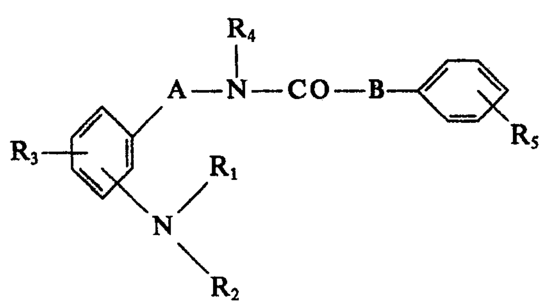

(VII)

in der

$R_1$ bis $R_5$, A und B wie eingangs definiert sind, mit Phosgen, einem Oxalylhalogenid, einem Alkyl- oder Alkanoylhalogenid mit jeweils 1 bis 3 Kohlenstoffatomen im Alkylteil oder mit Cyanwasserstoff/Halogenwasserstoff in Gegenwart einer Lewis-Säure umgesetzt wird, oder

e) zur Herstellung einer Verbindung der allgemeinen Formel I, in der W die Carboxygruppe darstellt, eine Verbindung der allgemeinen Formel

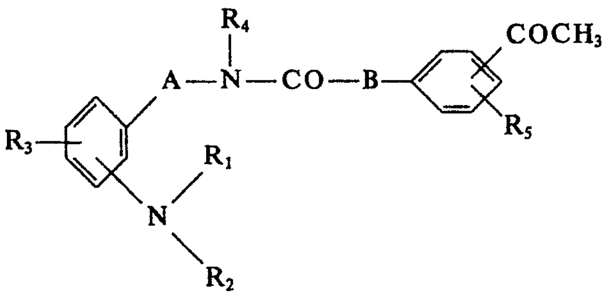

(VIII)

in der
$R_1$ bis $R_5$, A und B wie eingangs definiert sind,
mit einem gegebenenfalls im Reaktionsgemisch hergestellten Hypohalogenit in Gegenwart einer Alkalibase umgesetzt wird, oder

f) zur Herstellung einer Verbindung der allgemeinen Formel I, in der W die Carboxygruppe darstellt, eine Verbindung der allgemeinen Formel

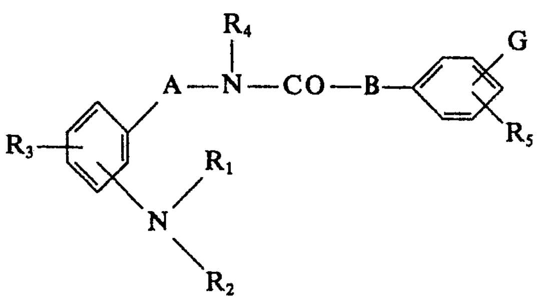

(IX)

in der
$R_1$ bis $R_5$, A und B wie eingangs definiert sind und G eine durch Oxidation in eine Carboxygruppe überführbare Gruppe darstellt, oxidiert wird, oder

g) zur Herstellung einer Verbindung der allgemeinen Formel I, in der $R_3$ die Nitrogruppe darstellt, eine Verbindung der allgemeinen Formel

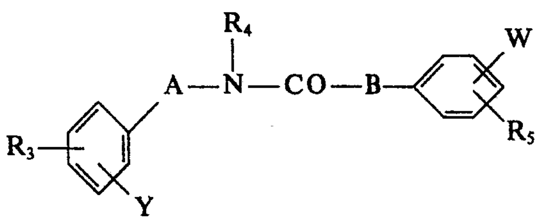

(X)

in der
$R_4$, $R_5$, A, B und W wie eingangs definiert sind,
$R_3$ eine Nitrogruppe und
Y einen nukleophil austauschbaren Rest bedeuten, mit einem Amin der allgemeinen Formel

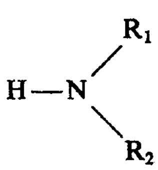

(XI)

in der
$R_1$ und $R_2$ wie eingangs definiert sind,
umgesetzt und gegebenenfalls anschließend hydrolysiert wird, oder

h) zur Herstellung einer Verbindung der allgemeinen Formel I, in der A eine Gruppe der Formel

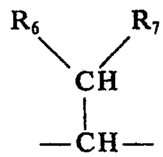

darstellt, wobei $R_6$ und $R_7$ wie eingangs definiert sind, eine Verbindung der allgemeinen Formel

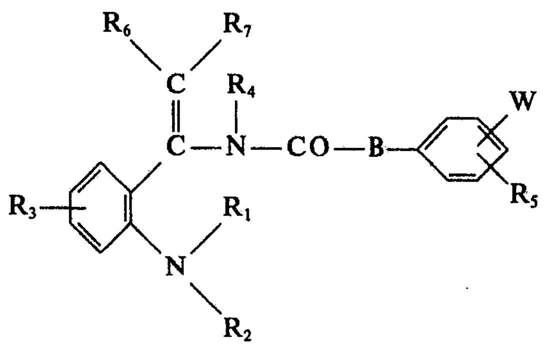

(XII)

in der
$R_1$ bis $R_7$, B und W wie eingangs definiert sind, mit Wasserstoff in Gegenwart eines Hydrierungskatalysators reduziert wird, oder

i) zur Herstellung einer Verbindung der allgemeinen Formel I, in der $R_4$ ein Wasserstoffatom und A eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierte Methylen- oder Äthylengruppe, eine durch eine Cycloalkylgruppe mit 3 bis 7 Kohlenstoffatomen, eine Alkoxyalkyl-, Carboxyl-, Alkoxycarbonyl-, Aryl- oder Aralkylgruppe substituierte Methylengruppe, wobei jeder der vorstehend erwähnten Alkylteile 1 bis 3 Kohlenstoffatome enthalten kann, oder eine Cycloalkylidengruppe mit 4 bis 7 Kohlenstoffatomen bedeuten, eine Verbindung der allgemeinen Formel

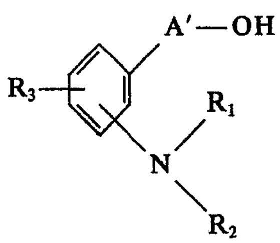

(XIII)

in der
$R_1$ bis $R_3$ wie eingangs definiert sind und
A' eine gegebenenfalls durch eine Alkylgruppe mit 1 bis 5 Kohlenstoffatomen substituierte Methylen- oder Äthylengruppe, eine durch zwei Alkylgruppen mit jeweils 1 bis 3 Kohlenstoffatomen substituierte Methylen- oder Äthylengruppe, eine durch eine Cycloalkylgruppe mit 3 bis 7 Kohlenstoffatomen, eine Alkoxyalkyl-, Carboxyl-, Alkoxycarbonyl-, Aryl- oder Aralkylgruppe substituierte Methylengruppe, wobei jeder der vorstehend erwähnten Alkylteile 1 bis 3 Kohlenstoffatome enthalten kann, oder eine Cycloalkylidengruppe mit 4 bis 7 Kohlenstoffatomen bedeutet,
mit einer Verbindung der allgemeinen Formel

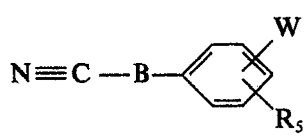

(XIV)

in der
$R_5$, B und W wie eingangs definiert sind,
in Gegenwart einer starken Säure umgesetzt wird
und gewünschtenfalls anschließend eine so erhaltene Verbindung der allgemeinen Formel I,

in der W die Carboxygruppe darstellt, mittels Veresterung oder Amidierung in eine entsprechende Verbindung der allgemeinen Formel I übergeführt wird und/oder
eine erhaltene Verbindung der allgemeinen Formel I, in der $R_3$ und/oder W eine Nitrogruppe darstellen, mittels Reduktion in eine entsprechende Verbindung der allgemeinen Formel I, in der $R_3$ und/oder W eine Aminogruppe darstellen, übergeführt wird, und/oder
eine erhaltene Verbindung der allgemeinen Formel I, in der $R_3$ und/oder W eine Aminogruppe darstellen, über ein entsprechendes Diazoniumsalz in eine entsprechende Verbindung der allgemeinen Formel I, in der $R_3$ eine Wasserstoff- oder Halogenatom, eine Hydroxy-, Alkoxy-, Mercapto-, Alkylmercapto-, Chlorsulfonyl- oder Cyangruppe und/oder W ein Wasserstoff- oder Halogenatom oder eine Cyangruppe darstellen, übergeführt wird, wobei eine so gegebenenfalls erhaltene Verbindung der allgemeinen Formel I, in der $R_3$ die Hydroxygruppe darstellt, mittels Alkylierung in eine entsprechende Verbindung der allgemeinen Formel I, in der $R_3$ eine Alkoxygruppe darstellt oder eine so gegebenenfalls erhaltene Verbindung der allgemeinen Formel I, in der $R_3$ die Chlorsulfonylgruppe darstellt, mittels Ammoniak in eine entsprechende Verbindung der allgemeinen Formel I, in der $R_3$ die Aminosulfonylgruppe darstellt, übergeführt werden kann, und/oder
eine erhaltene Verbindung der allgemeinen Formel I, in der $R_3$ eine Aminogruppe darstellt, mittels Acylierung in eine entsprechende Verbindung der allgemeinen Formel I, in der $R_3$ eine Alkanoylamino-, Aroylamino-, Alkoxycarbonylamino- oder Alkylsulfonylaminogruppe darstellt, übergeführt wird, und/oder
eine erhaltene Verbindung der allgemeinen Formel I, in der $R_3$ eine Aminogruppe darstellt, mittels Alkylierung in eine entsprechende Verbindung der allgemeinen Formel I, in der $R_3$ eine Alkylamino- oder Dialkylaminogruppe darstellt, übergeführt wird und/oder
eine erhaltene Verbindung der allgemeinen Formel I, in der $R_3$ ein Chlor- oder Bromatom darstellt, mittels Enthalogenierung in eine entsprechende Verbindung der allgemeinen Formel I, in der $R_3$ ein Wasserstoffatom darstellt, übergeführt wird, und/oder
eine erhaltene Verbindung der allgemeinen Formel I, in der $R_3$ eine Nitrilgruppe darstellt, mittels Hydrolyse bzw. Alkoholyse in eine entsprechende Verbindung der allgemeinen Formel I, in der $R_3$ eine Aminocarbonyl-, Carboxy- bzw. Alkoxycarbonylgruppe darstellt, übergeführt wird, und/oder
eine erhaltene Verbindung der allgemeinen Formel I, in der $R_3$ eine Carboxy- oder Alkoxycarbonylgruppe und/oder W eine gegebenenfalls veresterte Carboxygruppe darstellen, mittels Reduktion in eine entsprechende Verbindung der allgemeinen Formel I, in der $R_3$ und/oder W eine Formyl- oder Hydroxymethylgruppe darstellen, übergeführt wird und/oder
eine erhaltene Verbindung der allgemeinen Formel I, in der W eine Alkoxycarbonylgruppe darstellt, in der die Alkoxygruppe 2 bis 6 Kohlenstoffatome enthalten kann und mit Ausnahme der $\alpha$-Position durch eine Hydroxygruppe substituiert ist, mittels Acylierung in eine entsprechende Verbindung der allgemeinen Formel I übergeführt wird und/oder
eine erhaltene Verbindung der allgemeinen Formel I, in der W eine Hydroxymethylgruppe darstellt, mittels Halogenierung und anschließender Umsetzung mit einem Malonsäurediester in eine entsprechende Verbindung der allgemeinen Formel I, in der W eine durch zwei Alkoxycarbonylgruppen substituierte Äthylgruppe darstellt, übergeführt wird und/oder
eine erhaltene Verbindung der allgemeinen Formel I, in der W eine Formylgruppe darstellt, mittels Kondensation und gegebenenfalls anschließender Hydrolyse und/oder Decarboxylierung in eine entsprechende Verbindung der allgemeinen Formel I, in der W eine durch eine Hydroxycarbonyl- oder Alkoxycarbonylgruppe substituierte Vinylgruppe darstellt, übergeführt wird und/oder
eine erhaltene Verbindung der allgemeinen Formel I, in der W eine durch zwei Alkoxycarbonylgruppen substituierte Äthylgruppe darstellt, mittels Hydrolyse und Decarboxylierung in eine entsprechende Verbindung der allgemeinen Formel I, in der W eine durch eine Carboxygruppe substituierte Äthylgruppe darstellt, übergeführt wird, und/oder
eine erhaltene Verbindung der allgemeinen Formel I, in der W eine Carboxygruppe darstellt, mittels Überführung in ein Sulfonsäurehydrazid und anschließend Disproportionierung in eine entsprechende Verbindung der allgemeinen Formel I, in der W eine Formylgruppe dargestellt, übergeführt wird, und/oder
eine erhaltene Verbindung der allgemeinen Formel I, in der $R_1$ und $R_2$ zusammen mit dem dazwischenliegenden Stickstoffatom eine Aza-1,4-dioxa-spiro-alkylgruppe mit 6 bis 8 Kohlenstoffatomen darstellen, mittels Hydrolyse in eine entsprechende Verbindung der allgemeinen Formel I, in der $R_1$ und $R_2$ zusammen mit dem dazwischenliegenden Stickstoffatom eine unverzweigte Alkyleniminogruppe mit 4 bis 6 Kohlenstoffatomen darstellen, in der eine Methylengruppe durch eine Carbonylgruppe ersetzt ist, übergeführt wird, und/oder
eine erhaltene Verbindung der allgemeinen Formel I, in der $R_1$ und $R_2$ zusammen mit dem dazwischenliegenden Stickstoffatom eine unverzweigte Alkyleniminogruppe mit 4 bis 6 Kohlenstoffatomen darstellen, in der eine Methylengruppe durch eine Carbonylgruppe ersetzt ist, mittels Reduktion in eine entsprechende Hydroxyalkylen-íminoverbindung der allgemeinen

Formel I übergeführt wird, und/oder
eine erhaltene Verbindung der allgemeinen Formel I, in der W eine Aminocarbonylgruppe darstellt, mittels Dehydratisierung in eine entsprechende Verbindung der allgemeinen Formel I, in der W eine Cyangruppe darstellt, übergeführt wird, und/oder
eine erhaltene Verbindung der allgemeinen Formel I in ihre Salze, insbesondere in ihre physiologisch verträglichen Salze mit anorganischen oder organsichen Säuren und Basen übergeführt wird.

2. Verfahren nach Anspruch 1 zur Herstellung von neuen Cyrbonsäuren allgemeinen Formel

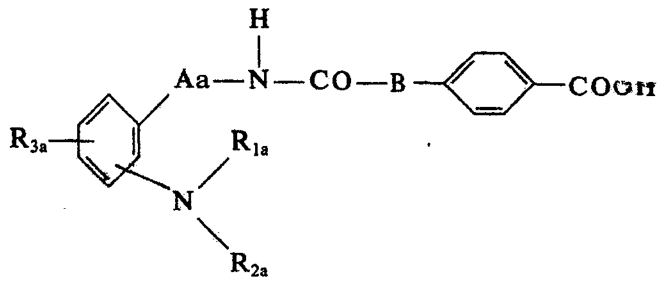

(Ia)

in der

B wie im Anspruch 1 definiert ist,
$R_{1a}$ und $R_{2a}$, die gleich oder verschieden sein können, Alkylgruppen mit 1 bis 6 Kohlenstoffatomen oder Cycloalkylgruppen mit 5 bis 7 Kohlenstoffatomen oder
$R_{1a}$ und $R_{2a}$ zusammen mit dem dazwischenliegenden Stickstoffatom eine gegebenenfalls durch eine oder zwei Alkylgruppen mit jeweils 1 bis 3 Kohlenstoffatomen substituierte Alkyleniminogruppe mit 4 bis 10 Kohlenstoffatomen im Alkylenring, eine Morpholino- oder Thiomorpholinogruppe,
$R_{3a}$ ein Wasserstoff- oder Halogenatom, eine Trifluormethyl-, Alkyl-, Hydroxy-, Alkoxy-, Mercapto-, Alkylmercapto-, Cyano-, Nitro-, Amino-, Aminocarbonyl-, Alkylamino-, Dialkylamino-, Alkanoylamino- oder Alkylsulfonylaminogruppe, wobei jeder Alkylteil in den vorstehend genannten Gruppen jeweils 1 bis 3 Kohlenstoffatome enthalten kann,und
Aa eine gegebenenfalls durch eine oder zwei Alkylgruppen mit jeweils 1 bis 3 Kohlenstoffatomen substituierte Methylen- oder Äthylengruppe bedeuten, sowie von deren Estern, von deren optisch aktiven Antipoden und von deren Salzen, insbesondere von deren physiologisch verträglichen Salzen mit anorganischen oder organischen Säuren oder Basen, dadurch gekennzeichnet, daß

a) ein Amin der allgemeinen Formel

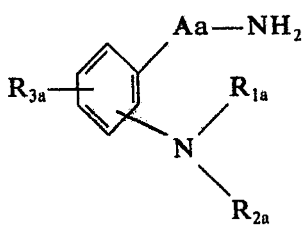

(IIa)

in der
Aa und $R_{1a}$ bis $R_{3a}$ wie eingangs definiert sind, mit einer Carbonsäure der allgemeinen Formel

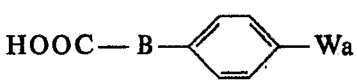

(IIIa)

in der
B wie im Anspruch 1 definiert ist und
Wa eine gegebenenfalls durch einen Schutzrest geschützte Carboxylgruppe darstellt, oder mit deren gegebenenfalls im Reaktionsgemisch hergestellten reaktionsfähigen Derivaten umgesetzt und erforderlichenfalls ein verwendeter Schutzrest abgespalten wird, oder

b) eine Verbindung der allgemeinen Formel

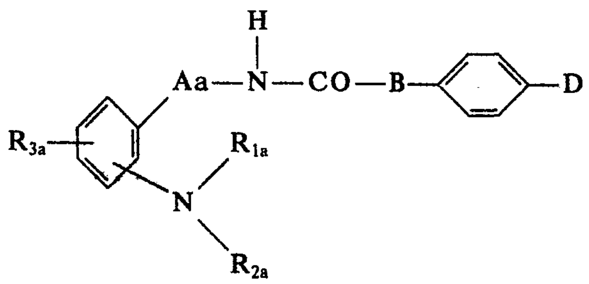

(IVa)

in der
B und D wie im Anspruch 1 und
Aa und $R_{1a}$ bis $R_{3a}$ wie eingangs definiert sind, hydrolysiert wird, oder

c) eine gegebenenfalls im Reaktionsgemisch gebildete Verbindung der allgemeinen Formel

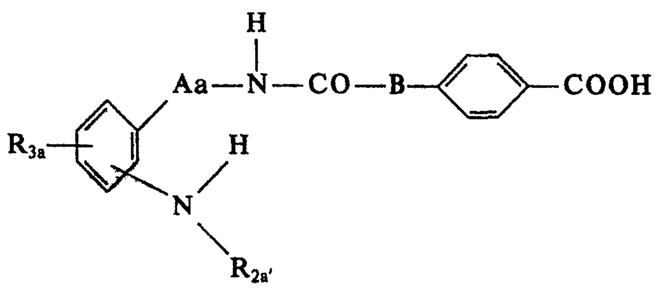

(Va)

in der
B wie im Anspruch 1 und
$R_{3a}$ und Aa wie eingangs definiert sind,
$R_{2a}'$ ein Wasserstoffatom darstellt oder die für $R_{2a}$ eingangs erwähnten Bedeutungen besitzt, oder deren Ester mit einer Verbindung der allgemeinen Formel

$$R_{1a}'-E \qquad \text{(VIa)}$$

in der
$R_{1a}'$ die für $R_{1a}$ eingangs erwähnten Bedeutungen besitzt oder zusamen mit dem Rest $R_{2a}'$ der Formel Va eine gegebenenfalls durch eine oder zwei Alkylgruppen mit jeweils 1 bis 3 Kohlenstoffatomen substituierte geradkettige Alkylengruppe mit 4 bis 6 Kohlenstoffatomen oder eine n-Pentylengruppe, in der die 3. Methylengruppe durch ein Sauerstoff- oder Schwefelatom erstzt ist, und
E eine nukleophile Austrittsgruppe bedeuten, umgesetzt und gegebenenfalls anschließend hydrolysiert wird, oder

d) eine Verbindung der allgemeinen Formel

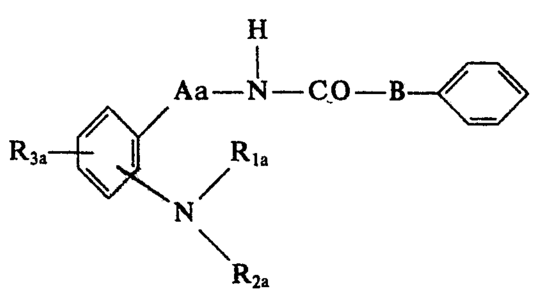

(VIIa)

in der
B wie im Anspruch 1 und
Aa und $R_{1a}$ bis $R_{3a}$ wie eingangs definiert sind, mit einem Oxalylhalogenid oder Phosgen in Gegenwart einer Lewis-Säure acyliert wird, oder

e) eine Verbindung der allgemeinen Formel

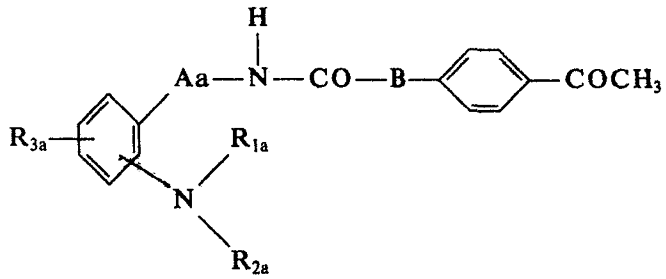

(VIIIa)

in der
B wie im Anspruch 1und
Aa und $R_{1a}$ bis $R_{3a}$ wie eingangs definiert sind,mit einem gegebenenfalls im Reaktionsgemisch hergestellten Hypohalogenit in Gegenwart einer Alkalibase umgesetzt wird, oder

f) eine Verbindung der allgemeinen Formel

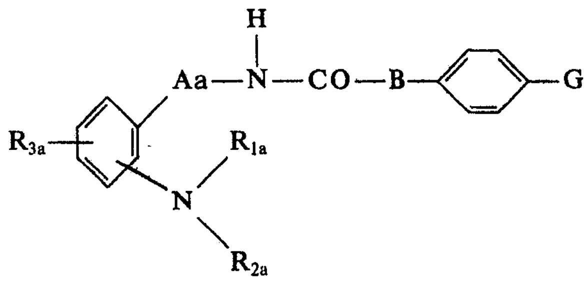

(IXa)

in der
B und G wie im Anspruch 1 und
Aa und $R_{1a}$ bis $R_{3a}$ wie eingangs definiert sind, oxidiert wird, oder

g) eine Verbindung der allgemeinen Formel

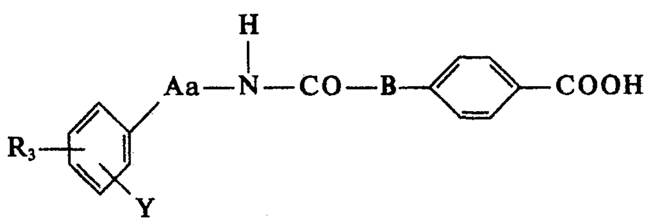

(Xa)

in der
$R_3$, B und Y wie im Anspruch 1 und
Aa wie eingangs definiert ist, oder deren Ester mit einem Amin der allgemeinen Formel

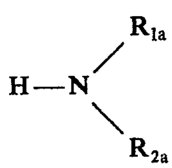

(XIa)

in der
$R_{1a}$ und $R_{2a}$ wie eingangs definiert sind, umgesetzt und gegebenenfalls anschließend hydrolysiert wird,
und gewünschtenfalls anschließend ein so erhaltenes Carbonsäureamid der allgemeinen Formel I mittels Veresterung in einen entsprechenden Ester übergeführt wird und/oder
eine erhaltene Verbindung der allgemeinen Formel I, in der $R_{3a}$ eine Nitrogruppe darstellt, mittels Reduktion in eine entsprechende Verbindung der allgemeinen Formel I, in der $R_{3a}$ eine Aminogruppe darstellt, übergeführt wird, und/oder
eine erhaltene Verbindung der allgemeinen Formel I, in der $R_{3a1}$ eine Aminogruppe darstellt, über ein entsprechendes Diazoniumsalz in eine entsprechende Verbindung der allgemeinen Formel I, in der $R_{3a}$ ein Wasserstoff- oder Halogenatom, eine Hydroxy-, Alkoxy-, Mercapto-, Alkylmercapto- oder Cyangruppe darstellt, übergeführt wird, wobei eine so gegebenenfalls

erhaltene Verbindung der allgemeinen Formel I, in der $R_{3a}$ die Hydroxygruppe darstellt, mittels Alkylierung in eine entsprechende Verbindung der allgemeinen Formel I, in der $R_{3a}$ eine Alkoxygruppe darstellt, übergeführt werden kann, und/oder
eine erhaltene Verbindung der allgemeinen Formel I, in $R_{3a}$ eine Aminogruppe darstellt, mittels Acylierung in eine entsprechende Verbindung der allgemeinen Formel I, in der $R_{3a}$ eine Alkanoylamino- oder Alkylsulfonylaminogruppe darstellt, übergeführt wird, und/oder
eine erhaltene Verbindung der allgemeinen Formel I, in der $R_{3a}$ ein Chlor- oder Bromatom darstellt, mittels Enthalogenierung in eine entsprechende Verbindung der allgemeinen Formel I, in der $R_{3a}$ ein Wasserstoffatom darstellt, übergeführt wird, und/oder
eine erhaltene Verbindung der allgemeinen Formel I, in der $R_{3a}$ eine Aminogruppe darstellt, mittels Alkylierung in eine entsprechende Verbindung der allgemeinen Formel I, in der $R_{3a}$ eine Alkylamino- oder Dialkylaminogruppe darstellt, übergeführt wird und/oder
eine erhaltene Verbindung der allgemeinen Formel I, in der $R_{3a}$ eine Nitrilgruppe darstellt, mittels Hydrolyse in eine entsprechende Verbindung der allgemeinen Formel I, in der $R_{3a}$ eine Aminocarbonylgruppe darstellt, übergeführt wird, und/oder
eine erhaltene Verbindung der allgemeinen Formel I in ihre Salze, insbesondere in ihre physiologisch verträglichen Salze mit anorganischen oder organischen Säuren und Basen übergeführt wird.

Priorität: 10. Januar 1981 (P 31 00 575.6)

3. Verfahren gemäß den Ansprüchen 1 und 2, dadurch gekennzeichnet, daß die Umsetzung in einem Lösungsmittel durchgeführt wird.

4. Verfahren gemäß den Ansprüchen 1a, 2a und 3, dadurch gekennzeichnet, daß die Umsetzung bei Temperaturen zwischen —25 und 250° C, vorzugsweise jedoch bei Temperaturen zwischen —10° C und der Siedetemperatur des verwendeten Lösungsmittels, durchgeführt wird.

5. Verfahren gemäß den Ansprüchen 1b, 2b und 3, dadurch gekennzeichnet, daß die Umsetzung bei Temperaturen zwischen —10 und 120° C, vorzugsweise bei Temperaturen zwischen Raumtemperatur und der Siedetemperatur des Reaktionsgemisches, durchgeführt wird.

6. Verfahren gemäß den Ansprüchen 1c, 2c und 3, dadurch gekennzeichnet, daß die Umsetzung bei Temperaturen zwischen 0 und 150° C, vorzugsweise jedoch bei Temperaturen zwischen 20 und 75° C, durchgeführt wird.

7. Verfahren gemäß den Ansprüchen 1d, 2d und 3, dadurch gekennzeichnet, daß die Umsetzung bei Temperaturen zwischen 0 und 120° C, vorzugsweise jedoch bei Temperaturen zwischen 20 und 80° C, durchgeführt wird.

8. Verfahren gemäß den Ansprüchen 1e, 2e und 3, dadurch gekennzeichnet, daß die Umsetzung in Gegenwart einer Alkalibase und bei Temperaturen zwischen 0 und 80° C, vorzugsweise jedoch bei Temperaturen zwischen 25 und 50° C, durchgeführt wird.

9. Verfahren gemäß den Ansprüchen 1f, 2f und 3, dadurch gekennzeichnet, daß die Umsetzung bei Temperaturen zwischen 0 und 100° C, vorzugsweise jedoch bei Temperaturen zwischen 20 und 50° C, durchgeführt wird.

10. Verfahren gemäß den Ansprüchen 1g und 2g, dadurch gekennzeichnet, daß die Umsetzung bei Temperaturen zwischen 20 und 150° C, vorzugsweise jedoch bei der Siedetemperatur des Reaktionsgemisches, z. B. bei 100° C, durchgeführt wird.

**Claims for the contracting states: BE, CH, DE, FR, IT, LI, LU, NL, SE**

1. New carboxylic acid amides of general formula

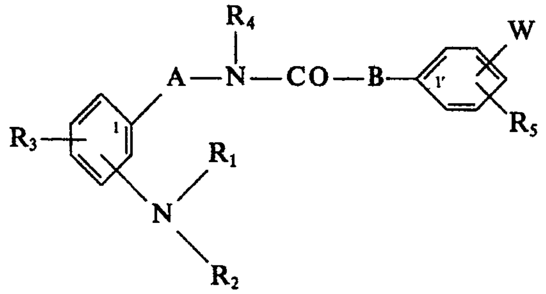

(I)

wherein

$R_1$ and $R_2$, which may be the same or different, represent alkyl groups with 1 to 6 carbon atoms or cycloalkyl groups with 5 to 7 carbon atoms, or

$R_1$ and $R_2$ together with the nitrogen atom to which they are attached, represent an unbranched alkyleneimino group with 3 to 6 carbon atoms, which may be substituted by one or two alkyl groups with 1 to 3 carbon atoms each or by a hydroxy group, or wherein a methylene group may be replaced by a carbonyl group, by an oxygen or sulfur atom or by an imino group, which may be substituted by an alkyl group with 1 to 3 carbon atoms, an aralkyl group with 7 to 10 carbon atoms, a phenyl or halophenyl group, or wherein an ethylene group may be replaced by an o-phenylene group, an unbranched alkenyleneimino group with 4 to 6 carbon atoms, a saturated or partly unsaturated azabicycloalkyl group with 6 to 10 carbon atoms, an aza-1,4-dioxa-spiro-alkyl group with 6 to 8 carbon atoms, a heptamethyleneimino, octamethyleneimino, nonamethyleneimino or decamethyleneimino group,

$R_3$ represents a hydrogen or halogen atom, a trifluoromethyl, alkyl, hydroxy, alkoxy, alkanoyloxy, mercapto, alkylmercapto, nitro, amino, cyano, alkanoyl, carboxy, alkoxycarbonyl, aminocarbonyl, alkylaminocarbonyl, dialkylaminocarbonyl, aminosulfonyl, alkylamino, dialkylamino, alkanoylamino, alkoxycarbonylamino or alkylsulfonylamino group, whereby each alkyl part in the above mentioned groups may contain from1 to 3 carbon atoms, an aralkoxy group with 7 to 10 carbon atoms or an arylcarbonylamino group,

$R_4$ represents a hydrogen atom or an alkyl group with 1 to 3 carbon atoms,

$R_5$ represents a hydrogen atom, a halogen atom or an alkyl group with 1 to 3 carbon atoms,

A represents a bond, a methylene or ethylene group optionally substituted by an alkyl group with 1 to 5 carbon atoms, a methylene or ethylene group substituted by two alkyl groups each with 1 to 3 carbon atoms, a methylene group substituted by a cycloalkyl group with 3 to 7 carbon atoms, by a hydroxyalkyl, alkoxyalkyl, cyano, carboxyl, alkoxycarbonyl, aminocarbonyl, alkylaminocarbonyl, dialkylaminocarbonyl, aryl or aralkyl group, whereby each of the above mentioned alkyl parts may contain from 1 to 3 carbon atoms, a cycloalkylidene group with 3 to 7 carbon atoms or a vinylidene group of formula

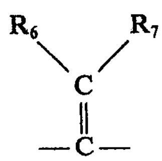

whereby

$R_6$ and $R_7$, which may be the same or different, represent hydrogen atoms or alkyl groups each with 1 to 3 carbon atoms or also one of the radicals $R_6$ or $R_7$ represents a cycloalkyl group with 3 to 7 carbon atoms, an aryl or aralkyl group or $R_6$ and $R_7$ together with the carbon atom to which they are attached, represent a cycloalkylidene radical with 5 to 7 carbon atoms,

B represents a methylene or ethylene group optionally substituted by an alkyl group with 1 to 3 carbon atoms, and

W represents a hydrogen or halogen atom, a nitro group, an amino group optionally substituted by an alkanoyl group with 1 to 3 carbon atoms, an alkyl group with 1 to 3 carbon atoms optionally substituted by a hydroxy, carboxy or alkoxycarbonyl group or two alkoxycarbonyl groups with altogether 2 to 4 carbon atoms each, an alkenyl group with 2 to 5 carbon atoms substituted by a carboxy or alkoxycarbonyl group with altogether 2 to 4 carbon atoms, an alkanoyl group with 1 to 3 carbon atoms, a dialkoxymethyl or trialkoxymethyl group with 1 to 3 carbon atoms in each alkyl part, an alkylenedioxymethyl group with 2 or 3 carbon atoms in the alkylene part, a 1,3-oxazolin-2-yl or cyano group, an aminocarbonyl group optionally substituted by one or two alkyl groups each with 1 to 4 carbon atoms in each alkyl part, an unbranched alkyleneiminocarbonyl group with altogether 5 to 8 carbon atoms, a morpholinocarbonyl group, a (dialkyldioxolan-yl)-alkoxycarbonyl group with altogether 7 to 10 carbon atoms or a carboxy group and their esters, whilst alkyl esters with 1 to 6 carbon atoms in the alkyl part with the exception of $\alpha$-position may each besubstituted by a hydroxy, alkoxy, amino, alkylamino, dialkylamino, 1,3-dimethylxanthin-7-yl, alkanoyloxy, aroyloxy, aralkanoyloxy or pyridine carbonyloxy group or by two hydroxy groups, whilst a methyl or methylene group in each case can be substituted only by one hydroxy group,
or by a group of formula

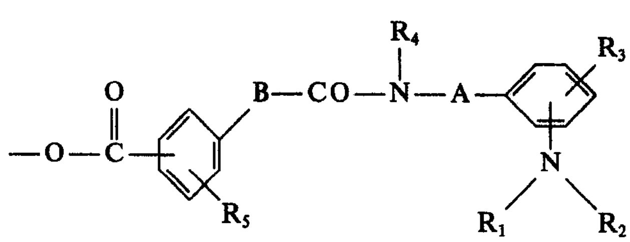

wherein

A, B and $R_1$ to $R_5$ are defined as hereinbefore whilst each alkyl substituent in the above mentioned alkyl esters may contain from 1 to 3 carbon atoms, and with the proviso that they contain an asymmetric carbon atom, their optically active antipodes, and their salts, especially their physiologically acceptable salts with inorganic or organic acids or bases.

2. New carboxylic acid amides of general formula I as claimed in claim 1, wherein

$R_1$ and $R_2$ together with the nitrogen atom to which they are attached represent a dialkylamino or N-alkyl-cyclohexyl-amino group, wherein each alkyl part may contain from 1 to 4 carbon atoms, an unbranched alkyleneimino group with 3 to 6 carbon atoms optionally substituted by one or two methyl groups, a hydroxypiperidino, piperidon-1-yl, tetrahydro-pyridino, morpholino, thiomorpholino, N-methyl-piperazino, N-benzyl-piperazino, N-chlorophenyl-piperazino, heptamethyleneimino or octamethyleneimino group, a saturated or partly unsaturated azabicycloalkyl group with 7 to 9 carbon atoms, an unbranched alkyleneimino group with 4 to 6 carbon atoms, wherein an ethylene group is replaced by an o-phenylene group, or a 1,4-dioxa-azaspiro-alkyl group with 7 to 8 carbon atoms,

$R_3$ represents a hydrogen, fluorine, chlorine, bromine, or iodine atom, a methyl, trifluoromethyl, hydroxy, methoxy, benzyloxy, acetoxy,mercapto, methylmercapto, nitro, amino, dimethylamino, acetylamino, methylsulfonylamino,benzoylamino, ethoxycarbonylamino, cyano, carboxy, methoxycarbonyl, ethoxycarbonyl, aminocarbonyl, acetyl or aminosulfonyl group,

$R_4$ represents a hydrogen atom or a methyl group,

$R_5$ represents a hydrogen atom, a chlorine atom or a methyl group,

A represents a bond, a methylene group optionally substituted by an alkyl group with 1 to 3 carbon atoms, a phenyl, cyclohexyl, carboxy, methoxycarbonyl or hydroxymethyl group, a dimethylmethylene, cyclopropylidene or ethylene group or a vinylidene group of formula

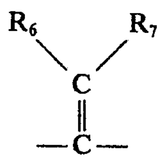

wherein

$R_6$ and $R_7$, which may be the same or different, represent hydrogen atoms or methyl groups or $R_6$ and $R_7$ together with the carbon atoms to which they are attached represent a cycloalkylidene radical with 5 or 6 carbon atoms,

B represents a methylene, ethylidene or ethylene group, and

W represents a hydrogen atom, a methyl, ethyl, hydroxymethyl, cyano or carboxyvinylene group, an alkyl group with 1 to 3 carbon atoms substituted by a carboxy group or by one or two alkoxycarbonyl groups with altogether 2 to 4 carbon atoms each, a carbonyl group substituted by a hydrogen atom, a methyl, ethyl, hydroxy, alkoxy, (2,2-dimethyldioxolan-4-yl)-methoxy, benzyloxy, pyridylmethoxy, amino, alkylamino, dialkylamino, piperidino or morpholino group, whereby each alkyl part in the above mentioned groups may contain from 1 to 3 carbon atoms, or a group of formula

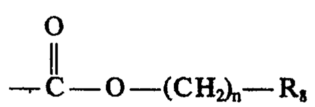

wherein

n represents the numbers 2, 3, oder 4, and

$R_8$ represents a hydroxy, methoxy, ethoxy, acetoxy, benzoyloxy, pyridine carbonyloxy, a dialkylamino group with 1 to 3 carbon atoms in each alkyl part, a 1,3-dimethylxanthin-7-yl group or a group of formula

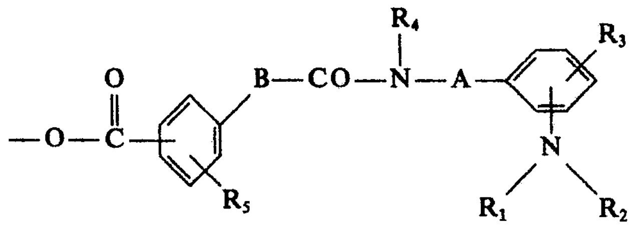

wherein

A, B and $R_1$ to $R_5$ are defined as mentioned in claim 2, and with the proviso that they contain an asymmetric carbon atom, their optically active antipodes and their salts, especially their physiologically acceptable salts with inorganic or organic acids or bases.

3. New carboxylic acid amides of general formula I as claimed in claim 1, wherein $R_1$ to $R_5$, A, B and W are defined as mentioned in claim 2,and the radical

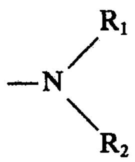

is present in 2-position and the radical W is present in 4'-position, and with the proviso that they contain an asymmetric carbon atom, their optically active antipodes, and their physiologically acceptable addition salts with inorganic or organic acids or bases.

4. New carboxylic acid amides of general formula

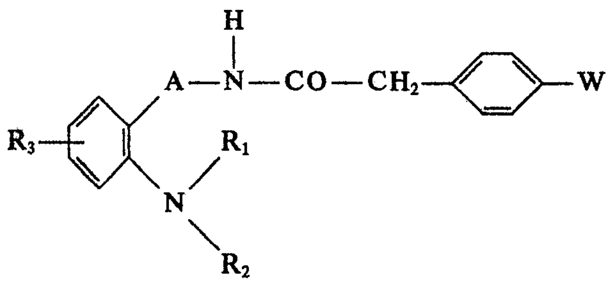

(Ia)

wherein

$R_1$ and $R_2$ together with the nitrogen atom to which they are attached, represent a dimethylamino, pyrrolidino, methylpyrrolidino, piperidino, methylpiperidino, dimethyl-piperidino,tetrahydro-pyridino, 2-octahydro-isoindolo, or hexamethyleneimino group,

$R_3$ represents a hydrogen, fluorine or chlorine atom or a methyl group,

A represents a methylene group optionally substituted by a cyclohexyl, phenyl, methoxycarbonyl, ethoxycarbonyl or an alkyl group with 1 to 3 carbon atoms, a dimethylmethylene or a vinylidene group of formula

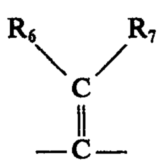

wherein

$R_6$ and $R_7$ each represents a hydrogen atom or together with the carbon atom to which they are attached, represent a cyclohexylidene group, and

W represents a methyl, hydroxymethyl or carboxymethyl group, a carbonyl group substituted by a hydrogen atom, a methyl, hydroxy, methoxy, ethoxy, propoxy, isopropoxy, 2-hydroxyethoxy, 2-methoxyethoxy or (2,2-dimethyl-dioxolan-4-yl)-methoxy or 2-diethylaminoethoxy group, and with the proviso that they contain an asymmetric carbon atom, their optically active antipodes, and their physiologically compatible addition salts with inorganic or organic acids or bases.

5. New carboxylic acid amides of general formula Ia as claimed in claim 4, wherein

$R_1$ and $R_2$ together with the nitrogen atom to which they are attached, represent a pyrrolidino, piperidino, methylpiperidino, hexamethyleneimino tetrahydro-pyridino or 2-octahydro-isoindolo group,
$R_3$ represents a hydrogen, fluorine or chlorine atom or a methyl group,
A represents a methylene group optionally substituted by a methyl, isopropyl, phenyl or methoxycarbonyl group, a dimethyl-methylene or vinylidene group and
W represents a methyl, hydroxymethyl, carboxymethyl, formyl or carboxy group or an alkoxycarbonyl group optionally substituted by a 2,2-dimethyl-dioxolan-4-yl group, wherein the alkoxy group may contain from 1 to 3 carbon atoms, and with the proviso that they contain an asymmetric carbon atom, their optically active antipodes, and their physiologically compatible addition salts with inorganic or organic acids or bases.

6. 4-[(1-(2-Piperidino-phenyl)-ethyl)-aminocarbonylmethyl]-benzoic acid, their alkyl esters with 1 to 3 carbon atoms, their optically active antipodes and their salts.

7. 4-[(2-Piperidino-benzhydrile)-aminocarbonylmethyl]benzoic acid, their alkyl esters with 1 to 3 carbon atoms, their optically active antipodes and their salts.

8. Pharmaceutical compositions containing a compound as claimed in claims 1 to 7 besides one or more inert carriers and/or diluting agents.

9. Pharmaceutical compositions as claimed in claim 8 with a blood-sugar lowering activity.

10. A process for the preparation of new carboxylic acid amides of general formula

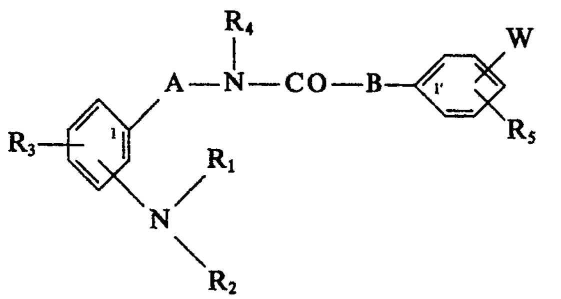

(I)

wherein

$R_1$ and $R_2$, which may be the same or different, represent alkyl groups with 1 to 6 carbon atoms or cycloalkyl groups with 5 to 7 carbon atoms, or
$R_1$ and $R_2$ together with the nitrogen atom to which they are attached, represent an unbranched alkyleneimino group with 3 to 6 carbon atoms, which may be substituted by one or two alkyl groups with 1 to 3 carbon atoms each or by a hydroxy group, or wherein a methylene group may be replaced by a carbonyl group, by an oxygen or sulfur atom or by an imino group, which may be substituted by an alkyl group with 1 to 3 carbon atoms, an aralkyl group with 7 to 10 carbon atoms, a phenyl or halophenyl group, or wherein an ethylene group may be replaced by an o-phenylene group, an unbranched alkenyleneimino group with 4 to 6 carbon atoms, a saturated or partly unsaturated azabicycloalkyl group with 6 to 10 carbon atoms, an aza-1,4-dioxa-spiro-alkyl group with 6 to 8 carbon atoms, a heptamethyleneimino, octamethyleneimino, nonamethyleneimino or decamethyleneimino group,
$R_3$ represents a hydrogen or halogen atom, a trifluoromethyl, alkyl, hydroxy, alkoxy, alkanoyloxy, mercapto, alkylmercapto, nitro, amino, cyano, alkanoyl, carboxy, alkoxycarbonyl, aminocarbonyl, alkylaminocarbonyl, dialkylaminocarbonyl, aminosulfonyl, alkylamino, dialkylamino, alkanoylamino, alkoxycarbonylamino or alkylsulfonylamino group, whilst each alkyl part in the above-mentioned groups may contain from 1 to 3 carbon atoms, an aralkoxy group with 7 to 10 carbon atoms or an arylcarbonylamino group,
$R_4$ represents a hydrogen atom or an alkyl group with 1 to 3 carbon atoms,
$R_5$ represents a hydrogen atom, a halogen atom or an alkyl group with 1 to 3 carbon atoms,
A represents a bond, a methylene or ethylene group optionally substituted by an alkyl group with 1 to 5 carbon atoms, a methylene or ethylene group substituted by two alkyl groups each with 1 to 3 carbon atoms, a methylene group substituted by a cycloalkyl group with 3 to 7 carbon atoms, by a hydroxyalkyl, alkoxyalkyl, cyano, carboxyl, alkoxycarbonyl, aminocarbonyl, alkylaminocarbonyl, dialkylaminocarbonyl, aryl or aralkyl group, whilst each of the abovementioned alkyl parts may contain from 1 to 3 carbon atoms, a cycloalkylidene group with 3 to 7 carbon atoms or a vinylidene group of formula

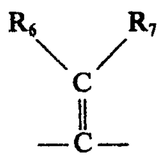

wherein

$R_6$ and $R_7$, which may be the same or different, represent hydrogen atoms or alkyl groups each with 1 to 3 carbon atoms or also one of the radicals $R_6$ or $R_7$ represents a cycloalkyl group with 3 to 7 carbon atoms, an aryl or alalkyl group or $R_6$ and $R_7$ together with the carbon atom to which they are attached represent a cycloalkylidene radical with 5 to 7 carbon atoms,

B represents a methylene or ethylene group optionally substituted by an alkyl group with 1 to 3 carbon atoms, and

W represents a hydrogen or halogen atom, a nitro group, an amino group optionally substituted by an alkanoyl group with 1 to 3 carbon atoms, an alkyl group with 1 to 3 carbon atoms optionally substituted by a hydroxy, carboxy or alkoxycarbonyl group or two alkoxycarbonyl groups with altogether 2 to 4 carbon atoms each, an alkenyl group with 2 to 5 carbon atoms substituted by a carboxy or alkoxycarbonyl group with altogether 2 to 4 carbon atoms, an alkanoyl group with 1 to 3 carbon atoms, a dialkoxymethyl or trialkoxymethyl group with 1 to 3 carbon atoms in each alkyl part, an alkylenedioxymethyl group with 2 or 3 carbon atoms in the alkylene part, a 1,3-oxazolin-2-yl or cyano group, an aminocarbonyl group optionally substituted by one or two alkyl groups each with 1 to 4 carbon atoms in each alkyl part, an unbranched alkyleneimino group with altogether 5 to 8 carbon atoms, a morpholinocarbonyl group, a (dialkyl-dioxolan-yl)-alkoxy group with altogether 7 to 10 carbon atoms or a carboxy group and their esters, whilst alkyl esters with 1 to 6 carbon atoms in the alkyl part with the exception of the $\alpha$-position may each be substituted by a hydroxy, alkoxy, amino, alkylamino, dialkylamino, 1,3-dimethyl-xanthin-7-yl, alkanoyloxy, arylcarbonyloxy, aralkanoyloxy or pyridine carbonyloxy group or by two hydroxy groups, whilst a methyl or methylene group in each case may be substituted only by a hydroxy group, or by a group or formula

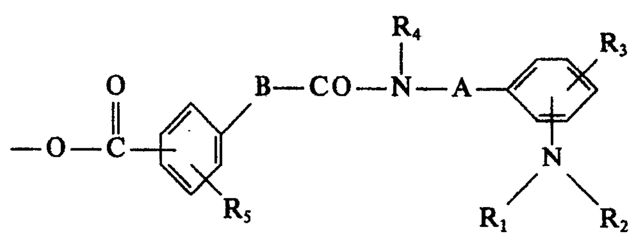

wherein

A, B and $R_1$ to $R_5$ are defined as hereinbefore,
whereby each alkyl substituent in the above-mentioned alkyl esters may contain from 1 to 3 carbon atoms, as well as of their optically active antipodes and of their salts, especially of their physiologically acceptable salts with inorganic or organic acids or bases,
characterised in that

a) an amine of general formula

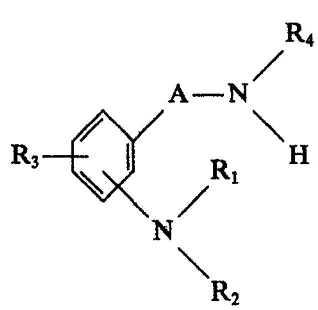

(II)

wherein

A and $R_1$ to $R_4$ are defined as mentioned before, or, if A represents one of the above-mentioned vinylidene groups, its tautomers, its lithium or magnesium halide complex, is reacted with a carboxylic acid of general formula

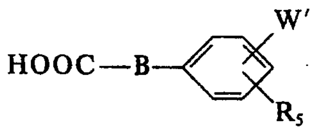

(III)

wherein

$R_5$ and B are defined as mentioned before and
W' has the meanings mentioned before for W or represents a carboxyl group protected by a protecting radical, or with the reactive derivatives thereof optionally prepared in the reactive mixture, and if necessary, any protective radical used is split off, or

b) for the preparation of a compound of general formula I wherein W represents a carboxy group, a compound of general formula

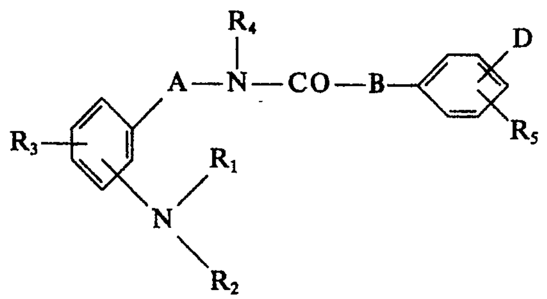

(IV)

wherein

$R_1$ to $R_5$, A and B are defined as mentioned before and D represents a group which can be converted into a carboxy group by means of hydrolysis, thermolysis or hydrogenolysis, is cleaved hydrolytically, thermolytically or hydrogeno-lytically, or

c) a compound of formula

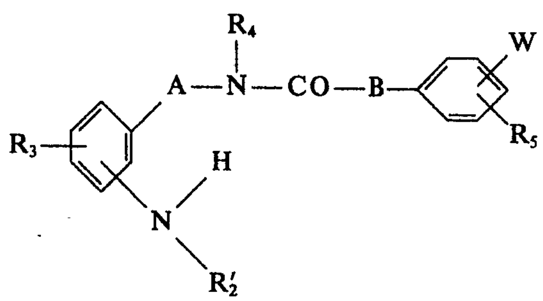

(V)

(wherein

$R_3$ to $R_5$, A, B and W are defined as mentioned before and $R_2$ represents a hydrogen atom or has the meanings mentioned before for $R_2$), optionally formed in the reaction mixture, is alkylated with a compound of general formula

$$R_1' - E \qquad \text{(VI)}$$

wherein

$R_1'$ has the meanings mentioned before for $R_1$ or together with the radical $R_2'$ of formula V represents a straight-chained alkylene group with 4 to 6 carbon atoms optionally substituted by one or two alkyl groups with 1 to 3 carbon atoms or an n-pentylene group, wherein the third methylene group is replaced by an oxygen or sulfur atom, and
E represents a nucleophilically exchangeable group or also a hydrogen atom, if in the radical $R_1'$ a methylene group is replaced by an aldehyde or ketonecarbonyl group, if necessary, in the presence of a reducing agent, and is optionally subsequently hydrolysed, or

d) for the preparation of a compound of general formula I wherein W represents a carboxy

group, and alkanoyl group with 1 to 3 carbon atoms or an alkyl group with 1 to 3 carbon atoms, a compound of general formula

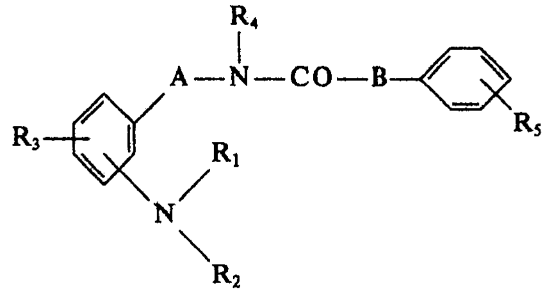

(VII)

wherein

$R_1$ to $R_5$, A and B are defined as mentioned before, is reacted with phosgene, an oxalyl halide, an alkyl or alkanoyl halide with 1 to 3 carbon atoms each in the alkyl part or with hydrogen cyanide/hydrogen halide in the presence of a Lewis acid, or

e) for the preparation of a compound of general formula I wherein W represents a carboxy group, a compound of general formula

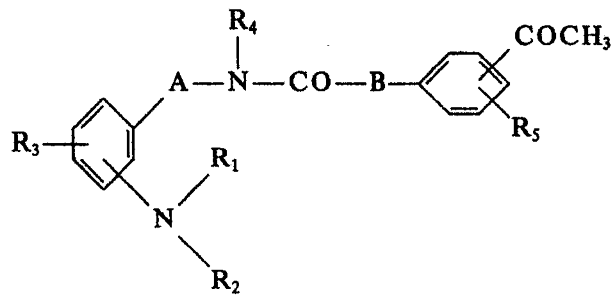

(VIII)

wherein

$R_1$ to $R_5$, A and B are defined as mentioned before, is reacted with a hypohalite optionally prepared in the reaction mixture, in the presence of an alkali metal base, or

f) for the preparation of a compound of general formula I wherein W represents the carboxy group, a compound of general formula

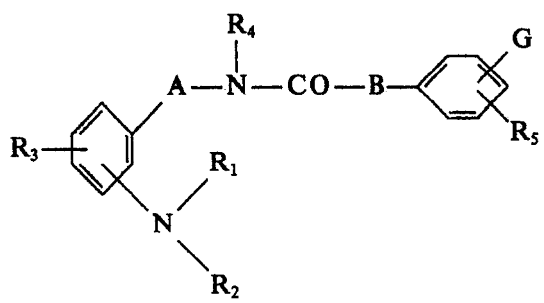

(IX)

wherein

$R_1$ to $R_5$, A and B are defined as mentioned before and G represents a group which can be converted into a carboxy group by means of oxidation, is oxidised, or

g) for the preparation of a compound of general formula I wherein $R_3$ represents a nitro group, a compound of general formula

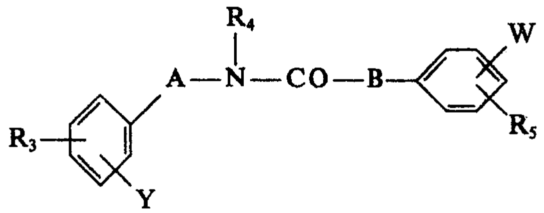

(X)

wherein

$R_4$, $R_5$, A, B and W are defined as mentioned before,
$R_3$ represents a nitro group and
Y represents a nucleophilically exchangeable group, is reacted with an amine of general formula

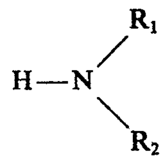

(XI)

wherein

$R_1$ and $R_2$ are defined as mentioned before, and optionally subsequently hydrolyzed, or

h) for the preparation of a compound of general formula I wherein A represents a group of formula

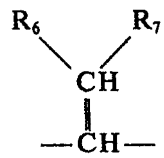

whereby

$R_6$ and $R_7$ are defined as mentioned before, a compound of general formula

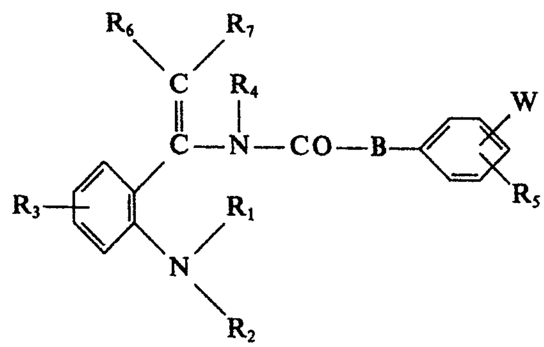

(XII)

wherein

$R_1$ to $R_7$, B and W are defined as mentioned before, is reduced with hydrogen in the presence of a hydrogenation catalyst, or

i) for the preparation of a compound of general formula I wherein $R_4$ represents a hydrogen atom and A represents a methylene or ethylene group optionally substituted by an alkyl group with 1 to 5 carbon atoms, a methylene or ethylene group substituted by two alkyl groups with 1 to 3 carbon atoms each, a methylene group substituted by a cycloalkyl group with 3 to 7 carbon atoms, an alkoxyalkyl, carboxyl, alkoxycarbonyl, aryl or aralkyl group, whilst each of the above-mentioned alkyl parts may contain from 1 to 3 carbon atoms, or a cycloalkylidene group with 4 to 7 carbon atoms, a compound of general formula

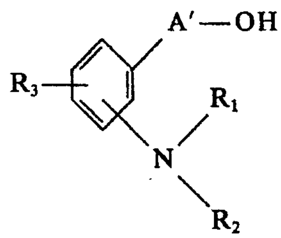

(XIII)

wherein

$R_1$ to $R_3$ are defined as hereinbefore and
A' represents a methylene or ethylene group optionally substituted by an alkyl group with 1 to 5 carbon atoms, a methylene or ethylene group substituted by two alkyl groups with 1 to 3 carbon atoms each, a methylene group substituted by a cycloalkyl group with 3 to 7 carbon atoms, an alkoxyalkyl, carboxyl, alkoxycarbonyl, aryl or aralkyl group, whereby each of the above mentioned alkyl parts may contain from 1 to 3 carbon atoms, or a cycloalkylidene group with 4 to 7 carbon atoms,
is reacted with a compound of general formula

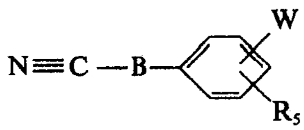

(XIV)

wherein

$R_5$, B and W are defined as mentioned before, in the presence of a strong acid,
and, if desired, subsequently, a compound of general formula I thus obtained wherein W represents a carboxy group is converted by esterification or amidation into a corresponding compound of general formula I, and/or
a compound of general formula I thus obtained wherein $R_3$ and/or W represent a nitro group, is converted by reduction into a corresponding compound of general formula I wherein $R_3$ and/or W represent an amino group, and/or
A compound of general formula I thus obtained wherein $R_3$ and/or W represent an amino group, is converted via a corresponding diazonium salt into a corresponding compound of general formula I, wherein $R_3$ represents a hydrogen or halogen atom, a hydroxy, alkoxy, mercapto, alkylmercapto, chlorosulfonyl or cyano group and/or W represents a hydrogen or halogen atom or a cyano group, whilst optionally a compound of general formula I thus obtained wherein $R_3$ represents the hydroxy group, can be converted by means of alkylation into a corresponding compound of general formula I, wherein $R_3$ represents an alkoxy group, or optionally a compound of general formula I thus obtained wherein $R_3$ represents the chlorosulfonyl group, can be converted by means of ammonia into a corresponding compound of general formula I $R_3$ represents the aminosulfonyl group, and/or
a compound of general formula I thus obtained wherein $R_3$ represents an amino group, is converted by acylation into a corresponding compound of general formula I wherein $R_3$ represents an alkanoylamino, aroylamino, alkoxycarbonylamino or alkylsulfonylamino group, and/or
a resulting compound of general formula I wherein $R_3$ represents an amino group, is converted by alkylation into a correspond compound of general formula I wherein $R_3$ represents an alkylamino or dialkylamino group, and/or
a resulting compound of general formula I wherein $R_3$ represents a chlorine or bromine atom, is converted by dehalogenation into a corresponding compound of general formula I wherein $R_3$ represents a hydrogen atom, and/or
a resulting compound of general formula I wherein $R_3$ represents a nitrile group, is converted by hydrolysis or alcoholysis into a corresponding compound of general formula I wherein $R_3$ represents an aminocarbonyl, carboxy or alkoxycarbonyl group, and/or
a resulting compound of general formula I wherein $R_3$ represents a carboxy or alkoxycarbonyl group and/or W represents a optionally esterified carboxy group, is converted by reduction into a corresponding compound of general formula I wherein $R_3$ and/or W represent a formyl or hydroxymethyl group, and/or
a resulting compound of general formula I wherein W represents an alkoxycarbonyl group, wherein the alkoxy group may contain from 2 to 6 carbon atoms and with the exception of the $\alpha$-position is substituted by a hydroxy group, is converted by acylation into a corresponding compound of general formula I, and/or
a resulting compound of general formula I wherein W represents a hydroxymethyl group, is converted by halogenation and subsequent reaction with a malonic acid diester into a corresponding compound of general formula I wherein W represents an ethyl group substituted by two alkoxycarbonyl groups, and/or
a resulting compound of general formula I wherein W represents a formyl group, is converted by condensation and optional subsequent hydrolysis and/or decarboxylation into a corresponding compound of general formula I wherein W represents a vinyl group substituted by a hydroxycarbonyl or alkoxycarbonyl group, and/or
a resulting compound of general formula I wherein W represents an ethyl group substituted by two alkoxycarbonyl groups, is converted by hydrolysis and decarboxylation into a corresponding compound of general formula I wherein W represents an ethyl group substituted by

a carboxy group, and/or
a resulting compound of general formula I wherein W represents a carboxy group, is converted by conversion into a sulfonic acid hydrazide and subsequent disproportionation into a corresponding compound of general formula I wherein W represents a formyl group, and/or
a resulting compound of formula I wherein $R_1$ and $R_2$ together with the nitrogen atom which they are attached represent an aza-1,4-dioxa-spiro-alkyl group with 6 to 8 carbon atoms, is converted by hydrolysis into a corresponding compound of general formula I wherein $R_1$ and $R_2$ together with the nitrogen atom to which they are attached represent an unbranched alkyleneimino group with 4 to 6 carbon atoms, wherein a methylene group is replaced by a carbonyl group, and/or
a resulting compound of general formula I wherein $R_1$ and $R_2$ together with the nitrogen atom to which they are attached represent an unbranched alkyleneimino group with 4 to 6 carbon atoms, wherein a methylene group is replaced by a carbonyl group, is converted by reduction into a corresponding hydroxy-alkyleneimino compound of general formula I, and/or
a resulting compound of general formula I wherein W represents an aminocarbonyl group, is converted by dehydration into a corresponding compound of general formula I wherein W represents a cyano group, and/or
a resulting compound of general formula I is converted into its salts, especially into its physiologically compatible salts with inorganic or organic acids and bases.

**Claims for the contracting state: AT**

1. A process for the preparation of new carboxylic acid amides of general formula

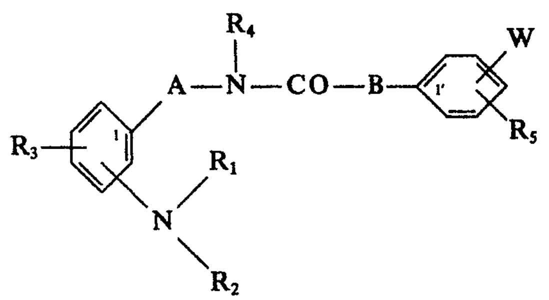

(I)

wherein

$R_1$ and $R_2$, which may be the same or different, represent alkyl groups with 1 to 6 carbon atoms or cycloalkyl groups with 5 to 7 carbon atoms, or
$R_1$ and $R_2$ together with the nitrogen atom to which they are attached, represent an unbranched alkyleneimino group with 3 to 6 carbon atoms, which may be substituted by one or two alkyl groups with 1 to 3 carbon atoms each or by a hydroxy group, or wherein a methylene group may be replaced by a carbonyl group, by an oxygen or sulfur atom or by an imino group, which may be substituted by an alkyl group with 1 to 3 carbon atoms, an aralkyl group with 7 to 10 carbon atoms, a phenyl or halophenyl group, or wherein an ethylene group may be replaced by an o-phenylene group, an unbranched alkenyleneimino group with 4 to 6 carbon atoms, a saturated or partly unsaturated azabicycloalkyl group with 6 to 10 carbon atoms, an aza-1,4-dioxa-spiro-alkyl group with 6 to 8 carbon atoms, a heptamethyleneimino, octamethyleneimino, nonamethyleneimino or decamethyleneimino group,
$R_3$ represents a hydrogen or halogen atom, a trifluoromethyl, alkyl, hydroxy, alkoxy, alkanoyloxy, mercapto, alkylmercapto, nitro, amino, cyano, alkanoyl, carboxy, alkoxycarbonyl, aminocarbonyl, alkylaminocarbonyl, dialkylaminocarbonyl, aminosulfonyl, alkylamino, dialkylamino, alkanoylamino, alkoxycarbonylamino or alkylsulfonylamino group, whilst each alkyl part in the above-mentioned groups may contain from1 to 3 carbon atoms, an aralkoxy group with 7 to 10 carbon atoms or an arylcarbonylamino group,
$R_4$ represents a hydrogen atom or an alkyl group with 1 to 3 carbon atoms,
$R_5$ represents a hydrogen atom, a halogen atom or an alkyl group with 1 to 3 carbon atoms,
A represents a bond, a methylene or ethylene group optionally substituted by an alkyl group with 1 to 5 carbon atoms, a methylene or ethylene group substituted by two alkyl groups each with 1 to 3 carbon atoms, a methylene group substituted by a cycloalkyl group with 3 to 7 carbon atoms, by a hydroxyalkyl, alkoxyalkyl, cyano, carboxyl, alkoxycarbonyl, aminocarbonyl, alkylaminocarbonyl, dialkylaminocarbonyl, aryl or aralkyl group, whilst each of the above-mentioned alkyl parts may contain from 1 to 3 carbon atoms, a cycloalkylidene group with 3 to 7 carbon atoms or a vinylidene group of formula

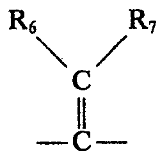

wherein

$R_6$ and $R_7$, which may be the same or different, represent hydrogen atoms or alkyl groups each with 1 to 3 carbon atoms or also one of the radicals $R_6$ or $R_7$ represents a cycloalkyl group with 3 to 7 carbon atoms, an aryl or alalkyl group or $R_6$ and $R_7$ together with the carbon atom to which they are attached represent a cycloalkylidene radical with 5 to 7 carbon atoms,

B represents a methylene or ethylene group optionally substituted by an alkyl group with 1 to 3 carbon atoms, and

W represents a hydrogen or halogen atom, a nitro group, an amino group optionally substituted by an alkanoyl group with 1 to 3 carbon atoms, an alkyl group with 1 to 3 carbon atoms optionally substituted by a hydroxy, carboxy or alkoxycarbonyl group or two alkoxycarbonyl groups with altogether 2 to 4 carbon atoms each, an alkenyl group with 2 to 5 carbon atoms substituted by a carboxy or alkoxycarbonyl group with altogether 2 to 4 carbon atoms, an alkanoyl group with 1 to 3 carbon atoms, a dialkoxymethyl or trialkoxymethyl group with 1 to 3 carbon atoms in each alkyl part, an alkylenedioxymethyl group with 2 or 3 carbon atoms in the alkylene part, a 1,3-oxazolin-2-yl or cyano group, an aminocarbonyl group optionally substituted by one or two alkyl groups each with 1 to 4 carbon atoms in each alkyl part, an unbranched alkyleneimino group with altogether 5 to 8 carbon atoms, a morpholinocarbonyl group, a (dialkyl-dioxolan-yl)-alkoxy group with altogether 7 to 10 carbon atoms or a carboxy group and their esters, whilst alkyl esters with 1 to 6 carbon atoms in the alkyl part with the exception of the $\alpha$-position may each be substituted by a hydroxy, alkoxy, amino, alkylamino, dialkylamino, 1,3-dimethyl-xanthin-7-yl, alkanoyloxy, arylcarbonyloxy, aralkanoyloxy or pyridine carbonyloxy group or by two hydroxy groups, whilst a methyl or methylene group in each case may be substituted only by a hydroxy group, or by a group or formula

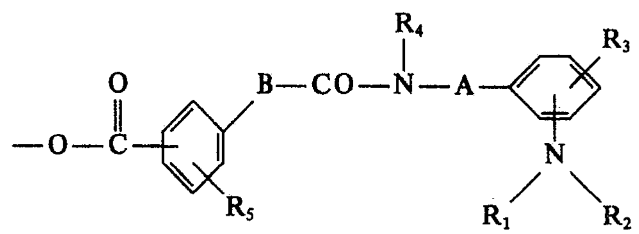

wherein

A, B and $R_1$ to $R_5$ are defined as hereinbefore,
whereby each alkyl substituent in the above-mentioned alkyl esters may contain from 1 to 3 carbon atoms, as well as of their optically active antipodes and of their salts, especially of their physiologically acceptable salts with inorganic or organic acids or bases,
characterised in that

a) an amine of general formula

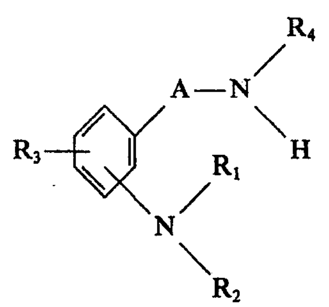

(II)

wherein

A and $R_1$ to $R_4$ are defined as mentioned before, or, if A represents one of the above-mentioned vinylidene groups, its tautomers, its lithium or magnesium halide complex, is reacted with a carboxylic acid of general formula

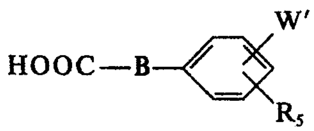

(III)

wherein

$R_5$ and B are defined as mentioned before and
W' has the meanings mentioned before for W or represents a carboxyl group protected by a protecting radical, or with the reactive derivatives thereof optionally prepared in the reactive mixture, and if necessary, any protective radical used is split off, or

b) for the preparation of a compound of general formula I wherein W represents a carboxy group, a compound of general formula

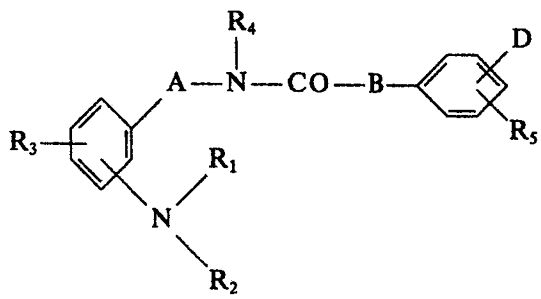

(IV)

wherein

$R_1$ to $R_5$, A and B are defined as mentioned before and D represents a group which can be converted into a carboxy group by means of hydrolysis, thermolysis or hydrogenolysis, is cleaved hydrolytically, thermolytically or hydrogenolytically, or

c) a compound of formula

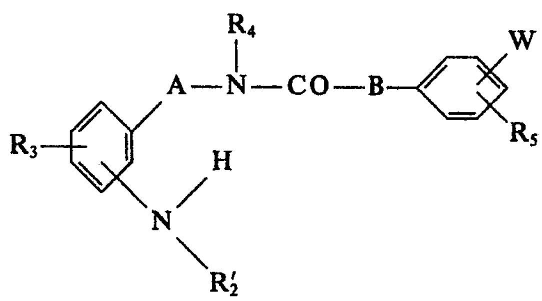

(V)

(wherein

$R_3$ to $R_5$, A, B and W are defined as mentioned before and $R_2$ represents a hydrogen atom or has the meanings mentioned before for $R_2$), optionally formed in the reaction mixture, is alkylated with a compound of general formula

$$R_1' - E \qquad (VI)$$

wherein

$R_1'$ has the meanings mentioned before for $R_1$ or together with the radical $R_2'$ of formula V represents a straight-chained alkylene group with 4 to 6 carbon atoms optionally substituted by one or two alkyl groups with 1 to 3 carbon atoms or an n-pentylene group, wherein the third methylene group is replaced by an oxygen or sulfur atom, and
E represents a nucleophilically exchangeable group or also a hydrogen atom, if in the radical $R_1'$ a methylene group is replaced by an aldehyde or ketonecarbonyl group, if necessary, in the presence of a reducing agent, and is optionally subsequently hydrolysed, or

d) for the preparation of a compound of general formula I wherein W represents a carboxy

group, and alkanoyl group with 1 to 3 carbon atoms or an alkyl group with 1 to 3 carbon atoms, a compound of general formula

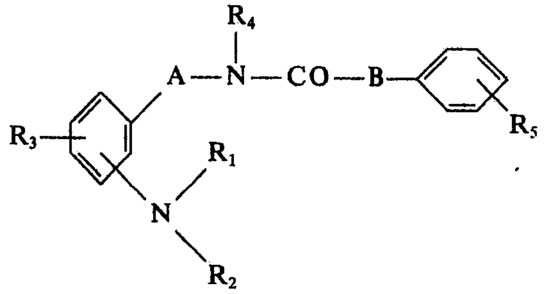

(VII)

wherein

$R_1$ to $R_5$, A and B are defined as mentioned before, is reacted with phosgene, an oxalyl halide, an alkyl or alkanoyl halide with 1 to 3 carbon atoms each in the alkyl part or with hydrogen cyanide/hydrogen halide in the presence of a Lewis acid, or

e) for the preparation of a compound of general formula I wherein W represents a carboxy group, a compound of general formula

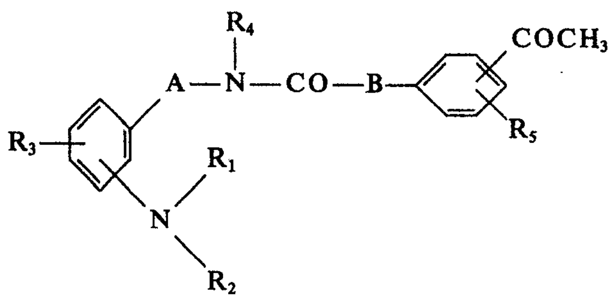

(VIII)

wherein

$R_1$ to $R_5$, A and B are defined as mentioned before, is reacted with a hypohalite optionally prepared in the reaction mixture, in the presence of an alkali metal base, or

f) for the preparation of a compound of general formula I wherein W represents the carboxy group, a compound of general formula

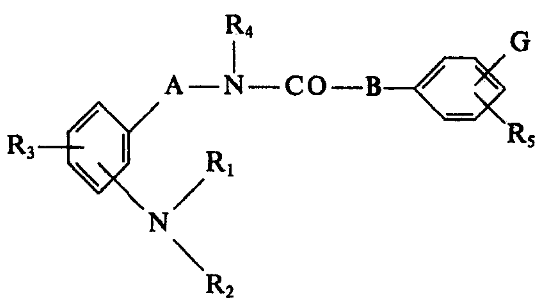

(IX)

wherein

$R_1$ to $R_5$, A and B are defined as mentioned before and G represents a group which can be converted into a carboxy group by means of oxidation, is oxidised, or

g) for the preparation of a compound of general formula I wherein $R_3$ represents a nitro group, a compound of general formula

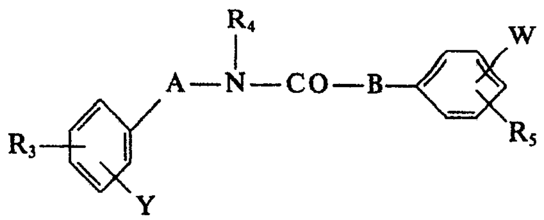

(X)

wherein

$R_4$, $R_5$, A, B and W are defined as mentioned before,
$R_3$ represents a nitro group and
Y represents a nucleophilically exchangeable group, is reacted with an amine of general formula

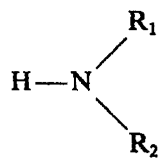

(XI)

wherein

$R_1$ and $R_2$ are defined as mentioned before, and optionally subsequently hydrolyzed, or

h) for the preparation of a compound of general formula I wherein A represents a group of formula

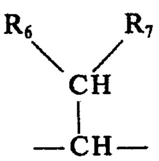

whereby

$R_6$ and $R_7$ are defined as mentioned before, a compound of general formula

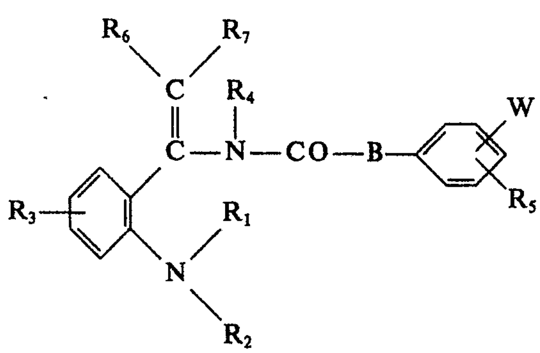

(XII)

wherein

$R_1$ to $R_7$, B and W are defined as mentioned before, is reduced with hydrogen in the presence of a hydrogenation catalyst, or

i) for the preparation of a compound of general formula I wherein $R_4$ represents a hydrogen atom and A represents a methylene or ethylene group optionally substituted by an alkyl group with 1 to 5 carbon atoms, a methylene or ethylene group substituted by two alkyl groups with 1 to 3 carbon atoms each, a methylene group substituted by a cycloalkyl group with 3 to 7 carbon atoms, an alkoxyalkyl, carboxyl, alkoxycarbonyl, aryl or aralkyl group, whilst each of the above-mentioned alkyl parts may contain from 1 to 3 carbon atoms, or a cycloalkylidene group with 4 to 7 carbon atoms, a compound of general formula

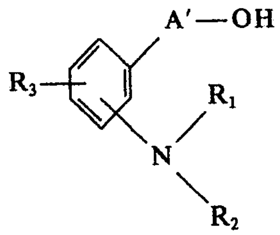

(XIII)

wherein

$R_1$ to $R_3$ are defined as hereinbefore and
A′ represents a methylene or ethylene group optionally substituted by an alkyl group with 1 to 5 carbon atoms, a methylene or ethylene group substituted by two alkyl groups with 1 to 3 carbon atoms each, a methylene group substituted by a cycloalkyl group with 3 to 7 carbon atoms, an alkoxyalkyl, carboxyl, alkoxycarbonyl, aryl or aralkyl group, whereby each of the above mentioned alkyl parts may contain from 1 to 3 carbon atoms, or a cycloalkylidene group with 4 to 7 carbon atoms,
is reacted with a compound of general formula

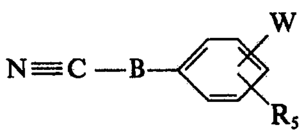

(XIV)

wherein

$R_5$, B and W are defined as mentioned before, in the presence of a strong acid,
and, if desired, subsequently, a compound of general formula I thus obtained wherein W represents a carboxy group is converted by esterification or amidation into a corresponding compound of general formula I, and/or
a compound of general formula I thus obtained wherein $R_3$ and/or W represent a nitro group, is converted by reduction into a corresponding compound of general formula I wherein $R_3$ and/or W represent an amino group, and/or
A compound of general formula I thus obtained wherein $R_3$ and/or W represent an amino group, is converted via a corresponding diazonium salt into a corresponding compound of general formula I, wherein $R_3$ represents a hydrogen or halogen atom, a hydroxy, alkoxy, mercapto, alkylmercapto, chlorosulfonyl or cyano group and/or W represents a hydrogen or halogen atom or a cyano group, whilst optionally a compound of general formula I thus obtained wherein $R_3$ represents the hydroxy group, can be converted by means of alkylation into a corresponding compound of general formula I, wherein $R_3$ represents an alkoxy group, or optionally a compound of general formula I thus obtained wherein $R_3$ represents the chlorosulfonyl group, can be converted by means of ammonia into a corresponding compound of general formula I $R_3$ represents the aminosulfonyl group, and/or
a compound of general formula I thus obtained wherein $R_3$ represents an amino group, is converted by acylation into a corresponding compound of general formula I wherein $R_3$ represents an alkanoylamino, aroylamino, alkoxycarbonylamino or alkylsulfonylamino group, and/or
a resulting compound of general formula I wherein $R_3$ represents an amino group, is converted by alkylation into a correspond compound of general formula I wherein $R_3$ represents an alkylamino or dialkylamino group, and/or
a resulting compound of general formula I wherein $R_3$ represents a chlorine or bromine atom, is converted by dehalogenation into a corresponding compound of general formula I wherein $R_3$ represents a hydrogen atom, and/or
a resulting compound of general formula I wherein $R_3$ represents a nitrile group, is converted by hydrolysis or alcoholysis into a corresponding compound of general formula I wherein $R_3$ represents an aminocarbonyl, carboxy or alkoxycarbonyl group, and/or
a resulting compound of general formula I wherein $R_3$ represents a carboxy or alkoxycarbonyl group and/or W represents a optionally esterified carboxy group, is converted by reduction into a corresponding compound of general formula I wherein $R_3$ and/or W represent a formyl or hydroxymethyl group, and/or
a resulting compound of general formula I wherein W represents an alkoxycarbonyl group, wherein the alkoxy group may contain from 2 to 6 carbon atoms and with the exception of the $\alpha$-position is substituted by a hydroxy group, is converted by acylation into a corresponding compound of general formula I, and/or

a resulting compound of general formula I wherein W represents a hydroxymethyl group, is converted by halogenation and subsequent reaction with a malonic acid diester into a corresponding compound of general formula I wherein W represents an ethyl group substituted by two alkoxycarbonyl groups, and/or

a resulting compound of general formula I wherein W represents a formyl group, is converted by condensation and optional subsequent hydrolysis and/or decarboxylation into a corresponding compound of general formula I wherein W represents a vinyl group substituted by a hydroxycarbonyl or alkoxycarbonyl group, and/or

a resulting compound of general formula I wherein W represents an ethyl group substituted by two alkoxycarbonyl groups, is converted by hydrolysis and decarboxylation into a corresponding compound of general formula I wherein W represents an ethyl group substituted by a carboxy group, and/or

a resulting compound of general formula I wherein W represents a carboxy group, is converted by conversion into a sulfonic acid hydrazide and subsequent disproportionation into a corresponding compound of general formula I wherein W represents a formyl group, and/or

a resulting compound of formula I wherein $R_1$ and $R_2$ together with the nitrogen atom which they are attached represent an aza-1,4-dioxa-spiro-alkyl group with 6 to 8 carbon atoms, is converted by hydrolysis into a corresponding compound of general formula I wherein $R_1$ and $R_2$ together with the nitrogen atom to which they are attached represent an unbranched alkyleneimino group with 4 to 6 carbon atoms, wherein a methylene group is replaced by a carbonyl group, and/or

a resulting compound of general formula I wherein $R_1$ and $R_2$ together with the nitrogen atom to which they are attached represent an unbranched alkyleneimino group with 4 to 6 carbon atoms, wherein a methylene group is replaced by a carbonyl group, is converted by reduction into a corresponding hydroxy-alkyleneimino compound of general formula I, and/or

a resulting compound of general formula I wherein W represents an aminocarbonyl group, is converted by dehydration into a corresponding compound of general formula I wherein W represents a cyano group, and/or

a resulting compound of general formula I is converted into its salts, especially into its physiologically compatible salts with inorganic or organic acids and bases.

2. Process as claimed in claim 1 for preparing new carboxylic acides of general formula

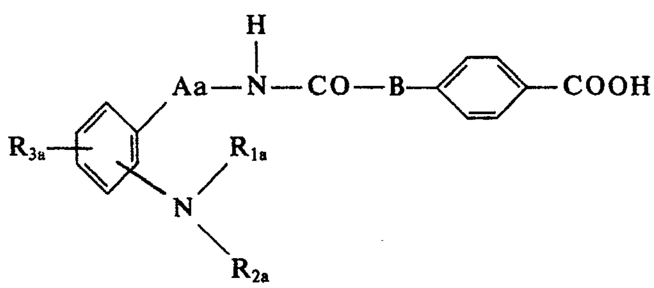

(I)

wherein

B is defined as in claim 1,

$R_{1a}$ and $R_{2a}$, which may be identical or different, represent alkyl groups with 1 to 6 carbon atoms or cycloalkyl groups with 5 to 7 carbon atoms or

$R_{1a}$ and $R_{2a}$ together with the nitrogen atom between them represent an alkyleneimino group with 4 to 10 carbon atoms in the alkylene ring optionally substituted by one or two alkyl groups with 1 to 3 carbon atoms, or a morpholino or thiomorpholino group,

$R_{3a}$ represents a hydrogen or halogen atom, a trifluoromethyl, alkyl, hydroxy, alkoxy, mercapto, alkylmercapto, cyano, nitro, amino, aminocarbonyl, alkylamino, dialkylamino, alkanoylamino or alkylsulphonylamino group, whilst each alkyl moiety in the above-mentioned groups may contain 1 to 3 carbon atoms, and

Aa represents a methylene or ethylene group optionally substituted by one or two alkyl groups each with 1 to 3 carbon atoms, and of the esters, optically active antipodes and salts thereof, particularly the physiologically acceptable salts thereof with inorganic or organic acids or bases, characterised in that

a) an amine of general formula

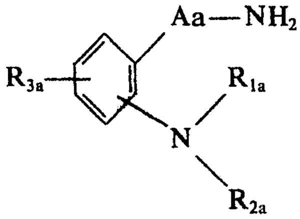

(IIa)

wherein

Aa and $R_{1a}$ to $R_{3a}$ are as hereinbefore defined, is reacted with a carboxylic acid of general formula

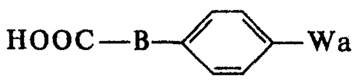

(IIIa)

wherein

B is defined as in claim 1
Wa represents a carboxyl group optionally protected by a protecting group, or with reactive derivatives thereof optionally prepared in the reaction mixture, and if necessary any protecting group used is split off, or

b) a compound of general formula

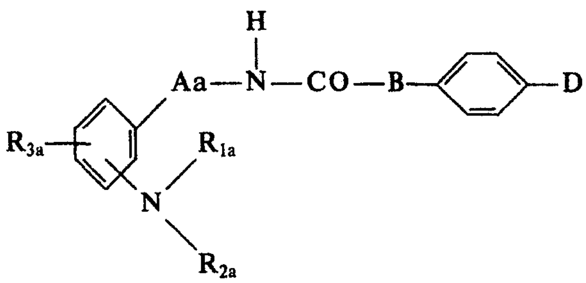

(IVa)

wherein

B and D are defined as in claim 1 and
Aa and $R_{1a}$ to $R_{3a}$ are defined as hereinbefore, is hydrolysed, or

c) a compound of general formula

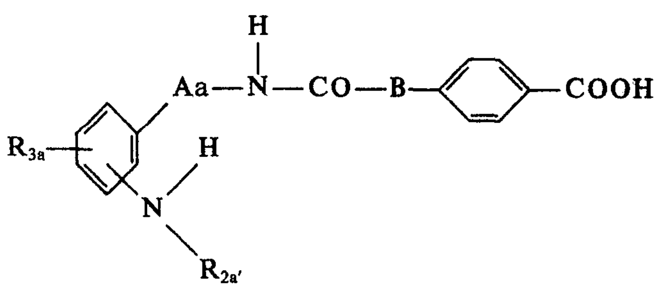

(Va)

optionally formed in the reaction mixture,

wherein

B is defined as in claim 1 and
$R_{3a}$ and Aa are as hereinbefore defined,

$R_{2a}$, represents a hydrogen atom or has the meanings given for $R_{2a}$ hereinbefore, or an ester thereof, is reacted with a compound of general formula

$$R_{1a}' - E \qquad (VIa)$$

wherein

$R_{1a}'$ has the meanings given for $R_{1a}$, hereinbefore or, together with the group $R_{2a}$, of formula Va, represents a straight-chained alkylene group with 4 to 6 carbon atoms optionally substituted by one or two alkyl groups, each with 1 to 3 carbon atoms, or an n-pentylene group wherein the third methylene group is replaced by an oxygen or sulphur atom, and
E represents a nucleophilically exchangeable group, and subsequently, if desired, it is hydrolysed, or

d) a compound of general formula

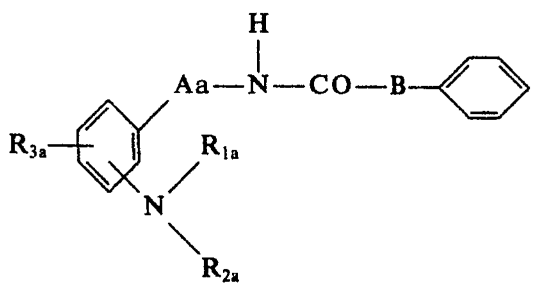

(VIIa)

wherein

B is defined as in claim 1 and
Aa and $R_{1a}$ to $R_{3a}$ are as hereinbefore defined, is acylated with an oxalyl halide or phosgene in the presence of a Lewis acid, or

e) a compound of general formula

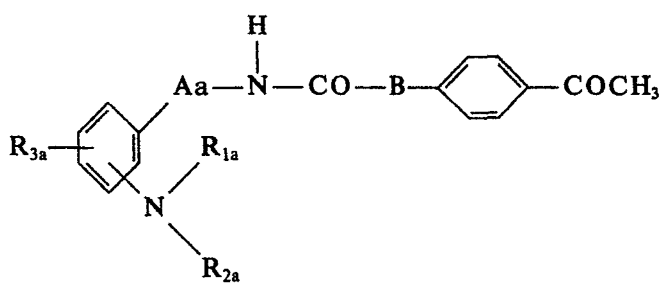

(VIIIa)

wherein

B is defined as in claim 1 and
Aa and $R_{1a}$ to $R_{3a}$ are as hereinbefore defined, is reacted with a hypohalite optionally prepared in the reaction mixture, in the presence of an alkali metal base, or

f) a compound of general formula

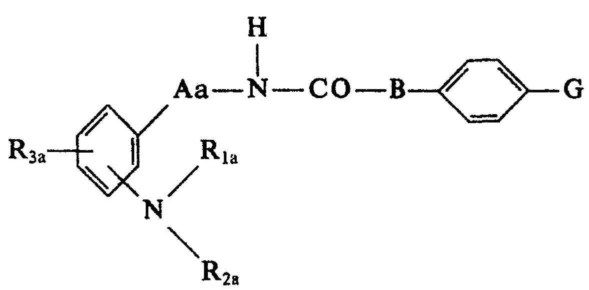

(IXa)

wherein

B and G are defined as in claim 1 and
Aa and $R_{1a}$ to $R_{3a}$ are as hereinbefore defined, is oxidised, or

g) a compound of general formula

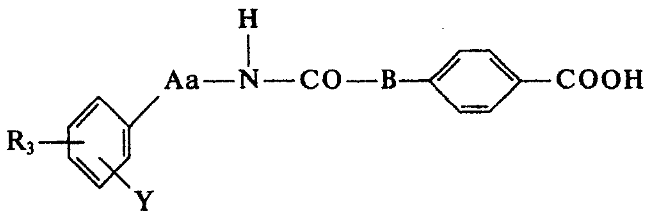

(Xa)

wherein

$R_3$, B and Y are defined as in claim 1 and
Aa is as hereinbefore defined, or an ester thereof, is reacted with an amine of general formula

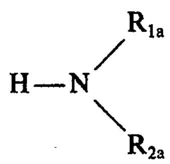

(XIa)

wherein $R_{1a}$ and $R_{2a}$ are as hereinbefore defined, and subsequently hydrolysed, if desired, and subsequently, if desired, a carboxylic acid amide of general formula I thus obtained is converted by esterification into a corresponding ester, and/or
a compound of general formula I obtained wherein $R_{3a}$ represents a nitro group is converted by reduction into a corresponding compound of general formula I wherein $R_{3a}$ represents an amino group, and/or
a compound of general formula I obtained wherein $R_{3a}$ represents an amino group is converted, via a corresponding diazonium salt, into a corresponding compound of general formula I wherein $R_{3a}$ represents a hydrogen or halogen atom, a hydroxy, alkoxy, mercapto, alkylmercapto or cyano group, whilst a compound of general formula I thus obtained wherein $R_{3a}$ represents a hydroxy group may be optionally converted by alkylation into a corresponding compound of general formula I wherein $R_{3a}$ represents an alkoxy group, and/or
a compound of general formula I obtained wherein $R_{3a}$ represents an amino group is converted by acylation into a corresponding compound of general formula I wherein $R_{3a}$ represents an alkanoylamino or alkylsulfonylamino group, and/or
a compound of general formula I obtained wherein $R_{3a}$ represents a chlorine or bromine atom is converted by dehalogenation into a corresponding compound of general formula I wherein $R_{3a}$ represents a hydrogen atom, and/or
a compound of general formula I obtained wherein $R_{3a}$ represents an amino group is converted by alkylation into a corresponding compound of general formula I wherein $R_{3a}$ represents an alkylamino or dialkylamino group, and/or
a compound of general formula I obtained wherein $R_{3a}$ represents a nitrile group is converted by hydrolysis into a corresponding compound of general formula I wherein $R_{3a}$ represents an aminocarbonyl group, and/or
a compound of general formula I obtained is converted into the salts thereof, particularly the physiologically acceptable salts with inorganic or organic acids and bases.

Priority: 10th January 1981 (P 31 00 575.6)

3. Process as claimed in claims 1 and 2, characterised in that the reaction is carried out in a solvent.
4. A process as claimed in claims 1a, 2a and 3, characterised in that the reaction is carried out at temperatures between −25 and 250° C, preferably, however, at temperatures between −10° C and the boiling temperature of the solvent used.
5. A process as claimed in claims 1b, 2b and 3, characterised in that the reaction is carried out at temperatures between −10 and 120° C, preferably at temperatures between room temperature and the boiling temperature of the reaction mixture
6. A process as claimed in claims 1c, 2c and 3, characterised in that the reaction is carried out at temperatures between 0 and 150° C, preferably, however, at temperatures between 20 and 75° C.
7. A process as claimed in claims 1d, 2d and 3, characterised in that the reaction is carried out at temperatures between 0 and 120° C, preferably, however, at temperatures between 20 and 180° C.
8. A process as claimed in claims 1e, 2e and 3, characterised in that the reaction is carried out in the presence of an alkali metal base and at temperatures between 0 and 80° C, preferably, however, at temperatures between 25 and 50° C.
9. A process as claimed in claims 1f, 2f and 3, characterised in that the reaction is carried out at

temperatures between 0 and 100° C, preferably, however, at temperatures between 20 and 50° C.

10. A process as claimed in claims 1g and 2g, characterised in that the reaction is carried out at temperatures between 20 and 150° C, preferably, however, at the boiling temperature of the reaction mixture, e. g. at 100° C.

**Revendications pour les Etats contractants: BE, CH, DE, FR, IT, LI, LU, NL, SE**

1. Nouveaux carboxamides de formule générale

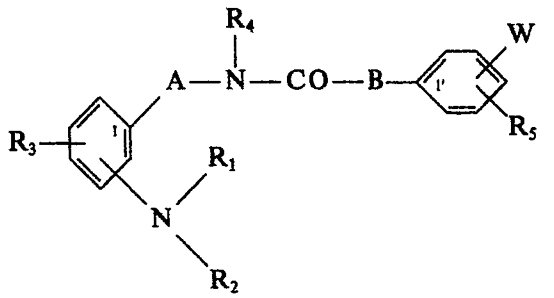

(I)

dans laquelle

$R_1$ et $R_2$, qui peuvent être identiques ou différents, représentent des groupes alcoyle avec 1 à 6 atomes de carbone ou des groupes cycloalcoyle avec 5 à 7 atomes de carbone, ou

$R_1$ et $R_2$ représentent ensemble avec l'atome d'azote se trouvant entre eux un groupe alcoylène-imino non ramifié avec 3 à 6 atomes de carbone qui peut être substitué par un ou deux groupes alcoyle avec chacun 1 à 3 atomes de carbone ou par un groupe hydroxy, ou dans lequel un groupe méthylène peut être remplacé par un groupe carbonyle, par un atome d'oxygène ou de soufre, ou par un groupe imino,lequel peut être substitué par un groupe alcoyle avec 1 à 3 atomes de carbone, un groupe aralcoyle avec 7 à 10 atomes de carbone, un groupe phényle ou halogénophényle, ou dans lequel un groupe éthylène peut être remplacé par un groupe o-phénylène, ou représente un groupe alcoylène-imino non ramifié avec 4 à 6 atomes de carbone, un groupe azabicycloalcoyle saturé ou en partie non saturé avec 6 à 10 atomes de carbone, un groupe aza-1,4-dioxaspiro-alcoyle avec 6 à 8 atomes de carbone, un groupe heptaméthylène-imino, octaméthylène-imino, nonaméthylène-imino ou décaméthylène-imino,

$R_3$ représente un atome d'hydrogène ou d'halogène, un groupe trifluorométhyle, alcoyle, hydroxy, alcoxy, alcanoyloxy, mercapto, alcoylmercapto, nitro, amino, cyano, alcanoyle, carboxy, alcoxycarbonyle, aminocarbonyle, alcoylaminocarbonyle, dialcoylaminocarbonyle, aminosulfonyle, alcoylamino, dialcoylamino, alcanoylamino, alcoxycarbonylamino ou alcoylsulfonylamino, chaque partie alcoyle dans les groupes mentionnés plus haut pouvant contenir à chaque fois 1 à 3 atomes de carbone, ou représente un groupe aralcoxy avec 7 à 10 atomes de carbone, ou un groupe arylcarbonylamino,

$R_4$ représente un atome d'hydrogène ou un groupe alcoyle avec 1 à 3 atomes de carbone,

$R_5$ représente un atome d'hydrogène, un atome d'halogène ou un groupe alcoyle avec 1 à 3 atomes de carbone,

A représente une liaison, un groupe méthylène ou éthylène éventuellement substitué par un groupe alcoyle avec 1 à 5 atomes de carbone, un groupe méthylène ou éthylène substitué par deux groupes alcoyle avec chacun 1 à 3 atomes de carbone, un groupe méthylène substitué par un groupe hydroxyalcoyle, alcoxyalcoyle, cyano, carboxyle, alcoxycarbonyle, aminocarbonyle, alcoylaminocarbonyle, dialcoylaminocarbonyle, aryle ou aralcoyle, chacune des parties alcoyle mentionnées plus haut pouvant contenir 1 à 3 atomes de carbone, un groupe cycloalcoylidène avec 3 à 7 atomes de carbone ou un groupe vinylidène de formule

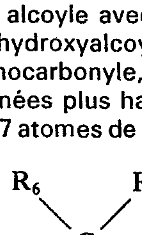

dans laquelle

$R_6$ et $R_7$, qui peuvent être identiques ou différents, représentent des atomes d'hydrogène ou des groupes alcoyle avec chacun 1 à 3 atomes de carbone ou également l'un des radicaux $R_6$ ou $R_7$ représente un groupe cycloalcoyle avec 3 à 7 atomes de carbone, un groupe aryle ou aralcoyle, ou

$R_6$ et $R_7$ représentent ensemble avec l'atome d'azote se trouvant entre eux un radical cycloalcoylidène avec 5 à 7 atomes de carbone,

B représente un groupe méthylène éventuellement substitué par un groupe alcoyle avec 1 à 3 atomes de carbone, et

W représente un atome d'hydrogène ou d'halogène, un groupe nitro, un groupe amino éventuellement substitué par un groupe alcanoyle avec 1 à 3 atomes de carbone, un groupe alcoyle avec 1 à 3 atomes de carbone éventuellement substitué par un groupe hydroxy, carboxy ou alcoxycarbonyle ou deux groupes alcoxycarbonyle avec chacun en tout 2 à 4 atomes de carbone, ou représente un groupe alcényle avec 2 à 5 atomes de carbone substitué par un groupe carboxy ou alcoxycarbonyle avec en tout 2 à 4 atomes de carbone, ou représente un groupe alcanoyle avec 1 à 3 atomes de carbone, un groupe dialcoxyméthyle ou trialcoxyméthyle avec 1 à 3 atomes de carbone dans chaque partie alcoyle, un groupe alcoylènedioxyméthyle avec 2 ou 3 atomes de carbone dans la partie alcoylène, un groupe 1,3-oxazoline-2-yle ou cyano, un groupe aminocarbonyle éventuellement substitué par un ou deux groupes alcoyle avec chacun 1 à 4 atomes de carbone dans chaque partie alcoyle, ou représente un groupe alcoylène-iminocarbonyle non ramifié avec en tout 5 à 8 atomes de carbone, un groupe morpholinocarbonyle, un groupe dialcoyldioxolane-alcoxycarbonyle avec en tout 7 à 10 atomes de carbone ou un groupe carboxy et ses esters, dans lesquels les esters d'alcoyle avec 1 à 6 atomes de carbone dans la partie alcoyle, à l'exception de la position $\alpha$, peuvent être à chaque fois substitués par un groupe hydroxy, alcoxy, amino, alcoylamino, dialcoylamino, 1,3-diméthyl-xanthine-7-yle, alcanoyloxy, aroyloxy, aralcanoyloxy ou pyridinecarbonyloxy, ou par deux groupes hydroxy, un groupe méthyle ou respectivement méthylène ne pouvant à chaque fois être substitué que par un groupe hydroxy, ou peuvent être substitués par un groupe formule

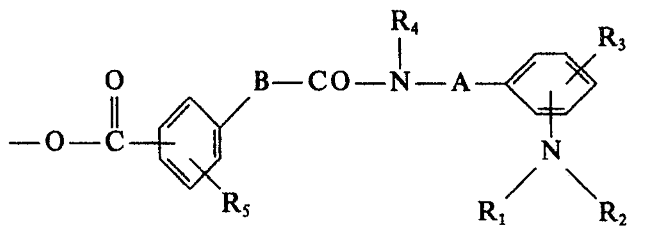

dans laquelle

A, B et $R_1$ à $R_5$ sont définis comme au début, chaque substituant alcoyle dans les esters d'alcoyle mentionnés plus haut pouvant contenir 1 à 3 atomes de carbone, et, dans la mesure où ils contiennent un atome de carbone asymétrique, leurs antipodes actifs optiquement et leurs sels, en particulier leurs sels physiologiquement supportables avec des acides ou des bases minéraux ou organiques.

2. Nouveaux carboxamides de formule générale I selon la revendication 1, dans laquelle:

$R_1$ et $R_2$ représentent ensemble avec l'atome d'azote se trouvant entre eux un groupe dialcoylamino ou N-alcoylcyclohexylamino, dans lequel chaque partie alcoyle peut contenir 1 à 4 atomes de carbone, un groupe alcoylèneimino non ramifié avec 3 à 6 atomes de carbone éventuellement substitué par un ou deux groupes méthyle, ou représentent un groupe hydroxypipéridino, pipéridone-1-yle, tétrahydropyridino, morpholino, thiomorpholino, N-méthyl-pipérazino, N-benzyl-pipérazino, N-chlorophényl-pipérazino, heptaméthylèneimino ou octaméthylèneimino, un groupe azabicycloalcoyle saturé ou en partie non saturé avec 7 à 9 atomes de carbone, un groupe alcoylèneimino non ramifié avec 4 à 6 atomes de carbone dans lequel un groupe éthylène est remplacé par un groupe o-phénylène, ou représent un groupe 1,4-dioxa-aza-spiro-alcoyle avec 7 ou 8 atomes de carbone,

$R_3$ représente un atome d'hydrogène, de fluor, de chlore, de brome ou d'iode, un groupe méthyle, trifluorométhyle, hydroxy, méthoxy, benzyloxy, acétoxy, mercapto, méthylmercapto, nitro, amino, diméthylamino, acétylamino, méthylsulfonylamino, benzoylamino, éthoxy-carbonylamino, cyano, carboxy, méthoxycarbonyle, éthoxycarbonyle, aminocarbonyle, acétyle ou aminosulfonyle,

$R_4$ représente un atome d'hydrogène ou un groupe méthyle,

$R_5$ représente un atome d'hydrogène, un atome de chlore ou un groupe méthyle,

A représente une liaison, un groupe méthylène éventuellement substitué par un groupe alcoyle avec 1 à 3 atomes de carbone, un groupe phényle, cyclohexyle, carboxy, méthoxycarbonyle ou hydroxyméthyle, ou représente un groupe diméthyl-méthylène, cyclopropylidène ou éthylène ou un groupe vinylidène de formule

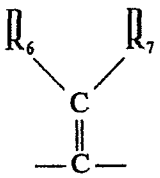

dans laquelle $R_6$ et $R_7$, qui peuvent être identiques ou différents, représentent des atomes d'hydrogène ou des groupes méthyle, ou $R_6$ et $R_7$ représentent ensemble avec l'atome d'azote se trouvant entre eux un radical cycloalcoylidène avec 5 ou 6 atomes de carbone,

B représente un groupe méthylène, éthylidène ou éthylène et

W représente un atome d'hydrogène, un groupe méthyle, éthyle, hydroxyméthyle, cyano ou carboxyvinylène, un groupe alcoyle avec 1 à 3 atomes de carbone substitué par un groupe carboxy ou par un ou deux groupes alcoxycarbonyle avec chacun en tout 2 à 4 atomes de carbone, ou représente un groupe carbonyle substitué par un atome d'hydrogène,un groupe méthyle, éthyle, hydroxy, alcoxy (2,2-diméthyl-dioxolane-4-yl)-méthoxy, benzyloxy, pyridylméthoxy, amino, alcoylamino, dialcoylamino, pipéridino ou morpholino, chaque partie alcoyle dans les groupes mentionnés plus haut pouvant contenir 1 à 3 atomes de carbone, ou représente un groupe de formule

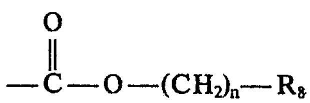

dans laquelle

n représente le nombre 2, 3 ou 4, et

$R_8$ représente un groupe hydroxy, méthoxy, éthoxy, acétoxy, benzoyloxy, pyridinecarbonyloxy, ou un groupe dialcoylamino avec 1 à 3 atomes de carbone dans chaque partie alcoyle, un groupe 1,3-diméthyl-xanthine-7-yle ou un groupe de formule

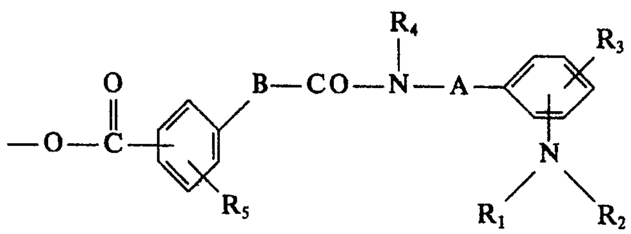

dans laquelle

A, B et $R_1$ à $R_5$ sont définis comme indiqué plus haut dans la revendication 2, et, dans la mesure où ils contiennent un atome de carbone asymétrique, leurs antipodes actifs optiquement et leurs sels, en particulier leurs sels physiologiquement supportables avec des acides ou des bases minéraux ou organiques.

3. Nouveaux carboxamides de formule générale I selon la revendication 1, dans laquelle $R_1$ à $R_5$, A, B et W sont définis comme dans la revendication 2, et le radical

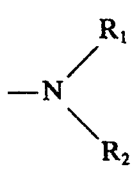

se trouve en position 2, et le radical W en position 4', et, dans la mesure où ils contiennent un atome de carbone asymétrique, leurs antipodes actifs optiquement et leurs sels d'addition physiologiquement supportables avec des acides ou des bases minéraux ou organiques.

4. Noveaux carboxamides de formule générale

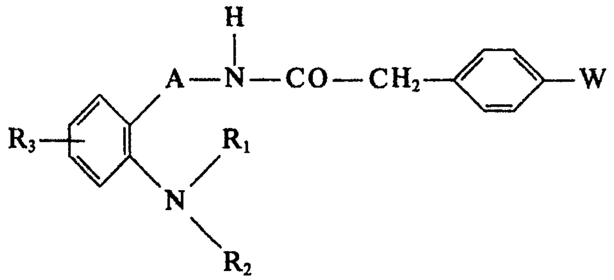

(Ia)

dans laquelle

$R_1$ et $R_2$ représentent ensemble avec l'atome d'azote se trouvant entre eux un groupe diméthylamino, pyrrolidino, méthylpyrrolidino, pipéridino, méthylpipéridino, diméthylpipéridino, tétrahydro-pyridino, 2-octahydro-isoindolo ou hexaméthylèneimino,
$R_3$ représente un atome d'hydrogène, de fluor ou de chlore ou un groupe méthyle,
A représente un groupe méthylène éventuellement substitué par un groupe cyclohexyle, phényle, méthoxycarbonyle, éthoxycarbonyle ou un groupe alcoyle avec 1 à 3 atomes de carbone, ou représente un groupe diméthyl-méthylène ou un groupe vinylidène de formule

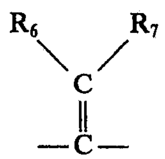

dans laquelle $R_6$ et $R_7$ représentent chacun un atome d'hydrogène ou ensemble avec l'atome de carbone se trouvant entre eux un groupe cyclohexylidène, et W représente un groupe méthyle, hydroxyméthyle ou carboxyméthyle, un groupe carbonyle substitué par un atome d'hydrogène, un groupe méthyle, hydroxy, méthoxy, éthoxy, propoxy, isopropoxy, 2-hydroxyéthoxy, 2-méthoxy-éthoxy, ou (2,2-diméthyl-dioxolane-4-yl)-méthoxy ou 2-diéthylaminoéthoxy, et, dans la mesure où ils contiennent un atome de carbone asymétrique, leurs antipodes actifs optiquement et leurs sels d'addition physiologiquement supportables avec des acides ou des bases minéraux ou organiques.

5. Nouveaux carboxamides de formule générale la selon la revendication 4,dans laquelle

$R_1$ et $R_2$ représentent ensemble avec l'atome d'azote se trouvant entre eux un groupe pyrrolidino, pipéridino, méthyl-pipéridino, hexaméthylèneimino, tétrahydropyridino ou 2-octahydro-isoindolo,
$R_3$ représente un atome d'hydrogène, de fluor ou de chlore ou un groupe méthyle,
A représente un groupe méthylène éventuellement substitué par un groupe méthyle,isopropyle, phényle ou méthoxycarbonyle,ou représente un groupe diméthyl-méthylène ou vinylidène et
W représente un groupe méthyle, hydroxyméthyle, carboxyméthyle,formyle ou carboxyle ou un groupe alcoxycarbonyle éventuellement substitué par un groupe (2,2-diméthyl-dioxolane-4-yle), dans lequel le groupe alcoxy peut contenir 1 à 3 atomes de carbone, et, dans la mesure où ils contiennent un atome de carbone asymétrique, leurs antipodes actifs optiquement et leurs sels d'addition physiologiquement supportables avec des acides ou des bases minéraux ou organiques.

6. L'acide 4-[(1-(2-pipéridino-phényl)-éthyl)-aminocarbonylméthyl]-benzoïque, ses esters alcoyliques avec 1 à 3 atomes de carbone, ses antipodes actifs optiquement et ses sels.

7. L'acide 4-[(2-pipéridino-benzhydryl)-amino-carbonylméthyl]-benzoïque, ses esters alcoyliques avec 1 à 3 atomes de carbone, ses antipodes actifs optiquement et ses sels.

8. Médicament contenant un composé selon les revendications 1 à 7 conjointement à un ou plusieurs excipients et/ou diluants inertes.

9. Médicament selon la revendication 8, avec une activité hypoglycémiante.

10. Procédé pour la préparation de nouveaux carboxamides de formule générale

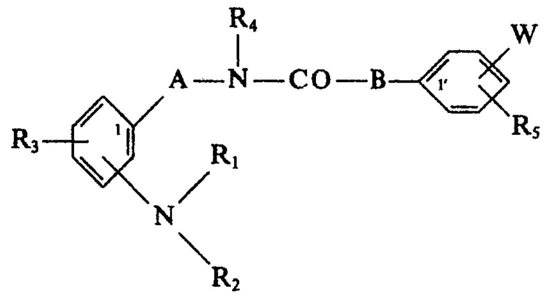

(I)

dans laquelle

$R_1$ et $R_2$ qui peuvent être identiques ou différents, représentent des groupes alcoyle avec 1 à 6 atomes de carbone, ou des groupes cycloalcoyle avec 5 à 7 atomes de carbone, ou

$R_1$ et $R_2$ représentent ensemble avec l'atome d'azote se trouvant entre eux un groupe alcoylèneimino non ramifié avec 3 à 6 atomes de carbone, qui peut être substitué par un ou deux groupes alcoyle avec chacun 1 à 3 atomes de carbone ou par un groupe hydroxy, ou dans lequel un groupe méthylène peut être remplacé par un groupe carbonyle, par un atome d'oxygène ou de soufre ou par un groupe imino, lequel peut être substitué par un groupe alcoyle avec 1 à 3 atomes de carbone, un groupe aralcoyle avec 7 à 10 atomes de carbone, un groupe phényle ou halogénophényle, ou dans lequel un groupe éthylène peut être remplacé par un groupe o-phénylène, ou représente un groupe alcénylèneimino non ramifié avec 4 à 6 atomes de carbone, un groupe azabicycloalcoyle saturé ou en partie non saturé avec 6 à 10 atomes de carbone, un groupe aza-1, 4-dioxa-spiro-alcoyle avec 6 à 8 atomes de carbone, un groupe heptaméthylèneimino, octaméthylèneimino, nonaméthylèneimino ou décaméthylèneimino,

$R_3$ représente un atome d'hydrogène ou d'halogène, un groupe trifluorométhyle, alcoyle, hydroxy, alcoxy, alcanoyloxy, mercapto, alcoylmercapto, nitro, amino, cyano, alcanoyle, carboxy, alcoxycarbonyle, aminocarbonyle, alcoylaminocarbonyle, dialcoylaminocarbonyle, aminosulfonyle, alcoylamino, dialcoylamino, alcanoylamino, alcoxycarbonylamino ou alcoylsulfonylamino, chaque partie alcoyle dans les groupes mentionnés plus haut pouvant contenir à chaque fois 1 à 3 atomes de carbone, ou représente un groupe aralcoxy avec 7 à 10 atomes de carbone,ou un groupe arylcarbonylamino,

$R_4$ représente un atome d'hydrogène ou un groupe alcoyle avec 1 à 3 atomes de carbone,

$R_5$ représente un atome d'hydrogène, un atome d'halogène ou un groupe alcoyle avec 1 à 3 atomes de carbone,

A représente une liaison, un groupe méthylène ou éthylène éventuellement substitué par un groupe alcoyle avec 1 à 5 atomes de carbone, ou représente un groupe méthylène ou éthylène substitué par deux groupes alcoyle avec chacun 1 à 3 atomes de carbone, ou représente un groupe méthylène substitué par un groupe cycloalcoyle avec 3 à 7 atomes de carbone, par un groupe hydroxyalcoyle, alcoxyalcoyle, cyano, carboxyle, alcoxycarbonyle, aminocarbonyle, alcoylaminocarbonyle, dialcoylaminocarbonyle, aryle ou aralcoyle, chacune des parties alcoyle mentionnées plus haut pouvant contenir 1 à 3 atomes de carbone, ou représente un groupe cycloalcoylidène avec 3 à 7 atomes de carbone ou un groupe vinylidène de formule

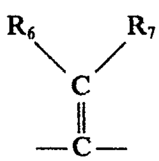

dans laquelle

$R_6$ et $R_7$ qui peuvent être identiques ou différents représentent des atomes d'hydrogène ou des groupes alcoyle avec chacun 1 à 3 atomes de carbone ou également l'un des radicaux $R_6$ et $R_7$ représente un groupe cycloalcoyle avec 3 à 7 atomes de carbone, un groupe aryle ou aralcoyle ou $R_6$ et $R_7$ représentent ensemble avec l'atome d'azote se trouvant entre eux un radical cycloalcoylidène avec 5 à 7 atomes de carbone,

B représente un groupe méthylène ou éthylène éventuellement substitué par un groupe alcoyle avec 1 à 3 atomes de carbone, et

W représente un atome d'hydrogène ou d'halogène, un groupe nitro, un groupe amino éventuellement substitué par un groupe alcanoyle avec 1 à 3 atomes de carbone, ou représente un groupe alcoyle avec 1 à 3 atomes de carbone éventuellement substitué par un groupe hydroxy, carboxy ou

alcoxycarbonyle ou deux groupes alcoxycarbonyle avec chacun en tout 2 à 4 atomes de carbone, ou représente un groupe alcényle avec 2 à 5 atomes de carbone substitué par un groupe carboxy ou alcoxycarbonyle avec en tout 2 à 4 atomes de carbone, ou représente un groupe alcanoyle avec 1 à 3 atomes de carbone un groupe dialcoxyméthyle ou trialcoxyméthyle avec 1 à 3 atomes de carbone dans chaque partie alcoyle, un groupe alcoylènedioxyméthyle avec 2 ou 3 atomes de carbone dans la partie alcoylène, un groupe 1,3-oxazoline-2-yle ou cyano, un groupe aminocarbonyle éventuellement substitué par un ou deux groupes alcoyle avec chacun 1 à 4 atomes de carbone dans chaque partie alcoyle, ou représente un groupe alcoylèneiminocarbonyle non ramifié avec en tout 5 à 8 atomes de carbone, un groupe morpholinocarbonyle, un groupe (dialcoyldioxolane-yl)-alcoxycarbonyle avec en tout 7 à 10 atomes de carbone, ou un groupe carboxy et ses esters, dans lesquels les esters d'alcoyle avec 1 à 6 atomes de carbone dans la partie alcoyle, à l'exception de la position *α*, peuvent être à chaque fois substitués par un groupe hydroxy, alcoxy, amino, alcoylamino,dialcoylamino, 1,3-diméthylxanthine-7-yle, alcanoyloxy, arylcarbonyloxy, aralcanoyloxy ou pyridinecarbonyloxy ou par deux groupes hydroxy, un groupe méthyl ou respectivement méthylène ne pouvant à chaque fois être substitué que par un groupe hydroxy, ou peuvent être substitués par un groupe de formule

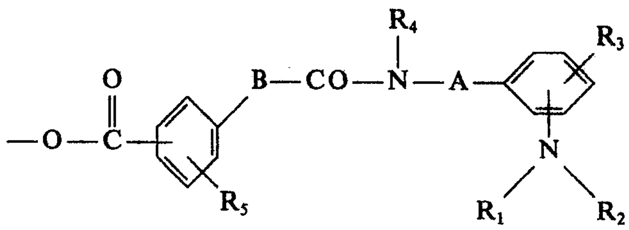

dans laquelle

A, B et $R_1$ à $R_5$ sont définis comme au début, chaque substituant alcoyle dans les esters alcoyliques mentionnés plus haut pouvant contenir 1 à 3 atomes de carbone, ainsi que de leurs antipodes actifs optiquement et de leurs sels, en particulier leurs sels physiologiquement supportables avec des acides ou des bases minéraux ou organiques,
caractérisé en ce que

a) on fait réagir une amine de formule générale

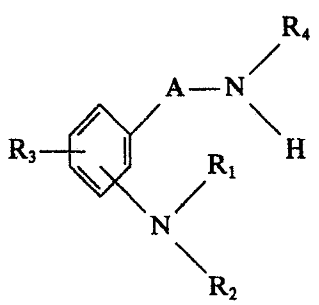

(II)

dans laquelle

A et $R_1$ à $R_4$ sont définis comme au début, ou lorsque A représente l'un des groupes vinylidène mentionnés au début, son tautomère, son complexe d'halogènure de lithium ou de magnésium avec un acide carboxylique de formule générale

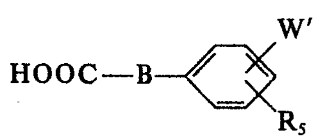

(III)

dans laquelle

$R_5$ et B sont définis comme au début et
W' possède les significations mentionnées au début pour W ou représente un groupe carboxyle protégé par un radical protecteur,
ou avec ses dérivés réactifs éventuellement préparés dans le mélange réactionnel et si nécessaire on clive un groupe protecteur utilisé, ou

b) pour la préparation d'un composé de formule générale I dans laquelle W représente un groupe carboxy, on clive hydrolytiquement, thermolytiquement ou hydrogénolytiquement un composé de formule générale

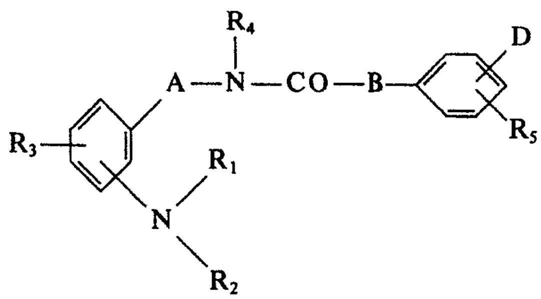

(IV)

dans laquelle

$R_1$ à $R_5$, A et B sont définis comme au début et D représente un groupe transformable en un groupe carboxy par hydrolyse, thermolyse ou d'hydrogénolyse, ou

c) on alcoyle si nécessaire en présence d'un agent réducteur, un composé éventuellement formé dans le mélange réactionnel de formule générale

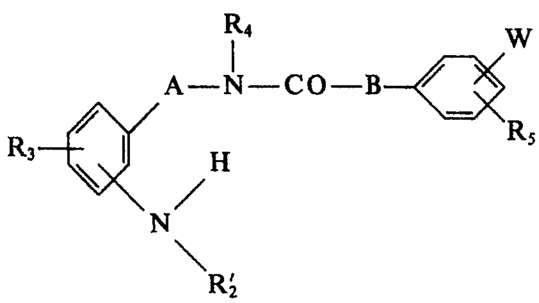

(V)

dans laquelle

$R_3$ à $R_5$, A, B et W sont définis comme au début, et $R_2'$ représente un atome d'hydrogène ou possède les significations mentionnées au début pour $R_2$, au moyen d'un composé de formule générale

$$R_1'-E \qquad (VI)$$

dans laquelle

$R_1'$ possède les significations mentionnées au début pour $R_1$ ou ensemble avec le radical $R_2'$ de la formule V représente un groupe alcoylène rectiligne avec 4 à 6 atomes de carbone éventuellement substitué par un ou deux groupes alcoyle avec chacun 1 à 3 atomes de carbone, ou représente un groupe n-pentylène dans lequel le troisième groupe méthylène est remplacé par un atome d'oxygène ou de soufre, et

E représente un groupe partant nucléophile ou également un atome d'hydrogène lorsque dans $R_1'$, un groupe méthylène est remplacé par un groupe aldéhyde ou cétonecarbonyle, et éventuellement, on hydrolyse ensuite, ou

d) pour la préparation d'un composé de formule générale I dans laquelle W représente un groupe carboxy, un groupe alcanoyle avec 1 à 3 atomes de carbone ou un groupe alcoyle avec 1 à 3 atomes de carbone, on fait réagir un composé de formule générale

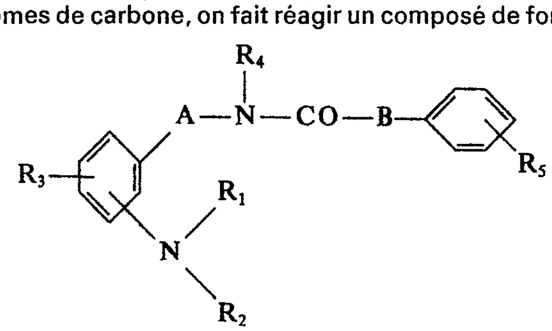

(VII)

dans laquelle

$R_1$ à $R_5$, A et B sont définis comme au début, avec le phosgène, un halogénure d'oxalyle, un halogénure d'alcoyle ou d'alcanoyle avec chacun 1 à 3 atomes de carbone dans la partie alcoyle ou avec le cyanure d'hydrogène/d'halogénure d'hydrogène en présence d'un acide de Lewis, ou

e) pour la préparation d'un composé de formule générale I dans laquelle W représente le groupe carboxy, on fait réagir un composé de formule générale

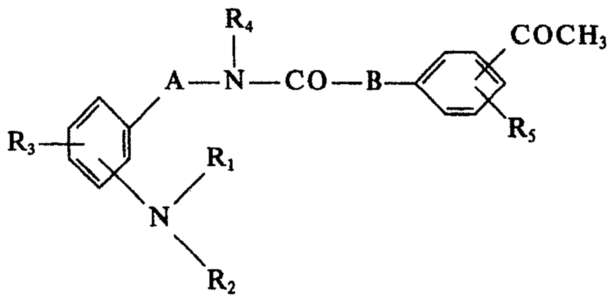

(VIII)

dans laquelle

$R_1$ à $R_5$, A et B sont définis comme au début, avec un hypohalogénite éventuellement préparé dans le mélange réactionnel en présence d'une base alcaline, ou

f) pour la prépration d'un composé de formule générale I dans laquelle W représente le groupe carboxy, on oxyde un composé de formule générale

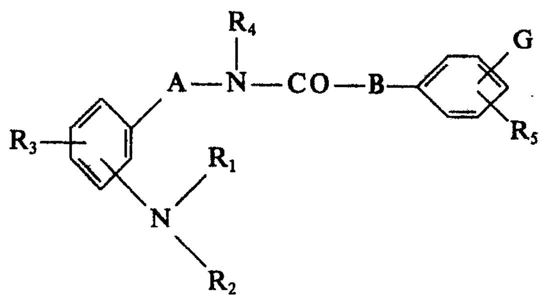

(IX)

dans laquelle

$R_1$ à $R_5$, A et B sont définis comme au début et G représente un groupe transformable en un groupe carboxy par oxydation, ou

g) pour la préparation d'un composé de formule générale I dans laquelle $R_3$ représente le groupe nitro, on fait réagir un composé de formule générale

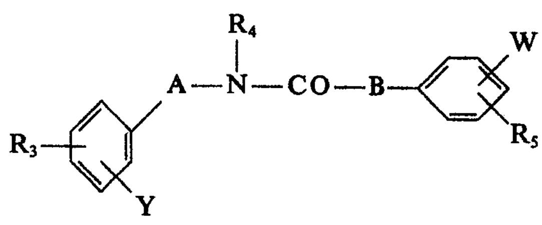

(X)

dans laquelle

$R_4$, $R_5$, A, B et W sont définis comme au début,
$R_3$ représente un groupe nitro et
Y représente un radical échangeable de façon nucléophile, avec une amine de formule générale

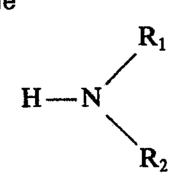

(XI)

dans laquelle

$R_1$ et $R_2$ sont définis comme au début, et éventuellement ensuite, on hydrolyse ou

h) pour la préparation d'un composé de formule générale I dans laquelle A représente un groupe de formule

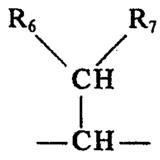

où $R_6$ et $R_7$ sont définis comme au début, on réduit un composé de formule générale

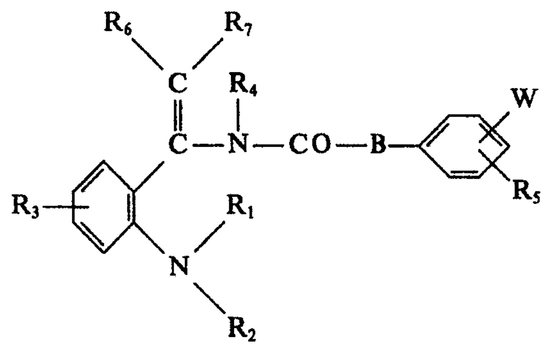

(XII)

dans laquelle

$R_1$ à $R_7$, B et W sont définis comme au début, au moyen d'hydrogène en présence d'un catalyseur d'hydrogènation,ou

i) pour la préparation d'un composé de formule générale I dans laquelle $R_4$ représente un atome d'hydrogène et A représente un groupe méthylène ou éthylène éventuellement substitué par un groupe alcoyle avec 1 à 5 atomes de carbone, ou représente un groupe méthylène ou éthylène substitué par deux groupes alcoyle avec chacun 1 à 3 atomes de carbone, ou représente un groupe méthylène substitué par un groupe cycloalcoyle avec 3 à 7 atomes de carbone, un groupe alcoxyalcoyle, carboxyle, alcoxycarbonyle, aryle ou aralcoyle, chacune des parties alcoyle mentionnées plus haut pouvant contenir 1 à 3 atomes de carbone, ou représente un groupe cycloalcoylidène avec 4 à 7 atomes de carbone, on fait réagir un composé de formule générale

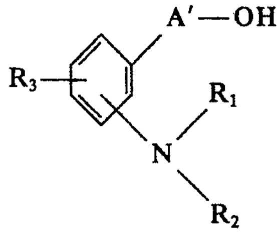

(XIII)

dans laquelle

$R_1$ à $R_3$ sont définis comme au début et

A' représente un groupe méthylène ou éthylène éventuellement substitué par un groupe alcoyle avec 1 à 5 atomes de carbone, ou représente un groupe méthylène ou éthylène substitué par deux groupes alcoyle avec chacun 1 à 3 atomes de carbone, ou représente un groupe méthylène substitué par un groupe cycloalcoyle avec 3 à 7 atomes de carbone, un groupe alcoxyalcoyle, carboxyle, alcoxycarbonyle, aryle ou aralcoyle, chacune des parties alcoyle mentionnées plus haut pouvant contenir 1 à 3 atomes de carbone, ou représente un groupe cycloalcoylidène avec 4 à 7 atomes de carbone,

avec un composé de formule générale

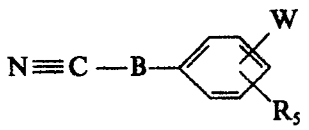

(XIV)

dans laquelle

$R_5$, B et W sont définis comme au début, en présence d'un acide fort,
et si on le désire ensuite, on transforme un composé ainsi obtenu de formule générale I dans laquelle W représente le groupe carboxy, au moyen d'une estérification ou d'une amidation, en un composé correspondant de formule générale I et/ou
on transforme un composé obtenu de formule générale I dans laquelle $R_3$ et/ou W représentent un groupe nitro, au moyen d'une réduction, en un composé correspondant de formule générale I dans laquelle $R_3$ et/ou représentent un groupe amino, et/ou
on transforme un composé obtenu de formule générale I dans laquelle $R_3$ et/ou W représentent un groupe amino, par l'intermédiaire d'un sel de diazonium correspondant, en un composé correspondant de formule générale I dans laquelle $R_3$ représente un atome d'hydrogène ou d'halogène, un groupe hydroxy, alcoxy, mercapto, alcoylmercapto, chlorosulfonyle ou cyano et/ou W représente un atome d'hydrogène ou d'halogène ou un groupe cyano, un composé ainsi éventuellement obtenu de formule générale I dans laquelle $R_3$ représente le groupe hydroxy pouvant être transformé au moyen d'une alcoylation en un composé correspondant de formule générale I dans laquelle $R_3$ représente un groupe alcoxy ou un composé ainsi éventuellement obtenu de formule générale I dans laquelle $R_3$ représente le groupe chlorosulfonyle pouvant être transformé au moyen d'ammoniac en un composé correspondant de formule générale I dans laquelle $R_3$ représente le groupe aminosulfonyle, et/ou
un composé obtenu de formule générale I dans laquelle $R_3$ représente un groupe amino est transformé au moyen d'une acylation en un composé correspondant de formule générale I dans laquelle $R_3$ représente un groupe alcanoylamino, aroylamino, alcoxycarbonylamino ou alcoylsulfonylamino, et/ou
un composé obtenu de formule générale I dans laquelle $R_3$ représente un groupe amino est transformé au moyen d'une alcoylation en un composé correspondant de formule générale I dans laquelle $R_3$ représente un groupe alcoylamino ou dialcoylamino, et/ou
un composé obtenu de formule générale I dans laquelle $R_3$ représente un atome de chlore ou de brome est transformé au moyen d'une déshalogénation en un composé correspondant de formule générale I dans laquelle $R_3$ représente un atome d'hydrogène, et/ou
un composé obtenu de formule générale I dans laquelle $R_3$ représente un groupe nitrile est transformé au moyen d'une hydrolyse ou d'une alcoolyse en un composé correspondant de formule générale I dans laquelle $R_3$ représente un groupe aminocarbonyle, carboxy ou alcoxycarbonyle, et/ou
un composé obtenu de formule générale I dans laquelle $R_3$ représente un groupe carboxy ou alcoxycarbonyle et/ou W représente un groupe carboxy éventuellement estérifié, est transformé au moyen d'une réduction en un composé correspondant de formule générale I dans laquelle $R_3$ et/ou W représentent un groupe formyle ou hydroxyméthyle, et/ou
un composé obtenu de formule générale I dans laquelle W représente un groupe alcoxycarbonyle dans lequel le groupe alcoxy peut contenir 2 à 6 atomes de carbone et, à l'exception de la position a, est substitué par un groupe hydroxy, est transformé au moyen d'une acylation en un composé correspondant de formule générale I et/ou un composé obtenu de formule générale I dans laquelle W représente un groupe hydroxyméthyle est transformé au moyen d'une halogénation et réaction subséquente avec un diester d'acide malonique en un composé correspondant de formule générale I dans laquelle W représente un groupe éthyle substitué par deux groupes alcoxycarbonyle et/ou
un composé obtenu de formule générale I dans laquelle W représente un groupe formyle est transformé au moyen d'une condensation et éventuellement d'une hydrolyse subséquente et/ou décarboxylation en un composé correspondant de formule générale I dans laquelle W représente un groupe vinyle substitué par un groupe hydroxycarbonyle ou alcoxycarbonyle et/ou
un composé obtenu de formule générale I dans laquelle W représente un groupe éthyle substitué par deux groupes alcoxycarbonyle est transformé au moyen d'une hydrolyse et d'une décarboxylation en un composé correspondant de formule générale I dans laquelle W représente un groupe éthyle substitué par un groupe carboxy et/ou
un composé obtenu de formule générale I dans laquelle W représente un groupe carboxy est transformé au moyen d'une transformation en un hydrazide d'acide sulfonique et ensuite d'une dismutation en un composé correspondant de formule générale I dans laquelle W

représente un groupe formyle et/ou
un composé obtenu de formule générale I dans laquelle $R_1$ et $R_2$ représentent ensemble avec l'atome d'azote se trouvant entre eux un groupe aza-1,4-dioxa-spiro-alcoyle avec 6 à 8 atomes de carbone est transformé au moyen d'une hydrolyse en un composé correspondant de formule générale I dans laquelle $R_1$ et $R_2$ représentent ensemble avec l'atome d'azote se trouvant entre eux un groupe alcoylèneimino non ramifié avec 4 à 6 atomes de carbone dans lequel un groupe méthylène est remplacé par un groupe carbonyle et/ou
un composé obtenu de formule générale I dans laquelle $R_1$ et $R_2$ représentent ensemble avec l'atome d'azote se trouvant entre eux un groupe alcoylèneimino non ramifié avec 4 à 6 atomes de carbone dans lequel un groupe méthylène est remplacé par un groupe carbonyle, est transformé au moyen d'une réduction en un composé hydroxyalcoylèneimino correspondant de formule générale I et/ou
un composé obtenu de formule générale I dans laquelle W représente un groupe aminocarbonyle est transformé au moyen d'une déshydratation en un composé correspondant de formule générale I dans laquelle W représente un groupe cyano et/ou
un composé obtenu de formule générale I est transformé en ses sels, en particulier en ses sels physiologiquement supportables avec des acides et des bases minéraux ou organiques.

**Revendications pour les Etats contractants: AT**

1. Procédé pour la préparation de nouveaux carboxamides de formule générale

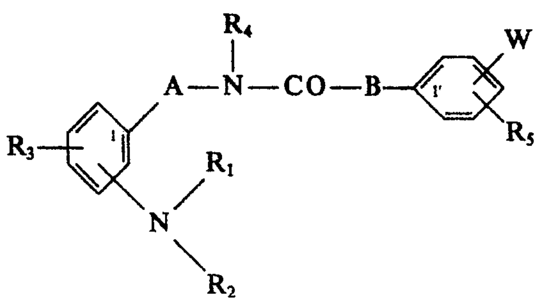

(I)

dans laquelle

$R_1$ et $R_2$, qui peuvent être identiques ou différents, représentent des groupes alcoyle avec 1 à 6 atomes de carbone ou des groupes cycloalcoyle avec 5 à 7 atomes de carbone, ou

$R_1$ et $R_2$ représentent ensemble avec l'atome d'azote se trouvant entre eux un groupe alcoylène-imino non ramifié avec 3 à 6 atomes de carbone qui peut être substitué par un ou deux groupes alcoyle avec chacun 1 à 3 atomes de carbone ou par un groupe hydroxy, ou dans lequel un groupe méthylène peut être remplacé par un groupe carbonyle, par un atome d'oxygène ou de soufre, ou par un groupe imino,lequel peut être substitué par un groupe alcoyle avec 1 à 3 atomes de carbone, un groupe aralcoyle avec 7 à 10 atomes de carbone, un groupe phényle ou halogénophényle, ou dans lequel un groupe éthylène peut être remplacé par un groupe o-phénylène, ou représente un groupe alcoylène-imino non ramifié avec 4 à 6 atomes de carbone, un groupe azabicycloalcoyle saturé ou en partie non saturé avec 6 à 10 atomes de carbone, un groupe aza-1,4-dioxaspiro-alcoyle avec 6 à 8 atomes de carbone, un groupe heptaméthylène-imino, octaméthylène-imino, nonaméthylène-imino ou décaméthylène-imino,

$R_3$ représente un atome d'hydrogène ou d'halogène, un groupe trifluorométhyle, alcoyle, hydroxy, alcoxy, alcanoyloxy, mercapto, alcoylmercapto, nitro, amino, cyano, alcanoyle, carboxy, alcoxycarbonyle, aminocarbonyle, alcoylaminocarbonyle, dialcoylaminocarbonyle, aminosulfonyle, alcoylamino, dialcoylamino, alcanoylamino, alcoxycarbonylamino ou alcoylsulfonylamino, chaque partie alcoyle dans les groupes mentionnés plus haut pouvant contenir à chaque fois 1 à 3 atomes de carbone, ou représente un groupe aralcoxy avec 7 à 10 atomes de carbone, ou un groupe arylcarbonylamino,

$R_4$ représente un atome d'hydrogène ou un groupe alcoyle avec 1 à 3 atomes de carbone,

$R_5$ représente un atome d'hydrogène, un atome d'halogène ou un groupe alcoyle avec 1 à 3 atomes de carbone,

A représente une liaison, un groupe méthylène ou éthylène éventuellement substitué par un groupe alcoyle avec 1 à 5 atomes de carbone, un groupe méthylène ou éthylène substitué par deux groupes alcoyle avec chacun 1 à 3 atomes de carbone, un groupe méthylène substitué par un groupe hydroxyalcoyle, alcoxyalcoyle, cyano, carboxyle, alcoxycarbonyle, aminocarbonyle, alcoylaminocarbonyle, dialcoylaminocarbonyle, aryle ou aralcoyle, chacune des parties alcoyle mentionnées plus haut pouvant contenir 1 à 3 atomes de carbone, un groupe cycloalcoylidène avec 3 à 7 atomes de carbone ou un groupe vinylidène de formule

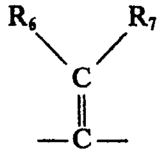

dans laquelle

$R_6$ et $R_7$, qui peuvent être identiques ou différents, représentent des atomes d'hydrogène ou des groupes alcoyle avec chacun 1 à 3 atomes de carbone ou également l'un des radicaux $R_6$ ou $R_7$ représente un groupe cycloalcoyle avec 3 à 7 atomes de carbone, un groupe aryle ou aralcoyle, ou $R_6$ et $R_7$ représentent ensemble avec l'atome d'azote se trouvant entre eux un radical cycloalcoylidène avec 5 à 7 atomes de carbone,

B représente un groupe méthylène éventuellement substitué par un groupe alcoyle avec 1 à 3 atomes de carbone, et

W représente un atome d'hydrogène ou d'halogène, un groupe nitro, un groupe amino éventuellement substitué par un groupe alcanoyle avec 1 à 3 atomes de carbone, un groupe alcoyle avec 1 à 3 atomes de carbone éventuellement substitué par un groupe hydroxy, carboxy ou alcoxycarbonyle ou deux groupes alcoxycarbonyle avec chacun en tout 2 à 4 atomes de carbone, ou représente un groupe alcényle avec 2 à 5 atomes de carbone substitué par un groupe carboxy ou alcoxycarbonyle avec en tout 2 à 4 atomes de carbone, ou représente un groupe alcanoyle avec 1 à 3 atomes de carbone, un groupe dialcoxyméthyle ou trialcoxyméthyle avec 1 à 3 atomes de carbone dans chaque partie alcoyle, un groupe alcoylènedioxyméthyle avec 2 ou 3 atomes de carbone dans la partie alcoylène, un groupe 1,3-oxazoline-2-yle ou cyano, un groupe aminocarbonyle éventuellement substitué par un ou deux groupes alcoyle avec chacun 1 à 4 atomes de carbone dans chaque partie alcoyle, ou représente un groupe alcoylène-iminocarbonyle non ramifié avec en tout 5 à 8 atomes de carbone, un groupe morpholinocarbonyle, un groupe dialcoyldioxolane-alcoxycarbonyle avec en tout 7 à 10 atomes de carbone ou un groupe carboxy et ses esters, dans lesquels les esters d'alcoyle avec 1 à 6 atomes de carbone dans la partie alcoyle, à l'exception de la position $\alpha$, peuvent être à chaque fois substitués par un groupe hydroxy, alcoxy, amino, alcoylamino, dialcoylamino, 1,3-diméthyl-xanthine-7-yle, alcanoyloxy, arylcarbonyloxy, aralcanoyloxy ou pyridinecarbonyloxy, ou par deux groupes hydroxy, un groupe méthyle ou respectivement méthylène ne pouvant à chaque fois être substitué que par un groupe hydroxy, ou pouvant être substitués par un groupe formule

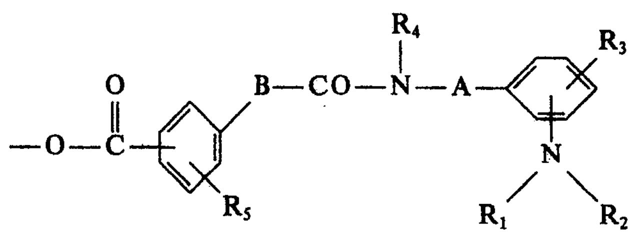

dans laquelle

A, B et $R_1$ à $R_5$ sont définis comme au début, chaque substituant alcoyle dans les esters d'alcoyle mentionnés plus haut pouvant contenir 1 à 3 atomes de carbone, et, dans la mesure où ils contiennent un atome de carbone asymétrique, de leurs antipodes actifs optiquement et de leurs sels, en particulier de leurs sels physiologiquement supportables avec des acides ou des bases minéraux ou organiques,

caractérisé en ce que

a) on fait réagir une amine de formule générale

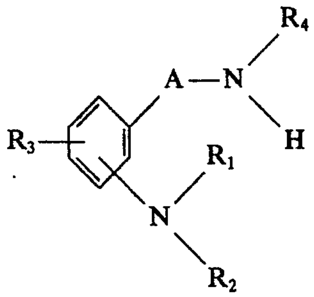

(II)

dans laquelle

A et $R_1$ à $R_4$ sont définis comme au début, ou lorsque A représente l'un des groupes vinylidène mentionnés au début, son tautomère, son complexe d'halogènure de lithium ou de magnésium avec un acide carboxylique de formule générale

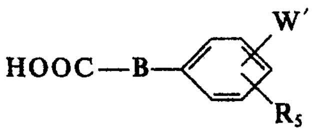

(III)

dans laquelle

$R_5$ et B sont définis comme au début et
W' possède les significations mentionnées au début pour W ou représente un groupe carboxyle protégé par un radical protecteur,
ou avec ses dérivés réactifs éventuellement préparés dans le mélange réactionnel et si nécessaire on clive un groupe protecteur utilisé, ou

b) pour la préparation d'un composé de formule générale I dans laquelle W représente un groupe carboxy, on clive hydrolytiquement, thermolytiquement ou hydrogénolytiquement un composé de formule générale

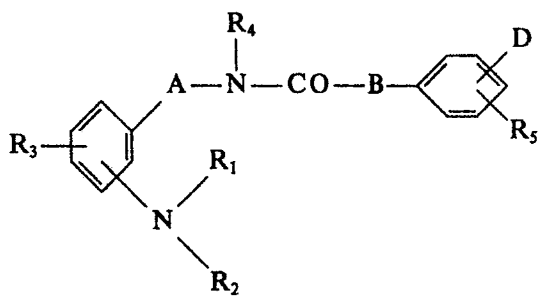

(IV)

dans laquelle

$R_1$ à $R_5$, A et B sont définis comme au début et D représente un groupe transformable en un groupe carboxy par hydrolyse, thermolyse ou d'hydrogènolyse, ou

c) on alcoyle si nécessaire en présence d'un agent réducteur, un composé éventuellement formé dans le mélange réactionnel de formule générale

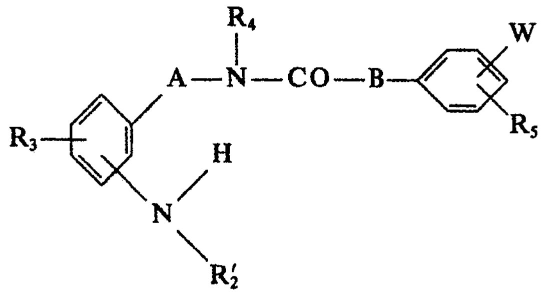

(V)

dans laquelle

$R_3$ à $R_5$, A, B et W sont définis comme au début, et $R_2'$ représente un atome d'hydrogène ou possède les significations mentionnées au début pour $R_2$, au moyen d'un composé de formule générale

$R_1'-E$ (VI)

dans laquelle

$R_1'$ possède les significations mentionnées au début pour $R_1$ ou ensemble avec le radical $R_2'$ de la formule V représente un groupe alcoylène rectiligne avec 4 à 6 atomes de carbone éventuellement substitué par un ou deux groupes alcoyle avec chacun 1 à 3 atomes de carbone, ou représente un groupe n-pentylène dans lequel le troisième groupe méthylène est remplacé par un atome d'oxygène ou de soufre, et
E représente un groupe partant nucléophile ou également un atome d'hydrogène lorsque dans $R_1'$, un groupe méthylène est remplacé par un groupe aldéhyde ou cétonecarbonyle, et éventuellement, on hydrolyse ensuite, ou

d) pour la préparation d'un composé de formule générale I dans laquelle W représente un groupe carboxy, un groupe alcanoyle avec 1 à 3 atomes de carbone ou un groupe alcoyle avec 1 à 3 atomes de carbone, on fait réagir un composé de formule générale

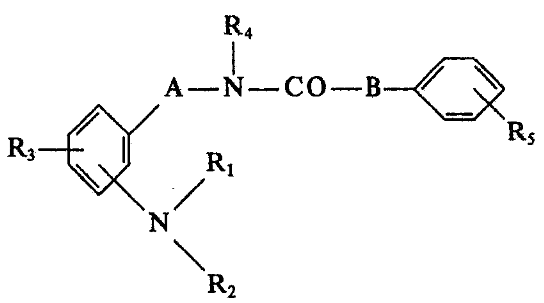

(VII)

dans laquelle

$R_1$ à $R_5$, A et B sont définis comme au début, avec le phosgène, un halogénure d'oxalyle, un halogénure d'alcoyle ou d'alcanoyle avec chacun 1 à 3 atomes de carbone dans la partie alcoyle ou avec le cyanure d'hydrogène/d'halogénure d'hydrogène en présence d'un acide de Lewis, ou

e) pour la préparation d'un composé de formule générale I dans laquelle W représente le groupe carboxy, on fait réagir un composé de formule générale

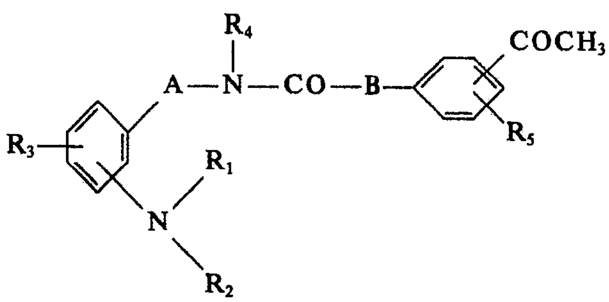

(VIII)

dans laquelle

$R_1$ à $R_5$, A et B sont définis comme au début, avec un hypohalogénite éventuellement préparé dans le mélange réactionnel en présence d'une base alcaline, ou

f) pour la préparation d'un composé de formule générale I dans laquelle W représente le groupe carboxy, on oxyde un composé de formule générale

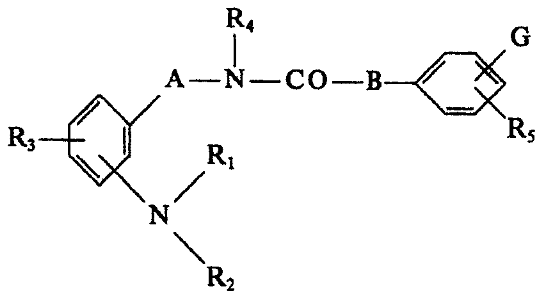

(IX)

dans laquelle

$R_1$ à $R_5$, A et B sont définis comme au début et G représente un groupe transformable en un groupe carboxy par oxydation, ou

g) pour la préparation d'un composé de formule générale I dans laquelle $R_3$ représente le groupe nitro, on fait réagir un composé de formule générale

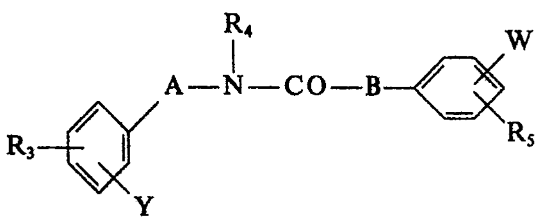

(X)

dans laquelle

$R_4$, $R_5$, A, B et W sont définis comme au début,
$R_3$ représente un groupe nitro et
Y représente un radical échangeable de façon nucléophile, avec une amine de formule générale

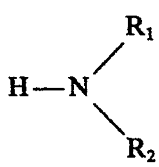

(XI)

dans laquelle
$R_1$ et $R_2$ sont définis comme au début, et éventuellement ensuite, on hydrolyse ou

h) pour la préparation d'un composé de formule générale I dans laquelle A représente un groupe de formule

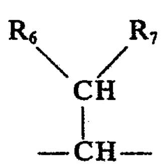

où $R_6$ et $R_7$ sont définis comme au début, on réduit un composé de formule générale

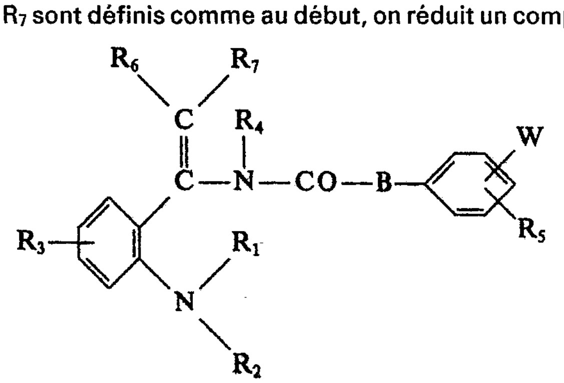

(XII)

dans laquelle

$R_1$ à $R_7$, B et W sont définis comme au début, au moyen d'hydrogène en présence d'un catalyseur d'hydrogènation,ou

i) pour la préparation d'un composé de formule générale I dans laquelle $R_4$ représente un atome d'hydrogène et A représente un groupe méthylène ou éthylène éventuellement substitué par un groupe alcoyle avec 1 à 5 atomes de carbone, ou représente un groupe méthylène ou éthylène substitué par deux groupes alcoyle avec chacun 1 à 3 atomes de carbone, ou représente un groupe méthylène substitué par un groupe cycloalcoyle avec 3 à 7 atomes de carbone, un groupe alcoxyalcoyle, carboxyle, alcoxycarbonyle, aryle ou aralcoyle, chacune des parties alcoyle mentionnées plus haut pouvant contenir 1 à 3 atomes de carbone, ou représente un groupe cycloalcoylidène avec 4 à 7 atomes de carbone, on fait réagir un composé de formule générale

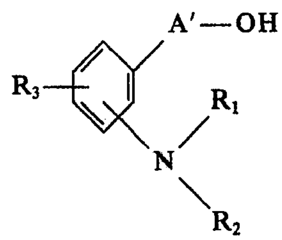

(XIII)

dans laquelle

$R_1$ à $R_3$ sont définis comme au début et
A' représente un groupe méthylène ou éthylène éventuellement substitué par un groupe alcoyle avec 1 à 5 atomes de carbone, ou représente un groupe méthylène ou éthylène substitué par deux groupes alcoyle avec chacun 1 à 3 atomes de carbone, ou représente un groupe méthylène substitué par un groupe cycloalcoyle avec 3 à 7 atomes de carbone, un groupe alcoxyalcoyle, carboxyle, alcoxycarbonyle, aryle ou aralcoyle, chacune des parties alcoyle mentionnées plus haut pouvant contenir 1 à 3 atomes de carbone, ou représente un groupe cycloalcoylidène avec 4 à 7 atomes de carbone, avec un composé de formule générale

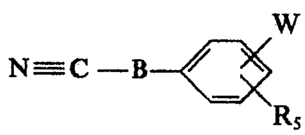

(XIV)

dans laquelle

$R_5$, B et W sont définis comme au début, en présence d'un acide fort,
et si on le désire ensuite, on transforme un composé ainsi obtenu de formule générale I dans laquelle W représente le groupe carboxy, au moyen d'une estérification ou d'une amidation, en un composé correspondant de formule générale I et/ou
on transforme un composé obtenu de formule générale I dans laquelle $R_3$ et/ou W représentent un groupe nitro, au moyen d'une réduction, en un composé correspondant de formule générale I dans laquelle $R_3$ et/ou représentent un groupe amino, et/ou
on transforme un composé obtenu de formule générale I dans laquelle $R_3$ et/ou W représentent un groupe amino, par l'intermédiaire d'un sel de diazonium correspondant, en un composé correspondant de formule générale I dans laquelle $R_3$ représente un atome d'hydrogène ou d'halogène, un groupe hydroxy, alcoxy, mercapto, alcoylmercapto, chlorosulfonyle ou cyano et/ou W représente un atome d'hydrogène ou d'halogène ou un groupe cyano, un composé ainsi éventuellement obtenu de formule générale I dans laquelle $R_3$ représente le groupe hydroxy pouvant être transformé au moyen d'une alcoylation en un composé correspondant de formule générale I dans laquelle $R_3$ représente un groupe alcoxy ou un composé ainsi éventuellement obtenu de formule générale I dans laquelle $R_3$ représente le groupe chlorosulfonyle pouvant être transformé au moyen d'ammoniac en un composé correspondant de formule générale I dans laquelle $R_3$ représente le groupe aminosulfonyle, et/ou
un composé obtenu de formule générale I dans laquelle $R_3$ représente un groupe amino est transformé au moyen d'une acylation en un composé correspondant de formule générale I dans laquelle $R_3$ représente un groupe alcanoylamino, aroylamino, alcoxycarbonylamino ou alcoylsulfonylamino, et/ou
un composé obtenu de formule générale I dans laquelle $R_3$ représente un groupe amino est transformé au moyen d'une alcoylation en un composé correspondant de formule générale I

dans laquelle $R_3$ représente un groupe alcoylamino ou dialcoylamino, et/ou
un composé obtenu de formule générale I dans laquelle $R_3$ représente un atome de chlore ou de brome est transformé au moyen d'une déshalogénation en un composé correspondant de formule générale I dans laquelle $R_3$ représente un atome d'hydrogène, et/ou
un composé obtenu de formule générale I dans laquelle $R_3$ représente un groupe nitrile est transformé au moyen d'une hydrolyse ou d'une alcoolyse en un composé correspondant de formule générale I dans laquelle $R_3$ représente un groupe aminocarbonyle, carboxy ou alcoxycarbonyle, et/ou
un composé obtenu de formule générale I dans laquelle $R_3$ représente un groupe carboxy ou alcoxycarbonyle et/ou W représente un groupe carboxy éventuellement estérifié, est transformé au moyen d'une réduction en un composé correspondant de formule générale I dans laquelle $R_3$ et/ou W représentent un groupe formyle ou hydroxyméthyle, et/ou
un composé obtenu de formule générale I dans laquelle W représente un groupe alcoxycarbonyle dans lequel le groupe alcoxy peut contenir 2 à 6 atomes de carbone et, à l'exception de la position a, est substitué par un groupe hydroxy, est transformé au moyen d'une acylation en un composé correspondant de formule générale I et/ou un composé obtenu de formule générale I dans laquelle W représente un groupe hydroxyméthyle est transformé au moyen d'une halogénation et réaction subséquente avec un diester d'acide malonique en un composé correspondant de formule générale I dans laquelle W représente un groupe éthyle substitué par deux groupes alcoxycarbonyle et/ou
un composé obtenu de formule générale I dans laquelle W représente un groupe formyle est transformé au moyen d'une condensation et éventuellement d'une hydrolyse subséquente et/ou décarboxylation en un composé correspondant de formule générale I dans laquelle W représente un groupe vinyle substitué par un groupe hydroxycarbonyle ou alcoxycarbonyle et/ou
un composé obtenu de formule générale I dans laquelle W représente un groupe éthyle substitué par deux groupes alcoxycarbonyle est transformé au moyen d'une hydrolyse et d'une décarboxylation en un composé correspondant de formule générale I dans laquelle W représente un groupe éthyle substitué par un groupe carboxy et/ou
un composé obtenu de formule générale I dans laquelle W représente un groupe carboxy est transformé au moyen d'une transformation en un hydrazide d'acide sulfonique et ensuite dismutation en un composé correspondant de formule générale I dans laquelle W représente un groupe formyle et/ou
un composé obtenu de formule générale I dans laquelle $R_1$ et $R_2$ représentent ensemble avec l'atome d'azote se trouvant entre eux un groupe aza-1,4-dioxa-spiro-alcoyle avec 6 à 8 atomes de carbone est transformé au moyen d'une hydrolyse en un composé correspondant de formule générale I dans laquelle $R_1$ et $R_2$ représentent ensemble avec l'atome d'azote se trouvant entre eux un groupe alcoylèneimino non ramifié avec 4 à 6 atomes de carbone dans lequel un groupe méthylène est remplacé par un groupe carbonyle et/ou
un composé obtenu de formule générale I dans laquelle $R_1$ et $R_2$ représentent ensemble avec l'atome d'azote se trouvant entre eux un groupe alcoylèneimino non ramifié avec 4 à 6 atomes de carbone dans lequel un groupe méthylène est remplacé par un groupe carbonyle, est transformé au moyen d'une réduction en un composé hydroxyalcoylèneimino correspondant de formule générale I et/ou
un composé obtenu de formule générale I dans laquelle W représente un groupe aminocarbonyle est transformé au moyen d'une déshydratation en un composé correspondant de formule générale I dans laquelle W représente un groupe cyano et/ou
un composé obtenu de formule générale I est transformé en ses sels, en particulier en ses sels physiologiquement supportables avec des acides et des bases minéraux ou organiques.

2. Procédé selon la revendication 1, pour la préparation de nouveaux carboxamides de formule générale

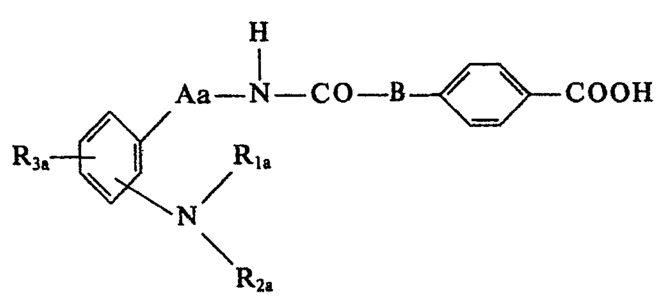

(I)

dans laquelle

B est défini comme dans la revendication 1,

$R_{1a}$ et $R_{2a}$ qui peuvent être identiques ou différents, représentent des groupes alcoyle avec 1 à 6 atomes de carbone ou des groupes cycloalcoyle avec 5 à 7 atomes de carbone ou

$R_{1a}$ et $R_{2a}$ représentent ensemble avec l'atome d'azote se trouvant entre eux un groupe alcoylèneimino avec 4 à 10 atomes de carbone dans le cycle alcoylène éventuellement substitué par un ou deux groupes alcoyle avec chacun 1 à 3 atomes de carbone, ou représentent un groupe morpholino ou thiomorpholino,

$R_{3a}$ représente un atome d'hydrogène ou d'halogène, un groupe trifluorométhyle, alcoyle, hydroxy, alcoxy, mercapto, alcoylmercapto, cyano, nitro, amino, aminocarbonyle, alcoylamino, dialcoylamino, alcanoylamino ou alcoylsulfonylamino, chaque partie alcoyle dans les groupes mentionnés plus haut pouvant contenir à chaque fois 1 à 3 atomes de carbone, et

Aa représente un groupe méthylène ou éthylène éventuellement substitué par un ou deux groupes alcoyle avec chacun 1 à 3 atomes de carbone, ainsi que de leurs esters, de leurs antipodes actifs optiquement et de leurs sels, en particulier de leurs sels physiologiquement supportables avec des acides ou bases minéraux ou organiques,
caractérisé en ce que

a) on fait réagir une amine de formule générale

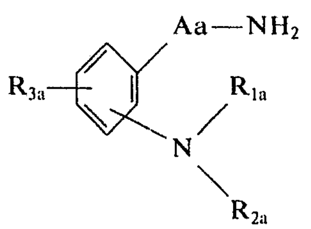

(IIa)

dans laquelle

Aa et $R_{1a}$ à $R_{3a}$ sont définis comme au début, avec un acide carboxylique de formule générale

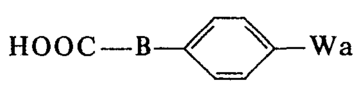

(IIIa)

dans laquelle

B est défini comme dans la revendication 1 et
Wa représente un groupe carboxyle éventuellement protégé par un groupe protecteur, ou avec ses dérivés réactifs éventuellement préparés dans le mélange réactionnel et si nécessaire, on clive un groupe protecteur utilisé ou

b) on hydrolyse un composé de formule générale

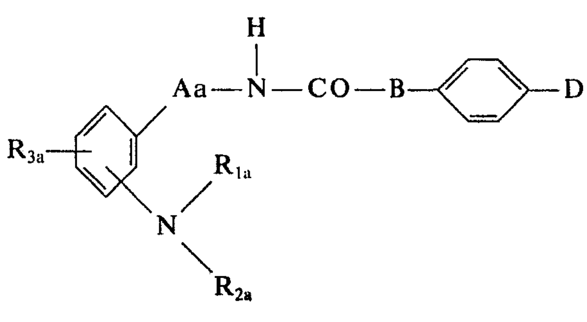

(IVa)

dans laquelle

B et D sont définis comme dans la revendication 1 et
Aa et $R_{1a}$ à $R_{3a}$ sont définis comme au début, ou

c) on fait réagir un composé éventuellement formé dans le mélange réactionnel de formule générale

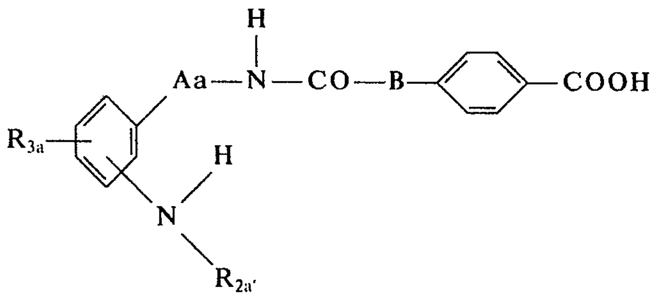

(Va)

dans laquelle

B est défini comme dans la revendication 1 et
$R_{3a}$ et Aa sont définis comme au début,
$R_{2a}'$ représente un atome d'hydrogène ou possède les significations mentionnées au début pour $R_{2a}$, ou ses esters avec un composé de formule générale:

$$R_{1a}' - E \quad \text{(VIa)}$$

dans laquelle

$R_{1a}'$ possède les significations mentionnées au début pour $R_{1a}$ ou ensemble avec le radical $R_{2a}'$ de la formule Va représente un groupe alcoylène rectiligne avec 4 à 6 atomes de carbone éventuellement substitué par un ou deux groupes alcoyle avec chacun 1 à 3 atomes de carbone, ou représente un groupe n-pentylène dans lequel le troisième groupe méthylène est remplacé par un atome d'oxygène ou de soufre, et
E représente un groupe partant nucléophile et éventuellement on hydrolyse ensuite, ou

d) on acyle un composé de formule générale

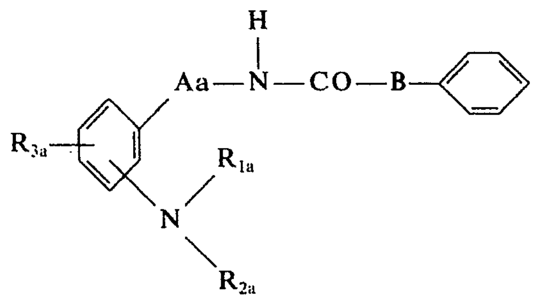

(VIIa)

dans laquelle

B est défini comme dans la revendication 1 et
Aa et $R_{1a}$ à $R_{3a}$ sont définis comme au début, au moyen d'un halogénure d'oxalyle ou de phosgène en présence d'un acide de Lewis, ou

e) on fait réagir un composé de formule générale

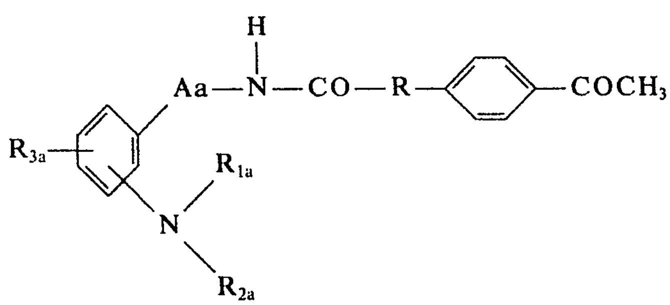

(VIIIa)

dans laquelle

B est défini comme dans la revendication 1 et
Aa et $R_{1a}$ à $R_{3a}$ sont définis comme au début, au moyen d'un hypohalogénite éventuellement préparé dans le mélange réactionnel en présence d'une base alcaline ou

f) on oxyde un composé de formule générale

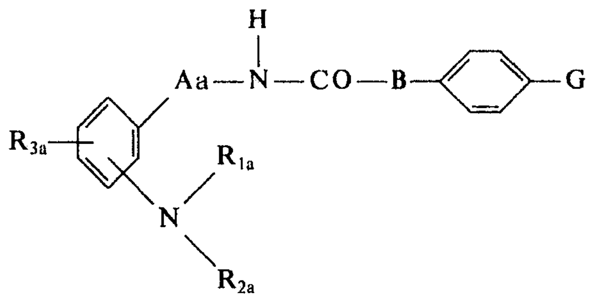

(IXa)

dans laquelle

B et G sont définis comme dans la revendication 1 et
Aa et $R_{1a}$ à $R_{3a}$ sont définis comme au début, ou

g) on fait réagir un composé de formule générale

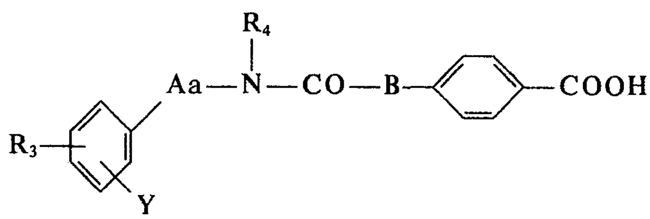

(Xa)

dans laquelle

$R_3$, B et Y sont définis comme dans la revendication 1 et
Aa est défini comme au début, ou son ester avec une amine de formule générale

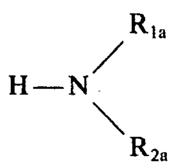

(XIa)

dans laquelle

$R_{1a}$ et $R_{2a}$ sont définis comme au début et éventuellement ensuite en hydrolyse,
et si on le désire ensuite, on transforme un carboxamide ainsi obtenu de formule générale I au moyen d'une estérification en un ester correspondant et/ou
on transforme un composé obtenu de formule générale I dans laquelle $R_{3a}$ représente un groupe nitro, au moyen d'une réduction, en un composé correspondant de formule générale I dans laquelle $R_{3a}$ représente un groupe amino, et/ou
on transforme un composé obtenu de formule générale I dans laquelle $R_{3a}$ représente un groupe amino, par l'intermédiaire d'un sel de diazonium correspondant, en un composé correspondant de formule générale I dans laquelle $R_{3a}$ représente un atome d'hydrogène ou d'halogène, un groupe hydroxy, alcoxy, mercapto, alcoylmercapto ou cyano, un composé ainsi obtenu de formule générale I dans laquelle $R_{3a}$ représente le groupe hydroxy pouvant être transformé au moyen d'une alcoylation en un composé correspondant de formule générale I dans laquelle $R_{3a}$ représente un groupe alcoxy et/ou un composé obtenu de formule générale I dans laquelle $R_{3a}$ représente un groupe amino est transformé au moyen d'une acylation en un composé correspondant de formule générale I dans laquelle $R_{3a}$ représente un groupe alcanoylamino ou alcoylsulfonylamino, et/ou
un composé obtenu de formule générale I dans laquelle $R_{3a}$ représente un atome de chlore ou de brome est transformé au moyen d'une déshalogénation en un composé correspondant de formule générale I dans laquelle $R_{3a}$ représente un atome d'hydrogène, et/ou
un composé obtenu de formule générale I dans laquelle $R_{3a}$ représente un groupe amino est transformé au moyen d'une alcoylation en un composé correspondant de formule générale I dans laquelle $R_{3a}$ représente un groupe alcoylamino ou dialcoylamino, et/ou
un composé obtenu de formule générale I dans laquelle $R_{3a}$ représente un groupe nitrile est transformé au moyen d'une hydrolyse en un composé correspondant de formule générale I

dans laquelle $R_{3a}$ représente un groupe aminocarbonyle, et/ou
un composé obtenu de formule générale I est transformé en ses sels, en particulier en ses sels physiologiquement supportables avec des acides et bases minéraux ou organiques.

Priorité: 10 Janvier 1981 (P 31 00 575.6)

3. Procédé selon les revendications 1 et 2, caractérisé en ce que la réaction est effectuée dans un solvant.

4. Procédé selon les revendications 1a, 2a et 3, caractérisé en ce que la réaction est effectuée à des températures entre — 25 et 250° C, de préférence cependant à des températures entre — 10° C et la température d'ébullition du solvant utilisé.

5. Procédé selon les revendications 1b, 2b et 3, caractérisé en ce que la réaction est effectuée à des températures entre — 10 et 120° C, de préférence à des températures entre la température ambiante et la température d'ébullition du mélange réactionnel.

6. Procédé selon les revendications 1c, 2c et 3, caractérisé en ce que la réaction est effectuée à des températures entre 0 et 150° C, de préférence cependant à des températures entre 20 et 75° C.

7. Procédé selon les revendications 1d, 2d et 3, caractérisé en ce que la réaction est effectuée à des températures entre 0 et 120° C, de préférence cependant à des températures entre 20 et 80° C.

8. Procédé selon les revendications 1e, 2e et 3, caractérisé en ce que la réaction est effectuée en présence d'une base alcaline et à des températures entre 0 et 80° C, de préférence cependant à des températures entre 25 et 50° C.

9. Procédé selon les revendications 1f, 2f et 3, caractérisé en ce que la réaction est effectuée à des températures entre 0 et 100° C, de préférence cependant à des températures entre 20 et 50° C.

10. Procédé selon les revendications 1g et 2g, caractérisé en ce que la réaction est effectuée à des températures entre 20 et 150° C, de préférence cependant à la température d'ébullition du mélange réactionnel, par exemple à 100° C.